# EUROPEAN PATENT SPECIFICATION

(11) **EP 2 607 497 B1**
(45) Date of publication and mention of the grant of the patent: **01.10.2014**
(21) Application number: 13159677.7
(22) Date of filing: 12.05.2009
(51) Int. Cl.: C12Q 1/68, G01N 33/53, G01N 33/574

(54) **Tests to predict responsiveness of cancer patients to chemotherapy treatment options**
Tests zur Vorhersage der Reaktionsfähigkeit von Krebspatienten auf Chemotherapiebehandlungsoptionen
Tests pour prédire une sensibilité de patients atteints de cancer à des options de traitement de chimiothérapie

(30) Priority: 12.05.2008 US 52573 P; 29.05.2008 US 57182 P
(43) Date of publication of application: 26.06.2013
(62) Divisional of application: 12195318.6
(73) Proprietor: Genomic Health, Inc., Redwood City, CA 94063 (US); Aventisub II Inc., Greenville, DE 19807 (US)
(72) Inventor: Shak, Steve, Hillsborough, CA 94010 (US); Baker, Joffre, Montara, CA 94037 (US); Yoshizawa, Carl, Redwood City, CA 94063 (US); Gray, Robert, Boston, MA 02115 (US); Sparano, Joseph, Pleasantville, NY 10570 (US)
(74) Representative: Ellis, Katherine Elizabeth Sleigh

(56) References cited:
- WO-A1-2006/052862
- WO-A2-2005/100606
- WO-A2-2007/085497
- US-A1- 2006 281 122
- US-A1- 2008 085 519
- TANG SHOU-CHING: "Predictive markers of tubulin-targeting agents in breast cancer", CLINICAL BREAST CANCER MAR 2008 LNKD- PUBMED:18637403,, vol. 8 Suppl 2, 1 March 2008 (2008-03-01), pages S79-S84, XP002636994,
- IVANOV OLGA ET AL: "alpha B-crystallin is a novel predictor of resistance to neoadjuvant chemotherapy in breast cancer", BREAST CANCER RESEARCH AND TREATMENT, SPRINGER, NEW YORK, NY, vol. 111, no. 3, 30 October 2007 (2007-10-30), pages 411-417, XP002516398, ISSN: 0167-6806, DOI: DOI:10.1007/S10549-007-9796-0 [retrieved on 2007-10-30]

### SEQUENCE LISTING

<110> Baker, Joffre B.
   Shak, Steve
   Yoshizawa, Carl
   Sparano, Joseph
   Gray, Robert
<120> TESTS TO PREDICT RESPONSIVENESS OF
   CANCER PATIENTS TO CHEMOTHERAPY TREATMENT OPTIONS
<130> KE/N29924
<140> US 12/464,797
   <141> 2009-05-12
<150> US 61/052,573
   <151> 2008-05-12
<150> US 61/057,182
   <151> 2008-05-29
<160> 1496
<170> FastSEQ for Windows Version 4.0
<210> 1
   <211> 20
   <212> DNA
   <213> Artificial Sequence
<220>
   <223> Synthetic Oligonucleotide
<400> 1
   ttacccgtgg gaactgtctc 20
<210> 2
   <211> 22
   <212> DNA
   <213> Artificial Sequence
<220>
   <223> Synthetic Oligonucleotide
<400> 2
   gaccagtaaa tgggtcagag ga 22
<210> 3
   <211> 25
   <212> DNA
   <213> Artificial Sequence
<220>
   <223> Synthetic Oligonucleotide
<400> 3
   tcctctcacc aggacaacaa ccaca 25
<210> 4
   <211> 20
   <212> DNA
   <213> Artificial Sequence
<220>
   <223> Synthetic Oligonucleotide
<400> 4
   aaacaccact ggagcattga 20
<210> 5
   <211> 20
   <212> DNA
   <213> Artificial Sequence
<220>
   <223> Synthetic Oligonucleotide
<400> 5
   caagcctgga acctatagcc 20
<210> 6
   <211> 24
   <212> DNA
   <213> Artificial Sequence
<220>
   <223> Synthetic Oligonucleotide
<400> 6
   ctcgccaatg atgctgctca agtt 24
<210> 7
   <211> 18
   <212> DNA
   <213> Artificial Sequence
<220>
   <223> Synthetic Oligonucleotide
<400> 7
   agacagtcgc cttggtcg 18
<210> 8
   <211> 20
   <212> DNA
   <213> Artificial Sequence
<220>
   <223> Synthetic Oligonucleotide
<400> 8
   aacctctgca gaagctggac 20
<210> 9
   <211> 24
   <212> DNA
   <213> Artificial Sequence
<220>
   <223> Synthetic Oligonucleotide
<400> 9
   ccgtactctt cccactgcca ttga 24
<210> 10
   <211> 18
   <212> DNA
   <213> Artificial Sequence
<220>
   <223> Synthetic Oligonucleotide
<400> 10
   accagtgcca caatgcag 18
<210> 11
   <211> 18
   <212> DNA
   <213> Artificial Sequence
<220>
   <223> Synthetic Oligonucleotide
<400> 11
   atagcgctga ccactgcc 18
<210> 12
   <211> 24
   <212> DNA
   <213> Artificial Sequence
<220>
   <223> Synthetic Oligonucleotide
<400> 12
   ccatgagctg tagccgaatg tcca 24
<210> 13
   <211> 18
   <212> DNA
   <213> Artificial Sequence
<220>
   <223> Synthetic Oligonucleotide
<400> 13
   aagccacagc ctccattg 18
<210> 14
   <211> 19
   <212> DNA
   <213> Artificial Sequence
<220>
   <223> Synthetic Oligonucleotide
<400> 14
   ggaaggcttc acggattgt 19
<210> 15
   <211> 24
   <212> DNA
   <213> Artificial Sequence
<220>
   <223> Synthetic Oligonucleotide
<400> 15
   tggagacaga caccctgatc cagc 24
<210> 16
   <211> 20
   <212> DNA
   <213> Artificial Sequence
<220>
   <223> Synthetic Oligonucleotide
<400> 16
   tgcagactgt accatgctga 20
<210> 17
   <211> 20
   <212> DNA
   <213> Artificial Sequence
<220>
   <223> Synthetic Oligonucleotide
<400> 17
   ggccagcacc ataatcctat 20
<210> 18
   <211> 22
   <212> DNA
   <213> Artificial Sequence
<220>
   <223> Synthetic Oligonucleotide
<400> 18
   ctgcacacgg ttctaggctc cg 22
<210> 19
   <211> 20
   <212> DNA
   <213> Artificial Sequence
<220>
   <223> Synthetic Oligonucleotide
<400> 19
   tctgtggccc acctctactc 20
<210> 20
   <211> 20
   <212> DNA
   <213> Artificial Sequence
<220>
   <223> Synthetic Oligonucleotide
<400> 20
   gggtgtagga agtcacagcc 20
<210> 21
   <211> 24
   <212> DNA
   <213> Artificial Sequence
<220>
   <223> Synthetic Oligonucleotide
<400> 21
   aacctgacca agccactcct ggac 24
<210> 22
   <211> 21
   <212> DNA
   <213> Artificial Sequence
<220>
   <223> Synthetic Oligonucleotide
<400> 22
   atgtacgtcg ccattcaagc t 21
<210> 23
   <211> 19
   <212> DNA
   <213> Artificial Sequence
<220>
   <223> Synthetic Oligonucleotide
<400> 23
   acgccatcac ctgaatcca 19
<210> 24
   <211> 20
   <212> DNA
   <213> Artificial Sequence
<220>
   <223> Synthetic Oligonucleotide
<400> 24
   ctggccgcac gacaggcatc 20
<210> 25
   <211> 18
   <212> DNA
   <213> Artificial Sequence
<220>
   <223> Synthetic Oligonucleotide
<400> 25
   atccgcattg aagaccca 18
<210> 26
   <211> 20
   <212> DNA
   <213> Artificial Sequence
<220>
   <223> Synthetic Oligonucleotide
<400> 26
   atccgctaga actgcaccac 20
<210> 27
   <211> 24
   <212> DNA
   <213> Artificial Sequence
<220>
   <223> Synthetic Oligonucleotide
<400> 27
   cccgcagaaa gcacatggta ttcc 24
<210> 28
   <211> 20
   <212> DNA
   <213> Artificial Sequence
<220>
   <223> Synthetic Oligonucleotide
<400> 28
   caactgctga gagaccgaga 20
<210> 29
   <211> 20
   <212> DNA
   <213> Artificial Sequence
<220>
   <223> Synthetic Oligonucleotide
<400> 29
   cgctccttta ctgccttagc 20
<210> 30
   <211> 26
   <212> DNA
   <213> Artificial Sequence
<220>
   <223> Synthetic Oligonucleotide
<400> 30
   aggaatccct ccagaacaat ccttgg 26
<210> 31
   <211> 27
   <212> DNA
   <213> Artificial Sequence
<220>
   <223> Synthetic Oligonucleotide
<400> 31
   ctgcatgtga ttgaataaga aacaaga 27
<210> 32
   <211> 22
   <212> DNA
   <213> Artificial Sequence
<220>
   <223> Synthetic Oligonucleotide
<400> 32
   tgtggacctg atccctgtac ac 22
<210> 33
   <211> 23
   <212> DNA
   <213> Artificial Sequence
<220>
   <223> Synthetic Oligonucleotide
<400> 33
   tgaccacacc aaagcctccc tgg 23
<210> 34
   <211> 20
   <212> DNA
   <213> Artificial Sequence
<220>
   <223> Synthetic Oligonucleotide
<400> 34
   cgcttctatg gcgctgagat 20
<210> 35
   <211> 20
   <212> DNA
   <213> Artificial Sequence
<220>
   <223> Synthetic Oligonucleotide
<400> 35
   tcccggtaca ccacgttctt 20
<210> 36
   <211> 24
   <212> DNA
   <213> Artificial Sequence
<220>
   <223> Synthetic Oligonucleotide
<400> 36
   cagccctgga ctacctgcac tcgg 24
<210> 37
   <211> 19
   <212> DNA
   <213> Artificial Sequence
<220>
   <223> Synthetic Oligonucleotide
<400> 37
   tcctgccacc cttcaaacc 19
<210> 38
   <211> 21
   <212> DNA
   <213> Artificial Sequence
<220>
   <223> Synthetic Oligonucleotide
<400> 38
   ggcggtaaat tcatcatcga a 21
<210> 39
   <211> 24
   <212> DNA
   <213> Artificial Sequence
<220>
   <223> Synthetic Oligonucleotide
<400> 39
   caggtcacgt ccgaggtcga caca 24
<210> 40
   <211> 25
   <212> DNA
   <213> Artificial Sequence
<220>
   <223> Synthetic Oligonucleotide
<400> 40
   ttgtctctgc cttggactat ctaca 25
<210> 41
   <211> 24
   <212> DNA
   <213> Artificial Sequence
<220>
   <223> Synthetic Oligonucleotide
<400> 41
   ccagcattag attctccaac ttga 24
<210> 42
   <211> 23
   <212> DNA
   <213> Artificial Sequence
<220>
   <223> Synthetic Oligonucleotide
<400> 42
   tcacggtaca caatctttcc gga 23
<210> 43
   <211> 19
   <212> DNA
   <213> Artificial Sequence
<220>
   <223> Synthetic Oligonucleotide
<400> 43
   tgggagggat gaaggaaat 19
<210> 44
   <211> 19
   <212> DNA
   <213> Artificial Sequence
<220>
   <223> Synthetic Oligonucleotide
<400> 44
   ctcatacagg tcctgggca 19
<210> 45
   <211> 24
   <212> DNA
   <213> Artificial Sequence
<220>
   <223> Synthetic Oligonucleotide
<400> 45
   tgtctcacga gagcatcgtc caga 24
<210> 46
   <211> 20
   <212> DNA
   <213> Artificial Sequence
<220>
   <223> Synthetic Oligonucleotide
<400> 46
   ggacagcagg aatgtgtttc 20
<210> 47
   <211> 20
   <212> DNA
   <213> Artificial Sequence
<220>
   <223> Synthetic Oligonucleotide
<400> 47
   acccactcga tttgtttctg 20
<210> 48
   <211> 22
   <212> DNA
   <213> Artificial Sequence
<220>
   <223> Synthetic Oligonucleotide
<400> 48
   cattggctcc ccgtgacctg ta 22
<210> 49
   <211> 20
   <212> DNA
   <213> Artificial Sequence
<220>
   <223> Synthetic Oligonucleotide
<400> 49
   gatgaagcct ttcgcaagtt 20
<210> 50
   <211> 20
   <212> DNA
   <213> Artificial Sequence
<220>
   <223> Synthetic Oligonucleotide
<400> 50
   aggtctccac acagcacaag 20
<210> 51
   <211> 21
   <212> DNA
   <213> Artificial Sequence
<220>
   <223> Synthetic Oligonucleotide
<400> 51
   ctttcgggaa gccaggccct t 21
<210> 52
   <211> 21
   <212> DNA
   <213> Artificial Sequence
<220>
   <223> Synthetic Oligonucleotide
<400> 52
   ggaaacacac tggaggacaa g 21
<210> 53
   <211> 19
   <212> DNA
   <213> Artificial Sequence
<220>
   <223> Synthetic Oligonucleotide
<400> 53
   cgcatcttgg cagaaagtt 19
<210> 54
   <211> 24
   <212> DNA
   <213> Artificial Sequence
<220>
   <223> Synthetic Oligonucleotide
<400> 54
   tcatcagccg catcaaacag agtg 24
<210> 55
   <211> 18
   <212> DNA
   <213> Artificial Sequence
<220>
   <223> Synthetic Oligonucleotide
<400> 55
   gtttatgcca tcggcacc 18
<210> 56
   <211> 22
   <212> DNA
   <213> Artificial Sequence
<220>
   <223> Synthetic Oligonucleotide
<400> 56
   ggaatacacg agggcatagt tc 22
<210> 57
   <211> 24
   <212> DNA
   <213> Artificial Sequence
<220>
   <223> Synthetic Oligonucleotide
<400> 57
   actggatcct ggccaccgac tatg 24
<210> 58
   <211> 18
   <212> DNA
   <213> Artificial Sequence
<220>
   <223> Synthetic Oligonucleotide
<400> 58
   gcctcaagag ctggttcg 18
<210> 59
   <211> 18
   <212> DNA
   <213> Artificial Sequence
<220>
   <223> Synthetic Oligonucleotide
<400> 59
   cctgcacctt ctccacca 18
<210> 60
   <211> 22
   <212> DNA
   <213> Artificial Sequence
<220>
   <223> Synthetic Oligonucleotide
<400> 60
   actggcgctg catgtcttcc ac 22
<210> 61
   <211> 18
   <212> DNA
   <213> Artificial Sequence
<220>
   <223> Synthetic Oligonucleotide
<400> 61
   gaggtcctgg agtgcgtg 18
<210> 62
   <211> 18
   <212> DNA
   <213> Artificial Sequence
<220>
   <223> Synthetic Oligonucleotide
<400> 62
   aggtgccagc ttctccct 18
<210> 63
   <211> 24
   <212> DNA
   <213> Artificial Sequence
<220>
   <223> Synthetic Oligonucleotide
<400> 63
   ccttaagcga ggtcagctcc acca 24
<210> 64
   <211> 18
   <212> DNA
   <213> Artificial Sequence
<220>
   <223> Synthetic Oligonucleotide
<400> 64
   ggtcctccgt cggttctc 18
<210> 65
   <211> 18
   <212> DNA
   <213> Artificial Sequence
<220>
   <223> Synthetic Oligonucleotide
<400> 65
   gtcgcaaact cggagacg 18
<210> 66
   <211> 24
   <212> DNA
   <213> Artificial Sequence
<220>
   <223> Synthetic Oligonucleotide
<400> 66
   ccacggtctg gtcttcagct accc 24
<210> 67
   <211> 21
   <212> DNA
   <213> Artificial Sequence
<220>
   <223> Synthetic Oligonucleotide
<400> 67
   agctgcagaa gagctgcaca t 21
<210> 68
   <211> 20
   <212> DNA
   <213> Artificial Sequence
<220>
   <223> Synthetic Oligonucleotide
<400> 68
   gcatctgcca actcctccat 20
<210> 69
   <211> 22
   <212> DNA
   <213> Artificial Sequence
<220>
   <223> Synthetic Oligonucleotide
<400> 69
   tgacgagcag cgaacagcca cg 22
<210> 70
   <211> 21
   <212> DNA
   <213> Artificial Sequence
<220>
   <223> Synthetic Oligonucleotide
<400> 70
   cagcagatgt ggatcagcaa g 21
<210> 71
   <211> 18
   <212> DNA
   <213> Artificial Sequence
<220>
   <223> Synthetic Oligonucleotide
<400> 71
   gcatttgcgg tggacgat 18
<210> 72
   <211> 23
   <212> DNA
   <213> Artificial Sequence
<220>
   <223> Synthetic Oligonucleotide
<400> 72
   aggagtatga cgagtccggc ccc 23
<210> 73
   <211> 22
   <212> DNA
   <213> Artificial Sequence
<220>
   <223> Synthetic Oligonucleotide
<400> 73
   ggctcttgtg cgtactgtcc tt 22
<210> 74
   <211> 23
   <212> DNA
   <213> Artificial Sequence
<220>
   <223> Synthetic Oligonucleotide
<400> 74
   tcagatgacg aagagcacag atg 23
<210> 75
   <211> 29
   <212> DNA
   <213> Artificial Sequence
<220>
   <223> Synthetic Oligonucleotide
<400> 75
   aggctcagtg atgtcttccc tgtcaccag 29
<210> 76
   <211> 19
   <212> DNA
   <213> Artificial Sequence
<220>
   <223> Synthetic Oligonucleotide
<400> 76
   gggtcaggtg cctcgagat 19
<210> 77
   <211> 21
   <212> DNA
   <213> Artificial Sequence
<220>
   <223> Synthetic Oligonucleotide
<400> 77
   ctgctcactc ggctcaaact c 21
<210> 78
   <211> 24
   <212> DNA
   <213> Artificial Sequence
<220>
   <223> Synthetic Oligonucleotide
<400> 78
   tgggcccaga gcatgttcca gatc 24
<210> 79
   <211> 23
   <212> DNA
   <213> Artificial Sequence
<220>
   <223> Synthetic Oligonucleotide
<400> 79
   cgttgtcagc acttggaata caa 23
<210> 80
   <211> 24
   <212> DNA
   <213> Artificial Sequence
<220>
   <223> Synthetic Oligonucleotide
<400> 80
   gttcaacctc ttcctgtgga ctgt 24
<210> 81
   <211> 26
   <212> DNA
   <213> Artificial Sequence
<220>
   <223> Synthetic Oligonucleotide
<400> 81
   cccaattaac atgacccggc aaccat 26
<210> 82
   <211> 20
   <212> DNA
   <213> Artificial Sequence
<220>
   <223> Synthetic Oligonucleotide
<400> 82
   ccattcccac cattctacct 20
<210> 83
   <211> 20
   <212> DNA
   <213> Artificial Sequence
<220>
   <223> Synthetic Oligonucleotide
<400> 83
   gggaacatag acccaccaat 20
<210> 84
   <211> 21
   <212> DNA
   <213> Artificial Sequence
<220>
   <223> Synthetic Oligonucleotide
<400> 84
   acaccccaga cgtcctggcc t 21
<210> 85
   <211> 18
   <212> DNA
   <213> Artificial Sequence
<220>
   <223> Synthetic Oligonucleotide
<400> 85
   ccgccgtgga cacagact 18
<210> 86
   <211> 21
   <212> DNA
   <213> Artificial Sequence
<220>
   <223> Synthetic Oligonucleotide
<400> 86
   ttgccgtcag aaaacatgtc a 21
<210> 87
   <211> 25
   <212> DNA
   <213> Artificial Sequence
<220>
   <223> Synthetic Oligonucleotide
<400> 87
   tgccactcgg aaaaagacct ctcgg 25
<210> 88
   <211> 20
   <212> DNA
   <213> Artificial Sequence
<220>
   <223> Synthetic Oligonucleotide
<400> 88
   cctggagggt cctgtacaat 20
<210> 89
   <211> 19
   <212> DNA
   <213> Artificial Sequence
<220>
   <223> Synthetic Oligonucleotide
<400> 89
   ctaattgggc tccatctcg 19
<210> 90
   <211> 24
   <212> DNA
   <213> Artificial Sequence
<220>
   <223> Synthetic Oligonucleotide
<400> 90
   catcatggga ctcctgccct tacc 24
<210> 91
   <211> 25
   <212> DNA
   <213> Artificial Sequence
<220>
   <223> Synthetic Oligonucleotide
<400> 91
   cagatggacc tagtacccac tgaga 25
<210> 92
   <211> 24
   <212> DNA
   <213> Artificial Sequence
<220>
   <223> Synthetic Oligonucleotide
<400> 92
   cctatgattt aagggcattt ttcc 24
<210> 93
   <211> 22
   <212> DNA
   <213> Artificial Sequence
<220>
   <223> Synthetic Oligonucleotide
<400> 93
   ttccacgccg aaggacagcg at 22
<210> 94
   <211> 20
   <212> DNA
   <213> Artificial Sequence
<220>
   <223> Synthetic Oligonucleotide
<400> 94
   aattaccaag cagccgaaga 20
<210> 95
   <211> 20
   <212> DNA
   <213> Artificial Sequence
<220>
   <223> Synthetic Oligonucleotide
<400> 95
   caggcggaca atgtaacgta 20
<210> 96
   <211> 28
   <212> DNA
   <213> Artificial Sequence
<220>
   <223> Synthetic Oligonucleotide
<400> 96
   ccacccacga atggttatct tacgactg 28
<210> 97
   <211> 20
   <212> DNA
   <213> Artificial Sequence
<220>
   <223> Synthetic Oligonucleotide
<400> 97
   cagcgacaac tctggacaag 20
<210> 98
   <211> 19
   <212> DNA
   <213> Artificial Sequence
<220>
   <223> Synthetic Oligonucleotide
<400> 98
   gctctcagac tgccaggaa 19
<210> 99
   <211> 24
   <212> DNA
   <213> Artificial Sequence
<220>
   <223> Synthetic Oligonucleotide
<400> 99
   ccccagagtc tccaactgtg acca 24
<210> 100
   <211> 24
   <212> DNA
   <213> Artificial Sequence
<220>
   <223> Synthetic Oligonucleotide
<400> 100
   cttttgtgga actctatggg aaca 24
<210> 101
   <211> 19
   <212> DNA
   <213> Artificial Sequence
<220>
   <223> Synthetic Oligonucleotide
<400> 101
   cagcggttga agcgttcct 19
<210> 102
   <211> 20
   <212> DNA
   <213> Artificial Sequence
<220>
   <223> Synthetic Oligonucleotide
<400> 102
   ttcggctctc ggctgctgca 20
<210> 103
   <211> 26
   <212> DNA
   <213> Artificial Sequence
<220>
   <223> Synthetic Oligonucleotide
<400> 103
   tgtactggcg aagaatattt ggtaaa 26
<210> 104
   <211> 20
   <212> DNA
   <213> Artificial Sequence
<220>
   <223> Synthetic Oligonucleotide
<400> 104
   gccacgtgat tcttccacaa 20
<210> 105
   <211> 24
   <212> DNA
   <213> Artificial Sequence
<220>
   <223> Synthetic Oligonucleotide
<400> 105
   cagggcatcg atctctcacc ctgg 24
<210> 106
   <211> 20
   <212> DNA
   <213> Artificial Sequence
<220>
   <223> Synthetic Oligonucleotide
<400> 106
   ggactgtgag gtcaacaacg 20
<210> 107
   <211> 20
   <212> DNA
   <213> Artificial Sequence
<220>
   <223> Synthetic Oligonucleotide
<400> 107
   ggaagccaaa caccagtagg 20
<210> 108
   <211> 24
   <212> DNA
   <213> Artificial Sequence
<220>
   <223> Synthetic Oligonucleotide
<400> 108
   tgtgatgcac tcatccctga ggct 24
<210> 109
   <211> 20
   <212> DNA
   <213> Artificial Sequence
<220>
   <223> Synthetic Oligonucleotide
<400> 109
   cctgcaaaag ggaacaagag 20
<210> 110
   <211> 20
   <212> DNA
   <213> Artificial Sequence
<220>
   <223> Synthetic Oligonucleotide
<400> 110
   cgtggttgac tctgatctcg 20
<210> 111
   <211> 21
   <212> DNA
   <213> Artificial Sequence
<220>
   <223> Synthetic Oligonucleotide
<400> 111
   cttcgcctcc agatggctcc c 21
<210> 112
   <211> 20
   <212> DNA
   <213> Artificial Sequence
<220>
   <223> Synthetic Oligonucleotide
<400> 112
   tcagggggct agaaatctgt 20
<210> 113
   <211> 20
   <212> DNA
   <213> Artificial Sequence
<220>
   <223> Synthetic Oligonucleotide
<400> 113
   ccattccagt tgatctgtgg 20
<210> 114
   <211> 23
   <212> DNA
   <213> Artificial Sequence
<220>
   <223> Synthetic Oligonucleotide
<400> 114
   ctatgggccc ttcaccaaca tgc 23
<210> 115
   <211> 20
   <212> DNA
   <213> Artificial Sequence
<220>
   <223> Synthetic Oligonucleotide
<400> 115
   agttcgtgct ttgcaagatg 20
<210> 116
   <211> 20
   <212> DNA
   <213> Artificial Sequence
<220>
   <223> Synthetic Oligonucleotide
<400> 116
   aaggtaagct gggtctgctg 20
<210> 117
   <211> 29
   <212> DNA
   <213> Artificial Sequence
<220>
   <223> Synthetic Oligonucleotide
<400> 117
   cattcttcac tgcttcataa agctctgca 29
<210> 118
   <211> 21
   <212> DNA
   <213> Artificial Sequence
<220>
   <223> Synthetic Oligonucleotide
<400> 118
   ccgaggttaa tccagcacgt a 21
<210> 119
   <211> 21
   <212> DNA
   <213> Artificial Sequence
<220>
   <223> Synthetic Oligonucleotide
<400> 119
   aagacatggc gctctcagtt c 21
<210> 120
   <211> 23
   <212> DNA
   <213> Artificial Sequence
<220>
   <223> Synthetic Oligonucleotide
<400> 120
   tgctgggagc ctacacttgg ccc 23
<210> 121
   <211> 24
   <212> DNA
   <213> Artificial Sequence
<220>
   <223> Synthetic Oligonucleotide
<400> 121
   tcaacagaag gctgaaccac taga 24
<210> 122
   <211> 22
   <212> DNA
   <213> Artificial Sequence
<220>
   <223> Synthetic Oligonucleotide
<400> 122
   caacagagtt tgccgagaca ct 22
<210> 123
   <211> 24
   <212> DNA
   <213> Artificial Sequence
<220>
   <223> Synthetic Oligonucleotide
<400> 123
   tacagtccca gcaccgacaa ttcc 24
<210> 124
   <211> 22
   <212> DNA
   <213> Artificial Sequence
<220>
   <223> Synthetic Oligonucleotide
<400> 124
   ctgaagcaga tggttcatca tt 22
<210> 125
   <211> 22
   <212> DNA
   <213> Artificial Sequence
<220>
   <223> Synthetic Oligonucleotide
<400> 125
   gctgattccc aagagtctaa cc 22
<210> 126
   <211> 24
   <212> DNA
   <213> Artificial Sequence
<220>
   <223> Synthetic Oligonucleotide
<400> 126
   cctcgctttg tttaacagcc cagg 24
<210> 127
   <211> 23
   <212> DNA
   <213> Artificial Sequence
<220>
   <223> Synthetic Oligonucleotide
<400> 127
   tgaggagtgg tattttggca aga 23
<210> 128
   <211> 21
   <212> DNA
   <213> Artificial Sequence
<220>
   <223> Synthetic Oligonucleotide
<400> 128
   ctctcgggtt ctctgcattg a 21
<210> 129
   <211> 24
   <212> DNA
   <213> Artificial Sequence
<220>
   <223> Synthetic Oligonucleotide
<400> 129
   aaccgctctg actcccgtct ggtg 24
<210> 130
   <211> 20
   <212> DNA
   <213> Artificial Sequence
<220>
   <223> Synthetic Oligonucleotide
<400> 130
   gtcagcgtgg tagcggtatt 20
<210> 131
   <211> 21
   <212> DNA
   <213> Artificial Sequence
<220>
   <223> Synthetic Oligonucleotide
<400> 131
   ggaagtcttg gctaaagagg c 21
<210> 132
   <211> 24
   <212> DNA
   <213> Artificial Sequence
<220>
   <223> Synthetic Oligonucleotide
<400> 132
   aacaattact gtcactgccg cgga 24
<210> 133
   <211> 20
   <212> DNA
   <213> Artificial Sequence
<220>
   <223> Synthetic Oligonucleotide
<400> 133
   tcagctgtga gctgcggata 20
<210> 134
   <211> 24
   <212> DNA
   <213> Artificial Sequence
<220>
   <223> Synthetic Oligonucleotide
<400> 134
   acggtcctag gtttgaggtt aaga 24
<210> 135
   <211> 19
   <212> DNA
   <213> Artificial Sequence
<220>
   <223> Synthetic Oligonucleotide
<400> 135
   caggtcccat tgccgggcg 19
<210> 136
   <211> 20
   <212> DNA
   <213> Artificial Sequence
<220>
   <223> Synthetic Oligonucleotide
<400> 136
   atcctagccc tggtttttgg 20
<210> 137
   <211> 20
   <212> DNA
   <213> Artificial Sequence
<220>
   <223> Synthetic Oligonucleotide
<400> 137
   ctgccttctc atctgcacaa 20
<210> 138
   <211> 20
   <212> DNA
   <213> Artificial Sequence
<220>
   <223> Synthetic Oligonucleotide
<400> 138
   tttgctgtca ccagcgtcgc 20
<210> 139
   <211> 26
   <212> DNA
   <213> Artificial Sequence
<220>
   <223> Synthetic Oligonucleotide
<400> 139
   cactaaggtt tgagacagtt ccagaa 26
<210> 140
   <211> 23
   <212> DNA
   <213> Artificial Sequence
<220>
   <223> Synthetic Oligonucleotide
<400> 140
   gcgaattagc cctctacaac tga 23
<210> 141
   <211> 26
   <212> DNA
   <213> Artificial Sequence
<220>
   <223> Synthetic Oligonucleotide
<400> 141
   aacccaagct caagacgcag gacgag 26
<210> 142
   <211> 20
   <212> DNA
   <213> Artificial Sequence
<220>
   <223> Synthetic Oligonucleotide
<400> 142
   tcgttggaga tcagagtgga 20
<210> 143
   <211> 18
   <212> DNA
   <213> Artificial Sequence
<220>
   <223> Synthetic Oligonucleotide
<400> 143
   ttaagcacgc caaccacc 18
<210> 144
   <211> 24
   <212> DNA
   <213> Artificial Sequence
<220>
   <223> Synthetic Oligonucleotide
<400> 144
   tcaccatcac accacctaca gccc 24
<210> 145
   <211> 20
   <212> DNA
   <213> Artificial Sequence
<220>
   <223> Synthetic Oligonucleotide
<400> 145
   gtggctcaac attgtgttcc 20
<210> 146
   <211> 20
   <212> DNA
   <213> Artificial Sequence
<220>
   <223> Synthetic Oligonucleotide
<400> 146
   caatggcctc cattttacag 20
<210> 147
   <211> 24
   <212> DNA
   <213> Artificial Sequence
<220>
   <223> Synthetic Oligonucleotide
<400> 147
   atttcagctg atcagtgggc ctcc 24
<210> 148
   <211> 20
   <212> DNA
   <213> Artificial Sequence
<220>
   <223> Synthetic Oligonucleotide
<400> 148
   ttcaggttgt tgcaggagac 20
<210> 149
   <211> 20
   <212> DNA
   <213> Artificial Sequence
<220>
   <223> Synthetic Oligonucleotide
<400> 149
   catcttcttg ggcacacaat 20
<210> 150
   <211> 27
   <212> DNA
   <213> Artificial Sequence
<220>
   <223> Synthetic Oligonucleotide
<400> 150
   tgtctccatt attgatcggt tcatgca 27
<210> 151
   <211> 21
   <212> DNA
   <213> Artificial Sequence
<220>
   <223> Synthetic Oligonucleotide
<400> 151
   gcatgttcgt ggcctctaag a 21
<210> 152
   <211> 22
   <212> DNA
   <213> Artificial Sequence
<220>
   <223> Synthetic Oligonucleotide
<400> 152
   cggtgtagat gcacagcttc tc 22
<210> 153
   <211> 23
   <212> DNA
   <213> Artificial Sequence
<220>
   <223> Synthetic Oligonucleotide
<400> 153
   aaggagacca tccccctgac ggc 23
<210> 154
   <211> 22
   <212> DNA
   <213> Artificial Sequence
<220>
   <223> Synthetic Oligonucleotide
<400> 154
   atgctgtggc tccttcctaa ct 22
<210> 155
   <211> 27
   <212> DNA
   <213> Artificial Sequence
<220>
   <223> Synthetic Oligonucleotide
<400> 155
   acccaaattg tgatatacaa aaaggtt 27
<210> 156
   <211> 30
   <212> DNA
   <213> Artificial Sequence
<220>
   <223> Synthetic Oligonucleotide
<400> 156
   taccaagcaa cctacatgtc aagaaagccc 30
<210> 157
   <211> 18
   <212> DNA
   <213> Artificial Sequence
<220>
   <223> Synthetic Oligonucleotide
<400> 157
   atccaaggcc atgactgg 18
<210> 158
   <211> 20
   <212> DNA
   <213> Artificial Sequence
<220>
   <223> Synthetic Oligonucleotide
<400> 158
   ggaatgacct ctagggcctg 20
<210> 159
   <211> 24
   <212> DNA
   <213> Artificial Sequence
<220>
   <223> Synthetic Oligonucleotide
<400> 159
   acagccctgt atggccattg ttcc 24
<210> 160
   <211> 19
   <212> DNA
   <213> Artificial Sequence
<220>
   <223> Synthetic Oligonucleotide
<400> 160
   gtgtgctagc gtactcccg 19
<210> 161
   <211> 18
   <212> DNA
   <213> Artificial Sequence
<220>
   <223> Synthetic Oligonucleotide
<400> 161
   gcatggtgcc ggttatct 18
<210> 162
   <211> 24
   <212> DNA
   <213> Artificial Sequence
<220>
   <223> Synthetic Oligonucleotide
<400> 162
   caaggaactg acgctcgagg acct 24
<210> 163
   <211> 25
   <212> DNA
   <213> Artificial Sequence
<220>
   <223> Synthetic Oligonucleotide
<400> 163
   tgtatttcaa gacctctgtg cactt 25
<210> 164
   <211> 23
   <212> DNA
   <213> Artificial Sequence
<220>
   <223> Synthetic Oligonucleotide
<400> 164
   ttagcctgag gaattgctgt gtt 23
<210> 165
   <211> 25
   <212> DNA
   <213> Artificial Sequence
<220>
   <223> Synthetic Oligonucleotide
<400> 165
   tttatgaacc tgccctgctc ccaca 25
<210> 166
   <211> 20
   <212> DNA
   <213> Artificial Sequence
<220>
   <223> Synthetic Oligonucleotide
<400> 166
   agatgaagtg gaaggcgctt 20
<210> 167
   <211> 21
   <212> DNA
   <213> Artificial Sequence
<220>
   <223> Synthetic Oligonucleotide
<400> 167
   tgcctctgta atcggcaact g 21
<210> 168
   <211> 18
   <212> DNA
   <213> Artificial Sequence
<220>
   <223> Synthetic Oligonucleotide
<400> 168
   caccgcggcc atcctgca 18
<210> 169
   <211> 18
   <212> DNA
   <213> Artificial Sequence
<220>
   <223> Synthetic Oligonucleotide
<400> 169
   agtgggactg attgccgt 18
<210> 170
   <211> 19
   <212> DNA
   <213> Artificial Sequence
<220>
   <223> Synthetic Oligonucleotide
<400> 170
   gggtagcccc ctggattat 19
<210> 171
   <211> 24
   <212> DNA
   <213> Artificial Sequence
<220>
   <223> Synthetic Oligonucleotide
<400> 171
   tctgactcag gacaagctgt gccc 24
<210> 172
   <211> 18
   <212> DNA
   <213> Artificial Sequence
<220>
   <223> Synthetic Oligonucleotide
<400> 172
   tggttcccag ccctgtgt 18
<210> 173
   <211> 19
   <212> DNA
   <213> Artificial Sequence
<220>
   <223> Synthetic Oligonucleotide
<400> 173
   ctcctccacc ctgggttgt 19
<210> 174
   <211> 28
   <212> DNA
   <213> Artificial Sequence
<220>
   <223> Synthetic Oligonucleotide
<400> 174
   ctccaagccc agattcagat tcgagtca 28
<210> 175
   <211> 18
   <212> DNA
   <213> Artificial Sequence
<220>
   <223> Synthetic Oligonucleotide
<400> 175
   gagagcgacg cggttgtt 18
<210> 176
   <211> 26
   <212> DNA
   <213> Artificial Sequence
<220>
   <223> Synthetic Oligonucleotide
<400> 176
   gtatggtaga tcccggctta ttattc 26
<210> 177
   <211> 18
   <212> DNA
   <213> Artificial Sequence
<220>
   <223> Synthetic Oligonucleotide
<400> 177
   tagctgccgc tgcggccg 18
<210> 178
   <211> 21
   <212> DNA
   <213> Artificial Sequence
<220>
   <223> Synthetic Oligonucleotide
<400> 178
   tggattggag ttctgggaat g 21
<210> 179
   <211> 22
   <212> DNA
   <213> Artificial Sequence
<220>
   <223> Synthetic Oligonucleotide
<400> 179
   gcttgcactc cacaggtaca ca 22
<210> 180
   <211> 23
   <212> DNA
   <213> Artificial Sequence
<220>
   <223> Synthetic Oligonucleotide
<400> 180
   actggccgtg gcactggaca aca 23
<210> 181
   <211> 21
   <212> DNA
   <213> Artificial Sequence
<220>
   <223> Synthetic Oligonucleotide
<400> 181
   aaacgagcag tttgccatca g 21
<210> 182
   <211> 20
   <212> DNA
   <213> Artificial Sequence
<220>
   <223> Synthetic Oligonucleotide
<400> 182
   gttggtgatg ttccgaagca 20
<210> 183
   <211> 24
   <212> DNA
   <213> Artificial Sequence
<220>
   <223> Synthetic Oligonucleotide
<400> 183
   cctcaccggc atagactgga agcg 24
<210> 184
   <211> 19
   <212> DNA
   <213> Artificial Sequence
<220>
   <223> Synthetic Oligonucleotide
<400> 184
   gaggcacagt attgcccag 19
<210> 185
   <211> 21
   <212> DNA
   <213> Artificial Sequence
<220>
   <223> Synthetic Oligonucleotide
<400> 185
   gagacggttg gagagcttct t 21
<210> 186
   <211> 24
   <212> DNA
   <213> Artificial Sequence
<220>
   <223> Synthetic Oligonucleotide
<400> 186
   atgtttccca aggcctctgg atcc 24
<210> 187
   <211> 21
   <212> DNA
   <213> Artificial Sequence
<220>
   <223> Synthetic Oligonucleotide
<400> 187
   tgagtgtccc ccggtatctt c 21
<210> 188
   <211> 21
   <212> DNA
   <213> Artificial Sequence
<220>
   <223> Synthetic Oligonucleotide
<400> 188
   cagccgcttt cagattttca t 21
<210> 189
   <211> 27
   <212> DNA
   <213> Artificial Sequence
<220>
   <223> Synthetic Oligonucleotide
<400> 189
   tgccaatccc gatgaaattg gaaattt 27
<210> 190
   <211> 20
   <212> DNA
   <213> Artificial Sequence
<220>
   <223> Synthetic Oligonucleotide
<400> 190
   aagccctatc cgatgtaccc 20
<210> 191
   <211> 19
   <212> DNA
   <213> Artificial Sequence
<220>
   <223> Synthetic Oligonucleotide
<400> 191
   ctgtggcatt gagtttggg 19
<210> 192
   <211> 24
   <212> DNA
   <213> Artificial Sequence
<220>
   <223> Synthetic Oligonucleotide
<400> 192
   cacaacatcc ctggtgaacg tcgt 24
<210> 193
   <211> 18
   <212> DNA
   <213> Artificial Sequence
<220>
   <223> Synthetic Oligonucleotide
<400> 193
   cggcgatcaa gaagctgt 18
<210> 194
   <211> 18
   <212> DNA
   <213> Artificial Sequence
<220>
   <223> Synthetic Oligonucleotide
<400> 194
   caggcgctga tctcttgc 18
<210> 195
   <211> 24
   <212> DNA
   <213> Artificial Sequence
<220>
   <223> Synthetic Oligonucleotide
<400> 195
   cgggcctctg atctccgatt tctt 24
<210> 196
   <211> 21
   <212> DNA
   <213> Artificial Sequence
<220>
   <223> Synthetic Oligonucleotide
<400> 196
   tgacaatcag cacacctgca t 21
<210> 197
   <211> 23
   <212> DNA
   <213> Artificial Sequence
<220>
   <223> Synthetic Oligonucleotide
<400> 197
   tgtgactaca gccgtgatcc tta 23
<210> 198
   <211> 20
   <212> DNA
   <213> Artificial Sequence
<220>
   <223> Synthetic Oligonucleotide
<400> 198
   caggccctct tccgagcggt 20
<210> 199
   <211> 21
   <212> DNA
   <213> Artificial Sequence
<220>
   <223> Synthetic Oligonucleotide
<400> 199
   taaattcact cgtggtgtgg a 21
<210> 200
   <211> 20
   <212> DNA
   <213> Artificial Sequence
<220>
   <223> Synthetic Oligonucleotide
<400> 200
   gcctcttgta gggccaatag 20
<210> 201
   <211> 21
   <212> DNA
   <213> Artificial Sequence
<220>
   <223> Synthetic Oligonucleotide
<400> 201
   cttcaattgg caagcccagg c 21
<210> 202
   <211> 20
   <212> DNA
   <213> Artificial Sequence
<220>
   <223> Synthetic Oligonucleotide
<400> 202
   ggatgctggt gacctcttct 20
<210> 203
   <211> 20
   <212> DNA
   <213> Artificial Sequence
<220>
   <223> Synthetic Oligonucleotide
<400> 203
   gccaaggcac caagtaactc 20
<210> 204
   <211> 25
   <212> DNA
   <213> Artificial Sequence
<220>
   <223> Synthetic Oligonucleotide
<400> 204
   tccctcacgt tgcaacagga attaa 25
<210> 205
   <211> 18
   <212> DNA
   <213> Artificial Sequence
<220>
   <223> Synthetic Oligonucleotide
<400> 205
   ctcccgtcaa cagcgttc 18
<210> 206
   <211> 18
   <212> DNA
   <213> Artificial Sequence
<220>
   <223> Synthetic Oligonucleotide
<400> 206
   gggtgagtct ggccttca 18
<210> 207
   <211> 24
   <212> DNA
   <213> Artificial Sequence
<220>
   <223> Synthetic Oligonucleotide
<400> 207
   acactggacc aggagtgcat ccag 24
<210> 208
   <211> 20
   <212> DNA
   <213> Artificial Sequence
<220>
   <223> Synthetic Oligonucleotide
<400> 208
   ctgaaggagc tccaagacct 20
<210> 209
   <211> 20
   <212> DNA
   <213> Artificial Sequence
<220>
   <223> Synthetic Oligonucleotide
<400> 209
   caaaaccgct gtgtttcttc 20
<210> 210
   <211> 22
   <212> DNA
   <213> Artificial Sequence
<220>
   <223> Synthetic Oligonucleotide
<400> 210
   tgctgatgtg ccctctcctt gg 22
<210> 211
   <211> 20
   <212> DNA
   <213> Artificial Sequence
<220>
   <223> Synthetic Oligonucleotide
<400> 211
   aaggaagtgg tccctctgtg 20
<210> 212
   <211> 20
   <212> DNA
   <213> Artificial Sequence
<220>
   <223> Synthetic Oligonucleotide
<400> 212
   gacgcagtct ttctgtctgg 20
<210> 213
   <211> 24
   <212> DNA
   <213> Artificial Sequence
<220>
   <223> Synthetic Oligonucleotide
<400> 213
   tgaagtctcc agctttgcct cagc 24
<210> 214
   <211> 26
   <212> DNA
   <213> Artificial Sequence
<220>
   <223> Synthetic Oligonucleotide
<400> 214
   gataaattgg tacaagggat cagctt 26
<210> 215
   <211> 24
   <212> DNA
   <213> Artificial Sequence
<220>
   <223> Synthetic Oligonucleotide
<400> 215
   gggtgccaag taactgacta ttca 24
<210> 216
   <211> 26
   <212> DNA
   <213> Artificial Sequence
<220>
   <223> Synthetic Oligonucleotide
<400> 216
   ccagcccaca tgtcctgatc atatgc 26
<210> 217
   <211> 21
   <212> DNA
   <213> Artificial Sequence
<220>
   <223> Synthetic Oligonucleotide
<400> 217
   atgtggaacc cccacctact t 21
<210> 218
   <211> 22
   <212> DNA
   <213> Artificial Sequence
<220>
   <223> Synthetic Oligonucleotide
<400> 218
   cagtccacag cacggttata cc 22
<210> 219
   <211> 29
   <212> DNA
   <213> Artificial Sequence
<220>
   <223> Synthetic Oligonucleotide
<400> 219
   agtcccaaca gaaacaagaa cttcaggcg 29
<210> 220
   <211> 24
   <212> DNA
   <213> Artificial Sequence
<220>
   <223> Synthetic Oligonucleotide
<400> 220
   ggatatttcc gtggctctta ttca 24
<210> 221
   <211> 25
   <212> DNA
   <213> Artificial Sequence
<220>
   <223> Synthetic Oligonucleotide
<400> 221
   cttctcatca aggcagaaaa atctt 25
<210> 222
   <211> 30
   <212> DNA
   <213> Artificial Sequence
<220>
   <223> Synthetic Oligonucleotide
<400> 222
   tctccatcaa atcctgtaaa ctccagagca 30
<210> 223
   <211> 18
   <212> DNA
   <213> Artificial Sequence
<220>
   <223> Synthetic Oligonucleotide
<400> 223
   tcattgacca cagtggcg 18
<210> 224
   <211> 19
   <212> DNA
   <213> Artificial Sequence
<220>
   <223> Synthetic Oligonucleotide
<400> 224
   tcgtgtactt ctccacccg 19
<210> 225
   <211> 24
   <212> DNA
   <213> Artificial Sequence
<220>
   <223> Synthetic Oligonucleotide
<400> 225
   cacgtcctca tactcaccaa ggcg 24
<210> 226
   <211> 23
   <212> DNA
   <213> Artificial Sequence
<220>
   <223> Synthetic Oligonucleotide
<400> 226
   ccccaggata cctaccacta cct 23
<210> 227
   <211> 18
   <212> DNA
   <213> Artificial Sequence
<220>
   <223> Synthetic Oligonucleotide
<400> 227
   tgcgggactt gggaaaga 18
<210> 228
   <211> 20
   <212> DNA
   <213> Artificial Sequence
<220>
   <223> Synthetic Oligonucleotide
<400> 228
   cccttcagcc tgccccaccg 20
<210> 229
   <211> 22
   <212> DNA
   <213> Artificial Sequence
<220>
   <223> Synthetic Oligonucleotide
<400> 229
   gacatttcca gtcctgcagt ca 22
<210> 230
   <211> 20
   <212> DNA
   <213> Artificial Sequence
<220>
   <223> Synthetic Oligonucleotide
<400> 230
   ctccgatcgc acacatttgt 20
<210> 231
   <211> 23
   <212> DNA
   <213> Artificial Sequence
<220>
   <223> Synthetic Oligonucleotide
<400> 231
   tgcctctctg ccccaccctt tgt 23
<210> 232
   <211> 21
   <212> DNA
   <213> Artificial Sequence
<220>
   <223> Synthetic Oligonucleotide
<400> 232
   tccctccact cggaaggact a 21
<210> 233
   <211> 22
   <212> DNA
   <213> Artificial Sequence
<220>
   <223> Synthetic Oligonucleotide
<400> 233
   cggttgttgc tgatctgtct ca 22
<210> 234
   <211> 27
   <212> DNA
   <213> Artificial Sequence
<220>
   <223> Synthetic Oligonucleotide
<400> 234
   tctgacactg tccaacttga ccctctt 27
<210> 235
   <211> 18
   <212> DNA
   <213> Artificial Sequence
<220>
   <223> Synthetic Oligonucleotide
<400> 235
   tccaccctcc tggctttg 18
<210> 236
   <211> 21
   <212> DNA
   <213> Artificial Sequence
<220>
   <223> Synthetic Oligonucleotide
<400> 236
   tcactcccac gttcaccttg t 21
<210> 237
   <211> 23
   <212> DNA
   <213> Artificial Sequence
<220>
   <223> Synthetic Oligonucleotide
<400> 237
   tcctttcgtc ttcgccatgc tgg 23
<210> 238
   <211> 18
   <212> DNA
   <213> Artificial Sequence
<220>
   <223> Synthetic Oligonucleotide
<400> 238
   gtggccatcc agctgacc 18
<210> 239
   <211> 23
   <212> DNA
   <213> Artificial Sequence
<220>
   <223> Synthetic Oligonucleotide
<400> 239
   cagtggtagg tgatgttctg gga 23
<210> 240
   <211> 21
   <212> DNA
   <213> Artificial Sequence
<220>
   <223> Synthetic Oligonucleotide
<400> 240
   tcctgcgcct gatgtccacc g 21
<210> 241
   <211> 24
   <212> DNA
   <213> Artificial Sequence
<220> ^{.}
   <223> Synthetic Oligonucleotide
<400> 241
   cagccaagaa ctggtatagg agct 24
<210> 242
   <211> 19
   <212> DNA
   <213> Artificial Sequence
<220>
   <223> Synthetic Oligonucleotide
<400> 242
   aaactggctg ccagcattg 19
<210> 243
   <211> 30
   <212> DNA
   <213> Artificial Sequence
<220>
   <223> Synthetic Oligonucleotide
<400> 243
   tctcctagcc agacgtgttt cttgtccttg 30
<210> 244
   <211> 21
   <212> DNA
   <213> Artificial Sequence
<220>
   <223> Synthetic Oligonucleotide
<400> 244
   gagagcaagc gagacattct g 21
<210> 245
   <211> 18
   <212> DNA
   <213> Artificial Sequence
<220>
   <223> Synthetic Oligonucleotide
<400> 245
   aacagggaac tggcccac 18
<210> 246
   <211> 24
   <212> DNA
   <213> Artificial Sequence
<220>
   <223> Synthetic Oligonucleotide
<400> 246
   cctctttgac ggctcagcca atct 24
<210> 247
   <211> 24
   <212> DNA
   <213> Artificial Sequence
<220>
   <223> Synthetic Oligonucleotide
<400> 247
   cgacagttgc gatgaaagtt ctaa 24
<210> 248
   <211> 22
   <212> DNA
   <213> Artificial Sequence
<220>
   <223> Synthetic Oligonucleotide
<400> 248
   ggctgctaga gaccatggac at 22
<210> 249
   <211> 27
   <212> DNA
   <213> Artificial Sequence
<220>
   <223> Synthetic Oligonucleotide
<400> 249
   cctcctcctg ttgctgccac taatgct 27
<210> 250
   <211> 23
   <212> DNA
   <213> Artificial Sequence
<220>
   <223> Synthetic Oligonucleotide
<400> 250
   tctgcagagt tggaagcact cta 23
<210> 251
   <211> 21
   <212> DNA
   <213> Artificial Sequence
<220>
   <223> Synthetic Oligonucleotide
<400> 251
   gccgaggctt ttctaccaga a 21
<210> 252
   <211> 28
   <212> DNA
   <213> Artificial Sequence
<220>
   <223> Synthetic Oligonucleotide
<400> 252
   caggatacag ctccacagca tcgatgtc 28
<210> 253
   <211> 20
   <212> DNA
   <213> Artificial Sequence
<220>
   <223> Synthetic Oligonucleotide
<400> 253
   acctctgtgg tccgtaggag 20
<210> 254
   <211> 20
   <212> DNA
   <213> Artificial Sequence
<220>
   <223> Synthetic Oligonucleotide
<400> 254
   cgaccacttg ttttccactg 20
<210> 255
   <211> 24
   <212> DNA
   <213> Artificial Sequence
<220>
   <223> Synthetic Oligonucleotide
<400> 255
   tcttcccagg gccctcctca tagt 24
<210> 256
   <211> 20
   <212> DNA
   <213> Artificial Sequence
<220>
   <223> Synthetic Oligonucleotide
<400> 256
   aacttcaagg tcggagaagg 20
<210> 257
   <211> 20
   <212> DNA
   <213> Artificial Sequence
<220>
   <223> Synthetic Oligonucleotide
<400> 257
   tggctaaact cctgcacttg 20
<210> 258
   <211> 21
   <212> DNA
   <213> Artificial Sequence
<220>
   <223> Synthetic Oligonucleotide
<400> 258
   ccgtccacgg tctcctcctc a 21
<210> 259
   <211> 18
   <212> DNA
   <213> Artificial Sequence
<220>
   <223> Synthetic Oligonucleotide
<400> 259
   gtgctacgcc accctgtt 18
<210> 260
   <211> 20
   <212> DNA
   <213> Artificial Sequence
<220>
   <223> Synthetic Oligonucleotide
<400> 260
   caggggcttc tcgtagatgt 20
<210> 261
   <211> 22
   <212> DNA
   <213> Artificial Sequence
<220>
   <223> Synthetic Oligonucleotide
<400> 261
   ccgatgttca cgcctttggg tc 22
<210> 262
   <211> 20
   <212> DNA
   <213> Artificial Sequence
<220>
   <223> Synthetic Oligonucleotide
<400> 262
   gatgtgattg aggtgcatgg 20
<210> 263
   <211> 21
   <212> DNA
   <213> Artificial Sequence
<220>
   <223> Synthetic Oligonucleotide
<400> 263
   gaactccctg gagatgaaac c 21
<210> 264
   <211> 24
   <212> DNA
   <213> Artificial Sequence
<220>
   <223> Synthetic Oligonucleotide
<400> 264
   tgttcatcct ggcgctcttc atgt 24
<210> 265
   <211> 18
   <212> DNA
   <213> Artificial Sequence
<220>
   <223> Synthetic Oligonucleotide
<400> 265
   tgcagcggct gattgaca 18
<210> 266
   <211> 25
   <212> DNA
   <213> Artificial Sequence
<220>
   <223> Synthetic Oligonucleotide
<400> 266
   caactgttcc tggtctacaa actca 25
<210> 267
   <211> 28
   <212> DNA
   <213> Artificial Sequence
<220>
   <223> Synthetic Oligonucleotide
<400> 267
   tcagatggag acctcgtgcc aaattaca 28
<210> 268
   <211> 20
   <212> DNA
   <213> Artificial Sequence
<220>
   <223> Synthetic Oligonucleotide
<400> 268
   agcttttccg gaatctgttc 20
<210> 269
   <211> 20
   <212> DNA
   <213> Artificial Sequence
<220>
   <223> Synthetic Oligonucleotide
<400> 269
   atttgagcat gttccagtcg 20
<210> 270
   <211> 24
   <212> DNA
   <213> Artificial Sequence
<220>
   <223> Synthetic Oligonucleotide
<400> 270
   catcgccagg gcttctccta tgac 24
<210> 271
   <211> 20
   <212> DNA
   <213> Artificial Sequence
<220>
   <223> Synthetic Oligonucleotide
<400> 271
   tggcagaact acctgcaaga 20
<210> 272
   <211> 19
   <212> DNA
   <213> Artificial Sequence
<220>
   <223> Synthetic Oligonucleotide
<400> 272
   tgcttcaagg tgtggttgg 19
<210> 273
   <211> 24
   <212> DNA
   <213> Artificial Sequence
<220>
   <223> Synthetic Oligonucleotide
<400> 273
   cacctgcgtc tggatgactg tgac 24
<210> 274
   <211> 24
   <212> DNA
   <213> Artificial Sequence
<220>
   <223> Synthetic Oligonucleotide
<400> 274
   gtacatgatc ccctgtgaga aggt 24
<210> 275
   <211> 21
   <212> DNA
   <213> Artificial Sequence
<220>
   <223> Synthetic Oligonucleotide
<400> 275
   gggacagctt gtagcctttg c 21
<210> 276
   <211> 22
   <212> DNA
   <213> Artificial Sequence
<220>
   <223> Synthetic Oligonucleotide
<400> 276
   accctgcccg cgatcacact ga 22
<210> 277
   <211> 23
   <212> DNA
   <213> Artificial Sequence
<220>
   <223> Synthetic Oligonucleotide
<400> 277
   gggaggctta tctcactgag tga 23
<210> 278
   <211> 19
   <212> DNA
   <213> Artificial Sequence
<220>
   <223> Synthetic Oligonucleotide
<400> 278
   ccattgcagc cttcattgc 19
<210> 279
   <211> 29
   <212> DNA
   <213> Artificial Sequence
<220>
   <223> Synthetic Oligonucleotide
<400> 279
   ttgaggccca gagcagtcta ccagattct 29
<210> 280
   <211> 21
   <212> DNA
   <213> Artificial Sequence
<220>
   <223> Synthetic Oligonucleotide
<400> 280
   tgtctcactg agcgagcaga a 21
<210> 281
   <211> 19
   <212> DNA
   <213> Artificial Sequence
<220>
   <223> Synthetic Oligonucleotide
<400> 281
   accattgcag ccctgattg 19
<210> 282
   <211> 24
   <212> DNA
   <213> Artificial Sequence
<220>
   <223> Synthetic Oligonucleotide
<400> 282
   cttgaggacg cgaacagtcc acca 24
<210> 283
   <211> 20
   <212> DNA
   <213> Artificial Sequence
<220>
   <223> Synthetic Oligonucleotide
<400> 283
   tgaccgcttc taccccaatg 20
<210> 284
   <211> 22
   <212> DNA
   <213> Artificial Sequence
<220>
   <223> Synthetic Oligonucleotide
<400> 284
   aggataaggc caaccatgat gt 22
<210> 285
   <211> 28
   <212> DNA
   <213> Artificial Sequence
<220>
   <223> Synthetic Oligonucleotide
<400> 285
   ctgaaactgg aacacaacca cccacaag 28
<210> 286
   <211> 19
   <212> DNA
   <213> Artificial Sequence
<220>
   <223> Synthetic Oligonucleotide
<400> 286
   ggtgcctact ccattgtgg 19
<210> 287
   <211> 20
   <212> DNA
   <213> Artificial Sequence
<220>
   <223> Synthetic Oligonucleotide
<400> 287
   gtggagccct tcttcctctt 20
<210> 288
   <211> 24
   <212> DNA
   <213> Artificial Sequence
<220>
   <223> Synthetic Oligonucleotide
<400> 288
   tactccagca ggcacacaaa cacg 24
<210> 289
   <211> 22
   <212> DNA
   <213> Artificial Sequence
<220>
   <223> Synthetic Oligonucleotide
<400> 289
   ccagctttgt gcctgtcact at 22
<210> 290
   <211> 20
   <212> DNA
   <213> Artificial Sequence
<220>
   <223> Synthetic Oligonucleotide
<400> 290
   gggaatgtgg tagcccaaga 20
<210> 291
   <211> 25
   <212> DNA
   <213> Artificial Sequence
<220>
   <223> Synthetic Oligonucleotide
<400> 291
   ctcatgccac cactgccaac acctc 25
<210> 292
   <211> 20
   <212> DNA
   <213> Artificial Sequence
<220>
   <223> Synthetic Oligonucleotide
<400> 292
   ccgtgttcaa gaggaagctc 20
<210> 293
   <211> 20
   <212> DNA
   <213> Artificial Sequence
<220>
   <223> Synthetic Oligonucleotide
<400> 293
   agtgggatca cagggtgaag 20
<210> 294
   <211> 24
   <212> DNA
   <213> Artificial Sequence
<220>
   <223> Synthetic Oligonucleotide
<400> 294
   ttttctcaac tcctccacaa ggca 24
<210> 295
   <211> 30
   <212> DNA
   <213> Artificial Sequence
<220>
   <223> Synthetic Oligonucleotide
<400> 295
   agaacaagga caacatagat ccttacatat 30
<210> 296
   <211> 19
   <212> DNA
   <213> Artificial Sequence
<220>
   <223> Synthetic Oligonucleotide
<400> 296
   gcaaacctca tgccaatgc 19
<210> 297
   <211> 26
   <212> DNA
   <213> Artificial Sequence
<220>
   <223> Synthetic Oligonucleotide
<400> 297
   cacacccttt ggaagtggac ccagaa 26
<210> 298
   <211> 18
   <212> DNA
   <213> Artificial Sequence
<220>
   <223> Synthetic Oligonucleotide
<400> 298
   ccgtgtggct cgctttct 18
<210> 299
   <211> 24
   <212> DNA
   <213> Artificial Sequence
<220>
   <223> Synthetic Oligonucleotide
<400> 299
   ggagatttcg ctgaagagta acca 24
<210> 300
   <211> 20
   <212> DNA
   <213> Artificial Sequence
<220>
   <223> Synthetic Oligonucleotide
<400> 300
   tgccgcttcc tctttgcggg 20
<210> 301
   <211> 19
   <212> DNA
   <213> Artificial Sequence
<220>
   <223> Synthetic Oligonucleotide
<400> 301
   cacaatggcg gctctgaag 19
<210> 302
   <211> 26
   <212> DNA
   <213> Artificial Sequence
<220>
   <223> Synthetic Oligonucleotide
<400> 302
   acacaaacac tgtctgtacc tgaaga 26
<210> 303
   <211> 23
   <212> DNA
   <213> Artificial Sequence
<220>
   <223> Synthetic Oligonucleotide
<400> 303
   aagttacgct gcgcgacagc caa 23
<210> 304
   <211> 20
   <212> DNA
   <213> Artificial Sequence
<220>
   <223> Synthetic Oligonucleotide
<400> 304
   caagcagtca aggagaacca 20
<210> 305
   <211> 20
   <212> DNA
   <213> Artificial Sequence
<220>
   <223> Synthetic Oligonucleotide
<400> 305
   agttttgctc gcctcatctt 20
<210> 306
   <211> 22
   <212> DNA
   <213> Artificial Sequence
<220>
   <223> Synthetic Oligonucleotide
<400> 306
   ttcttctgtc tcccgccgct tc 22
<210> 307
   <211> 20
   <212> DNA
   <213> Artificial Sequence
<220>
   <223> Synthetic Oligonucleotide
<400> 307
   tccaattcca gcatcactgt 20
<210> 308
   <211> 20
   <212> DNA
   <213> Artificial Sequence
<220>
   <223> Synthetic Oligonucleotide
<400> 308
   ggcagtgaag gcgataaagt 20
<210> 309
   <211> 25
   <212> DNA
   <213> Artificial Sequence
<220>
   <223> Synthetic Oligonucleotide
<400> 309
   agaaaagctg tttgtctccc cagca 25
<210> 310
   <211> 20
   <212> DNA
   <213> Artificial Sequence
<220>
   <223> Synthetic Oligonucleotide
<400> 310
   cagtgcttcc atggacaagt 20
<210> 311
   <211> 20
   <212> DNA
   <213> Artificial Sequence
<220>
   <223> Synthetic Oligonucleotide
<400> 311
   tggacaggga tgattgatgt 20
<210> 312
   <211> 24
   <212> DNA
   <213> Artificial Sequence
<220>
   <223> Synthetic Oligonucleotide
<400> 312
   atctccatca gcatgggcca gttt 24
<210> 313
   <211> 21
   <212> DNA
   <213> Artificial Sequence
<220>
   <223> Synthetic Oligonucleotide
<400> 313
   tgcacagagg gtgtgggtta c 21
<210> 314
   <211> 28
   <212> DNA
   <213> Artificial Sequence
<220>
   <223> Synthetic Oligonucleotide
<400> 314
   tcttcatctg atttacaagc tgtacatg 28
<210> 315
   <211> 29
   <212> DNA
   <213> Artificial Sequence
<220>
   <223> Synthetic Oligonucleotide
<400> 315
   caatgcttcc aacaatttgt ttgcttgcc 29
<210> 316
   <211> 24
   <212> DNA
   <213> Artificial Sequence
<220>
   <223> Synthetic Oligonucleotide
<400> 316
   ctctgagaca gtgcttcgat gact 24
<210> 317
   <211> 19
   <212> DNA
   <213> Artificial Sequence
<220>
   <223> Synthetic Oligonucleotide
<400> 317
   ccatgaggcc caacttcct 19
<210> 318
   <211> 23
   <212> DNA
   <213> Artificial Sequence
<220>
   <223> Synthetic Oligonucleotide
<400> 318
   cagacttggt gccctttgac tcc 23
<210> 319
   <211> 20
   <212> DNA
   <213> Artificial Sequence
<220>
   <223> Synthetic Oligonucleotide
<400> 319
   agacatcagc tcctggttca 20
<210> 320
   <211> 20
   <212> DNA
   <213> Artificial Sequence
<220>
   <223> Synthetic Oligonucleotide
<400> 320
   gacaaacacc cttcctccag 20
<210> 321
   <211> 26
   <212> DNA
   <213> Artificial Sequence
<220>
   <223> Synthetic Oligonucleotide
<400> 321
   cgaggccatt gacttcatag actcca 26
<210> 322
   <211> 22
   <212> DNA
   <213> Artificial Sequence
<220>
   <223> Synthetic Oligonucleotide
<400> 322
   cagaggatga cgaggatgat ga 22
<210> 323
   <211> 18
   <212> DNA
   <213> Artificial Sequence
<220>
   <223> Synthetic Oligonucleotide
<400> 323
   ctgtgccgcc tccttgtc 18
<210> 324
   <211> 28
   <212> DNA
   <213> Artificial Sequence
<220>
   <223> Synthetic Oligonucleotide
<400> 324
   ctcctcattg tcactgccaa acaggtca 28
<210> 325
   <211> 20
   <212> DNA
   <213> Artificial Sequence
<220>
   <223> Synthetic Oligonucleotide
<400> 325
   tgtcgatgga cttccagaac 20
<210> 326
   <211> 19
   <212> DNA
   <213> Artificial Sequence
<220>
   <223> Synthetic Oligonucleotide
<400> 326
   attgggacag cttggatca 19
<210> 327
   <211> 18
   <212> DNA
   <213> Artificial Sequence
<220>
   <223> Synthetic Oligonucleotide
<400> 327
   cacctgggca gctgccaa 18
<210> 328
   <211> 23
   <212> DNA
   <213> Artificial Sequence
<220>
   <223> Synthetic Oligonucleotide
<400> 328
   gatctaagat ggcgactgtc gaa 23
<210> 329
   <211> 25
   <212> DNA
   <213> Artificial Sequence
<220>
   <223> Synthetic Oligonucleotide
<400> 329
   ttagattccg ttttctcctc ttctg 25
<210> 330
   <211> 27
   <212> DNA
   <213> Artificial Sequence
<220>
   <223> Synthetic Oligonucleotide
<400> 330
   accaccccta ctcctaatcc cccgact 27
<210> 331
   <211> 18
   <212> DNA
   <213> Artificial Sequence
<220>
   <223> Synthetic Oligonucleotide
<400> 331
   aagccgcggt tgaatgtg 18
<210> 332
   <211> 20
   <212> DNA
   <213> Artificial Sequence
<220>
   <223> Synthetic Oligonucleotide
<400> 332
   tgacgccagc ttcaatgatg 20
<210> 333
   <211> 25
   <212> DNA
   <213> Artificial Sequence
<220>
   <223> Synthetic Oligonucleotide
<400> 333
   tgaccctctc cctctctgga tggca 25
<210> 334
   <211> 18
   <212> DNA
   <213> Artificial Sequence
<220>
   <223> Synthetic Oligonucleotide
<400> 334
   ctcggatcct agccctcg 18
<210> 335
   <211> 22
   <212> DNA
   <213> Artificial Sequence
<220>
   <223> Synthetic Oligonucleotide
<400> 335
   ggcattggaa tatccctctg ta 22
<210> 336
   <211> 24
   <212> DNA
   <213> Artificial Sequence
<220>
   <223> Synthetic Oligonucleotide
<400> 336
   cctccatgga ctcgagtgta ccga 24
<210> 337
   <211> 20
   <212> DNA
   <213> Artificial Sequence
<220>
   <223> Synthetic Oligonucleotide
<400> 337
   tggtccatcg ccagttatca 20
<210> 338
   <211> 23
   <212> DNA
   <213> Artificial Sequence
<220>
   <223> Synthetic Oligonucleotide
<400> 338
   tgttctagcg atcttgcttc aca 23
<210> 339
   <211> 30
   <212> DNA
   <213> Artificial Sequence
<220>
   <223> Synthetic Oligonucleotide
<400> 339
   atctgtatgc ggaacctcaa aagagtccct 30
<210> 340
   <211> 23
   <212> DNA
   <213> Artificial Sequence
<220>
   <223> Synthetic Oligonucleotide
<400> 340
   cggttatgtc atgccagata cac 23
<210> 341
   <211> 24
   <212> DNA
   <213> Artificial Sequence
<220>
   <223> Synthetic Oligonucleotide
<400> 341
   gaactgagac ccactgaaga aagg 24
<210> 342
   <211> 25
   <212> DNA
   <213> Artificial Sequence
<220>
   <223> Synthetic Oligonucleotide
<400> 342
   cctcaaaggt actccctcct cccgg 25
<210> 343
   <211> 25
   <212> DNA
   <213> Artificial Sequence
<220>
   <223> Synthetic Oligonucleotide
<400> 343
   tggctcttaa tcagtttcgt tacct 25
<210> 344
   <211> 25
   <212> DNA
   <213> Artificial Sequence
<220>
   <223> Synthetic Oligonucleotide
<400> 344
   caaggcatat cgatcctcat aaagt 25
<210> 345
   <211> 30
   <212> DNA
   <213> Artificial Sequence
<220>
   <223> Synthetic Oligonucleotide
<400> 345
   tgtcccacga ataatgcgta aattctccag 30
<210> 346
   <211> 20
   <212> DNA
   <213> Artificial Sequence
<220>
   <223> Synthetic Oligonucleotide
<400> 346
   gtccaggtgg atgtgaaaga 20
<210> 347
   <211> 20
   <212> DNA
   <213> Artificial Sequence
<220>
   <223> Synthetic Oligonucleotide
<400> 347
   cggccaggat acacatctta 20
<210> 348
   <211> 21
   <212> DNA
   <213> Artificial Sequence
<220>
   <223> Synthetic Oligonucleotide
<400> 348
   cagcaggccc tcaaggagct g 21
<210> 349
   <211> 20
   <212> DNA
   <213> Artificial Sequence
<220>
   <223> Synthetic Oligonucleotide
<400> 349
   acggatcaca gtggaggaag 20
<210> 350
   <211> 20
   <212> DNA
   <213> Artificial Sequence
<220>
   <223> Synthetic Oligonucleotide
<400> 350
   ctcatccgtc gggtcatagt 20
<210> 351
   <211> 20
   <212> DNA
   <213> Artificial Sequence
<220>
   <223> Synthetic Oligonucleotide
<400> 351
   cgctggctca cccctacctg 20
<210> 352
   <211> 18
   <212> DNA
   <213> Artificial Sequence
<220>
   <223> Synthetic Oligonucleotide
<400> 352
   acccccagac cggatcag 18
<210> 353
   <211> 23
   <212> DNA
   <213> Artificial Sequence
<220>
   <223> Synthetic Oligonucleotide
<400> 353
   tgtagggcag acttcctcaa aca 23
<210> 354
   <211> 23
   <212> DNA
   <213> Artificial Sequence
<220>
   <223> Synthetic Oligonucleotide
<400> 354
   ctggccctca tgtccccttc acg 23
<210> 355
   <211> 19
   <212> DNA
   <213> Artificial Sequence
<220>
   <223> Synthetic Oligonucleotide
<400> 355
   cgtggtgccc ctctatgac 19
<210> 356
   <211> 19
   <212> DNA
   <213> Artificial Sequence
<220>
   <223> Synthetic Oligonucleotide
<400> 356
   ggctagtggg cgcatgtag 19
<210> 357
   <211> 19
   <212> DNA
   <213> Artificial Sequence
<220>
   <223> Synthetic Oligonucleotide
<400> 357
   ctggagatgc tggacgccc 19
<210> 358
   <211> 21
   <212> DNA
   <213> Artificial Sequence
<220>
   <223> Synthetic Oligonucleotide
<400> 358
   ggattgctca acaaccatgc t 21
<210> 359
   <211> 24
   <212> DNA
   <213> Artificial Sequence
<220>
   <223> Synthetic Oligonucleotide
<400> 359
   ggcattaaca cttttggacg ataa 24
<210> 360
   <211> 30
   <212> DNA
   <213> Artificial Sequence
<220>
   <223> Synthetic Oligonucleotide
<400> 360
   tctggaccct cctacctctg gttcttacgt 30
<210> 361
   <211> 24
   <212> DNA
   <213> Artificial Sequence
<220>
   <223> Synthetic Oligonucleotide
<400> 361
   gcactttggg attctttcca ttat 24
<210> 362
   <211> 24
   <212> DNA
   <213> Artificial Sequence
<220>
   <223> Synthetic Oligonucleotide
<400> 362
   gcatgtaaga agaccctcac tgaa 24
<210> 363
   <211> 29
   <212> DNA
   <213> Artificial Sequence
<220>
   <223> Synthetic Oligonucleotide
<400> 363
   acaacattct cggtgcctgt aacaaagaa 29
<210> 364
   <211> 18
   <212> DNA
   <213> Artificial Sequence
<220>
   <223> Synthetic Oligonucleotide
<400> 364
   gcctcttcct gttcgacg 18
<210> 365
   <211> 18
   <212> DNA
   <213> Artificial Sequence
<220>
   <223> Synthetic Oligonucleotide
<400> 365
   gctttgcccg gtagctct 18
<210> 366
   <211> 24
   <212> DNA
   <213> Artificial Sequence
<220>
   <223> Synthetic Oligonucleotide
<400> 366
   tcgcccacct acgtactggc ctac 24
<210> 367
   <211> 25
   <212> DNA
   <213> Artificial Sequence
<220>
   <223> Synthetic Oligonucleotide
<400> 367
   ggattgtaga ctgtcaccga aattc 25
<210> 368
   <211> 28
   <212> DNA
   <213> Artificial Sequence
<220>
   <223> Synthetic Oligonucleotide
<400> 368
   ggctattcct cattttctct acaaagtg 28
<210> 369
   <211> 30
   <212> DNA
   <213> Artificial Sequence
<220>
   <223> Synthetic Oligonucleotide
<400> 369
   cctccaggag gctaccttct tcatgttcac 30
<210> 370
   <211> 19
   <212> DNA
   <213> Artificial Sequence
<220>
   <223> Synthetic Oligonucleotide
<400> 370
   aaggaaaggg tgcctcatc 19
<210> 371
   <211> 19
   <212> DNA
   <213> Artificial Sequence
<220>
   <223> Synthetic Oligonucleotide
<400> 371
   gagccacaag cagcacagt 19
<210> 372
   <211> 24
   <212> DNA
   <213> Artificial Sequence
<220>
   <223> Synthetic Oligonucleotide
<400> 372
   catcacccac aaggacaggt tgct 24
<210> 373
   <211> 20
   <212> DNA
   <213> Artificial Sequence
<220>
   <223> Synthetic Oligonucleotide
<400> 373
   cacgggacat tcaccacatc 20
<210> 374
   <211> 19
   <212> DNA
   <213> Artificial Sequence
<220>
   <223> Synthetic Oligonucleotide
<400> 374
   gggtgccatc cacttcaca 19
<210> 375
   <211> 27
   <212> DNA
   <213> Artificial Sequence
<220>
   <223> Synthetic Oligonucleotide
<400> 375
   ataaaaagac aaccaacggc cgactgc 27
<210> 376
   <211> 18
   <212> DNA
   <213> Artificial Sequence
<220>
   <223> Synthetic Oligonucleotide
<400> 376
   ccagtggagc gcttccat 18
<210> 377
   <211> 22
   <212> DNA
   <213> Artificial Sequence
<220>
   <223> Synthetic Oligonucleotide
<400> 377
   ctctctgggt cgtctgaaac aa 22
<210> 378
   <211> 23
   <212> DNA
   <213> Artificial Sequence
<220>
   <223> Synthetic Oligonucleotide
<400> 378
   tcggccactt catcaggacg cag 23
<210> 379
   <211> 22
   <212> DNA
   <213> Artificial Sequence
<220>
   <223> Synthetic Oligonucleotide
<400> 379
   ggttccagct ttctgtagct gt 22
<210> 380
   <211> 19
   <212> DNA
   <213> Artificial Sequence
<220>
   <223> Synthetic Oligonucleotide
<400> 380
   gccgcaggtt ctagttgct 19
<210> 381
   <211> 24
   <212> DNA
   <213> Artificial Sequence
<220>
   <223> Synthetic Oligonucleotide
<400> 381
   attggtggcc acaccacctc ctta 24
<210> 382
   <211> 21
   <212> DNA
   <213> Artificial Sequence
<220>
   <223> Synthetic Oligonucleotide
<400> 382
   gcgtatgatt tcccgaatga g 21
<210> 383
   <211> 21
   <212> DNA
   <213> Artificial Sequence
<220>
   <223> Synthetic Oligonucleotide
<400> 383
   cagtgacctc gtacccattg c 21
<210> 384
   <211> 25
   <212> DNA
   <213> Artificial Sequence
<220>
   <223> Synthetic Oligonucleotide
<400> 384
   atgttgatat gcccaaactt catga 25
<210> 385
   <211> 21
   <212> DNA
   <213> Artificial Sequence
<220>
   <223> Synthetic Oligonucleotide
<400> 385
   cgagcccttt gatgacttcc t 21
<210> 386
   <211> 18
   <212> DNA
   <213> Artificial Sequence
<220>
   <223> Synthetic Oligonucleotide
<400> 386
   ggagcgggct gtctcaga 18
<210> 387
   <211> 22
   <212> DNA
   <213> Artificial Sequence
<220>
   <223> Synthetic Oligonucleotide
<400> 387
   tcccagcatc atccaggccc ag 22
<210> 388
   <211> 19
   <212> DNA
   <213> Artificial Sequence
<220>
   <223> Synthetic Oligonucleotide
<400> 388
   ccaccccgag caaatctgt 19
<210> 389
   <211> 22
   <212> DNA
   <213> Artificial Sequence
<220>
   <223> Synthetic Oligonucleotide
<400> 389
   aaatccagtc cccctacttt gg 22
<210> 390
   <211> 23
   <212> DNA
   <213> Artificial Sequence
<220>
   <223> Synthetic Oligonucleotide
<400> 390
   cctgaatcct ggaggctcac gcc 23
<210> 391
   <211> 26
   <212> DNA
   <213> Artificial Sequence
<220>
   <223> Synthetic Oligonucleotide
<400> 391
   ggataattca gacaacaaca ccatct 26
<210> 392
   <211> 25
   <212> DNA
   <213> Artificial Sequence
<220>
   <223> Synthetic Oligonucleotide
<400> 392
   tgaagtaatc agccacagac tcaat 25
<210> 393
   <211> 29
   <212> DNA
   <213> Artificial Sequence
<220>
   <223> Synthetic Oligonucleotide
<400> 393
   tcaattgtaa cattctcacc caggccttg 29
<210> 394
   <211> 20
   <212> DNA
   <213> Artificial Sequence
<220>
   <223> Synthetic Oligonucleotide
<400> 394
   gaagcgcaga tcatgaagaa 20
<210> 395
   <211> 20
   <212> DNA
   <213> Artificial Sequence
<220>
   <223> Synthetic Oligonucleotide
<400> 395
   ctcctcagac accactgcat 20
<210> 396
   <211> 24
   <212> DNA
   <213> Artificial Sequence
<220>
   <223> Synthetic Oligonucleotide
<400> 396
   ctgaagcacg acaagctggt ccag 24
<210> 397
   <211> 20
   <212> DNA
   <213> Artificial Sequence
<220>
   <223> Synthetic Oligonucleotide
<400> 397
   cctccaactc ctagcctcaa 20
<210> 398
   <211> 18
   <212> DNA
   <213> Artificial Sequence
<220>
   <223> Synthetic Oligonucleotide
<400> 398
   ggcgcatgct tgtaatcc 18
<210> 399
   <211> 24
   <212> DNA
   <213> Artificial Sequence
<220>
   <223> Synthetic Oligonucleotide
<400> 399
   tgatcctcct gtctcaacct ccca 24
<210> 400
   <211> 18
   <212> DNA
   <213> Artificial Sequence
<220>
   <223> Synthetic Oligonucleotide
<400> 400
   gtgctggtga cgaatcca 18
<210> 401
   <211> 20
   <212> DNA
   <213> Artificial Sequence
<220>
   <223> Synthetic Oligonucleotide
<400> 401
   cccggcaaaa acaaataagt 20
<210> 402
   <211> 25
   <212> DNA
   <213> Artificial Sequence
<220>
   <223> Synthetic Oligonucleotide
<400> 402
   ttcatctcaa tggaaggatc ctgcc 25
<210> 403
   <211> 20
   <212> DNA
   <213> Artificial Sequence
<220>
   <223> Synthetic Oligonucleotide
<400> 403
   accctcgaca agaccacact 20
<210> 404
   <211> 20
   <212> DNA
   <213> Artificial Sequence
<220>
   <223> Synthetic Oligonucleotide
<400> 404
   tgggagttca tgggtacaga 20
<210> 405
   <211> 24
   <212> DNA
   <213> Artificial Sequence
<220>
   <223> Synthetic Oligonucleotide
<400> 405
   aacttcagcc ccagctccca agtc 24
<210> 406
   <211> 25
   <212> DNA
   <213> Artificial Sequence
<220>
   <223> Synthetic Oligonucleotide
<400> 406
   aagggcaatc acagtgttaa aagaa 25
<210> 407
   <211> 26
   <212> DNA
   <213> Artificial Sequence
<220>
   <223> Synthetic Oligonucleotide
<400> 407
   ggagaacttc aatgaagaat tttcca 26
<210> 408
   <211> 29
   <212> DNA
   <213> Artificial Sequence
<220>
   <223> Synthetic Oligonucleotide
<400> 408
   cataggagca gcctgcaaca tttcagcat 29
<210> 409
   <211> 20
   <212> DNA
   <213> Artificial Sequence
<220>
   <223> Synthetic Oligonucleotide
<400> 409
   attccaccca tggcaaattc 20
<210> 410
   <211> 22
   <212> DNA
   <213> Artificial Sequence
<220>
   <223> Synthetic Oligonucleotide
<400> 410
   gatgggattt ccattgatga ca 22
<210> 411
   <211> 22
   <212> DNA
   <213> Artificial Sequence
<220>
   <223> Synthetic Oligonucleotide
<400> 411
   ccgttctcag ccttgacggt gc 22
<210> 412
   <211> 23
   <212> DNA
   <213> Artificial Sequence
<220>
   <223> Synthetic Oligonucleotide
<400> 412
   caaaggagct cactgtggtg tct 23
<210> 413
   <211> 26
   <212> DNA
   <213> Artificial Sequence
<220>
   <223> Synthetic Oligonucleotide
<400> 413
   gagtcagaat ggcttattca cagatg 26
<210> 414
   <211> 24
   <212> DNA
   <213> Artificial Sequence
<220>
   <223> Synthetic Oligonucleotide
<400> 414
   tgttccaacc actgaatctg gacc 24
<210> 415
   <211> 20
   <212> DNA
   <213> Artificial Sequence
<220>
   <223> Synthetic Oligonucleotide
<400> 415
   ttgggaaata tttgggcatt 20
<210> 416
   <211> 20
   <212> DNA
   <213> Artificial Sequence
<220>
   <223> Synthetic Oligonucleotide
<400> 416
   agaagctagg gtggttgtcc 20
<210> 417
   <211> 26
   <212> DNA
   <213> Artificial Sequence
<220>
   <223> Synthetic Oligonucleotide
<400> 417
   ttgggacatt gtagacttgg ccagac 26
<210> 418
   <211> 19
   <212> DNA
   <213> Artificial Sequence
<220>
   <223> Synthetic Oligonucleotide
<400> 418
   gcatgggaac catcaacca 19
<210> 419
   <211> 21
   <212> DNA
   <213> Artificial Sequence
<220>
   <223> Synthetic Oligonucleotide
<400> 419
   tgaggagttt gccttgattc g 21
<210> 420
   <211> 30
   <212> DNA
   <213> Artificial Sequence
<220>
   <223> Synthetic Oligonucleotide
<400> 420
   ccatggacca acttcactat gtgacagagc 30
<210> 421
   <211> 20
   <212> DNA
   <213> Artificial Sequence
<220>
   <223> Synthetic Oligonucleotide
<400> 421
   ctgttgcagg aaggcattga 20
<210> 422
   <211> 28
   <212> DNA
   <213> Artificial Sequence
<220>
   <223> Synthetic Oligonucleotide
<400> 422
   gtcagtgggt ctctaataaa gagatgag 28
<210> 423
   <211> 21
   <212> DNA
   <213> Artificial Sequence
<220>
   <223> Synthetic Oligonucleotide
<400> 423
   catctcctgg cccagcatgt t 21
<210> 424
   <211> 21
   <212> DNA
   <213> Artificial Sequence
<220>
   <223> Synthetic Oligonucleotide
<400> 424
   cgctccagac ctatgatgac t 21
<210> 425
   <211> 20
   <212> DNA
   <213> Artificial Sequence
<220>
   <223> Synthetic Oligonucleotide
<400> 425
   acagtggaag gaccaggact 20
<210> 426
   <211> 24
   <212> DNA
   <213> Artificial Sequence
<220>
   <223> Synthetic Oligonucleotide
<400> 426
   tgttagccaa agactgccac tgca 24
<210> 427
   <211> 18
   <212> DNA
   <213> Artificial Sequence
<220>
   <223> Synthetic Oligonucleotide
<400> 427
   ctccgacgtg gctttcca 18
<210> 428
   <211> 23
   <212> DNA
   <213> Artificial Sequence
<220>
   <223> Synthetic Oligonucleotide
<400> 428
   cgtaattggc agaattgatg aca 23
<210> 429
   <211> 25
   <212> DNA
   <213> Artificial Sequence
<220>
   <223> Synthetic Oligonucleotide
<400> 429
   tggcccacag catctatgga atccc 25
<210> 430
   <211> 23
   <212> DNA
   <213> Artificial Sequence
<220>
   <223> Synthetic Oligonucleotide
<400> 430
   ggagctctgc agtaaccaca gaa 23
<210> 431
   <211> 20
   <212> DNA
   <213> Artificial Sequence
<220>
   <223> Synthetic Oligonucleotide
<400> 431
   caatgccatc cagctcttga 20
<210> 432
   <211> 22
   <212> DNA
   <213> Artificial Sequence
<220>
   <223> Synthetic Oligonucleotide
<400> 432
   aggccccatg ctgacgtccc tc 22
<210> 433
   <211> 20
   <212> DNA
   <213> Artificial Sequence
<220>
   <223> Synthetic Oligonucleotide
<400> 433
   ggtgaaggtt gtctcttccg 20
<210> 434
   <211> 19
   <212> DNA
   <213> Artificial Sequence
<220>
   <223> Synthetic Oligonucleotide
<400> 434
   cagcccttcc acttgtctg 19
<210> 435
   <211> 24
   <212> DNA
   <213> Artificial Sequence
<220>
   <223> Synthetic Oligonucleotide
<400> 435
   aagagccctg tcttcagaag gccc 24
<210> 436
   <211> 20
   <212> DNA
   <213> Artificial Sequence
<220>
   <223> Synthetic Oligonucleotide
<400> 436
   tacccttcca tgtgctggat 20
<210> 437
   <211> 18
   <212> DNA
   <213> Artificial Sequence
<220>
   <223> Synthetic Oligonucleotide
<400> 437
   gctgaagagg cccaggtt 18
<210> 438
   <211> 24
   <212> DNA
   <213> Artificial Sequence
<220>
   <223> Synthetic Oligonucleotide
<400> 438
   cgggactccc tggtcagcta catc 24
<210> 439
   <211> 18
   <212> DNA
   <213> Artificial Sequence
<220>
   <223> Synthetic Oligonucleotide
<400> 439
   gcttatgacc gaccccaa 18
<210> 440
   <211> 20
   <212> DNA
   <213> Artificial Sequence
<220>
   <223> Synthetic Oligonucleotide
<400> 440
   aaagttccag gcaacatcgt 20
<210> 441
   <211> 24
   <212> DNA
   <213> Artificial Sequence
<220>
   <223> Synthetic Oligonucleotide
<400> 441
   ctcatcacct ggtctccggt gtgt 24
<210> 442
   <211> 20
   <212> DNA
   <213> Artificial Sequence
<220>
   <223> Synthetic Oligonucleotide
<400> 442
   ccatctgcat ccatcttgtt 20
<210> 443
   <211> 20
   <212> DNA
   <213> Artificial Sequence
<220>
   <223> Synthetic Oligonucleotide
<400> 443
   ggccaccagg gtattatctg 20
<210> 444
   <211> 23
   <212> DNA
   <213> Artificial Sequence
<220>
   <223> Synthetic Oligonucleotide
<400> 444
   ctccccaccc ttgagaagtg cct 23
<210> 445
   <211> 18
   <212> DNA
   <213> Artificial Sequence
<220>
   <223> Synthetic Oligonucleotide
<400> 445
   gacaaggacg gcagcaag 18
<210> 446
   <211> 18
   <212> DNA
   <213> Artificial Sequence
<220>
   <223> Synthetic Oligonucleotide
<400> 446
   ttgtggcctg tctggacc 18
<210> 447
   <211> 24
   <212> DNA
   <213> Artificial Sequence
<220>
   <223> Synthetic Oligonucleotide
<400> 447
   ccaggagttg ccaccactgt tgtc 24
<210> 448
   <211> 20
   <212> DNA
   <213> Artificial Sequence
<220>
   <223> Synthetic Oligonucleotide
<400> 448
   gtgatcccaa gcccacaata 20
<210> 449
   <211> 18
   <212> DNA
   <213> Artificial Sequence
<220>
   <223> Synthetic Oligonucleotide
<400> 449
   tgtggcgatc aggatgtg 18
<210> 450
   <211> 24
   <212> DNA
   <213> Artificial Sequence
<220>
   <223> Synthetic Oligonucleotide
<400> 450
   tcagtgggaa aaagtacacc gccc 24
<210> 451
   <211> 20
   <212> DNA
   <213> Artificial Sequence
<220>
   <223> Synthetic Oligonucleotide
<400> 451
   ggcccagctt gaatttttca 20
<210> 452
   <211> 27
   <212> DNA
   <213> Artificial Sequence
<220>
   <223> Synthetic Oligonucleotide
<400> 452
   aagctatgag gaaaagaagt acacgat 27
<210> 453
   <211> 30
   <212> DNA
   <213> Artificial Sequence
<220>
   <223> Synthetic Oligonucleotide
<400> 453
   tcagccactg gcttctgtca taatcaggag 30
<210> 454
   <211> 20
   <212> DNA
   <213> Artificial Sequence
<220>
   <223> Synthetic Oligonucleotide
<400> 454
   gagaccctgc tgtcccagaa 20
<210> 455
   <211> 23
   <212> DNA
   <213> Artificial Sequence
<220>
   <223> Synthetic Oligonucleotide
<400> 455
   ggttgtagtc agcgaaggag atc 23
<210> 456
   <211> 26
   <212> DNA
   <213> Artificial Sequence
<220>
   <223> Synthetic Oligonucleotide
<400> 456
   tcccacaatg aaggtcttgc ctccct 26
<210> 457
   <211> 20
   <212> DNA
   <213> Artificial Sequence
<220>
   <223> Synthetic Oligonucleotide
<400> 457
   cccactcagt agccaagtca 20
<210> 458
   <211> 20
   <212> DNA
   <213> Artificial Sequence
<220>
   <223> Synthetic Oligonucleotide
<400> 458
   cacgcaggtg gtatcagtct 20
<210> 459
   <211> 27
   <212> DNA
   <213> Artificial Sequence
<220>
   <223> Synthetic Oligonucleotide
<400> 459
   tcaagtaaac gggctgtttt ccaaaca 27
<210> 460
   <211> 18
   <212> DNA
   <213> Artificial Sequence
<220>
   <223> Synthetic Oligonucleotide
<400> 460
   tcctgtgctc tggaagcc 18
<210> 461
   <211> 21
   <212> DNA
   <213> Artificial Sequence
<220>
   <223> Synthetic Oligonucleotide
<400> 461
   ctccacggtc tcagttgatc t 21
<210> 462
   <211> 24
   <212> DNA
   <213> Artificial Sequence
<220>
   <223> Synthetic Oligonucleotide
<400> 462
   caagaacctc ccagaagggc tcaa 24
<210> 463
   <211> 20
   <212> DNA
   <213> Artificial Sequence
<220>
   <223> Synthetic Oligonucleotide
<400> 463
   cggtgtgaga agtgcagcaa 20
<210> 464
   <211> 19
   <212> DNA
   <213> Artificial Sequence
<220>
   <223> Synthetic Oligonucleotide
<400> 464
   cctctcgcaa gtgctccat 19
<210> 465
   <211> 24
   <212> DNA
   <213> Artificial Sequence
<220>
   <223> Synthetic Oligonucleotide
<400> 465
   ccagaccata gcacactcgg gcac 24
<210> 466
   <211> 24
   <212> DNA
   <213> Artificial Sequence
<220>
   <223> Synthetic Oligonucleotide
<400> 466
   tgaacataaa gtctgcaaca tgga 24
<210> 467
   <211> 28
   <212> DNA
   <213> Artificial Sequence
<220>
   <223> Synthetic Oligonucleotide
<400> 467
   tgaggttggt tactgttggt atcatata 28
<210> 468
   <211> 24
   <212> DNA
   <213> Artificial Sequence
<220>
   <223> Synthetic Oligonucleotide
<400> 468
   ttgcactgca caggccacat tcac 24
<210> 469
   <211> 24
   <212> DNA
   <213> Artificial Sequence
<220>
   <223> Synthetic Oligonucleotide
<400> 469
   tccaggatgt taggaactgt gaag 24
<210> 470
   <211> 22
   <212> DNA
   <213> Artificial Sequence
<220>
   <223> Synthetic Oligonucleotide
<400> 470
   gcgtgtctgc gtagtagctg tt 22
<210> 471
   <211> 24
   <212> DNA
   <213> Artificial Sequence
<220>
   <223> Synthetic Oligonucleotide
<400> 471
   agtcgctggt ttcatgccct tcca 24
<210> 472
   <211> 19
   <212> DNA
   <213> Artificial Sequence
<220>
   <223> Synthetic Oligonucleotide
<400> 472
   ggacgaatac gaccccact 19
<210> 473
   <211> 18
   <212> DNA
   <213> Artificial Sequence
<220>
   <223> Synthetic Oligonucleotide
<400> 473
   gcacgtctcc ccatcaat 18
<210> 474
   <211> 24
   <212> DNA
   <213> Artificial Sequence
<220>
   <223> Synthetic Oligonucleotide
<400> 474
   accacctgct tccggtagga atcc 24
<210> 475
   <211> 19
   <212> DNA
   <213> Artificial Sequence
<220>
   <223> Synthetic Oligonucleotide
<400> 475
   ctgctgcgac agtccacta 19
<210> 476
   <211> 18
   <212> DNA
   <213> Artificial Sequence
<220>
   <223> Synthetic Oligonucleotide
<400> 476
   caggttcgct ctgggaag 18
<210> 477
   <211> 24
   <212> DNA
   <213> Artificial Sequence
<220>
   <223> Synthetic Oligonucleotide
<400> 477
   agagtgactc ccgttgtccc aagg 24
<210> 478
   <211> 19
   <212> DNA
   <213> Artificial Sequence
<220>
   <223> Synthetic Oligonucleotide
<400> 478
   ggtccgcttc gtctttcga 19
<210> 479
   <211> 19
   <212> DNA
   <213> Artificial Sequence
<220>
   <223> Synthetic Oligonucleotide
<400> 479
   gcacaggttc gctctggaa 19
<210> 480
   <211> 20
   <212> DNA
   <213> Artificial Sequence
<220>
   <223> Synthetic Oligonucleotide
<400> 480
   tgactcccgc ggtcccaagg 20
<210> 481
   <211> 19
   <212> DNA
   <213> Artificial Sequence
<220>
   <223> Synthetic Oligonucleotide
<400> 481
   gcaggtgtga ttgctggac 19
<210> 482
   <211> 18
   <212> DNA
   <213> Artificial Sequence
<220>
   <223> Synthetic Oligonucleotide
<400> 482
   accataggca atggcagc 18
<210> 483
   <211> 24
   <212> DNA
   <213> Artificial Sequence
<220>
   <223> Synthetic Oligonucleotide
<400> 483
   aagaatcatc aatgagccca cggc 24
<210> 484
   <211> 24
   <212> DNA
   <213> Artificial Sequence
<220>
   <223> Synthetic Oligonucleotide
<400> 484
   ggctagtaga actggatccc aaca 24
<210> 485
   <211> 20
   <212> DNA
   <213> Artificial Sequence
<220>
   <223> Synthetic Oligonucleotide
<400> 485
   ggtctgccca aatgcttttc 20
<210> 486
   <211> 30
   <212> DNA
   <213> Artificial Sequence
<220>
   <223> Synthetic Oligonucleotide
<400> 486
   taattagacc taggcctcag ctgcactgcc 30
<210> 487
   <211> 19
   <212> DNA
   <213> Artificial Sequence
<220>
   <223> Synthetic Oligonucleotide
<400> 487
   ggccactaaa gatgctggc 19
<210> 488
   <211> 18
   <212> DNA
   <213> Artificial Sequence
<220>
   <223> Synthetic Oligonucleotide
<400> 488
   agcagctgtg ggctcatt 18
<210> 489
   <211> 24
   <212> DNA
   <213> Artificial Sequence
<220>
   <223> Synthetic Oligonucleotide
<400> 489
   atcacccgaa gcacattcag tcca 24
<210> 490
   <211> 19
   <212> DNA
   <213> Artificial Sequence
<220>
   <223> Synthetic Oligonucleotide
<400> 490
   ccgactggag gagcataaa 19
<210> 491
   <211> 19
   <212> DNA
   <213> Artificial Sequence
<220>
   <223> Synthetic Oligonucleotide
<400> 491
   atgctggctg actctgctc 19
<210> 492
   <211> 24
   <212> DNA
   <213> Artificial Sequence
<220>
   <223> Synthetic Oligonucleotide
<400> 492
   cgcacttttc tgagcagacg tcca 24
<210> 493
   <211> 20
   <212> DNA
   <213> Artificial Sequence
<220>
   <223> Synthetic Oligonucleotide
<400> 493
   caaaaggcag aggctgataa 20
<210> 494
   <211> 20
   <212> DNA
   <213> Artificial Sequence
<220>
   <223> Synthetic Oligonucleotide
<400> 494
   agcgcagttt cataaagcaa 20
<210> 495
   <211> 27
   <212> DNA
   <213> Artificial Sequence
<220>
   <223> Synthetic Oligonucleotide
<400> 495
   tgaccagatc cttcacagac ttgtcgt 27
<210> 496
   <211> 19
   <212> DNA
   <213> Artificial Sequence
<220>
   <223> Synthetic Oligonucleotide
<400> 496
   agaaccgcaa ggtgagcaa 19
<210> 497
   <211> 21
   <212> DNA
   <213> Artificial Sequence
<220>
   <223> Synthetic Oligonucleotide
<400> 497
   tccaactgaa ggtccctgat g 21
<210> 498
   <211> 26
   <212> DNA
   <213> Artificial Sequence
<220>
   <223> Synthetic Oligonucleotide
<400> 498
   tggagattct ccagcacgtc atcgac 26
<210> 499
   <211> 20
   <212> DNA
   <213> Artificial Sequence
<220>
   <223> Synthetic Oligonucleotide
<400> 499
   cacgcagaaa accacacttc 20
<210> 500
   <211> 20
   <212> DNA
   <213> Artificial Sequence
<220>
   <223> Synthetic Oligonucleotide
<400> 500
   catgttcctc cttgtgcatc 20
<210> 501
   <211> 24
   <212> DNA
   <213> Artificial Sequence
<220>
   <223> Synthetic Oligonucleotide
<400> 501
   caacacttcc ttccccaaag ccag 24
<210> 502
   <211> 21
   <212> DNA
   <213> Artificial Sequence
<220>
   <223> Synthetic Oligonucleotide
<400> 502
   gcatggtagc cgaagatttc a 21
<210> 503
   <211> 30
   <212> DNA
   <213> Artificial Sequence
<220>
   <223> Synthetic Oligonucleotide
<400> 503
   tttccggtaa tagtctgtct catagatatc 30
<210> 504
   <211> 28
   <212> DNA
   <213> Artificial Sequence
<220>
   <223> Synthetic Oligonucleotide
<400> 504
   cgcgtcatac caaaatctcc gattttga 28
<210> 505
   <211> 19
   <212> DNA
   <213> Artificial Sequence
<220>
   <223> Synthetic Oligonucleotide
<400> 505
   gtggacagca ccatgaaca 19
<210> 506
   <211> 20
   <212> DNA
   <213> Artificial Sequence
<220>
   <223> Synthetic Oligonucleotide
<400> 506
   ccttcatacc cgacttgagg 20
<210> 507
   <211> 20
   <212> DNA
   <213> Artificial Sequence
<220>
   <223> Synthetic Oligonucleotide
<400> 507
   cttccggcca gcactgcctc 20
<210> 508
   <211> 17
   <212> DNA
   <213> Artificial Sequence
<220>
   <223> Synthetic Oligonucleotide
<400> 508
   acgcaccggg tgtctga 17
<210> 509
   <211> 24
   <212> DNA
   <213> Artificial Sequence
<220>
   <223> Synthetic Oligonucleotide
<400> 509
   tgccctttct tgatgatgat tatc 24
<210> 510
   <211> 24
   <212> DNA
   <213> Artificial Sequence
<220>
   <223> Synthetic Oligonucleotide
<400> 510
   cccaagttcc accccctcca ttca 24
<210> 511
   <211> 22
   <212> DNA
   <213> Artificial Sequence
<220>
   <223> Synthetic Oligonucleotide
<400> 511
   tggacaagta cgggatgaag ct 22
<210> 512
   <211> 21
   <212> DNA
   <213> Artificial Sequence
<220>
   <223> Synthetic Oligonucleotide
<400> 512
   cgaaggtgtg gcactgaaag t 21
<210> 513
   <211> 24
   <212> DNA
   <213> Artificial Sequence
<220>
   <223> Synthetic Oligonucleotide
<400> 513
   cccgtcaacg tactccatgc ctgg 24
<210> 514
   <211> 19
   <212> DNA
   <213> Artificial Sequence
<220>
   <223> Synthetic Oligonucleotide
<400> 514
   gcggtgattc ggaaattcg 19
<210> 515
   <211> 19
   <212> DNA
   <213> Artificial Sequence
<220>
   <223> Synthetic Oligonucleotide
<400> 515
   ctctcctggg cacctgctt 19
<210> 516
   <211> 23
   <212> DNA
   <213> Artificial Sequence
<220>
   <223> Synthetic Oligonucleotide
<400> 516
   ctctggtcct catccaggtg cgc 23
<210> 517
   <211> 26
   <212> DNA
   <213> Artificial Sequence
<220>
   <223> Synthetic Oligonucleotide
<400> 517
   aaggaaccat ctcactgtgt gtaaac 26
<210> 518
   <211> 20
   <212> DNA
   <213> Artificial Sequence
<220>
   <223> Synthetic Oligonucleotide
<400> 518
   atcaggaagg ctgccaagag 20
<210> 519
   <211> 22
   <212> DNA
   <213> Artificial Sequence
<220>
   <223> Synthetic Oligonucleotide
<400> 519
   tgacttccaa gctggccgtg gc 22
<210> 520
   <211> 20
   <212> DNA
   <213> Artificial Sequence
<220>
   <223> Synthetic Oligonucleotide
<400> 520
   tctgcgtggt tcctttctca 20
<210> 521
   <211> 25
   <212> DNA
   <213> Artificial Sequence
<220>
   <223> Synthetic Oligonucleotide
<400> 521
   ttgaaggatg tttattaggc aacgt 25
<210> 522
   <211> 26
   <212> DNA
   <213> Artificial Sequence
<220>
   <223> Synthetic Oligonucleotide
<400> 522
   cgcttctgac tgctgattct cccgtt 26
<210> 523
   <211> 20
   <212> DNA
   <213> Artificial Sequence
<220>
   <223> Synthetic Oligonucleotide
<400> 523
   cctgaacctt ccaaagatgg 20
<210> 524
   <211> 20
   <212> DNA
   <213> Artificial Sequence
<220>
   <223> Synthetic Oligonucleotide
<400> 524
   accaggcaag tctcctcatt 20
<210> 525
   <211> 27
   <212> DNA
   <213> Artificial Sequence
<220>
   <223> Synthetic Oligonucleotide
<400> 525
   ccagattgga agcatccatc tttttca 27
<210> 526
   <211> 20
   <212> DNA
   <213> Artificial Sequence
<220>
   <223> Synthetic Oligonucleotide
<400> 526
   ccgctccgtc actgatgtct 20
<210> 527
   <211> 21
   <212> DNA
   <213> Artificial Sequence
<220>
   <223> Synthetic Oligonucleotide
<400> 527
   gcaaggtcag ggcaaagagt a 21
<210> 528
   <211> 24
   <212> DNA
   <213> Artificial Sequence
<220>
   <223> Synthetic Oligonucleotide
<400> 528
   cctgctgaac ctagccttgg ccga 24
<210> 529
   <211> 20
   <212> DNA
   <213> Artificial Sequence
<220>
   <223> Synthetic Oligonucleotide
<400> 529
   ctcaggattt tctcgcatcc 20
<210> 530
   <211> 19
   <212> DNA
   <213> Artificial Sequence
<220>
   <223> Synthetic Oligonucleotide
<400> 530
   aggagcaggt ggagactgg 19
<210> 531
   <211> 24
   <212> DNA
   <213> Artificial Sequence
<220>
   <223> Synthetic Oligonucleotide
<400> 531
   atgtgctccc agtgctaggt gcct 24
<210> 532
   <211> 20
   <212> DNA
   <213> Artificial Sequence
<220>
   <223> Synthetic Oligonucleotide
<400> 532
   agccatcact ctcagtgcag 20
<210> 533
   <211> 20
   <212> DNA
   <213> Artificial Sequence
<220>
   <223> Synthetic Oligonucleotide
<400> 533
   actgcagagt cagggtctcc 20
<210> 534
   <211> 22
   <212> DNA
   <213> Artificial Sequence
<220>
   <223> Synthetic Oligonucleotide
<400> 534
   caggtcctat cgtggcccct ga 22
<210> 535
   <211> 21
   <212> DNA
   <213> Artificial Sequence
<220>
   <223> Synthetic Oligonucleotide
<400> 535
   gccaggatac tggagtccat c 21
<210> 536
   <211> 18
   <212> DNA
   <213> Artificial Sequence
<220>
   <223> Synthetic Oligonucleotide
<400> 536
   cttgaccctt ggggtcct 18
<210> 537
   <211> 23
   <212> DNA
   <213> Artificial Sequence
<220>
   <223> Synthetic Oligonucleotide
<400> 537
   tgagctccct gatggctaca ccc 23
<210> 538
   <211> 18
   <212> DNA
   <213> Artificial Sequence
<220>
   <223> Synthetic Oligonucleotide
<400> 538
   ggatggagtt cgcctcct 18
<210> 539
   <211> 20
   <212> DNA
   <213> Artificial Sequence
<220>
   <223> Synthetic Oligonucleotide
<400> 539
   cgctccatgg acttgatctt 20
<210> 540
   <211> 24
   <212> DNA
   <213> Artificial Sequence
<220>
   <223> Synthetic Oligonucleotide
<400> 540
   cgtgatcaca gacctgaccc agct 24
<210> 541
   <211> 20
   <212> DNA
   <213> Artificial Sequence
<220>
   <223> Synthetic Oligonucleotide
<400> 541
   ccacagctca ccttctgtca 20
<210> 542
   <211> 20
   <212> DNA
   <213> Artificial Sequence
<220>
   <223> Synthetic Oligonucleotide
<400> 542
   cctcagtgcc agtctcttcc 20
<210> 543
   <211> 20
   <212> DNA
   <213> Artificial Sequence
<220>
   <223> Synthetic Oligonucleotide
<400> 543
   tccatcccag ctccagccag 20
<210> 544
   <211> 20
   <212> DNA
   <213> Artificial Sequence
<220>
   <223> Synthetic Oligonucleotide
<400> 544
   tcagaattgg atttggctca 20
<210> 545
   <211> 20
   <212> DNA
   <213> Artificial Sequence
<220>
   <223> Synthetic Oligonucleotide
<400> 545
   cctgagctta gctggtgttg 20
<210> 546
   <211> 30
   <212> DNA
   <213> Artificial Sequence
<220>
   <223> Synthetic Oligonucleotide
<400> 546
   tgctaatgta aggcatcaca gtcttttcca 30
<210> 547
   <211> 26
   <212> DNA
   <213> Artificial Sequence
<220>
   <223> Synthetic Oligonucleotide
<400> 547
   tgaggaagaa ggagaggaat actaat 26
<210> 548
   <211> 21
   <212> DNA
   <213> Artificial Sequence
<220>
   <223> Synthetic Oligonucleotide
<400> 548
   ctgaaattct gggagcatga c 21
<210> 549
   <211> 24
   <212> DNA
   <213> Artificial Sequence
<220>
   <223> Synthetic Oligonucleotide
<400> 549
   tatccattcc ttttggccct gcag 24
<210> 550
   <211> 20
   <212> DNA
   <213> Artificial Sequence
<220>
   <223> Synthetic Oligonucleotide
<400> 550
   gaggacgaag gcctctacac 20
<210> 551
   <211> 20
   <212> DNA
   <213> Artificial Sequence
<220>
   <223> Synthetic Oligonucleotide
<400> 551
   aaaaatgcct ccacttttgc 20
<210> 552
   <211> 24
   <212> DNA
   <213> Artificial Sequence
<220>
   <223> Synthetic Oligonucleotide
<400> 552
   caggcatgca gtgttcttgg ctgt 24
<210> 553
   <211> 19
   <212> DNA
   <213> Artificial Sequence
<220>
   <223> Synthetic Oligonucleotide
<400> 553
   cggactttgg gtgcgactt 19
<210> 554
   <211> 24
   <212> DNA
   <213> Artificial Sequence
<220>
   <223> Synthetic Oligonucleotide
<400> 554
   ttacaactct tccactggga cgat 24
<210> 555
   <211> 23
   <212> DNA
   <213> Artificial Sequence
<220>
   <223> Synthetic Oligonucleotide
<400> 555
   ccacttgtcg aaccaccgct cgt 23
<210> 556
   <211> 20
   <212> DNA
   <213> Artificial Sequence
<220>
   <223> Synthetic Oligonucleotide
<400> 556
   tggaggttgt aagccaatgt 20
<210> 557
   <211> 20
   <212> DNA
   <213> Artificial Sequence
<220>
   <223> Synthetic Oligonucleotide
<400> 557
   tgccttacgt ccatctgatt 20
<210> 558
   <211> 28
   <212> DNA
   <213> Artificial Sequence
<220>
   <223> Synthetic Oligonucleotide
<400> 558
   cagtgatgtc tgaacttgaa gcctcaca 28
<210> 559
   <211> 20
   <212> DNA
   <213> Artificial Sequence
<220>
   <223> Synthetic Oligonucleotide
<400> 559
   ctaaggcact tgctggaagg 20
<210> 560
   <211> 18
   <212> DNA
   <213> Artificial Sequence
<220>
   <223> Synthetic Oligonucleotide
<400> 560
   tcttcccagc tcctgtgg 18
<210> 561
   <211> 24
   <212> DNA
   <213> Artificial Sequence
<220>
   <223> Synthetic Oligonucleotide
<400> 561
   tccataggca agcacactgg catt 24
<210> 562
   <211> 25
   <212> DNA
   <213> Artificial Sequence
<220>
   <223> Synthetic Oligonucleotide
<400> 562
   aattcctgct ccaaaagaaa gtctt 25
<210> 563
   <211> 20
   <212> DNA
   <213> Artificial Sequence
<220>
   <223> Synthetic Oligonucleotide
<400> 563
   cgtgatgcga agctctgaga 20
<210> 564
   <211> 22
   <212> DNA
   <213> Artificial Sequence
<220>
   <223> Synthetic Oligonucleotide
<400> 564
   aagccgctcc actcgcatgt cc 22
<210> 565
   <211> 20
   <212> DNA
   <213> Artificial Sequence
<220>
   <223> Synthetic Oligonucleotide
<400> 565
   ccacagggtt gaagaaccag 20
<210> 566
   <211> 20
   <212> DNA
   <213> Artificial Sequence
<220>
   <223> Synthetic Oligonucleotide
<400> 566
   cacctgatgt gccagacttc 20
<210> 567
   <211> 24
   <212> DNA
   <213> Artificial Sequence
<220>
   <223> Synthetic Oligonucleotide
<400> 567
   agccagttcc tgctgttcct gtcc 24
<210> 568
   <211> 18
   <212> DNA
   <213> Artificial Sequence
<220>
   <223> Synthetic Oligonucleotide
<400> 568
   gcccagaggc tccatcgt 18
<210> 569
   <211> 23
   <212> DNA
   <213> Artificial Sequence
<220>
   <223> Synthetic Oligonucleotide
<400> 569
   cagaggtttg aacagtgcag aca 23
<210> 570
   <211> 23
   <212> DNA
   <213> Artificial Sequence
<220>
   <223> Synthetic Oligonucleotide
<400> 570
   cctcttcctc cccagtcggc tga 23
<210> 571
   <211> 20
   <212> DNA
   <213> Artificial Sequence
<220>
   <223> Synthetic Oligonucleotide
<400> 571
   caaacagagg gtggcagaag 20
<210> 572
   <211> 18
   <212> DNA
   <213> Artificial Sequence
<220>
   <223> Synthetic Oligonucleotide
<400> 572
   gaggctctca cggctcct 18
<210> 573
   <211> 24
   <212> DNA
   <213> Artificial Sequence
<220>
   <223> Synthetic Oligonucleotide
<400> 573
   cgcttctcca tgttctcagg gtca 24
<210> 574
   <211> 18
   <212> DNA
   <213> Artificial Sequence
<220>
   <223> Synthetic Oligonucleotide
<400> 574
   agccgaggct ttgttgaa 18
<210> 575
   <211> 19
   <212> DNA
   <213> Artificial Sequence
<220>
   <223> Synthetic Oligonucleotide
<400> 575
   tgggctatga gcacagctt 19
<210> 576
   <211> 24
   <212> DNA
   <213> Artificial Sequence
<220>
   <223> Synthetic Oligonucleotide
<400> 576
   ttcatatcca gtaccggcga tcgg 24
<210> 577
   <211> 20
   <212> DNA
   <213> Artificial Sequence
<220>
   <223> Synthetic Oligonucleotide
<400> 577
   atgtgccagt gagcttgagt 20
<210> 578
   <211> 20
   <212> DNA
   <213> Artificial Sequence
<220>
   <223> Synthetic Oligonucleotide
<400> 578
   tgagcccctg gttaacagta 20
<210> 579
   <211> 24
   <212> DNA
   <213> Artificial Sequence
<220>
   <223> Synthetic Oligonucleotide
<400> 579
   ccttggagaa acacaagcac ctgc 24
<210> 580
   <211> 20
   <212> DNA
   <213> Artificial Sequence
<220>
   <223> Synthetic Oligonucleotide
<400> 580
   ggcctgctga gatcaaagac 20
<210> 581
   <211> 20
   <212> DNA
   <213> Artificial Sequence
<220>
   <223> Synthetic Oligonucleotide
<400> 581
   gtccactgtg gctgtgagaa 20
<210> 582
   <211> 26
   <212> DNA
   <213> Artificial Sequence
<220>
   <223> Synthetic Oligonucleotide
<400> 582
   tgttcctcag gtcctcaatg gtcttg 26
<210> 583
   <211> 21
   <212> DNA
   <213> Artificial Sequence
<220>
   <223> Synthetic Oligonucleotide
<400> 583
   cgaggattgg ttcttcagca a 21
<210> 584
   <211> 24
   <212> DNA
   <213> Artificial Sequence
<220>
   <223> Synthetic Oligonucleotide
<400> 584
   cacctcgcgg ttcagttcct ctgt 24
<210> 585
   <211> 22
   <212> DNA
   <213> Artificial Sequence
<220>
   <223> Synthetic Oligonucleotide
<400> 585
   actctgcacc agctcactgt tg 22
<210> 586
   <211> 21
   <212> DNA
   <213> Artificial Sequence
<220>
   <223> Synthetic Oligonucleotide
<400> 586
   tgagcggcag aatcaggagt a 21
<210> 587
   <211> 19
   <212> DNA
   <213> Artificial Sequence
<220>
   <223> Synthetic Oligonucleotide
<400> 587
   tgcggtaggt ggcaatctc 19
<210> 588
   <211> 24
   <212> DNA
   <213> Artificial Sequence
<220>
   <223> Synthetic Oligonucleotide
<400> 588
   ctcatggaca tcaagtcgcg gctg 24
<210> 589
   <211> 20
   <212> DNA
   <213> Artificial Sequence
<220>
   <223> Synthetic Oligonucleotide
<400> 589
   tcagtggaga aggagttgga 20
<210> 590
   <211> 20
   <212> DNA
   <213> Artificial Sequence
<220>
   <223> Synthetic Oligonucleotide
<400> 590
   tgccatatcc agaggaaaca 20
<210> 591
   <211> 28
   <212> DNA
   <213> Artificial Sequence
<220>
   <223> Synthetic Oligonucleotide
<400> 591
   ccagtcaaca tctctgttgt cacaagca 28
<210> 592
   <211> 18
   <212> DNA
   <213> Artificial Sequence
<220>
   <223> Synthetic Oligonucleotide
<400> 592
   cttgctggcc aatgccta 18
<210> 593
   <211> 18
   <212> DNA
   <213> Artificial Sequence
<220>
   <223> Synthetic Oligonucleotide
<400> 593
   tgattgtccg cagtcagg 18
<210> 594
   <211> 24
   <212> DNA
   <213> Artificial Sequence
<220>
   <223> Synthetic Oligonucleotide
<400> 594
   atctacgttg tccagctgcc agcc 24
<210> 595
   <211> 21
   <212> DNA
   <213> Artificial Sequence
<220>
   <223> Synthetic Oligonucleotide
<400> 595
   actcaagcgg aaattgaagc a 21
<210> 596
   <211> 21
   <212> DNA
   <213> Artificial Sequence
<220>
   <223> Synthetic Oligonucleotide
<400> 596
   actccctgaa gccgagacac t 21
<210> 597
   <211> 28
   <212> DNA
   <213> Artificial Sequence
<220>
   <223> Synthetic Oligonucleotide
<400> 597
   aggtcttatc agcacagtct ccgcctcc 28
<210> 598
   <211> 18
   <212> DNA
   <213> Artificial Sequence
<220>
   <223> Synthetic Oligonucleotide
<400> 598
   agcgatgaag atggtcgc 18
<210> 599
   <211> 18
   <212> DNA
   <213> Artificial Sequence
<220>
   <223> Synthetic Oligonucleotide
<400> 599
   gacatggcag cacaagca 18
<210> 600
   <211> 24
   <212> DNA
   <213> Artificial Sequence
<220>
   <223> Synthetic Oligonucleotide
<400> 600
   ctggacgcgg ttctactcca acag 24
<210> 601
   <211> 20
   <212> DNA
   <213> Artificial Sequence
<220>
   <223> Synthetic Oligonucleotide
<400> 601
   gcttcaggtg ttgtgactgc 20
<210> 602
   <211> 20
   <212> DNA
   <213> Artificial Sequence
<220>
   <223> Synthetic Oligonucleotide
<400> 602
   aagagctgcc catccttctc 20
<210> 603
   <211> 24
   <212> DNA
   <213> Artificial Sequence
<220>
   <223> Synthetic Oligonucleotide
<400> 603
   tgcctccctg tcgcaccagt acta 24
<210> 604
   <211> 18
   <212> DNA
   <213> Artificial Sequence
<220>
   <223> Synthetic Oligonucleotide
<400> 604
   ctttgggcca ggaggaat 18
<210> 605
   <211> 18
   <212> DNA
   <213> Artificial Sequence
<220>
   <223> Synthetic Oligonucleotide
<400> 605
   ctcccacaat ccactgcc 18
<210> 606
   <211> 24
   <212> DNA
   <213> Artificial Sequence
<220>
   <223> Synthetic Oligonucleotide
<400> 606
   actcgaatcc acccaggaac tccc 24
<210> 607
   <211> 20
   <212> DNA
   <213> Artificial Sequence
<220>
   <223> Synthetic Oligonucleotide
<400> 607
   agaagctgtc cctgcaagag 20
<210> 608
   <211> 20
   <212> DNA
   <213> Artificial Sequence
<220>
   <223> Synthetic Oligonucleotide
<400> 608
   agccgtacca gctcagactt 20
<210> 609
   <211> 25
   <212> DNA
   <213> Artificial Sequence
<220>
   <223> Synthetic Oligonucleotide
<400> 609
   catgttcttc acaatcgctg catcc 25
<210> 610
   <211> 18
   <212> DNA
   <213> Artificial Sequence
<220>
   <223> Synthetic Oligonucleotide
<400> 610
   ccgggagcag ggaatcac 18
<210> 611
   <211> 20
   <212> DNA
   <213> Artificial Sequence
<220>
   <223> Synthetic Oligonucleotide
<400> 611
   atgctgttga tgccgaatga 20
<210> 612
   <211> 19
   <212> DNA
   <213> Artificial Sequence
<220>
   <223> Synthetic Oligonucleotide
<400> 612
   cggccacgat ttcggcgct 19
<210> 613
   <211> 20
   <212> DNA
   <213> Artificial Sequence
<220>
   <223> Synthetic Oligonucleotide
<400> 613
   ctgtcatgtg gcagaccttc 20
<210> 614
   <211> 20
   <212> DNA
   <213> Artificial Sequence
<220>
   <223> Synthetic Oligonucleotide
<400> 614
   taaatgtcac tggtgcctgg 20
<210> 615
   <211> 24
   <212> DNA
   <213> Artificial Sequence
<220>
   <223> Synthetic Oligonucleotide
<400> 615
   cgaaccacgg cttgggaaga ctac 24
<210> 616
   <211> 19
   <212> DNA
   <213> Artificial Sequence
<220>
   <223> Synthetic Oligonucleotide
<400> 616
   gcccagtgga gaaattcgt 19
<210> 617
   <211> 19
   <212> DNA
   <213> Artificial Sequence
<220>
   <223> Synthetic Oligonucleotide
<400> 617
   gcgagtctga gctgatgga 19
<210> 618
   <211> 24
   <212> DNA
   <213> Artificial Sequence
<220>
   <223> Synthetic Oligonucleotide
<400> 618
   tttgagatca cccagcctcc actg 24
<210> 619
   <211> 20
   <212> DNA
   <213> Artificial Sequence
<220>
   <223> Synthetic Oligonucleotide
<400> 619
   cctcagaaat tgccaggact 20
<210> 620
   <211> 24
   <212> DNA
   <213> Artificial Sequence
<220>
   <223> Synthetic Oligonucleotide
<400> 620
   ttcccgtgat cttcagcaaa gcct 24
<210> 621
   <211> 20
   <212> DNA
   <213> Artificial Sequence
<220>
   <223> Synthetic Oligonucleotide
<400> 621
   ccaaagacca gctgcaagta 20
<210> 622
   <211> 20
   <212> DNA
   <213> Artificial Sequence
<220>
   <223> Synthetic Oligonucleotide
<400> 622
   aggactccag caaccaagaa 20
<210> 623
   <211> 20
   <212> DNA
   <213> Artificial Sequence
<220>
   <223> Synthetic Oligonucleotide
<400> 623
   gagtgctgct tggaactcag 20
<210> 624
   <211> 24
   <212> DNA
   <213> Artificial Sequence
<220>
   <223> Synthetic Oligonucleotide
<400> 624
   caagcacctt ccctgacctg gagt 24
<210> 625
   <211> 18
   <212> DNA
   <213> Artificial Sequence
<220>
   <223> Synthetic Oligonucleotide
<400> 625
   cggaccacca ggtcagag 18
<210> 626
   <211> 20
   <212> DNA
   <213> Artificial Sequence
<220>
   <223> Synthetic Oligonucleotide
<400> 626
   caggggtagt gggtgttgag 20
<210> 627
   <211> 24
   <212> DNA
   <213> Artificial Sequence
<220>
   <223> Synthetic Oligonucleotide
<400> 627
   ccactcttcc ctgctctgcg aatt 24
<210> 628
   <211> 20
   <212> DNA
   <213> Artificial Sequence
<220>
   <223> Synthetic Oligonucleotide
<400> 628
   gccctccaat gtccttatca 20
<210> 629
   <211> 21
   <212> DNA
   <213> Artificial Sequence
<220>
   <223> Synthetic Oligonucleotide
<400> 629
   gtagccactg atgccaaagt c 21
<210> 630
   <211> 24
   <212> DNA
   <213> Artificial Sequence
<220>
   <223> Synthetic Oligonucleotide
<400> 630
   cacatcttca catggccctc cttg 24
<210> 631
   <211> 18
   <212> DNA
   <213> Artificial Sequence
<220>
   <223> Synthetic Oligonucleotide
<400> 631
   gccggtcagg cacacaag 18
<210> 632
   <211> 23
   <212> DNA
   <213> Artificial Sequence
<220>
   <223> Synthetic Oligonucleotide
<400> 632
   gcagcataca cacaacaaaa tgg 23
<210> 633
   <211> 24
   <212> DNA
   <213> Artificial Sequence
<220>
   <223> Synthetic Oligonucleotide
<400> 633
   accaaccagt ccacgctcca aggg 24
<210> 634
   <211> 18
   <212> DNA
   <213> Artificial Sequence
<220>
   <223> Synthetic Oligonucleotide
<400> 634
   ccctcaaccg gcaaatcc 18
<210> 635
   <211> 19
   <212> DNA
   <213> Artificial Sequence
<220>
   <223> Synthetic Oligonucleotide
<400> 635
   cgtccatgcc ctgaattca 19
<210> 636
   <211> 23
   <212> DNA
   <213> Artificial Sequence
<220>
   <223> Synthetic Oligonucleotide
<400> 636
   tggcgagtgc agtgagcagc tcc 23
<210> 637
   <211> 23
   <212> DNA
   <213> Artificial Sequence
<220>
   <223> Synthetic Oligonucleotide
<400> 637
   tgagtggaaa agcctgacct atg 23
<210> 638
   <211> 23
   <212> DNA
   <213> Artificial Sequence
<220>
   <223> Synthetic Oligonucleotide
<400> 638
   ggactccatc tcttcttggt caa 23
<210> 639
   <211> 27
   <212> DNA
   <213> Artificial Sequence
<220>
   <223> Synthetic Oligonucleotide
<400> 639
   tgaagtcatc agctttgtgc caccacc 27
<210> 640
   <211> 22
   <212> DNA
   <213> Artificial Sequence
<220>
   <223> Synthetic Oligonucleotide
<400> 640
   caacacccgt acatcaatgt ct 22
<210> 641
   <211> 22
   <212> DNA
   <213> Artificial Sequence
<220>
   <223> Synthetic Oligonucleotide
<400> 641
   tcatctaact gcttgtcagg ga 22
<210> 642
   <211> 24
   <212> DNA
   <213> Artificial Sequence
<220>
   <223> Synthetic Oligonucleotide
<400> 642
   ctgaagcaga agctccacca ccaa 24
<210> 643
   <211> 20
   <212> DNA
   <213> Artificial Sequence
<220>
   <223> Synthetic Oligonucleotide
<400> 643
   gaccttggaa cctttggaac 20
<210> 644
   <211> 20
   <212> DNA
   <213> Artificial Sequence
<220>
   <223> Synthetic Oligonucleotide
<400> 644
   cctaggccta tgagggttca 20
<210> 645
   <211> 26
   <212> DNA
   <213> Artificial Sequence
<220>
   <223> Synthetic Oligonucleotide
<400> 645
   cagccctgta agacctgttg acagca 26
<210> 646
   <211> 18
   <212> DNA
   <213> Artificial Sequence
<220>
   <223> Synthetic Oligonucleotide
<400> 646
   cacaagctga ccttccgc 18
<210> 647
   <211> 20
   <212> DNA
   <213> Artificial Sequence
<220>
   <223> Synthetic Oligonucleotide
<400> 647
   acttgtacac gatctccgcc 20
<210> 648
   <211> 24
   <212> DNA
   <213> Artificial Sequence
<220>
   <223> Synthetic Oligonucleotide
<400> 648
   agaacgccaa agccaagaca gacc 24
<210> 649
   <211> 23
   <212> DNA
   <213> Artificial Sequence
<220>
   <223> Synthetic Oligonucleotide
<400> 649
   cagatggcca ctttgagaac att 23
<210> 650
   <211> 22
   <212> DNA
   <213> Artificial Sequence
<220>
   <223> Synthetic Oligonucleotide
<400> 650
   ggcagcatta accacaagga tt 22
<210> 651
   <211> 28
   <212> DNA
   <213> Artificial Sequence
<220>
   <223> Synthetic Oligonucleotide
<400> 651
   agctgacaac agtgtgaacg accagacc 28
<210> 652
   <211> 20
   <212> DNA
   <213> Artificial Sequence
<220>
   <223> Synthetic Oligonucleotide
<400> 652
   cttcggaaac tggacatcaa 20
<210> 653
   <211> 20
   <212> DNA
   <213> Artificial Sequence
<220>
   <223> Synthetic Oligonucleotide
<400> 653
   gtcgctgaaa acatggatca 20
<210> 654
   <211> 26
   <212> DNA
   <213> Artificial Sequence
<220>
   <223> Synthetic Oligonucleotide
<400> 654
   tcactcgaga caacgatttc acatcg 26
<210> 655
   <211> 21
   <212> DNA
   <213> Artificial Sequence
<220>
   <223> Synthetic Oligonucleotide
<400> 655
   gacttttgcc cgctaccttt c 21
<210> 656
   <211> 26
   <212> DNA
   <213> Artificial Sequence
<220>
   <223> Synthetic Oligonucleotide
<400> 656
   gccactaact gcttcagtat gaagag 26
<210> 657
   <211> 24
   <212> DNA
   <213> Artificial Sequence
<220>
   <223> Synthetic Oligonucleotide
<400> 657
   acagctcatt gttgtcacgc cgga 24
<210> 658
   <211> 24
   <212> DNA
   <213> Artificial Sequence
<220>
   <223> Synthetic Oligonucleotide
<400> 658
   tgatggtcct atgtgtcaca ttca 24
<210> 659
   <211> 20
   <212> DNA
   <213> Artificial Sequence
<220>
   <223> Synthetic Oligonucleotide
<400> 659
   tgggacagga aacacaccaa 20
<210> 660
   <211> 30
   <212> DNA
   <213> Artificial Sequence
<220>
   <223> Synthetic Oligonucleotide
<400> 660
   caggtttcat accaacacag gcttcagcac 30
<210> 661
   <211> 19
   <212> DNA
   <213> Artificial Sequence
<220>
   <223> Synthetic Oligonucleotide
<400> 661
   cgctcagcca gatgcaatc 19
<210> 662
   <211> 25
   <212> DNA
   <213> Artificial Sequence
<220>
   <223> Synthetic Oligonucleotide
<400> 662
   gcactgagat cttcctattg gtgaa 25
<210> 663
   <211> 21
   <212> DNA
   <213> Artificial Sequence
<220>
   <223> Synthetic Oligonucleotide
<400> 663
   tgccccagtc acctgctgtt a 21
<210> 664
   <211> 20
   <212> DNA
   <213> Artificial Sequence
<220>
   <223> Synthetic Oligonucleotide
<400> 664
   gtgaaatgaa acgcaccaca 20
<210> 665
   <211> 20
   <212> DNA
   <213> Artificial Sequence
<220>
   <223> Synthetic Oligonucleotide
<400> 665
   gaccctgctc acaaccagac 20
<210> 666
   <211> 20
   <212> DNA
   <213> Artificial Sequence
<220>
   <223> Synthetic Oligonucleotide
<400> 666
   cagccctttg gggaagctgg 20
<210> 667
   <211> 19
   <212> DNA
   <213> Artificial Sequence
<220>
   <223> Synthetic Oligonucleotide
<400> 667
   ccaacgcttg ccaaatcct 19
<210> 668
   <211> 24
   <212> DNA
   <213> Artificial Sequence
<220>
   <223> Synthetic Oligonucleotide
<400> 668
   acggtagtga cagcatcaaa actc 24
<210> 669
   <211> 24
   <212> DNA
   <213> Artificial Sequence
<220>
   <223> Synthetic Oligonucleotide
<400> 669
   aaccagctct ctgtgacccc aatt 24
<210> 670
   <211> 20
   <212> DNA
   <213> Artificial Sequence
<220>
   <223> Synthetic Oligonucleotide
<400> 670
   ccatgatgga gaggcagaca 20
<210> 671
   <211> 21
   <212> DNA
   <213> Artificial Sequence
<220>
   <223> Synthetic Oligonucleotide
<400> 671
   ggagtccgtc cttaccgtca a 21
<210> 672
   <211> 24
   <212> DNA
   <213> Artificial Sequence
<220>
   <223> Synthetic Oligonucleotide
<400> 672
   ctgggagcat ggcgatggat accc 24
<210> 673
   <211> 20
   <212> DNA
   <213> Artificial Sequence
<220>
   <223> Synthetic Oligonucleotide
<400> 673
   gagaaccaat ctcaccgaca 20
<210> 674
   <211> 20
   <212> DNA
   <213> Artificial Sequence
<220>
   <223> Synthetic Oligonucleotide
<400> 674
   cacccgagtg taaccatagc 20
<210> 675
   <211> 24
   <212> DNA
   <213> Artificial Sequence
<220>
   <223> Synthetic Oligonucleotide
<400> 675
   acaggtattc ctctgccagc tgcc 24
<210> 676
   <211> 18
   <212> DNA
   <213> Artificial Sequence
<220>
   <223> Synthetic Oligonucleotide
<400> 676
   gccatgctgg ctcagtct 18
<210> 677
   <211> 20
   <212> DNA
   <213> Artificial Sequence
<220>
   <223> Synthetic Oligonucleotide
<400> 677
   cacccagacc cacctaactg 20
<210> 678
   <211> 24
   <212> DNA
   <213> Artificial Sequence
<220>
   <223> Synthetic Oligonucleotide
<400> 678
   cactagctga tgtggcccac agct 24
<210> 679
   <211> 18
   <212> DNA
   <213> Artificial Sequence
<220>
   <223> Synthetic Oligonucleotide
<400> 679
   tcatggtgcc cgtcaatg 18
<210> 680
   <211> 23
   <212> DNA
   <213> Artificial Sequence
<220>
   <223> Synthetic Oligonucleotide
<400> 680
   cgattgtctt tgctcttcat gtg 23
<210> 681
   <211> 29
   <212> DNA
   <213> Artificial Sequence
<220>
   <223> Synthetic Oligonucleotide
<400> 681
   acctgatacg tcttggtctt catcgccat 29
<210> 682
   <211> 20
   <212> DNA
   <213> Artificial Sequence
<220>
   <223> Synthetic Oligonucleotide
<400> 682
   aggggatgac ttggacacat 20
<210> 683
   <211> 20
   <212> DNA
   <213> Artificial Sequence
<220>
   <223> Synthetic Oligonucleotide
<400> 683
   aaaactgcat ggctttgtca 20
<210> 684
   <211> 25
   <212> DNA
   <213> Artificial Sequence
<220>
   <223> Synthetic Oligonucleotide
<400> 684
   ctgccattcg acatgactgc aattt 25
<210> 685
   <211> 22
   <212> DNA
   <213> Artificial Sequence
<220>
   <223> Synthetic Oligonucleotide
<400> 685
   tcatcctggc gatctacttc ct 22
<210> 686
   <211> 20
   <212> DNA
   <213> Artificial Sequence
<220>
   <223> Synthetic Oligonucleotide
<400> 686
   ccgttgagtg gaatcagcaa 20
<210> 687
   <211> 26
   <212> DNA
   <213> Artificial Sequence
<220>
   <223> Synthetic Oligonucleotide
<400> 687
   tctgtcctgg ctggagtcgc tttcat 26
<210> 688
   <211> 21
   <212> DNA
   <213> Artificial Sequence
<220>
   <223> Synthetic Oligonucleotide
<400> 688
   tgattaccat catggctcag a 21
<210> 689
   <211> 20
   <212> DNA
   <213> Artificial Sequence
<220>
   <223> Synthetic Oligonucleotide
<400> 689
   cttgtgaaaa tgccatccac 20
<210> 690
   <211> 24
   <212> DNA
   <213> Artificial Sequence
<220>
   <223> Synthetic Oligonucleotide
<400> 690
   tcccaattgt cgcttcttct gcag 24
<210> 691
   <211> 20
   <212> DNA
   <213> Artificial Sequence
<220>
   <223> Synthetic Oligonucleotide
<400> 691
   ggccaggatc tgtggtggta 20
<210> 692
   <211> 20
   <212> DNA
   <213> Artificial Sequence
<220>
   <223> Synthetic Oligonucleotide
<400> 692
   ctccacgtcg tggacattga 20
<210> 693
   <211> 23
   <212> DNA
   <213> Artificial Sequence
<220>
   <223> Synthetic Oligonucleotide
<400> 693
   ctctggcctt ccgagaaggt acc 23
<210> 694
   <211> 23
   <212> DNA
   <213> Artificial Sequence
<220>
   <223> Synthetic Oligonucleotide
<400> 694
   gaaggaatgg gaatcagtca tga 23
<210> 695
   <211> 26
   <212> DNA
   <213> Artificial Sequence
<220>
   <223> Synthetic Oligonucleotide
<400> 695
   gtctattaga gtcagatccg ggacat 26
<210> 696
   <211> 23
   <212> DNA
   <213> Artificial Sequence
<220>
   <223> Synthetic Oligonucleotide
<400> 696
   tcaccctgga gatcagctcc cga 23
<210> 697
   <211> 18
   <212> DNA
   <213> Artificial Sequence
<220>
   <223> Synthetic Oligonucleotide
<400> 697
   gccgagatcg ccaagatg 18
<210> 698
   <211> 27
   <212> DNA
   <213> Artificial Sequence
<220>
   <223> Synthetic Oligonucleotide
<400> 698
   cttttgatgg tagagttcca gtgattc 27
<210> 699
   <211> 24
   <212> DNA
   <213> Artificial Sequence
<220>
   <223> Synthetic Oligonucleotide
<400> 699
   cagcattgtc tgtcctccct ggca 24
<210> 700
   <211> 27
   <212> DNA
   <213> Artificial Sequence
<220>
   <223> Synthetic Oligonucleotide
<400> 700
   cggtacttct cagggctaat atatacg 27
<210> 701
   <211> 21
   <212> DNA
   <213> Artificial Sequence
<220>
   <223> Synthetic Oligonucleotide
<400> 701
   ccgagtagat gggcaggtgt t 21
<210> 702
   <211> 26
   <212> DNA
   <213> Artificial Sequence
<220>
   <223> Synthetic Oligonucleotide
<400> 702
   ctcttctgcg tggtggtcaa ccccta 26
<210> 703
   <211> 18
   <212> DNA
   <213> Artificial Sequence
<220>
   <223> Synthetic Oligonucleotide
<400> 703
   gtgaggcagc gcgactct 18
<210> 704
   <211> 23
   <212> DNA
   <213> Artificial Sequence
<220>
   <223> Synthetic Oligonucleotide
<400> 704
   tgccaatggt gtacaacact tca 23
<210> 705
   <211> 21
   <212> DNA
   <213> Artificial Sequence
<220>
   <223> Synthetic Oligonucleotide
<400> 705
   tgccttcccg ggctgaggac t 21
<210> 706
   <211> 20
   <212> DNA
   <213> Artificial Sequence
<220>
   <223> Synthetic Oligonucleotide
<400> 706
   cagaccaagg agatggacct 20
<210> 707
   <211> 18
   <212> DNA
   <213> Artificial Sequence
<220>
   <223> Synthetic Oligonucleotide
<400> 707
   agctgccagt gctatccg 18
<210> 708
   <211> 24
   <212> DNA
   <213> Artificial Sequence
<220>
   <223> Synthetic Oligonucleotide
<400> 708
   aagctgtaaa catgagccgc acca 24
<210> 709
   <211> 19
   <212> DNA
   <213> Artificial Sequence
<220>
   <223> Synthetic Oligonucleotide
<400> 709
   ctgccgggat ggcttctat 19
<210> 710
   <211> 22
   <212> DNA
   <213> Artificial Sequence
<220>
   <223> Synthetic Oligonucleotide
<400> 710
   ccaggttctg gaaactgtgg at 22
<210> 711
   <211> 21
   <212> DNA
   <213> Artificial Sequence
<220>
   <223> Synthetic Oligonucleotide
<400> 711
   ctgagctctg cccggaccgc t 21
<210> 712
   <211> 18
   <212> DNA
   <213> Artificial Sequence
<220>
   <223> Synthetic Oligonucleotide
<400> 712
   cactgacacc cgcaacac 18
<210> 713
   <211> 19
   <212> DNA
   <213> Artificial Sequence
<220>
   <223> Synthetic Oligonucleotide
<400> 713
   ggctgcaatc tctctgctg 19
<210> 714
   <211> 24
   <212> DNA
   <213> Artificial Sequence
<220>
   <223> Synthetic Oligonucleotide
<400> 714
   aaccacagag tccgaaccat gggt 24
<210> 715
   <211> 19
   <212> DNA
   <213> Artificial Sequence
<220>
   <223> Synthetic Oligonucleotide
<400> 715
   ctgcttcttt cccgagctt 19
<210> 716
   <211> 19
   <212> DNA
   <213> Artificial Sequence
<220>
   <223> Synthetic Oligonucleotide
<400> 716
   agcaggaatg tagctgcca 19
<210> 717
   <211> 24
   <212> DNA
   <213> Artificial Sequence
<220>
   <223> Synthetic Oligonucleotide
<400> 717
   acttcgttat ccgcgatgcg tttc 24
<210> 718
   <211> 20
   <212> DNA
   <213> Artificial Sequence
<220>
   <223> Synthetic Oligonucleotide
<400> 718
   ggctgtggct gaggctgtag 20
<210> 719
   <211> 21
   <212> DNA
   <213> Artificial Sequence
<220>
   <223> Synthetic Oligonucleotide
<400> 719
   ggagcattcg aggtcaaatc a 21
<210> 720
   <211> 28
   <212> DNA
   <213> Artificial Sequence
<220>
   <223> Synthetic Oligonucleotide
<400> 720
   ttcccagagt gtctcacctc cagcagag 28
<210> 721
   <211> 20
   <212> DNA
   <213> Artificial Sequence
<220>
   <223> Synthetic Oligonucleotide
<400> 721
   cggacctgaa tgtaatgcaa 20
<210> 722
   <211> 20
   <212> DNA
   <213> Artificial Sequence
<220>
   <223> Synthetic Oligonucleotide
<400> 722
   ctttgccagg tgactaagca 20
<210> 723
   <211> 28
   <212> DNA
   <213> Artificial Sequence
<220>
   <223> Synthetic Oligonucleotide
<400> 723
   aatgaacaga acaagcaaaa tgaccagc 28
<210> 724
   <211> 20
   <212> DNA
   <213> Artificial Sequence
<220>
   <223> Synthetic Oligonucleotide
<400> 724
   caaaggaaga gcaacggaag 20
<210> 725
   <211> 19
   <212> DNA
   <213> Artificial Sequence
<220>
   <223> Synthetic Oligonucleotide
<400> 725
   attcccaaaa cctttgctt 19
<210> 726
   <211> 24
   <212> DNA
   <213> Artificial Sequence
<220>
   <223> Synthetic Oligonucleotide
<400> 726
   ttctcctttc gttcttgctc gcgt 24
<210> 727
   <211> 21
   <212> DNA
   <213> Artificial Sequence
<220>
   <223> Synthetic Oligonucleotide
<400> 727
   tggagactct cagggtcgaa a 21
<210> 728
   <211> 22
   <212> DNA
   <213> Artificial Sequence
<220>
   <223> Synthetic Oligonucleotide
<400> 728
   ggcgtttgga gtggtagaaa tc 22
<210> 729
   <211> 20
   <212> DNA
   <213> Artificial Sequence
<220>
   <223> Synthetic Oligonucleotide
<400> 729
   cggcggcaga ccagcatgac 20
<210> 730
   <211> 21
   <212> DNA
   <213> Artificial Sequence
<220>
   <223> Synthetic Oligonucleotide
<400> 730
   cggtggacca cgaagagtta a 21
<210> 731
   <211> 19
   <212> DNA
   <213> Artificial Sequence
<220>
   <223> Synthetic Oligonucleotide
<400> 731
   ggctcgcctc ttccatgtc 19
<210> 732
   <211> 23
   <212> DNA
   <213> Artificial Sequence
<220>
   <223> Synthetic Oligonucleotide
<400> 732
   ccgggacttg gagaagcact gca 23
<210> 733
   <211> 20
   <212> DNA
   <213> Artificial Sequence
<220>
   <223> Synthetic Oligonucleotide
<400> 733
   tcactaccag ctgacatccg 20
<210> 734
   <211> 20
   <212> DNA
   <213> Artificial Sequence
<220>
   <223> Synthetic Oligonucleotide
<400> 734
   agatggggct attgagggtc 20
<210> 735
   <211> 24
   <212> DNA
   <213> Artificial Sequence
<220>
   <223> Synthetic Oligonucleotide
<400> 735
   cttctccagg tagccctcag gcag 24
<210> 736
   <211> 20
   <212> DNA
   <213> Artificial Sequence
<220>
   <223> Synthetic Oligonucleotide
<400> 736
   ccagctctcc ttccagctac 20
<210> 737
   <211> 20
   <212> DNA
   <213> Artificial Sequence
<220>
   <223> Synthetic Oligonucleotide
<400> 737
   gggtggctct cacttagctc 20
<210> 738
   <211> 24
   <212> DNA
   <213> Artificial Sequence
<220>
   <223> Synthetic Oligonucleotide
<400> 738
   atcaatgtcc ctgtccgagt gctg 24
<210> 739
   <211> 19
   <212> DNA
   <213> Artificial Sequence
<220>
   <223> Synthetic Oligonucleotide
<400> 739
   gagaagcacg ccatgaaac 19
<210> 740
   <211> 20
   <212> DNA
   <213> Artificial Sequence
<220>
   <223> Synthetic Oligonucleotide
<400> 740
   ggctgtgagc tgctgaatct 20
<210> 741
   <211> 24
   <212> DNA
   <213> Artificial Sequence
<220>
   <223> Synthetic Oligonucleotide
<400> 741
   tgactcatca tttccatgac ggcc 24
<210> 742
   <211> 24
   <212> DNA
   <213> Artificial Sequence
<220>
   <223> Synthetic Oligonucleotide
<400> 742
   agagccagtt gctgtagaac tcaa 24
<210> 743
   <211> 21
   <212> DNA
   <213> Artificial Sequence
<220>
   <223> Synthetic Oligonucleotide
<400> 743
   ctgggcctac acagtccttc a 21
<210> 744
   <211> 27
   <212> DNA
   <213> Artificial Sequence
<220>
   <223> Synthetic Oligonucleotide
<400> 744
   tctctgctgg gcaaggatgt tctgttc 27
<210> 745
   <211> 20
   <212> DNA
   <213> Artificial Sequence
<220>
   <223> Synthetic Oligonucleotide
<400> 745
   gacattgtgg taggggaagg 20
<210> 746
   <211> 20
   <212> DNA
   <213> Artificial Sequence
<220>
   <223> Synthetic Oligonucleotide
<400> 746
   cggcagtcaa agataattgg 20
<210> 747
   <211> 26
   <212> DNA
   <213> Artificial Sequence
<220>
   <223> Synthetic Oligonucleotide
<400> 747
   tcattttctc atcccgtggg tacaga 26
<210> 748
   <211> 23
   <212> DNA
   <213> Artificial Sequence
<220>
   <223> Synthetic Oligonucleotide
<400> 748
   cacactagca ttttctccgc ata 23
<210> 749
   <211> 21
   <212> DNA
   <213> Artificial Sequence
<220>
   <223> Synthetic Oligonucleotide
<400> 749
   ataccaatca ccgcacaaac c 21
<210> 750
   <211> 30
   <212> DNA
   <213> Artificial Sequence
<220>
   <223> Synthetic Oligonucleotide
<400> 750
   tgcgctgtga ctggacttaa caaatagcct 30
<210> 751
   <211> 20
   <212> DNA
   <213> Artificial Sequence
<220>
   <223> Synthetic Oligonucleotide
<400> 751
   ctgctcaagg acaccgtcta 20
<210> 752
   <211> 18
   <212> DNA
   <213> Artificial Sequence
<220>
   <223> Synthetic Oligonucleotide
<400> 752
   ggatccactc tggagggc 18
<210> 753
   <211> 24
   <212> DNA
   <213> Artificial Sequence
<220>
   <223> Synthetic Oligonucleotide
<400> 753
   tacactcggg tcccatcgaa gtcc 24
<210> 754
   <211> 19
   <212> DNA
   <213> Artificial Sequence
<220>
   <223> Synthetic Oligonucleotide
<400> 754
   tggggacatt ccctttgag 19
<210> 755
   <211> 18
   <212> DNA
   <213> Artificial Sequence
<220>
   <223> Synthetic Oligonucleotide
<400> 755
   gacatgggct gggaagtg 18
<210> 756
   <211> 24
   <212> DNA
   <213> Artificial Sequence
<220>
   <223> Synthetic Oligonucleotide
<400> 756
   cagcttccag aatctcctgg tccc 24
<210> 757
   <211> 20
   <212> DNA
   <213> Artificial Sequence
<220>
   <223> Synthetic Oligonucleotide
<400> 757
   cccatggatg ctcctctgaa 20
<210> 758
   <211> 20
   <212> DNA
   <213> Artificial Sequence
<220>
   <223> Synthetic Oligonucleotide
<400> 758
   ccggtggcta ccagacattg 20
<210> 759
   <211> 22
   <212> DNA
   <213> Artificial Sequence
<220>
   <223> Synthetic Oligonucleotide
<400> 759
   cattgactgc cgaggcccca tg 22
<210> 760
   <211> 18
   <212> DNA
   <213> Artificial Sequence
<220>
   <223> Synthetic Oligonucleotide
<400> 760
   ccaagttctg ggtggtgg 18
<210> 761
   <211> 19
   <212> DNA
   <213> Artificial Sequence
<220>
   <223> Synthetic Oligonucleotide
<400> 761
   gtgaacgcca gtccatgtt 19
<210> 762
   <211> 24
   <212> DNA
   <213> Artificial Sequence
<220>
   <223> Synthetic Oligonucleotide
<400> 762
   ccagacccac ttctacctgg gcag 24
<210> 763
   <211> 26
   <212> DNA
   <213> Artificial Sequence
<220>
   <223> Synthetic Oligonucleotide
<400> 763
   aatgaataca gtattcccaa gcacat 26
<210> 764
   <211> 23
   <212> DNA
   <213> Artificial Sequence
<220>
   <223> Synthetic Oligonucleotide
<400> 764
   tgtctgaagc atcttctgga tga 23
<210> 765
   <211> 18
   <212> DNA
   <213> Artificial Sequence
<220>
   <223> Synthetic Oligonucleotide
<400> 765
   aaccccgtgg ccgcctcc 18
<210> 766
   <211> 21
   <212> DNA
   <213> Artificial Sequence
<220>
   <223> Synthetic Oligonucleotide
<400> 766
   cttacggttt tcgtggagaa g 21
<210> 767
   <211> 20
   <212> DNA
   <213> Artificial Sequence
<220>
   <223> Synthetic Oligonucleotide
<400> 767
   agcagccgtt cttgttgtaa 20
<210> 768
   <211> 29
   <212> DNA
   <213> Artificial Sequence
<220>
   <223> Synthetic Oligonucleotide
<400> 768
   cctcagctat acaacaaatt gaccccaag 29
<210> 769
   <211> 20
   <212> DNA
   <213> Artificial Sequence
<220>
   <223> Synthetic Oligonucleotide
<400> 769
   gatgtggact gccattcaaa 20
<210> 770
   <211> 20
   <212> DNA
   <213> Artificial Sequence
<220>
   <223> Synthetic Oligonucleotide
<400> 770
   tgcgagatta gttggctgag 20
<210> 771
   <211> 28
   <212> DNA
   <213> Artificial Sequence
<220>
   <223> Synthetic Oligonucleotide
<400> 771
   tcgaaattta catccggtat cttcctgg 28
<210> 772
   <211> 19
   <212> DNA
   <213> Artificial Sequence
<220>
   <223> Synthetic Oligonucleotide
<400> 772
   ccacataccg tccagccta 19
<210> 773
   <211> 18
   <212> DNA
   <213> Artificial Sequence
<220>
   <223> Synthetic Oligonucleotide
<400> 773
   gaggtcatgt gcgggagt 18
<210> 774
   <211> 24
   <212> DNA
   <213> Artificial Sequence
<220>
   <223> Synthetic Oligonucleotide
<400> 774
   ccgctcctct tcttcgttct ccag 24
<210> 775
   <211> 20
   <212> DNA
   <213> Artificial Sequence
<220>
   <223> Synthetic Oligonucleotide
<400> 775
   gcaatcatgg aacttgacga 20
<210> 776
   <211> 18
   <212> DNA
   <213> Artificial Sequence
<220>
   <223> Synthetic Oligonucleotide
<400> 776
   atgtggctcg cctctacg 18
<210> 777
   <211> 24
   <212> DNA
   <213> Artificial Sequence
<220>
   <223> Synthetic Oligonucleotide
<400> 777
   tttcttgcag tttgacccag cacc 24
<210> 778
   <211> 20
   <212> DNA
   <213> Artificial Sequence
<220>
   <223> Synthetic Oligonucleotide
<400> 778
   gcatcaggct gtcattatgg 20
<210> 779
   <211> 20
   <212> DNA
   <213> Artificial Sequence
<220>
   <223> Synthetic Oligonucleotide
<400> 779
   agtagttgtg ctgcccttcc 20
<210> 780
   <211> 28
   <212> DNA
   <213> Artificial Sequence
<220>
   <223> Synthetic Oligonucleotide
<400> 780
   tgtccttacc tgtgggagct gtaaggtc 28
<210> 781
   <211> 19
   <212> DNA
   <213> Artificial Sequence
<220>
   <223> Synthetic Oligonucleotide
<400> 781
   aggactggga cccatgaac 19
<210> 782
   <211> 20
   <212> DNA
   <213> Artificial Sequence
<220>
   <223> Synthetic Oligonucleotide
<400> 782
   cccataatcc tgagcaatgg 20
<210> 783
   <211> 24
   <212> DNA
   <213> Artificial Sequence
<220>
   <223> Synthetic Oligonucleotide
<400> 783
   tcctttggta tcagacccga agcg 24
<210> 784
   <211> 20
   <212> DNA
   <213> Artificial Sequence
<220>
   <223> Synthetic Oligonucleotide
<400> 784
   ggtgtccttc gccagatcac 20
<210> 785
   <211> 18
   <212> DNA
   <213> Artificial Sequence
<220>
   <223> Synthetic Oligonucleotide
<400> 785
   cagccgcaga gcctcatc 18
<210> 786
   <211> 26
   <212> DNA
   <213> Artificial Sequence
<220>
   <223> Synthetic Oligonucleotide
<400> 786
   ttaatgattt gcctgtggga cgctcc 26
<210> 787
   <211> 21
   <212> DNA
   <213> Artificial Sequence
<220>
   <223> Synthetic Oligonucleotide
<400> 787
   caagcaatgc gtcatcaatg t 21
<210> 788
   <211> 20
   <212> DNA
   <213> Artificial Sequence
<220>
   <223> Synthetic Oligonucleotide
<400> 788
   gtaaatccgc cccctcttct 20
<210> 789
   <211> 25
   <212> DNA
   <213> Artificial Sequence
<220>
   <223> Synthetic Oligonucleotide
<400> 789
   cagcctctgc ggaatggatc acact 25
<210> 790
   <211> 20
   <212> DNA
   <213> Artificial Sequence
<220>
   <223> Synthetic Oligonucleotide
<400> 790
   ctgacacttg ccgcagagaa 20
<210> 791
   <211> 20
   <212> DNA
   <213> Artificial Sequence
<220>
   <223> Synthetic Oligonucleotide
<400> 791
   aggtggtcct tggtctggaa 20
<210> 792
   <211> 26
   <212> DNA
   <213> Artificial Sequence
<220>
   <223> Synthetic Oligonucleotide
<400> 792
   ccctttctca cccacctcat ctgcac 26
<210> 793
   <211> 18
   <212> DNA
   <213> Artificial Sequence
<220>
   <223> Synthetic Oligonucleotide
<400> 793
   gggttctaga cgcccctc 18
<210> 794
   <211> 21
   <212> DNA
   <213> Artificial Sequence
<220>
   <223> Synthetic Oligonucleotide
<400> 794
   ggacggctgt agaggctgta t 21
<210> 795
   <211> 24
   <212> DNA
   <213> Artificial Sequence
<220>
   <223> Synthetic Oligonucleotide
<400> 795
   caagcgttcc tggccttctg aact 24
<210> 796
   <211> 21
   <212> DNA
   <213> Artificial Sequence
<220>
   <223> Synthetic Oligonucleotide
<400> 796
   ctccacctat gagcgtctgt c 21
<210> 797
   <211> 20
   <212> DNA
   <213> Artificial Sequence
<220>
   <223> Synthetic Oligonucleotide
<400> 797
   cacactttcc ctccttttgg 20
<210> 798
   <211> 24
   <212> DNA
   <213> Artificial Sequence
<220>
   <223> Synthetic Oligonucleotide
<400> 798
   tcctgttaac atcccaagcc caca 24
<210> 799
   <211> 19
   <212> DNA
   <213> Artificial Sequence
<220>
   <223> Synthetic Oligonucleotide
<400> 799
   gccctcccag tgtgcaaat 19
<210> 800
   <211> 25
   <212> DNA
   <213> Artificial Sequence
<220>
   <223> Synthetic Oligonucleotide
<400> 800
   cgtcgatggt attaggatag aagca 25
<210> 801
   <211> 23
   <212> DNA
   <213> Artificial Sequence
<220>
   <223> Synthetic Oligonucleotide
<400> 801
   tgctgtttcg acgacaccgt tcg 23
<210> 802
   <211> 25
   <212> DNA
   <213> Artificial Sequence
<220>
   <223> Synthetic Oligonucleotide
<400> 802
   tggctaagtg aagatgacaa tcatg 25
<210> 803
   <211> 25
   <212> DNA
   <213> Artificial Sequence
<220>
   <223> Synthetic Oligonucleotide
<400> 803
   tgcacatatc attacaccag ttcgt 25
<210> 804
   <211> 29
   <212> DNA
   <213> Artificial Sequence
<220>
   <223> Synthetic Oligonucleotide
<400> 804
   cctttccagc tttacagtga attgctgca 29
<210> 805
   <211> 23
   <212> DNA
   <213> Artificial Sequence
<220>
   <223> Synthetic Oligonucleotide
<400> 805
   cgcttgccta actcatactt tcc 23
<210> 806
   <211> 20
   <212> DNA
   <213> Artificial Sequence
<220>
   <223> Synthetic Oligonucleotide
<400> 806
   ccattcagac tgcgccactt 20
<210> 807
   <211> 19
   <212> DNA
   <213> Artificial Sequence
<220>
   <223> Synthetic Oligonucleotide
<400> 807
   tccacgcagc gtggcactg 19
<210> 808
   <211> 28
   <212> DNA
   <213> Artificial Sequence
<220>
   <223> Synthetic Oligonucleotide
<400> 808
   ggctactctg atctatgttg ataaggaa 28
<210> 809
   <211> 20
   <212> DNA
   <213> Artificial Sequence
<220>
   <223> Synthetic Oligonucleotide
<400> 809
   gcttcagccc atccttagca 20
<210> 810
   <211> 22
   <212> DNA
   <213> Artificial Sequence
<220>
   <223> Synthetic Oligonucleotide
<400> 810
   cacacgggtg cctggttctc ca 22
<210> 811
   <211> 18
   <212> DNA
   <213> Artificial Sequence
<220>
   <223> Synthetic Oligonucleotide
<400> 811
   gggacactgc gggacaag 18
<210> 812
   <211> 18
   <212> DNA
   <213> Artificial Sequence
<220>
   <223> Synthetic Oligonucleotide
<400> 812
   gcccatggcg tctctgaa 18
<210> 813
   <211> 25
   <212> DNA
   <213> Artificial Sequence
<220>
   <223> Synthetic Oligonucleotide
<400> 813
   cggttccgga gtctcaccac tgcat 25
<210> 814
   <211> 19
   <212> DNA
   <213> Artificial Sequence
<220>
   <223> Synthetic Oligonucleotide
<400> 814
   ctgaaggacc ctacgctcg 19
<210> 815
   <211> 19
   <212> DNA
   <213> Artificial Sequence
<220>
   <223> Synthetic Oligonucleotide
<400> 815
   atgcaaagcc agtgtgctc 19
<210> 816
   <211> 24
   <212> DNA
   <213> Artificial Sequence
<220>
   <223> Synthetic Oligonucleotide
<400> 816
   cttctcaaag tgaggtgcca ggcc 24
<210> 817
   <211> 21
   <212> DNA
   <213> Artificial Sequence
<220>
   <223> Synthetic Oligonucleotide
<400> 817
   gcgacagctc ctctagttcc a 21
<210> 818
   <211> 19
   <212> DNA
   <213> Artificial Sequence
<220>
   <223> Synthetic Oligonucleotide
<400> 818
   ttcccgaagt ctccgcccg 19
<210> 819
   <211> 22
   <212> DNA
   <213> Artificial Sequence
<220>
   <223> Synthetic Oligonucleotide
<400> 819
   ggaacaccag cttgaatttc ct 22
<210> 820
   <211> 20
   <212> DNA
   <213> Artificial Sequence
<220>
   <223> Synthetic Oligonucleotide
<400> 820
   gaggagtggt gacagtctgc 20
<210> 821
   <211> 20
   <212> DNA
   <213> Artificial Sequence
<220>
   <223> Synthetic Oligonucleotide
<400> 821
   gctgcagaaa tcaaagtcca 20
<210> 822
   <211> 27
   <212> DNA
   <213> Artificial Sequence
<220>
   <223> Synthetic Oligonucleotide
<400> 822
   tcaatacaca ggaacctgag gagaccc 27
<210> 823
   <211> 23
   <212> DNA
   <213> Artificial Sequence
<220>
   <223> Synthetic Oligonucleotide
<400> 823
   agctagcctc agtgacacac atg 23
<210> 824
   <211> 18
   <212> DNA
   <213> Artificial Sequence
<220>
   <223> Synthetic Oligonucleotide
<400> 824
   ccggatctga cggctgtt 18
<210> 825
   <211> 24
   <212> DNA
   <213> Artificial Sequence
<220>
   <223> Synthetic Oligonucleotide
<400> 825
   acacaacgtc ggcagtgcaa cctg 24
<210> 826
   <211> 20
   <212> DNA
   <213> Artificial Sequence
<220>
   <223> Synthetic Oligonucleotide
<400> 826
   cgtcgtatgc gagagtctgt 20
<210> 827
   <211> 20
   <212> DNA
   <213> Artificial Sequence
<220>
   <223> Synthetic Oligonucleotide
<400> 827
   tgaaggcgtg aggtgtagaa 20
<210> 828
   <211> 26
   <212> DNA
   <213> Artificial Sequence
<220>
   <223> Synthetic Oligonucleotide
<400> 828
   tccaggatgc ctgttagttc tcagca 26
<210> 829
   <211> 26
   <212> DNA
   <213> Artificial Sequence
<220>
   <223> Synthetic Oligonucleotide
<400> 829
   ggtgtcagat ataaatgtgc aaatgc 26
<210> 830
   <211> 24
   <212> DNA
   <213> Artificial Sequence
<220>
   <223> Synthetic Oligonucleotide
<400> 830
   ttcgatattg ccagcagcta taaa 24
<210> 831
   <211> 28
   <212> DNA
   <213> Artificial Sequence
<220>
   <223> Synthetic Oligonucleotide
<400> 831
   tgctgtcctg tcggtctcag tacgttca 28
<210> 832
   <211> 20
   <212> DNA
   <213> Artificial Sequence
<220>
   <223> Synthetic Oligonucleotide
<400> 832
   tgtggatgct ggattgattt 20
<210> 833
   <211> 20
   <212> DNA
   <213> Artificial Sequence
<220>
   <223> Synthetic Oligonucleotide
<400> 833
   aagcagcact tcctggtctt 20
<210> 834
   <211> 25
   <212> DNA
   <213> Artificial Sequence
<220>
   <223> Synthetic Oligonucleotide
<400> 834
   ccactggtgc agctgctaaa tagca 25
<210> 835
   <211> 20
   <212> DNA
   <213> Artificial Sequence
<220>
   <223> Synthetic Oligonucleotide
<400> 835
   agtgggagac acctgacctt 20
<210> 836
   <211> 20
   <212> DNA
   <213> Artificial Sequence
<220>
   <223> Synthetic Oligonucleotide
<400> 836
   tgatctgggc attgtactcc 20
<210> 837
   <211> 29
   <212> DNA
   <213> Artificial Sequence
<220>
   <223> Synthetic Oligonucleotide
<400> 837
   ttgatcttct gctcaatctc agcttgaga 29
<210> 838
   <211> 20
   <212> DNA
   <213> Artificial Sequence
<220>
   <223> Synthetic Oligonucleotide
<400> 838
   cgaagccctt acaagtttcc 20
<210> 839
   <211> 20
   <212> DNA
   <213> Artificial Sequence
<220>
   <223> Synthetic Oligonucleotide
<400> 839
   ggactcttca ggggtgaaat 20
<210> 840
   <211> 24
   <212> DNA
   <213> Artificial Sequence
<220>
   <223> Synthetic Oligonucleotide
<400> 840
   cccttacgga ttcctggagg gaac 24
<210> 841
   <211> 20
   <212> DNA
   <213> Artificial Sequence
<220>
   <223> Synthetic Oligonucleotide
<400> 841
   cccagtgtac gaggaacaag 20
<210> 842
   <211> 20
   <212> DNA
   <213> Artificial Sequence
<220>
   <223> Synthetic Oligonucleotide
<400> 842
   ttagcgagga aggagttgct 20
<210> 843
   <211> 24
   <212> DNA
   <213> Artificial Sequence
<220>
   <223> Synthetic Oligonucleotide
<400> 843
   acagaccgag atctccaacc ggac 24
<210> 844
   <211> 18
   <212> DNA
   <213> Artificial Sequence
<220>
   <223> Synthetic Oligonucleotide
<400> 844
   gggctgggtg agaatgtg 18
<210> 845
   <211> 19
   <212> DNA
   <213> Artificial Sequence
<220>
   <223> Synthetic Oligonucleotide
<400> 845
   cacaacatcc tccggaatg 19
<210> 846
   <211> 24
   <212> DNA
   <213> Artificial Sequence
<220>
   <223> Synthetic Oligonucleotide
<400> 846
   ataccagggc cggcttcttc ctct 24
<210> 847
   <211> 18
   <212> DNA
   <213> Artificial Sequence
<220>
   <223> Synthetic Oligonucleotide
<400> 847
   cctacaagtc ctggggca 18
<210> 848
   <211> 20
   <212> DNA
   <213> Artificial Sequence
<220>
   <223> Synthetic Oligonucleotide
<400> 848
   tgaggtcagg ctcacacagt 20
<210> 849
   <211> 24
   <212> DNA
   <213> Artificial Sequence
<220>
   <223> Synthetic Oligonucleotide
<400> 849
   tggagcccca agcagtgtta atcc 24
<210> 850
   <211> 20
   <212> DNA
   <213> Artificial Sequence
<220>
   <223> Synthetic Oligonucleotide
<400> 850
   gcgaggagct ctacttgctc 20
<210> 851
   <211> 18
   <212> DNA
   <213> Artificial Sequence
<220>
   <223> Synthetic Oligonucleotide
<400> 851
   gccctgctga acaccact 18
<210> 852
   <211> 24
   <212> DNA
   <213> Artificial Sequence
<220>
   <223> Synthetic Oligonucleotide
<400> 852
   tgtcctcttt ctgcaccttg tcgc 24
<210> 853
   <211> 21
   <212> DNA
   <213> Artificial Sequence
<220>
   <223> Synthetic Oligonucleotide
<400> 853
   aagcatgaac aggacttgac c 21
<210> 854
   <211> 18
   <212> DNA
   <213> Artificial Sequence
<220>
   <223> Synthetic Oligonucleotide
<400> 854
   cctccccaag tcagttgc 18
<210> 855
   <211> 24
   <212> DNA
   <213> Artificial Sequence
<220>
   <223> Synthetic Oligonucleotide
<400> 855
   ctttccaacc cctggggaag acat 24
<210> 856
   <211> 18
   <212> DNA
   <213> Artificial Sequence
<220>
   <223> Synthetic Oligonucleotide
<400> 856
   cccgttcggt ctgaggaa 18
<210> 857
   <211> 22
   <212> DNA
   <213> Artificial Sequence
<220>
   <223> Synthetic Oligonucleotide
<400> 857
   gagcactcaa ggtagccaaa gg 22
<210> 858
   <211> 19
   <212> DNA
   <213> Artificial Sequence
<220>
   <223> Synthetic Oligonucleotide
<400> 858
   tccggttcgc catgtcccg 19
<210> 859
   <211> 20
   <212> DNA
   <213> Artificial Sequence
<220>
   <223> Synthetic Oligonucleotide
<400> 859
   ccagacgagc gattagaagc 20
<210> 860
   <211> 20
   <212> DNA
   <213> Artificial Sequence
<220>
   <223> Synthetic Oligonucleotide
<400> 860
   tcctcctctt cctcctcctc 20
<210> 861
   <211> 23
   <212> DNA
   <213> Artificial Sequence
<220>
   <223> Synthetic Oligonucleotide
<400> 861
   tgtgaggtga atgatttggg gga 23
<210> 862
   <211> 22
   <212> DNA
   <213> Artificial Sequence
<220>
   <223> Synthetic Oligonucleotide
<400> 862
   tgtgcacata ggaatgagct tc 22
<210> 863
   <211> 20
   <212> DNA
   <213> Artificial Sequence
<220>
   <223> Synthetic Oligonucleotide
<400> 863
   gtttagcacg caattgctca 20
<210> 864
   <211> 24
   <212> DNA
   <213> Artificial Sequence
<220>
   <223> Synthetic Oligonucleotide
<400> 864
   tcactctctt tgctggccaa ctgc 24
<210> 865
   <211> 19
   <212> DNA
   <213> Artificial Sequence
<220>
   <223> Synthetic Oligonucleotide
<400> 865
   gatctggcac tgtggttcc 19
<210> 866
   <211> 20
   <212> DNA
   <213> Artificial Sequence
<220>
   <223> Synthetic Oligonucleotide
<400> 866
   tgacagcgga agtggtattg 20
<210> 867
   <211> 22
   <212> DNA
   <213> Artificial Sequence
<220>
   <223> Synthetic Oligonucleotide
<400> 867
   caccgccagc cactgtcttc at 22
<210> 868
   <211> 24
   <212> DNA
   <213> Artificial Sequence
<220>
   <223> Synthetic Oligonucleotide
<400> 868
   ccattctatc atcaacgggt acaa 24
<210> 869
   <211> 23
   <212> DNA
   <213> Artificial Sequence
<220>
   <223> Synthetic Oligonucleotide
<400> 869
   tcagcaagtg ggaaggtgta atc 23
<210> 870
   <211> 25
   <212> DNA
   <213> Artificial Sequence
<220>
   <223> Synthetic Oligonucleotide
<400> 870
   tctccacaga caaggccagg actcg 25
<210> 871
   <211> 19
   <212> DNA
   <213> Artificial Sequence
<220>
   <223> Synthetic Oligonucleotide
<400> 871
   ctgtcttcct gttggccct 19
<210> 872
   <211> 20
   <212> DNA
   <213> Artificial Sequence
<220>
   <223> Synthetic Oligonucleotide
<400> 872
   gtgaggtagt gagtgggcgt 20
<210> 873
   <211> 24
   <212> DNA
   <213> Artificial Sequence
<220>
   <223> Synthetic Oligonucleotide
<400> 873
   acaatcatag ccagcacctg ggct 24
<210> 874
   <211> 25
   <212> DNA
   <213> Artificial Sequence
<220>
   <223> Synthetic Oligonucleotide
<400> 874
   gctcattatg aaaaacatcc caaac 25
<210> 875
   <211> 26
   <212> DNA
   <213> Artificial Sequence
<220>
   <223> Synthetic Oligonucleotide
<400> 875
   aagaaacaga agttgtctgg ctttct 26
<210> 876
   <211> 24
   <212> DNA
   <213> Artificial Sequence
<220>
   <223> Synthetic Oligonucleotide
<400> 876
   cacaccaacc aataatttcg catt 24
<210> 877
   <211> 20
   <212> DNA
   <213> Artificial Sequence
<220>
   <223> Synthetic Oligonucleotide
<400> 877
   gctctgttgt gtcctgttgc 20
<210> 878
   <211> 21
   <212> DNA
   <213> Artificial Sequence
<220>
   <223> Synthetic Oligonucleotide
<400> 878
   gtacggagaa gccacttcac a 21
<210> 879
   <211> 23
   <212> DNA
   <213> Artificial Sequence
<220>
   <223> Synthetic Oligonucleotide
<400> 879
   tcagtcacag gaaggccaga gcc 23
<210> 880
   <211> 18
   <212> DNA
   <213> Artificial Sequence
<220>
   <223> Synthetic Oligonucleotide
<400> 880
   cgaggagatg cctgtgga 18
<210> 881
   <211> 18
   <212> DNA
   <213> Artificial Sequence
<220>
   <223> Synthetic Oligonucleotide
<400> 881
   caacgccctg gtcactct 18
<210> 882
   <211> 24
   <212> DNA
   <213> Artificial Sequence
<220>
   <223> Synthetic Oligonucleotide
<400> 882
   ctgttccaca gcaagctctg cctc 24
<210> 883
   <211> 20
   <212> DNA
   <213> Artificial Sequence
<220>
   <223> Synthetic Oligonucleotide
<400> 883
   acaccaaaat gccatctcaa 20
<210> 884
   <211> 20
   <212> DNA
   <213> Artificial Sequence
<220>
   <223> Synthetic Oligonucleotide
<400> 884
   tttatcccca gcgaatttgt 20
<210> 885
   <211> 24
   <212> DNA
   <213> Artificial Sequence
<220>
   <223> Synthetic Oligonucleotide
<400> 885
   cacgccatgg aaaccatgat gttt 24
<210> 886
   <211> 18
   <212> DNA
   <213> Artificial Sequence
<220>
   <223> Synthetic Oligonucleotide
<400> 886
   cttctgagcc cgtctccc 18
<210> 887
   <211> 18
   <212> DNA
   <213> Artificial Sequence
<220>
   <223> Synthetic Oligonucleotide
<400> 887
   gagagctccc cttccgag 18
<210> 888
   <211> 24
   <212> DNA
   <213> Artificial Sequence
<220>
   <223> Synthetic Oligonucleotide
<400> 888
   cgcttctgga gcagcttcct caac 24
<210> 889
   <211> 20
   <212> DNA
   <213> Artificial Sequence
<220>
   <223> Synthetic Oligonucleotide
<400> 889
   cgcgagcccc tcattataca 20
<210> 890
   <211> 19
   <212> DNA
   <213> Artificial Sequence
<220>
   <223> Synthetic Oligonucleotide
<400> 890
   cactcgccgt tgacatcct 19
<210> 891
   <211> 22
   <212> DNA
   <213> Artificial Sequence
<220>
   <223> Synthetic Oligonucleotide
<400> 891
   ctccccacag cgcatcgagg aa 22
<210> 892
   <211> 20
   <212> DNA
   <213> Artificial Sequence
<220>
   <223> Synthetic Oligonucleotide
<400> 892
   gagagagcca gtctgatcca 20
<210> 893
   <211> 19
   <212> DNA
   <213> Artificial Sequence
<220>
   <223> Synthetic Oligonucleotide
<400> 893
   aggctgccat gtcctcata 19
<210> 894
   <211> 22
   <212> DNA
   <213> Artificial Sequence
<220>
   <223> Synthetic Oligonucleotide
<400> 894
   ctgctctgcc agcttggcct tc 22
<210> 895
   <211> 23
   <212> DNA
   <213> Artificial Sequence
<220>
   <223> Synthetic Oligonucleotide
<400> 895
   cagtggagac cagttgggta gtg 23
<210> 896
   <211> 20
   <212> DNA
   <213> Artificial Sequence
<220>
   <223> Synthetic Oligonucleotide
<400> 896
   ccttgaagag cgtcccatca 20
<210> 897
   <211> 28
   <212> DNA
   <213> Artificial Sequence
<220>
   <223> Synthetic Oligonucleotide
<400> 897
   cacacatgca gagcttgtag cgtaccca 28
<210> 898
   <211> 18
   <212> DNA
   <213> Artificial Sequence
<220>
   <223> Synthetic Oligonucleotide
<400> 898
   tccgcaagac acctcctg 18
<210> 899
   <211> 18
   <212> DNA
   <213> Artificial Sequence
<220>
   <223> Synthetic Oligonucleotide
<400> 899
   tgaagtcatc cttggccc 18
<210> 900
   <211> 23
   <212> DNA
   <213> Artificial Sequence
<220>
   <223> Synthetic Oligonucleotide
<400> 900
   tgtcctgtcc ttctgcagga ggc 23
<210> 901
   <211> 20
   <212> DNA
   <213> Artificial Sequence
<220>
   <223> Synthetic Oligonucleotide
<400> 901
   tgcattcgtt ggtttctctt 20
<210> 902
   <211> 20
   <212> DNA
   <213> Artificial Sequence
<220>
   <223> Synthetic Oligonucleotide
<400> 902
   tttctgaatg gcaaactgct 20
<210> 903
   <211> 30
   <212> DNA
   <213> Artificial Sequence
<220>
   <223> Synthetic Oligonucleotide
<400> 903
   tgcacctcct tcagaagact tatttttgtg 30
<210> 904
   <211> 20
   <212> DNA
   <213> Artificial Sequence
<220>
   <223> Synthetic Oligonucleotide
<400> 904
   ccaacacctt cttggcttct 20
<210> 905
   <211> 20
   <212> DNA
   <213> Artificial Sequence
<220>
   <223> Synthetic Oligonucleotide
<400> 905
   ctgttatccc aacccaaacc 20
<210> 906
   <211> 24
   <212> DNA
   <213> Artificial Sequence
<220>
   <223> Synthetic Oligonucleotide
<400> 906
   cctgtgttct tgctgagcac cctc 24
<210> 907
   <211> 20
   <212> DNA
   <213> Artificial Sequence
<220>
   <223> Synthetic Oligonucleotide
<400> 907
   agctggggtg tctgtttcat 20
<210> 908
   <211> 20
   <212> DNA
   <213> Artificial Sequence
<220>
   <223> Synthetic Oligonucleotide
<400> 908
   acagcaaggc gagcataaat 20
<210> 909
   <211> 23
   <212> DNA
   <213> Artificial Sequence
<220>
   <223> Synthetic Oligonucleotide
<400> 909
   cctgacttca ggtcaaggga tgg 23
<210> 910
   <211> 18
   <212> DNA
   <213> Artificial Sequence
<220>
   <223> Synthetic Oligonucleotide
<400> 910
   gtggggagcc catcatct 18
<210> 911
   <211> 20
   <212> DNA
   <213> Artificial Sequence
<220>
   <223> Synthetic Oligonucleotide
<400> 911
   ccagtccaca ctgagtgcat 20
<210> 912
   <211> 24
   <212> DNA
   <213> Artificial Sequence
<220>
   <223> Synthetic Oligonucleotide
<400> 912
   ccaagccatc acaggctctg cata 24
<210> 913
   <211> 20
   <212> DNA
   <213> Artificial Sequence
<220>
   <223> Synthetic Oligonucleotide
<400> 913
   tctgccagtg ctgaattctt 20
<210> 914
   <211> 20
   <212> DNA
   <213> Artificial Sequence
<220>
   <223> Synthetic Oligonucleotide
<400> 914
   ttcgaacctt agcagcttcc 20
<210> 915
   <211> 24
   <212> DNA
   <213> Artificial Sequence
<220>
   <223> Synthetic Oligonucleotide
<400> 915
   tgctgacatt gccctggctc ctat 24
<210> 916
   <211> 20
   <212> DNA
   <213> Artificial Sequence
<220>
   <223> Synthetic Oligonucleotide
<400> 916
   cccaactcag gtccttggta 20
<210> 917
   <211> 18
   <212> DNA
   <213> Artificial Sequence
<220>
   <223> Synthetic Oligonucleotide
<400> 917
   caagagggtc accccaag 18
<210> 918
   <211> 24
   <212> DNA
   <213> Artificial Sequence
<220>
   <223> Synthetic Oligonucleotide
<400> 918
   atcctgcaag ccaatcccag tcat 24
<210> 919
   <211> 22
   <212> DNA
   <213> Artificial Sequence
<220>
   <223> Synthetic Oligonucleotide
<400> 919
   agatgcatac ttggaagcac ag 22
<210> 920
   <211> 22
   <212> DNA
   <213> Artificial Sequence
<220>
   <223> Synthetic Oligonucleotide
<400> 920
   aacctaggac caggtaacca ca 22
<210> 921
   <211> 24
   <212> DNA
   <213> Artificial Sequence
<220>
   <223> Synthetic Oligonucleotide
<400> 921
   catatcacac tgggaggcaa tgca 24
<210> 922
   <211> 18
   <212> DNA
   <213> Artificial Sequence
<220>
   <223> Synthetic Oligonucleotide
<400> 922
   gcgcagaggc cagttaaa 18
<210> 923
   <211> 18
   <212> DNA
   <213> Artificial Sequence
<220>
   <223> Synthetic Oligonucleotide
<400> 923
   agctgagctg tgggttgc 18
<210> 924
   <211> 24
   <212> DNA
   <213> Artificial Sequence
<220>
   <223> Synthetic Oligonucleotide
<400> 924
   agatcacctc ctcgaaccca ctcc 24
<210> 925
   <211> 19
   <212> DNA
   <213> Artificial Sequence
<220>
   <223> Synthetic Oligonucleotide
<400> 925
   ggctggccaa acataagca 19
<210> 926
   <211> 26
   <212> DNA
   <213> Artificial Sequence
<220>
   <223> Synthetic Oligonucleotide
<400> 926
   tccttgtcac agtatttaca gctgaa 26
<210> 927
   <211> 29
   <212> DNA
   <213> Artificial Sequence
<220>
   <223> Synthetic Oligonucleotide
<400> 927
   ctgcactgcg atgcccagtc tagaaaatc 29
<210> 928
   <211> 21
   <212> DNA
   <213> Artificial Sequence
<220>
   <223> Synthetic Oligonucleotide
<400> 928
   agtgacaaat gttggaggag c 21
<210> 929
   <211> 19
   <212> DNA
   <213> Artificial Sequence
<220>
   <223> Synthetic Oligonucleotide
<400> 929
   ccctccactg tcttctggg 19
<210> 930
   <211> 24
   <212> DNA
   <213> Artificial Sequence
<220>
   <223> Synthetic Oligonucleotide
<400> 930
   tactgctgtc acacccgtca ccac 24
<210> 931
   <211> 19
   <212> DNA
   <213> Artificial Sequence
<220>
   <223> Synthetic Oligonucleotide
<400> 931
   acccaccatg gatgtcttc 19
<210> 932
   <211> 19
   <212> DNA
   <213> Artificial Sequence
<220>
   <223> Synthetic Oligonucleotide
<400> 932
   cctgcttggt cttttccac 19
<210> 933
   <211> 24
   <212> DNA
   <213> Artificial Sequence
<220>
   <223> Synthetic Oligonucleotide
<400> 933
   aagaagggct tctccatcgc caag 24
<210> 934
   <211> 20
   <212> DNA
   <213> Artificial Sequence
<220>
   <223> Synthetic Oligonucleotide
<400> 934
   tgaagagagg catgttggag 20
<210> 935
   <211> 20
   <212> DNA
   <213> Artificial Sequence
<220>
   <223> Synthetic Oligonucleotide
<400> 935
   aatagacaca tcggccacac 20
<210> 936
   <211> 24
   <212> DNA
   <213> Artificial Sequence
<220>
   <223> Synthetic Oligonucleotide
<400> 936
   tttgtcagca gtcacattgc ccaa 24
<210> 937
   <211> 24
   <212> DNA
   <213> Artificial Sequence
<220>
   <223> Synthetic Oligonucleotide
<400> 937
   atacagcacc aatggaaaga tcac 24
<210> 938
   <211> 24
   <212> DNA
   <213> Artificial Sequence
<220>
   <223> Synthetic Oligonucleotide
<400> 938
   tgtctgtaag agccctcagt ttga 24
<210> 939
   <211> 24
   <212> DNA
   <213> Artificial Sequence
<220>
   <223> Synthetic Oligonucleotide
<400> 939
   ttcgacgact acatcgcctg ctgc 24
<210> 940
   <211> 20
   <212> DNA
   <213> Artificial Sequence
<220>
   <223> Synthetic Oligonucleotide
<400> 940
   gggctcagct ttcagaagtg 20
<210> 941
   <211> 20
   <212> DNA
   <213> Artificial Sequence
<220>
   <223> Synthetic Oligonucleotide
<400> 941
   acatgttcag ctggtccaca 20
<210> 942
   <211> 25
   <212> DNA
   <213> Artificial Sequence
<220>
   <223> Synthetic Oligonucleotide
<400> 942
   tggcagtttt cttctgtcac caaaa 25
<210> 943
   <211> 20
   <212> DNA
   <213> Artificial Sequence
<220>
   <223> Synthetic Oligonucleotide
<400> 943
   tcacatgcca ctttggtgtt 20
<210> 944
   <211> 20
   <212> DNA
   <213> Artificial Sequence
<220>
   <223> Synthetic Oligonucleotide
<400> 944
   cttgcaggaa gcggctatac 20
<210> 945
   <211> 22
   <212> DNA
   <213> Artificial Sequence
<220>
   <223> Synthetic Oligonucleotide
<400> 945
   tcctgggaga gattgaccag ca 22
<210> 946
   <211> 19
   <212> DNA
   <213> Artificial Sequence
<220>
   <223> Synthetic Oligonucleotide
<400> 946
   caagagggac tcggactgc 19
<210> 947
   <211> 21
   <212> DNA
   <213> Artificial Sequence
<220>
   <223> Synthetic Oligonucleotide
<400> 947
   caggtcagtg gagagattgg t 21
<210> 948
   <211> 24
   <212> DNA
   <213> Artificial Sequence
<220>
   <223> Synthetic Oligonucleotide
<400> 948
   cctctgcacc tctgagagca tgga 24
<210> 949
   <211> 18
   <212> DNA
   <213> Artificial Sequence
<220>
   <223> Synthetic Oligonucleotide
<400> 949
   ggactcggag acgctgtg 18
<210> 950
   <211> 20
   <212> DNA
   <213> Artificial Sequence
<220>
   <223> Synthetic Oligonucleotide
<400> 950
   gggatccttc gcaacttctt 20
<210> 951
   <211> 24
   <212> DNA
   <213> Artificial Sequence
<220>
   <223> Synthetic Oligonucleotide
<400> 951
   ttcttgagga tcttgacggc cctc 24
<210> 952
   <211> 20
   <212> DNA
   <213> Artificial Sequence
<220>
   <223> Synthetic Oligonucleotide
<400> 952
   catcttccag gaggaccact 20
<210> 953
   <211> 20
   <212> DNA
   <213> Artificial Sequence
<220>
   <223> Synthetic Oligonucleotide
<400> 953
   tccgaccttc aatcatttca 20
<210> 954
   <211> 24
   <212> DNA
   <213> Artificial Sequence
<220>
   <223> Synthetic Oligonucleotide
<400> 954
   ctctgtggca ccctggacta cctg 24
<210> 955
   <211> 20
   <212> DNA
   <213> Artificial Sequence
<220>
   <223> Synthetic Oligonucleotide
<400> 955
   aatacccaac gcacaaatga 20
<210> 956
   <211> 20
   <212> DNA
   <213> Artificial Sequence
<220>
   <223> Synthetic Oligonucleotide
<400> 956
   ggagacaatg caaaccacac 20
<210> 957
   <211> 23
   <212> DNA
   <213> Artificial Sequence
<220>
   <223> Synthetic Oligonucleotide
<400> 957
   cacgttctct gccccgtttc ttg 23
<210> 958
   <211> 20
   <212> DNA
   <213> Artificial Sequence
<220>
   <223> Synthetic Oligonucleotide
<400> 958
   cctggaggct gcaacatacc 20
<210> 959
   <211> 23
   <212> DNA
   <213> Artificial Sequence
<220>
   <223> Synthetic Oligonucleotide
<400> 959
   tacaatggct ttggaggata gca 23
<210> 960
   <211> 25
   <212> DNA
   <213> Artificial Sequence
<220>
   <223> Synthetic Oligonucleotide
<400> 960
   atcctcctga agcccttttc gcagc 25
<210> 961
   <211> 20
   <212> DNA
   <213> Artificial Sequence
<220>
   <223> Synthetic Oligonucleotide
<400> 961
   tgttttgatt cccgggctta 20
<210> 962
   <211> 24
   <212> DNA
   <213> Artificial Sequence
<220>
   <223> Synthetic Oligonucleotide
<400> 962
   caaagctgtc agctctagca aaag 24
<210> 963
   <211> 28
   <212> DNA
   <213> Artificial Sequence
<220>
   <223> Synthetic Oligonucleotide
<400> 963
   tgccttcttc ctccctcact tctcacct 28
<210> 964
   <211> 20
   <212> DNA
   <213> Artificial Sequence
<220>
   <223> Synthetic Oligonucleotide
<400> 964
   cacccttgga ctggaaaact 20
<210> 965
   <211> 20
   <212> DNA
   <213> Artificial Sequence
<220>
   <223> Synthetic Oligonucleotide
<400> 965
   ccttgatgag ctgttggttc 20
<210> 966
   <211> 24
   <212> DNA
   <213> Artificial Sequence
<220>
   <223> Synthetic Oligonucleotide
<400> 966
   cacacccggt ctggacacag aaag 24
<210> 967
   <211> 19
   <212> DNA
   <213> Artificial Sequence
<220>
   <223> Synthetic Oligonucleotide
<400> 967
   gatcctcgcg tgagacaga 19
<210> 968
   <211> 21
   <212> DNA
   <213> Artificial Sequence
<220>
   <223> Synthetic Oligonucleotide
<400> 968
   cactttttct ttgaccaacc g 21
<210> 969
   <211> 23
   <212> DNA
   <213> Artificial Sequence
<220>
   <223> Synthetic Oligonucleotide
<400> 969
   ttcaccacgc cggtcttgat ctt 23
<210> 970
   <211> 20
   <212> DNA
   <213> Artificial Sequence
<220>
   <223> Synthetic Oligonucleotide
<400> 970
   ctgttttcct ggaggactgc 20
<210> 971
   <211> 20
   <212> DNA
   <213> Artificial Sequence
<220>
   <223> Synthetic Oligonucleotide
<400> 971
   actgtgtatg cggagctgtt 20
<210> 972
   <211> 21
   <212> DNA
   <213> Artificial Sequence
<220>
   <223> Synthetic Oligonucleotide
<400> 972
   ccactgccag cacgcagtca c 21
<210> 973
   <211> 21
   <212> DNA
   <213> Artificial Sequence
<220>
   <223> Synthetic Oligonucleotide
<400> 973
   cagccaggtg tacgagacat t 21
<210> 974
   <211> 18
   <212> DNA
   <213> Artificial Sequence
<220>
   <223> Synthetic Oligonucleotide
<400> 974
   acctcgtcca gcacatcc 18
<210> 975
   <211> 24
   <212> DNA
   <213> Artificial Sequence
<220>
   <223> Synthetic Oligonucleotide
<400> 975
   ctcacctaca gtgacgtcgt gggc 24
<210> 976
   <211> 19
   <212> DNA
   <213> Artificial Sequence
<220>
   <223> Synthetic Oligonucleotide
<400> 976
   tcccgagaga ggaaagcat 19
<210> 977
   <211> 18
   <212> DNA
   <213> Artificial Sequence
<220>
   <223> Synthetic Oligonucleotide
<400> 977
   gtcgggtgcc tgcattta 18
<210> 978
   <211> 24
   <212> DNA
   <213> Artificial Sequence
<220>
   <223> Synthetic Oligonucleotide
<400> 978
   atacacagtc ccttcgtggc tccc 24
<210> 979
   <211> 22
   <212> DNA
   <213> Artificial Sequence
<220>
   <223> Synthetic Oligonucleotide
<400> 979
   cctggttgaa gcctgttaat gc 22
<210> 980
   <211> 26
   <212> DNA
   <213> Artificial Sequence
<220>
   <223> Synthetic Oligonucleotide
<400> 980
   tgcagacttc tcatatttgc taaagg 26
<210> 981
   <211> 23
   <212> DNA
   <213> Artificial Sequence
<220>
   <223> Synthetic Oligonucleotide
<400> 981
   ccgctgggtt ttccacacgt tga 23
<210> 982
   <211> 22
   <212> DNA
   <213> Artificial Sequence
<220>
   <223> Synthetic Oligonucleotide
<400> 982
   ccagtgtgtg taacagggtc ac 22
<210> 983
   <211> 18
   <212> DNA
   <213> Artificial Sequence
<220>
   <223> Synthetic Oligonucleotide
<400> 983
   tatagccttg ggccaccc 18
<210> 984
   <211> 24
   <212> DNA
   <213> Artificial Sequence
<220>
   <223> Synthetic Oligonucleotide
<400> 984
   agaattcgac agccagatgc tcca 24
<210> 985
   <211> 20
   <212> DNA
   <213> Artificial Sequence
<220>
   <223> Synthetic Oligonucleotide
<400> 985
   gccaactgct ttcatttgtg 20
<210> 986
   <211> 20
   <212> DNA
   <213> Artificial Sequence
<220>
   <223> Synthetic Oligonucleotide
<400> 986
   actcaggccc atttccttta 20
<210> 987
   <211> 28
   <212> DNA
   <213> Artificial Sequence
<220>
   <223> Synthetic Oligonucleotide
<400> 987
   agggatctga accaatacag agcagaca 28
<210> 988
   <211> 22
   <212> DNA
   <213> Artificial Sequence
<220>
   <223> Synthetic Oligonucleotide
<400> 988
   catccgcaaa gtgactgaag ag 22
<210> 989
   <211> 27
   <212> DNA
   <213> Artificial Sequence
<220>
   <223> Synthetic Oligonucleotide
<400> 989
   gtactgaact ccgttgtgat agcatag 27
<210> 990
   <211> 22
   <212> DNA
   <213> Artificial Sequence
<220>
   <223> Synthetic Oligonucleotide
<400> 990
   ccaatgagct gaggcggcct cc 22
<210> 991
   <211> 20
   <212> DNA
   <213> Artificial Sequence
<220>
   <223> Synthetic Oligonucleotide
<400> 991
   gccgggaaga ccgtaattgt 20
<210> 992
   <211> 18
   <212> DNA
   <213> Artificial Sequence
<220>
   <223> Synthetic Oligonucleotide
<400> 992
   cagcggcacc aggttcag 18
<210> 993
   <211> 26
   <212> DNA
   <213> Artificial Sequence
<220>
   <223> Synthetic Oligonucleotide
<400> 993
   caaatggctt cctctggaag gtccca 26
<210> 994
   <211> 20
   <212> DNA
   <213> Artificial Sequence
<220>
   <223> Synthetic Oligonucleotide
<400> 994
   aatccaaggg ggagagtgat 20
<210> 995
   <211> 20
   <212> DNA
   <213> Artificial Sequence
<220>
   <223> Synthetic Oligonucleotide
<400> 995
   gtacagattt tgcccgagga 20
<210> 996
   <211> 26
   <212> DNA
   <213> Artificial Sequence
<220>
   <223> Synthetic Oligonucleotide
<400> 996
   catatggact ttgactcagc tgtggc 26
<210> 997
   <211> 18
   <212> DNA
   <213> Artificial Sequence
<220>
   <223> Synthetic Oligonucleotide
<400> 997
   gtgatgcctt ccctgtgg 18
<210> 998
   <211> 19
   <212> DNA
   <213> Artificial Sequence
<220>
   <223> Synthetic Oligonucleotide
<400> 998
   tcaggtagtc gggtgggtt 19
<210> 999
   <211> 24
   <212> DNA
   <213> Artificial Sequence
<220>
   <223> Synthetic Oligonucleotide
<400> 999
   tgcttctcca gcatcttcac ctcg 24
<210> 1000
   <211> 24
   <212> DNA
   <213> Artificial Sequence
<220>
   <223> Synthetic Oligonucleotide
<400> 1000
   ctatatgcag ccagagatgt gaca 24
<210> 1001
   <211> 24
   <212> DNA
   <213> Artificial Sequence
<220>
   <223> Synthetic Oligonucleotide
<400> 1001
   ccacgagttt cttactgaga atgg 24
<210> 1002
   <211> 23
   <212> DNA
   <213> Artificial Sequence
<220>
   <223> Synthetic Oligonucleotide
<400> 1002
   acagcctgcc actcatcaca gcc 23
<210> 1003
   <211> 20
   <212> DNA
   <213> Artificial Sequence
<220>
   <223> Synthetic Oligonucleotide
<400> 1003
   tgctgttgct gagtctgttg 20
<210> 1004
   <211> 20
   <212> DNA
   <213> Artificial Sequence
<220>
   <223> Synthetic Oligonucleotide
<400> 1004
   cttgcctggc ttcacagata 20
<210> 1005
   <211> 24
   <212> DNA
   <213> Artificial Sequence
<220>
   <223> Synthetic Oligonucleotide
<400> 1005
   ccagtcccca gaagaccatg tctg 24
<210> 1006
   <211> 22
   <212> DNA
   <213> Artificial Sequence
<220>
   <223> Synthetic Oligonucleotide
<400> 1006
   ggtgtcgata ttgtcatgaa cc 22
<210> 1007
   <211> 27
   <212> DNA
   <213> Artificial Sequence
<220>
   <223> Synthetic Oligonucleotide
<400> 1007
   gtaatctttg atgtacttct tgtaggc 27
<210> 1008
   <211> 27
   <212> DNA
   <213> Artificial Sequence
<220>
   <223> Synthetic Oligonucleotide
<400> 1008
   tcacctgcag gaaacaagtt tcacaaa 27
<210> 1009
   <211> 21
   <212> DNA
   <213> Artificial Sequence
<220>
   <223> Synthetic Oligonucleotide
<400> 1009
   gacgctggtc tggtgaagat g 21
<210> 1010
   <211> 20
   <212> DNA
   <213> Artificial Sequence
<220>
   <223> Synthetic Oligonucleotide
<400> 1010
   tgggtggttg ttggacaatg 20
<210> 1011
   <211> 23
   <212> DNA
   <213> Artificial Sequence
<220>
   <223> Synthetic Oligonucleotide
<400> 1011
   ccaggaaacc acgagcctcc agc 23
<210> 1012
   <211> 22
   <212> DNA
   <213> Artificial Sequence
<220>
   <223> Synthetic Oligonucleotide
<400> 1012
   cttcacagtg ctcctgcagt ct 22
<210> 1013
   <211> 21
   <212> DNA
   <213> Artificial Sequence
<220>
   <223> Synthetic Oligonucleotide
<400> 1013
   catctgcttc agctcgttgg t 21
<210> 1014
   <211> 26
   <212> DNA
   <213> Artificial Sequence
<220>
   <223> Synthetic Oligonucleotide
<400> 1014
   aagtacacgt aagttacagc cacaca 26
<210> 1015
   <211> 18
   <212> DNA
   <213> Artificial Sequence
<220>
   <223> Synthetic Oligonucleotide
<400> 1015
   gcctcggtgt gcctttca 18
<210> 1016
   <211> 19
   <212> DNA
   <213> Artificial Sequence
<220>
   <223> Synthetic Oligonucleotide
<400> 1016
   cgtgatgtgc gcaatcatg 19
<210> 1017
   <211> 22
   <212> DNA
   <213> Artificial Sequence
<220>
   <223> Synthetic Oligonucleotide
<400> 1017
   catcgccagc tacgccctgc tc 22
<210> 1018
   <211> 19
   <212> DNA
   <213> Artificial Sequence
<220>
   <223> Synthetic Oligonucleotide
<400> 1018
   ctggtcagcc ttcagggac 19
<210> 1019
   <211> 18
   <212> DNA
   <213> Artificial Sequence
<220>
   <223> Synthetic Oligonucleotide
<400> 1019
   cttcagttct gggctggc 18
<210> 1020
   <211> 23
   <212> DNA
   <213> Artificial Sequence
<220>
   <223> Synthetic Oligonucleotide
<400> 1020
   ctgagcaccg cctggtctct ttc 23
<210> 1021
   <211> 23
   <212> DNA
   <213> Artificial Sequence
<220>
   <223> Synthetic Oligonucleotide
<400> 1021
   tgcttgtcag ttgtcaacac ctt 23
<210> 1022
   <211> 23
   <212> DNA
   <213> Artificial Sequence
<220>
   <223> Synthetic Oligonucleotide
<400> 1022
   tggagtggca agtatttgat gct 23
<210> 1023
   <211> 23
   <212> DNA
   <213> Artificial Sequence
<220>
   <223> Synthetic Oligonucleotide
<400> 1023
   tggccaacct gcctgtttcc agc 23
<210> 1024
   <211> 19
   <212> DNA
   <213> Artificial Sequence
<220>
   <223> Synthetic Oligonucleotide
<400> 1024
   tgtcaccccg actcaacgt 19
<210> 1025
   <211> 22
   <212> DNA
   <213> Artificial Sequence
<220>
   <223> Synthetic Oligonucleotide
<400> 1025
   acgtggactg agatgcattc ac 22
<210> 1026
   <211> 21
   <212> DNA
   <213> Artificial Sequence
<220>
   <223> Synthetic Oligonucleotide
<400> 1026
   agacgcaccg cccggactca c 21
<210> 1027
   <211> 20
   <212> DNA
   <213> Artificial Sequence
<220>
   <223> Synthetic Oligonucleotide
<400> 1027
   agctcaacat gcgtgagtgt 20
<210> 1028
   <211> 19
   <212> DNA
   <213> Artificial Sequence
<220>
   <223> Synthetic Oligonucleotide
<400> 1028
   attgccgatc tggactcct 19
<210> 1029
   <211> 23
   <212> DNA
   <213> Artificial Sequence
<220>
   <223> Synthetic Oligonucleotide
<400> 1029
   atctctatcc acgtggggca ggc 23
<210> 1030
   <211> 23
   <212> DNA
   <213> Artificial Sequence
<220>
   <223> Synthetic Oligonucleotide
<400> 1030
   ctcttacatc gaccgcctaa gag 23
<210> 1031
   <211> 18
   <212> DNA
   <213> Artificial Sequence
<220>
   <223> Synthetic Oligonucleotide
<400> 1031
   gctgatggcg gagacgaa 18
<210> 1032
   <211> 27
   <212> DNA
   <213> Artificial Sequence
<220>
   <223> Synthetic Oligonucleotide
<400> 1032
   cgcgctgtaa gaagcaacaa cctctcc 27
<210> 1033
   <211> 20
   <212> DNA
   <213> Artificial Sequence
<220>
   <223> Synthetic Oligonucleotide
<400> 1033
   gaggagggtg agttctccaa 20
<210> 1034
   <211> 20
   <212> DNA
   <213> Artificial Sequence
<220>
   <223> Synthetic Oligonucleotide
<400> 1034
   atgcccacct ccttgtaatc 20
<210> 1035
   <211> 24
   <212> DNA
   <213> Artificial Sequence
<220>
   <223> Synthetic Oligonucleotide
<400> 1035
   ccatgaggat atgactgccc tgga 24
<210> 1036
   <211> 19
   <212> DNA
   <213> Artificial Sequence
<220>
   <223> Synthetic Oligonucleotide
<400> 1036
   gtcccttcgc ctccttcac 19
<210> 1037
   <211> 20
   <212> DNA
   <213> Artificial Sequence
<220>
   <223> Synthetic Oligonucleotide
<400> 1037
   cgtggatgga gatgcactca 20
<210> 1038
   <211> 28
   <212> DNA
   <213> Artificial Sequence
<220>
   <223> Synthetic Oligonucleotide
<400> 1038
   ccgcagaccc cttcaagttc tagtcatg 28
<210> 1039
   <211> 20
   <212> DNA
   <213> Artificial Sequence
<220>
   <223> Synthetic Oligonucleotide
<400> 1039
   cgccctacct ataccaacct 20
<210> 1040
   <211> 20
   <212> DNA
   <213> Artificial Sequence
<220>
   <223> Synthetic Oligonucleotide
<400> 1040
   cggagagaag cagtgattga 20
<210> 1041
   <211> 25
   <212> DNA
   <213> Artificial Sequence
<220>
   <223> Synthetic Oligonucleotide
<400> 1041
   caaccgcctc atcagtcaga ttgtg 25
<210> 1042
   <211> 20
   <212> DNA
   <213> Artificial Sequence
<220>
   <223> Synthetic Oligonucleotide
<400> 1042
   cgaggacgag gcttaaaaac 20
<210> 1043
   <211> 20
   <212> DNA
   <213> Artificial Sequence
<220>
   <223> Synthetic Oligonucleotide
<400> 1043
   accatgcttg aggacaacag 20
<210> 1044
   <211> 29
   <212> DNA
   <213> Artificial Sequence
<220>
   <223> Synthetic Oligonucleotide
<400> 1044
   tctcagatca atcgtgcatc cttagtgaa 29
<210> 1045
   <211> 21
   <212> DNA
   <213> Artificial Sequence
<220>
   <223> Synthetic Oligonucleotide
<400> 1045
   cgccctcctg cagtatttat g 21
<210> 1046
   <211> 22
   <212> DNA
   <213> Artificial Sequence
<220>
   <223> Synthetic Oligonucleotide
<400> 1046
   acagagacag gagcagctca ca 22
<210> 1047
   <211> 23
   <212> DNA
   <213> Artificial Sequence
<220>
   <223> Synthetic Oligonucleotide
<400> 1047
   cctcgtcctc cccacctagg cca 23
<210> 1048
   <211> 21
   <212> DNA
   <213> Artificial Sequence
<220>
   <223> Synthetic Oligonucleotide
<400> 1048
   acactgactg gcatcctgct t 21
<210> 1049
   <211> 25
   <212> DNA
   <213> Artificial Sequence
<220>
   <223> Synthetic Oligonucleotide
<400> 1049
   gctctgtagc tccccatgta ctagt 25
<210> 1050
   <211> 24
   <212> DNA
   <213> Artificial Sequence
<220>
   <223> Synthetic Oligonucleotide
<400> 1050
   agcctccaga agagccaggt gcct 24
<210> 1051
   <211> 20
   <212> DNA
   <213> Artificial Sequence
<220>
   <223> Synthetic Oligonucleotide
<400> 1051
   gtggcctaga gccttcagtc 20
<210> 1052
   <211> 21
   <212> DNA
   <213> Artificial Sequence
<220>
   <223> Synthetic Oligonucleotide
<400> 1052
   caggctggga gtgaataaag a 21
<210> 1053
   <211> 24
   <212> DNA
   <213> Artificial Sequence
<220>
   <223> Synthetic Oligonucleotide
<400> 1053
   ttcacactgc ttccctgctt tccc 24
<210> 1054
   <211> 18
   <212> DNA
   <213> Artificial Sequence
<220>
   <223> Synthetic Oligonucleotide
<400> 1054
   acaggcccca tgcatcct 18
<210> 1055
   <211> 25
   <212> DNA
   <213> Artificial Sequence
<220>
   <223> Synthetic Oligonucleotide
<400> 1055
   tgtttctctc ccagataagc taagg 25
<210> 1056
   <211> 21
   <212> DNA
   <213> Artificial Sequence
<220>
   <223> Synthetic Oligonucleotide
<400> 1056
   tgcctcactc ccctcagccc c 21
<210> 1057
   <211> 19
   <212> DNA
   <213> Artificial Sequence
<220>
   <223> Synthetic Oligonucleotide
<400> 1057
   ccctatggcc ctgaatggt 19
<210> 1058
   <211> 26
   <212> DNA
   <213> Artificial Sequence
<220>
   <223> Synthetic Oligonucleotide
<400> 1058
   actaattaca tgacttggct gcattt 26
<210> 1059
   <211> 23
   <212> DNA
   <213> Artificial Sequence
<220>
   <223> Synthetic Oligonucleotide
<400> 1059
   tgaggggccg acaccaacac aat 23
<210> 1060
   <211> 20
   <212> DNA
   <213> Artificial Sequence
<220>
   <223> Synthetic Oligonucleotide
<400> 1060
   agtggaatcc ttccctttcc 20
<210> 1061
   <211> 20
   <212> DNA
   <213> Artificial Sequence
<220>
   <223> Synthetic Oligonucleotide
<400> 1061
   cccttgatcc ctttctctga 20
<210> 1062
   <211> 24
   <212> DNA
   <213> Artificial Sequence
<220>
   <223> Synthetic Oligonucleotide
<400> 1062
   cctcactcag ctcctttccc ctga 24
<210> 1063
   <211> 24
   <212> DNA
   <213> Artificial Sequence
<220>
   <223> Synthetic Oligonucleotide
<400> 1063
   ggaaggaaag aagcatggtc tact 24
<210> 1064
   <211> 24
   <212> DNA
   <213> Artificial Sequence
<220>
   <223> Synthetic Oligonucleotide
<400> 1064
   aaaaagtgac aggcaacagt gaag 24
<210> 1065
   <211> 24
   <212> DNA
   <213> Artificial Sequence
<220>
   <223> Synthetic Oligonucleotide
<400> 1065
   caccagagac ccagcgcaca ccta 24
<210> 1066
   <211> 19
   <212> DNA
   <213> Artificial Sequence
<220>
   <223> Synthetic Oligonucleotide
<400> 1066
   gatgccgagg gaaatcatc 19
<210> 1067
   <211> 20
   <212> DNA
   <213> Artificial Sequence
<220>
   <223> Synthetic Oligonucleotide
<400> 1067
   ccagaactcg aacccaatct 20
<210> 1068
   <211> 24
   <212> DNA
   <213> Artificial Sequence
<220>
   <223> Synthetic Oligonucleotide
<400> 1068
   attgccgcac tggcccaact gtag 24
<210> 1069
   <211> 18
   <212> DNA
   <213> Artificial Sequence
<220>
   <223> Synthetic Oligonucleotide
<400> 1069
   gcgctgcgga agatcatc 18
<210> 1070
   <211> 22
   <212> DNA
   <213> Artificial Sequence
<220>
   <223> Synthetic Oligonucleotide
<400> 1070
   gcttgagggt ctgaatcttg ct 22
<210> 1071
   <211> 21
   <212> DNA
   <213> Artificial Sequence
<220>
   <223> Synthetic Oligonucleotide
<400> 1071
   ccacgctgcc ctcggacaag c 21
<210> 1072
   <211> 18
   <212> DNA
   <213> Artificial Sequence
<220>
   <223> Synthetic Oligonucleotide
<400> 1072
   cagtgtggag gggatgga 18
<210> 1073
   <211> 19
   <212> DNA
   <213> Artificial Sequence
<220>
   <223> Synthetic Oligonucleotide
<400> 1073
   caagttctgg accacagcc 19
<210> 1074
   <211> 24
   <212> DNA
   <213> Artificial Sequence
<220>
   <223> Synthetic Oligonucleotide
<400> 1074
   cttcacccac tggatgtcag gctc 24
<210> 1075
   <211> 23
   <212> DNA
   <213> Artificial Sequence
<220>
   <223> Synthetic Oligonucleotide
<400> 1075
   agttgtcgtg tgttctggat tca 23
<210> 1076
   <211> 22
   <212> DNA
   <213> Artificial Sequence
<220>
   <223> Synthetic Oligonucleotide
<400> 1076
   cgtcagcgtg atcttaaagg aa 22
<210> 1077
   <211> 22
   <212> DNA
   <213> Artificial Sequence
<220>
   <223> Synthetic Oligonucleotide
<400> 1077
   tccggcacca ccatgtcgaa gg 22
<210> 1078
   <211> 19
   <212> DNA
   <213> Artificial Sequence
<220>
   <223> Synthetic Oligonucleotide
<400> 1078
   gtggatgtgc cctgaagga 19
<210> 1079
   <211> 20
   <212> DNA
   <213> Artificial Sequence
<220>
   <223> Synthetic Oligonucleotide
<400> 1079
   ctgcggatcc agggtaagaa 20
<210> 1080
   <211> 28
   <212> DNA
   <213> Artificial Sequence
<220>
   <223> Synthetic Oligonucleotide
<400> 1080
   aagccaggcg tctacacgag agtctcac 28
<210> 1081
   <211> 24
   <212> DNA
   <213> Artificial Sequence
<220>
   <223> Synthetic Oligonucleotide
<400> 1081
   ggttgtgctc tacgagctta tgac 24
<210> 1082
   <211> 22
   <212> DNA
   <213> Artificial Sequence
<220>
   <223> Synthetic Oligonucleotide
<400> 1082
   cggcccacca taaagataat ct 22
<210> 1083
   <211> 24
   <212> DNA
   <213> Artificial Sequence
<220>
   <223> Synthetic Oligonucleotide
<400> 1083
   tgccttacag ccacattggc tgcc 24
<210> 1084
   <211> 21
   <212> DNA
   <213> Artificial Sequence
<220>
   <223> Synthetic Oligonucleotide
<400> 1084
   tggcttcagg agctgaatac c 21
<210> 1085
   <211> 24
   <212> DNA
   <213> Artificial Sequence
<220>
   <223> Synthetic Oligonucleotide
<400> 1085
   tgctgtcgtg atgagaaaat agtg 24
<210> 1086
   <211> 26
   <212> DNA
   <213> Artificial Sequence
<220>
   <223> Synthetic Oligonucleotide
<400> 1086
   caggcacaca caggtgggac acaaat 26
<210> 1087
   <211> 20
   <212> DNA
   <213> Artificial Sequence
<220>
   <223> Synthetic Oligonucleotide
<400> 1087
   ctgctgtctt gggtgcattg 20
<210> 1088
   <211> 18
   <212> DNA
   <213> Artificial Sequence
<220>
   <223> Synthetic Oligonucleotide
<400> 1088
   gcagcctggg accacttg 18
<210> 1089
   <211> 25
   <212> DNA
   <213> Artificial Sequence
<220>
   <223> Synthetic Oligonucleotide
<400> 1089
   ttgccttgct gctctacctc cacca 25
<210> 1090
   <211> 19
   <212> DNA
   <213> Artificial Sequence
<220>
   <223> Synthetic Oligonucleotide
<400> 1090
   tgacgatggc ctggagtgt 19
<210> 1091
   <211> 22
   <212> DNA
   <213> Artificial Sequence
<220>
   <223> Synthetic Oligonucleotide
<400> 1091
   ggtaccggat catgaggatc tg 22
<210> 1092
   <211> 21
   <212> DNA
   <213> Artificial Sequence
<220>
   <223> Synthetic Oligonucleotide
<400> 1092
   ctgggcagca ccaagtccgg a 21
<210> 1093
   <211> 25
   <212> DNA
   <213> Artificial Sequence
<220>
   <223> Synthetic Oligonucleotide
<400> 1093
   cctcagcaag acgttatttg aaatt 25
<210> 1094
   <211> 24
   <212> DNA
   <213> Artificial Sequence
<220>
   <223> Synthetic Oligonucleotide
<400> 1094
   aagtgtgatt ggcaaaactg attg 24
<210> 1095
   <211> 26
   <212> DNA
   <213> Artificial Sequence
<220>
   <223> Synthetic Oligonucleotide
<400> 1095
   cctctctctc aaggccccaa accagt 26
<210> 1096
   <211> 19
   <212> DNA
   <213> Artificial Sequence
<220>
   <223> Synthetic Oligonucleotide
<400> 1096
   cggttggtga cttgtctgc 19
<210> 1097
   <211> 20
   <212> DNA
   <213> Artificial Sequence
<220>
   <223> Synthetic Oligonucleotide
<400> 1097
   aagacttgtc cctgcctcac 20
<210> 1098
   <211> 24
   <212> DNA
   <213> Artificial Sequence
<220>
   <223> Synthetic Oligonucleotide
<400> 1098
   atgcctcagt cttcccaaag cagg 24
<210> 1099
   <211> 21
   <212> DNA
   <213> Artificial Sequence
<220>
   <223> Synthetic Oligonucleotide
<400> 1099
   tgcccttaaa ggaaccaatg a 21
<210> 1100
   <211> 22
   <212> DNA
   <213> Artificial Sequence
<220>
   <223> Synthetic Oligonucleotide
<400> 1100
   gcttcaacgg caaagttctc tt 22
<210> 1101
   <211> 22
   <212> DNA
   <213> Artificial Sequence
<220>
   <223> Synthetic Oligonucleotide
<400> 1101
   atttcacgca tctggcgttc ca 22
<210> 1102
   <211> 18
   <212> DNA
   <213> Artificial Sequence
<220>
   <223> Synthetic Oligonucleotide
<400> 1102
   ctctccagtg tgggcacc 18
<210> 1103
   <211> 19
   <212> DNA
   <213> Artificial Sequence
<220>
   <223> Synthetic Oligonucleotide
<400> 1103
   gcggtgtagc tcccagagt 19
<210> 1104
   <211> 24
   <212> DNA
   <213> Artificial Sequence
<220>
   <223> Synthetic Oligonucleotide
<400> 1104
   ccagaacaga tgcgagcagt ccat 24
<210> 1105
   <211> 25
   <212> DNA
   <213> Artificial Sequence
<220>
   <223> Synthetic Oligonucleotide
<400> 1105
   gtatcaggac cacatgcagt acatc 25
<210> 1106
   <211> 22
   <212> DNA
   <213> Artificial Sequence
<220>
   <223> Synthetic Oligonucleotide
<400> 1106
   tgtcggaatt gatactggca tt 22
<210> 1107
   <211> 24
   <212> DNA
   <213> Artificial Sequence
<220>
   <223> Synthetic Oligonucleotide
<400> 1107
   ttgatgcctg tcttcgcgcc ttct 24
<210> 1108
   <211> 23
   <212> DNA
   <213> Artificial Sequence
<220>
   <223> Synthetic Oligonucleotide
<400> 1108
   gcagttggaa gacacaggaa agt 23
<210> 1109
   <211> 21
   <212> DNA
   <213> Artificial Sequence
<220>
   <223> Synthetic Oligonucleotide
<400> 1109
   tgcgtggcac tattttcaag a 21
<210> 1110
   <211> 27
   <212> DNA
   <213> Artificial Sequence
<220>
   <223> Synthetic Oligonucleotide
<400> 1110
   tccccaaatt gcagatttat caacggc 27
<210> 1111
   <211> 20
   <212> DNA
   <213> Artificial Sequence
<220>
   <223> Synthetic Oligonucleotide
<400> 1111
   caggtcaggc agaggaagtc 20
<210> 1112
   <211> 20
   <212> DNA
   <213> Artificial Sequence
<220>
   <223> Synthetic Oligonucleotide
<400> 1112
   cctaacaagc cccaaatcaa 20
<210> 1113
   <211> 27
   <212> DNA
   <213> Artificial Sequence
<220>
   <223> Synthetic Oligonucleotide
<400> 1113
   tgcattgcat gagctaaacc tatctga 27
<210> 1114
   <211> 20
   <212> DNA
   <213> Artificial Sequence
<220>
   <223> Synthetic Oligonucleotide
<400> 1114
   tggtcagatg ctgctgaagt 20
<210> 1115
   <211> 20
   <212> DNA
   <213> Artificial Sequence
<220>
   <223> Synthetic Oligonucleotide
<400> 1115
   atcacagcat gaagggatgg 20
<210> 1116
   <211> 24
   <212> DNA
   <213> Artificial Sequence
<220>
   <223> Synthetic Oligonucleotide
<400> 1116
   tatccttagg ccgctggcat cttg 24
<210> 1117
   <211> 19
   <212> DNA
   <213> Artificial Sequence
<220>
   <223> Synthetic Oligonucleotide
<400> 1117
   gagggagcag acagtgggt 19
<210> 1118
   <211> 21
   <212> DNA
   <213> Artificial Sequence
<220>
   <223> Synthetic Oligonucleotide
<400> 1118
   tcagagccct tgctaagtca c 21
<210> 1119
   <211> 24
   <212> DNA
   <213> Artificial Sequence
<220>
   <223> Synthetic Oligonucleotide
<400> 1119
   ccacgatctc cgtaaccatt tgca 24
<210> 1120
   <211> 20
   <212> DNA
   <213> Artificial Sequence
<220>
   <223> Synthetic Oligonucleotide
<400> 1120
   tagaggccat caaaattggc 20
<210> 1121
   <211> 18
   <212> DNA
   <213> Artificial Sequence
<220>
   <223> Synthetic Oligonucleotide
<400> 1121
   tccgtttcct ctgggctt 18
<210> 1122
   <211> 24
   <212> DNA
   <213> Artificial Sequence
<220>
   <223> Synthetic Oligonucleotide
<400> 1122
   accaaggccc tgactcagat ggag 24
<210> 1123
   <211> 82
   <212> DNA
   <213> Artificial Sequence
<220>
   <223> Synthetic Oligonucleotide
<400> 1123
   ttacccgtgg gaactgtctc caaatacata cttcctctca ccaggacaac aaccacagga 60
   tcctctgacc catttactgg tc 82
<210> 1124
   <211> 77
   <212> DNA
   <213> Artificial Sequence
<220>
   <223> Synthetic Oligonucleotide
<400> 1124
   aaacaccact ggagcattga ctaccaggct cgccaatgat gctgctcaag ttaaaggggc 60
   tataggttcc aggcttg 77
<210> 1125
   <211> 67
   <212> DNA
   <213> Artificial Sequence
<220>
   <223> Synthetic Oligonucleotide
<400> 1125
   agacagtcgc cttggtcggt ctcaatggca gtgggaagag tacggtagtc cagcttctgc 60
   agaggtt 67
<210> 1126
   <211> 68
   <212> DNA
   <213> Artificial Sequence
<220>
   <223> Synthetic Oligonucleotide
<400> 1126
   accagtgcca caatgcagtg gctggacatt cggctacagc tcatgggggc ggcagtggtc 60
   agcgctat 68
<210> 1127
   <211> 66
   <212> DNA
   <213> Artificial Sequence
<220>
   <223> Synthetic Oligonucleotide
<400> 1127
   aagccacagc ctccattgac atggagacag acaccctgat ccagcgcaca atccgtgaag 60
   ccttcc 66
<210> 1128
   <211> 76
   <212> DNA
   <213> Artificial Sequence
<220>
   <223> Synthetic Oligonucleotide
<400> 1128
   tgcagactgt accatgctga ccattgccca tcgcctgcac acggttctag gctccgatag 60
   gattatggtg ctggcc 76
<210> 1129
   <211> 67
   <212> DNA
   <213> Artificial Sequence
<220>
   <223> Synthetic Oligonucleotide
<400> 1129
   tctgtggccc acctctactc caacctgacc aagccactcc tggacgtggc tgtgacttcc 60
   tacaccc 67
<210> 1130
   <211> 83
   <212> DNA
   <213> Artificial Sequence
<220>
   <223> Synthetic Oligonucleotide
<400> 1130
   atgtacgtcg ccattcaagc tgtgctctcc ctctatgcct ctggccgcac gacaggcatc 60
   gtcctggatt caggtgatgg cgt 83
<210> 1131
   <211> 66
   <212> DNA
   <213> Artificial Sequence
<220>
   <223> Synthetic Oligonucleotide
<400> 1131
   atccgcattg aagacccacc ccgcagaaag cacatggtat tcctgggtgg tgcagttcta 60
   gcggat 66
<210> 1132
   <211> 74
   <212> DNA
   <213> Artificial Sequence
<220>
   <223> Synthetic Oligonucleotide
<400> 1132
   caactgctga gagaccgaga agtaggaatc cctccagaac aatccttgga aactgctaag 60
   gcagtaaagg agcg 74
<210> 1133
   <211> 78
   <212> DNA
   <213> Artificial Sequence
<220>
   <223> Synthetic Oligonucleotide
<400> 1133
   ctgcatgtga ttgaataaga aacaagaaag tgaccacacc aaagcctccc tggctggtgt 60
   acagggatca ggtccaca 78
<210> 1134
   <211> 71
   <212> DNA
   <213> Artificial Sequence
<220>
   <223> Synthetic Oligonucleotide
<400> 1134
   cgcttctatg gcgctgagat tgtgtcagcc ctggactacc tgcactcgga gaagaacgtg 60
   gtgtaccggg a 71
<210> 1135
   <211> 71
   <212> DNA
   <213> Artificial Sequence
<220>
   <223> Synthetic Oligonucleotide
<400> 1135
   tcctgccacc cttcaaacct caggtcacgt ccgaggtcga cacaaggtac ttcgatgatg 60
   aatttaccgc c 71
<210> 1136
   <211> 75
   <212> DNA
   <213> Artificial Sequence
<220>
   <223> Synthetic Oligonucleotide
<400> 1136
   ttgtctctgc cttggactat ctacattccg gaaagattgt gtaccgtgat ctcaagttgg 60
   agaatctaat gctgg 75
<210> 1137
   <211> 67
   <212> DNA
   <213> Artificial Sequence
<220>
   <223> Synthetic Oligonucleotide
<400> 1137
   tgggagggat gaaggaaatt atctggacga tgctctcgtg agacaggatg cccaggacct 60
   gtatgag 67
<210> 1138
   <211> 69
   <212> DNA
   <213> Artificial Sequence
<220>
   <223> Synthetic Oligonucleotide
<400> 1138
   ggacagcagg aatgtgtttc tccatacagg tcacggggag ccaatggttc agaaacaaat 60
   cgagtgggt 69
<210> 1139
   <211> 68
   <212> DNA
   <213> Artificial Sequence
<220>
   <223> Synthetic Oligonucleotide
<400> 1139
   gatgaagcct ttcgcaagtt cctgaagggc ctggcttccc gaaagcccct tgtgctgtgt 60
   ggagacct 68
<210> 1140
   <211> 74
   <212> DNA
   <213> Artificial Sequence
<220>
   <223> Synthetic Oligonucleotide
<400> 1140
   ggaaacacac tggaggacaa ggctcgggaa ctcatcagcc gcatcaaaca gagtgaactt 60
   tctgccaaga tgcg 74
<210> 1141
   <211> 67
   <212> DNA
   <213> Artificial Sequence
<220>
   <223> Synthetic Oligonucleotide
<400> 1141
   gtttatgcca tcggcaccgt actggatcct ggccaccgac tatgagaact atgccctcgt 60
   gtattcc 67
<210> 1142
   <211> 75
   <212> DNA
   <213> Artificial Sequence
<220>
   <223> Synthetic Oligonucleotide
<400> 1142
   gcctcaagag ctggttcgag cccctggtgg aagacatgca gcgccagtgg gccgggctgg 60
   tggagaaggt gcagg 75
<210> 1143
   <211> 66
   <212> DNA
   <213> Artificial Sequence
<220>
   <223> Synthetic Oligonucleotide
<400> 1143
   gaggtcctgg agtgcgtgag cctggtggag ctgacctcgc ttaagggcag ggagaagctg 60
   gcacct 66
<210> 1144
   <211> 73
   <212> DNA
   <213> Artificial Sequence
<220>
   <223> Synthetic Oligonucleotide
<400> 1144
   ggtcctccgt cggttctctc attagtccac ggtctggtct tcagctaccc gccttcgtct 60
   ccgagtttgc gac 73
<210> 1145
   <211> 67
   <212> DNA
   <213> Artificial Sequence
<220>
   <223> Synthetic Oligonucleotide
<400> 1145
   agctgcagaa gagctgcaca tttgacgagc agcgaacagc cacgatcatg gaggagttgg 60
   cagatgc 67
<210> 1146
   <211> 66
   <212> DNA
   <213> Artificial Sequence
<220>
   <223> Synthetic Oligonucleotide
<400> 1146
   cagcagatgt ggatcagcaa gcaggagtat gacgagtccg gcccctccat cgtccaccgc 60
   aaatgc 66
<210> 1147
   <211> 73
   <212> DNA
   <213> Artificial Sequence
<220>
   <223> Synthetic Oligonucleotide
<400> 1147
   gggtcaggtg cctcgagatc gggcttgggc ccagagcatg ttccagatcc cagagtttga 60
   gccgagtgag cag 73
<210> 1148
   <211> 81
   <212> DNA
   <213> Artificial Sequence
<220>
   <223> Synthetic Oligonucleotide
<400> 1148
   cgttgtcagc acttggaata caagatggtt gccgggtcat gttaattggg aaaaagaaca 60
   gtccacagga agaggttgaa c 81
<210> 1149
   <211> 66
   <212> DNA
   <213> Artificial Sequence
<220>
   <223> Synthetic Oligonucleotide
<400> 1149
   ccattcccac cattctacct gaggccagga cgtctggggt gtggggattg gtgggtctat 60
   gttccc 66
<210> 1150
   <211> 70
   <212> DNA
   <213> Artificial Sequence
<220>
   <223> Synthetic Oligonucleotide
<400> 1150
   ccgccgtgga cacagactcc ccccgagagg tctttttccg agtggcagct gacatgtttt 60
   ctgacggcaa 70
<210> 1151
   <211> 83
   <212> DNA
   <213> Artificial Sequence
<220>
   <223> Synthetic Oligonucleotide
<400> 1151
   cctggagggt cctgtacaat ctcatcatgg gactcctgcc cttacccagg ggccacagag 60
   cccccgagat ggagcccaat tag 83
<210> 1152
   <211> 80
   <212> DNA
   <213> Artificial Sequence
<220>
   <223> Synthetic Oligonucleotide
<400> 1152
   ggctcttgtg cgtactgtcc ttcgggctgg tgacagggaa gacatcactg agcctgccat 60
   ctgtgctctt cgtcatctga 80
<210> 1153
   <211> 73
   <212> DNA
   <213> Artificial Sequence
<220>
   <223> Synthetic Oligonucleotide
<400> 1153
   cagatggacc tagtacccac tgagatttcc acgccgaagg acagcgatgg gaaaaatgcc 60
   cttaaatcat agg 73
<210> 1154
   <211> 69
   <212> DNA
   <213> Artificial Sequence
<220>
   <223> Synthetic Oligonucleotide
<400> 1154
   aattaccaag cagccgaaga ccacccacga atggttatct tacgactgtt acgttacatt 60
   gtccgcctg 69
<210> 1155
   <211> 70
   <212> DNA
   <213> Artificial Sequence
<220>
   <223> Synthetic Oligonucleotide
<400> 1155
   cagcgacaac tctggacaag tcagtccccc agagtctcca actgtgacca cttcctggca 60
   gtctgagagc 70
<210> 1156
   <211> 70
   <212> DNA
   <213> Artificial Sequence
<220>
   <223> Synthetic Oligonucleotide
<400> 1156
   cttttgtgga actctatggg aacaatgcag cagccgagag ccgaaagggc caggaacgct 60
   tcaaccgctg 70
<210> 1157
   <211> 74
   <212> DNA
   <213> Artificial Sequence
<220>
   <223> Synthetic Oligonucleotide
<400> 1157
   tgtactggcg aagaatattt ggtaaagcag ggcatcgatc tctcaccctg gggcttgtgg 60
   aagaatcacg tggc 74
<210> 1158
   <211> 71
   <212> DNA
   <213> Artificial Sequence
<220>
   <223> Synthetic Oligonucleotide
<400> 1158
   ggactgtgag gtcaacaacg gttccagcct cagggatgag tgcatcacaa acctactggt 60
   gtttggcttc c 71
<210> 1159
   <211> 76
   <212> DNA
   <213> Artificial Sequence
<220>
   <223> Synthetic Oligonucleotide
<400> 1159
   cctgcaaaag ggaacaagag cccttcgcct ccagatggct cccctgccgc cacccccgag 60
   atcagagtca accacg 76
<210> 1160
   <211> 65
   <212> DNA
   <213> Artificial Sequence
<220>
   <223> Synthetic Oligonucleotide
<400> 1160
   tcagggggct agaaatctgt tgctatgggc ccttcaccaa catgcccaca gatcaactgg 60
   aatgg                                                             65
<210> 1161
   <211> 70
   <212> DNA
   <213> Artificial Sequence
<220>
   <223> Synthetic Oligonucleotide
<400> 1161
   agttcgtgct ttgcaagatg gtgcagagct ttatgaagca gtgaagaatg cagcagaccc 60
   agcttacctt                                                        70
<210> 1162
   <211> 68
   <212> DNA
   <213> Artificial Sequence
<220>
   <223> Synthetic Oligonucleotide
<400> 1162
   ccgaggttaa tccagcacgt atggggccaa gtgtaggctc ccagcaggaa ctgagagcgc 60
   catgtctt                                                          68
<210> 1163
   <211> 82
   <212> DNA
   <213> Artificial Sequence
<220>
   <223> Synthetic Oligonucleotide
<400> 1163
   tcaacagaag gctgaaccac tagaaagact acagtcccag caccgacaat tccaagctcg 60
   agtgtctcgg caaactctgt tg                                          82
<210> 1164
   <211> 73
   <212> DNA
   <213> Artificial Sequence
<220>
   <223> Synthetic Oligonucleotide
<400> 1164
   ctgaagcaga tggttcatca tttcctgggc tgttaaacaa agcgaggtta aggttagact 60
   cttgggaatc agc 73
<210> 1165
   <211> 69
   <212> DNA
   <213> Artificial Sequence
<220>
   <223> Synthetic Oligonucleotide
<400> 1165
   gtcagcgtgg tagcggtatt ctccgcggca gtgacagtaa ttgtttttgc ctctttagcc 60
   aagacttcc 69
<210> 1166
   <211> 65
   <212> DNA
   <213> Artificial Sequence
<220>
   <223> Synthetic Oligonucleotide
<400> 1166
   tcagctgtga gctgcggata ccgcccggca atgggacctg ctcttaacct caaacctagg 60
   accgt 65
<210> 1167
   <211> 72
   <212> DNA
   <213> Artificial Sequence
<220>
   <223> Synthetic Oligonucleotide
<400> 1167
   atcctagccc tggtttttgg cctccttttt gctgtcacca gcgtcgcgtt ccttgtgcag 60
   atgagaaggc ag 72
<210> 1168
   <211> 78
   <212> DNA
   <213> Artificial Sequence
<220>
   <223> Synthetic Oligonucleotide
<400> 1168
   cactaaggtt tgagacagtt ccagaaagaa cccaagctca agacgcagga cgagctcagt 60
   tgtagagggc taattcgc 78
<210> 1169
   <211> 66
   <212> DNA
   <213> Artificial Sequence
<220>
   <223> Synthetic Oligonucleotide
<400> 1169
   tcgttggaga tcagagtgga agttcaccat cacaccacct acagcccagg tggttggcgt 60
   gcttaa 66
<210> 1170
   <211> 74
   <212> DNA
   <213> Artificial Sequence
<220>
   <223> Synthetic Oligonucleotide
<400> 1170
   gtggctcaac attgtgttcc catttcagct gatcagtggg cctccaagga ggggctgtaa 60
   aatggaggcc attg 74
<210> 1171
   <211> 84
   <212> DNA
   <213> Artificial Sequence
<220>
   <223> Synthetic Oligonucleotide
<400> 1171
   ttcaggttgt tgcaggagac catgtacatg actgtctcca ttattgatcg gttcatgcag 60
   aataattgtg tgcccaagaa gatg 84
<210> 1172
   <211> 69
   <212> DNA
   <213> Artificial Sequence
<220>
   <223> Synthetic Oligonucleotide
<400> 1172
   gcatgttcgt ggcctctaag atgaaggaga ccatccccct gacggccgag aagctgtgca 60
   tctacaccg 69
<210> 1173
   <211> 82
   <212> DNA
   <213> Artificial Sequence
<220>
   <223> Synthetic Oligonucleotide
<400> 1173
   atgctgtggc tccttcctaa ctggggcttt cttgacatgt aggttgcttg gtaataacct 60
   ttttgtatat cacaatttgg gt 82
<210> 1174
   <211> 69
   <212> DNA
   <213> Artificial Sequence
<220>
   <223> Synthetic Oligonucleotide
<400> 1174
   atccaaggcc atgactggtg tggaacaatg gccatacagg gctgttgccc aggccctaga 60
   ggtcattcc 69
<210> 1175
   <211> 66
   <212> DNA
   <213> Artificial Sequence
<220>
   <223> Synthetic Oligonucleotide
<400> 1175
   gtgtgctagc gtactcccgc ctcaaggaac tgacgctcga ggacctaaag ataaccggca 60
   ccatgc 66
<210> 1176
   <211> 75
   <212> DNA
   <213> Artificial Sequence
<220>
   <223> Synthetic Oligonucleotide
<400> 1176
   tgtatttcaa gacctctgtg cacttattta tgaacctgcc ctgctcccac agaacacagc 60
   aattcctcag gctaa 75
<210> 1177
   <211> 65
   <212> DNA
   <213> Artificial Sequence
<220>
   <223> Synthetic Oligonucleotide
<400> 1177
   agatgaagtg gaaggcgctt ttcaccgcgg ccatcctgca ggcacagttg ccgattacag 60
   aggca 65
<210> 1178
   <211> 71
   <212> DNA
   <213> Artificial Sequence
<220>
   <223> Synthetic Oligonucleotide
<400> 1178
   agtgggactg attgccgtgg gtgtcggggc acagcttgtc ctgagtcaga ccataatcca 60
   gggggctacc c 71
<210> 1179
   <211> 74
   <212> DNA
   <213> Artificial Sequence
<220>
   <223> Synthetic Oligonucleotide
<400> 1179
   tggttcccag ccctgtgtcc acctccaagc ccagattcag attcgagtca tgtacacaac 60
   ccagggtgga ggag 74
<210> 1180
   <211> 68
   <212> DNA
   <213> Artificial Sequence
<220>
   <223> Synthetic Oligonucleotide
<400> 1180
   gagagcgacg cggttgttgt agctgccgct gcggccgccg cggaataata agccgggatc 60
   taccatac 68
<210> 1181
   <211> 68
   <212> DNA
   <213> Artificial Sequence
<220>
   <223> Synthetic Oligonucleotide
<400> 1181
   tggattggag ttctgggaat gtactggccg tggcactgga caacagtgtg tacctgtgga 60
   gtgcaagc 68
<210> 1182
   <211> 85
   <212> DNA
   <213> Artificial Sequence
<220>
   <223> Synthetic Oligonucleotide
<400> 1182
   aaacgagcag tttgccatca gacgcttcca gtctatgccg gtgaggctgc tgggccacag 60
   ccccgtgctt cggaacatca ccaac 85
<210> 1183
   <211> 67
   <212> DNA
   <213> Artificial Sequence
<220>
   <223> Synthetic Oligonucleotide
<400> 1183
   gaggcacagt attgcccagc tggatccaga ggccttggga aacattaaga agctctccaa 60
   ccgtctc 67
<210> 1184
   <211> 81
   <212> DNA
   <213> Artificial Sequence
<220>
   <223> Synthetic Oligonucleotide
<400> 1184
   tgagtgtccc ccggtatctt ccccgccctg ccaatcccga tgaaattgga aattttattg 60
   atgaaaatct gaaagcggct g 81
<210> 1185
   <211> 67
   <212> DNA
   <213> Artificial Sequence
<220>
   <223> Synthetic Oligonucleotide
<400> 1185
   aagccctatc cgatgtaccc ggccacaaca tccctggtga acgtcgtgcc caaactcaat 60
   gccacag 67
<210> 1186
   <211> 69
   <212> DNA
   <213> Artificial Sequence
<220>
   <223> Synthetic Oligonucleotide
<400> 1186
   cggcgatcaa gaagctgtcc gggcctctga tctccgattt cttcgccaag cgcaagagat 60
   cagcgcctg 69
<210> 1187
   <211> 77
   <212> DNA
   <213> Artificial Sequence
<220>
   <223> Synthetic Oligonucleotide
<400> 1187
   tgacaatcag cacacctgca ttcaccgctc ggaagagggc ctgagctgca tgaataagga 60
   tcacggctgt agtcaca 77
<210> 1188
   <211> 63
   <212> DNA
   <213> Artificial Sequence
<220>
   <223> Synthetic Oligonucleotide
<400> 1188
   taaattcact cgtggtgtgg acttcaattg gcaagcccag gccctattgg ccctacaaga 60
   ggc 63
<210> 1189
   <211> 83
   <212> DNA
   <213> Artificial Sequence
<220>
   <223> Synthetic Oligonucleotide
<400> 1189
   ggatgctggt gacctcttct tccctcacgt tgcaacagga attaaaggct aaaagaaaac 60
   gaagagttac ttggtgcctt ggc 83
<210> 1190
   <211> 68
   <212> DNA
   <213> Artificial Sequence
<220>
   <223> Synthetic Oligonucleotide
<400> 1190
   ctcccgtcaa cagcgttctt tccaaacact ggaccaggag tgcatccaga tgaaggccag 60
   actcaccc 68
<210> 1191
   <211> 76
   <212> DNA
   <213> Artificial Sequence
<220>
   <223> Synthetic Oligonucleotide
<400> 1191
   ctgaaggagc tccaagacct cgctctccaa ggcgccaagg agagggcaca tcagcagaag 60
   aaacacagcg gttttg 76
<210> 1192
   <211> 76
   <212> DNA
   <213> Artificial Sequence
<220>
   <223> Synthetic Oligonucleotide
<400> 1192
   aaggaagtgg tccctctgtg gcaagtgatg aagtctccag ctttgcctca gctctcccag 60
   acagaaagac tgcgtc 76
<210> 1193
   <211> 82
   <212> DNA
   <213> Artificial Sequence
<220>
   <223> Synthetic Oligonucleotide
<400> 1193
   gataaattgg tacaagggat cagcttttcc cagcccacat gtcctgatca tatgcttttg 60
   aatagtcagt tacttggcac cc 82
<210> 1194
   <211> 78
   <212> DNA
   <213> Artificial Sequence
<220>
   <223> Synthetic Oligonucleotide
<400> 1194
   atgtggaacc cccacctact tggcgcctga agttcttgtt tctgttggga ctgctgggta 60
   taaccgtgct gtggactg 78
<210> 1195
   <211> 86
   <212> DNA
   <213> Artificial Sequence
<220>
   <223> Synthetic Oligonucleotide
<400> 1195
   ggatatttcc gtggctctta ttcaaactct ccatcaaatc ctgtaaactc cagagcaaat 60
   caagattttt ctgccttgat gagaag 86
<210> 1196
   <211> 66
   <212> DNA
   <213> Artificial Sequence
<220>
   <223> Synthetic Oligonucleotide
<400> 1196
   tcattgacca cagtggcgcc cgccttggtg agtatgagga cgtgtcccgg gtggagaagt 60
   acacga 66
<210> 1197
   <211> 76
   <212> DNA
   <213> Artificial Sequence
<220>
   <223> Synthetic Oligonucleotide
<400> 1197
   ccccaggata cctaccacta cctgcccttc agcctgcccc accggaggcc tcacttcttc 60
   tttcccaagt cccgca 76
<210> 1198
   <211> 86
   <212> DNA
   <213> Artificial Sequence
<220>
   <223> Synthetic Oligonucleotide
<400> 1198
   gacatttcca gtcctgcagt caatgcctct ctgccccacc ctttgttcag tgtggctggt 60
   gccacgacaa atgtgtgcga tcggag 86
<210> 1199
   <211> 84
   <212> DNA
   <213> Artificial Sequence
<220>
   <223> Synthetic Oligonucleotide
<400> 1199
   tccctccact cggaaggact atcctgctgc caagagggtc aagttggaca gtgtcagagt 60
   cctgagacag atcagcaaca accg 84
<210> 1200
   <211> 64
   <212> DNA
   <213> Artificial Sequence
<220>
   <223> Synthetic Oligonucleotide
<400> 1200
   tccaccctcc tggctttggc cagcatggcg aagacgaaag gaaacaaggt gaacgtggga 60
   gtga 64
<210> 1201
   <211> 68
   <212> DNA
   <213> Artificial Sequence
<220>
   <223> Synthetic Oligonucleotide
<400> 1201
   gtggccatcc agctgacctt cctgcgcctg atgtccaccg aggcctccca gaacatcacc 60
   taccactg 68
<210> 1202
   <211> 80
   <212> DNA
   <213> Artificial Sequence
<220>
   <223> Synthetic Oligonucleotide
<400> 1202
   cagccaagaa ctggtatagg agctccaagg acaagaaaca cgtctggcta ggagaaacta 60
   tcaatgctgg cagccagttt 80
<210> 1203
   <211> 66
   <212> DNA
   <213> Artificial Sequence
<220>
   <223> Synthetic Oligonucleotide
<400> 1203
   gagagcaagc gagacattct gttcctcttt gacggctcag ccaatcttgt gggccagttc 60
   cctgtt 66
<210> 1204
   <211> 81
   <212> DNA
   <213> Artificial Sequence
<220>
   <223> Synthetic Oligonucleotide
<400> 1204
   cgacagttgc gatgaaagtt ctaatctctt ccctcctcct gttgctgcca ctaatgctga 60
   tgtccatggt ctctagcagc c 81
<210> 1205
   <211> 79
   <212> DNA
   <213> Artificial Sequence
<220>
   <223> Synthetic Oligonucleotide
<400> 1205
   tctgcagagt tggaagcact ctatggtgac atcgatgctg tggagctgta tcctgccctt 60
   ctggtagaaa agcctcggc 79
<210> 1206
   <211> 78
   <212> DNA
   <213> Artificial Sequence
<220>
   <223> Synthetic Oligonucleotide
<400> 1206
   acctctgtgg tccgtaggag ccactatgag gagggccctg ggaagaattt gccattttca 60
   gtggaaaaca agtggtcg 78
<210> 1207
   <211> 67
   <212> DNA
   <213> Artificial Sequence
<220>
   <223> Synthetic Oligonucleotide
<400> 1207
   aacttcaagg tcggagaagg ctttgaggag gagaccgtgg acggacgcaa gtgcaggagt 60
   ttagcca 67
<210> 1208
   <211> 76
   <212> DNA
   <213> Artificial Sequence
<220>
   <223> Synthetic Oligonucleotide
<400> 1208
   gtgctacgcc accctgttcg gacccaaagg cgtgaacatc gggggcgcgg gctcctacat 60
   ctacgagaag cccctg 76
<210> 1209
   <211> 69
   <212> DNA
   <213> Artificial Sequence
<220>
   <223> Synthetic Oligonucleotide
<400> 1209
   gatgtgattg aggtgcatgg aaaacatgaa gagcgccagg atgaacatgg tttcatctcc 60
   agggagttc 69
<210> 1210
   <211> 74
   <212> DNA
   <213> Artificial Sequence
<220>
   <223> Synthetic Oligonucleotide
<400> 1210
   tgcagcggct gattgacagt cagatggaga cctcgtgcca aattacattt gagtttgtag 60
   accaggaaca gttg 74
<210> 1211
   <211> 72
   <212> DNA
   <213> Artificial Sequence
<220>
   <223> Synthetic Oligonucleotide
<400> 1211
   agcttttccg gaatctgttc catcgccagg gcttctccta tgactacgtg ttcgactgga 60
   acatgctcaa at 72
<210> 1212
   <211> 78
   <212> DNA
   <213> Artificial Sequence
<220>
   <223> Synthetic Oligonucleotide
<400> 1212
   tggcagaact acctgcaaga aaaaccagca cctgcgtctg gatgactgtg acttccaaac 60
   caaccacacc ttgaagca 78
<210> 1213
   <211> 80
   <212> DNA
   <213> Artificial Sequence
<220>
   <223> Synthetic Oligonucleotide
<400> 1213
   gtacatgatc ccctgtgaga aggtgtccac cctgcccgcg atcacactga agctgggagg 60
   caaaggctac aagctgtccc 80
<210> 1214
   <211> 74
   <212> DNA
   <213> Artificial Sequence
<220>
   <223> Synthetic Oligonucleotide
<400> 1214
   gggaggctta tctcactgag tgagcagaat ctggtagact gctctgggcc tcaaggcaat 60
   gaaggctgca atgg 74
<210> 1215
   <211> 67
   <212> DNA
   <213> Artificial Sequence
<220>
   <223> Synthetic Oligonucleotide
<400> 1215
   tgtctcactg agcgagcaga atctggtgga ctgttcgcgt cctcaaggca atcagggctg 60
   caatggt 67
<210> 1216
   <211> 72
   <212> DNA
   <213> Artificial Sequence
<220>
   <223> Synthetic Oligonucleotide
<400> 1216
   tgaccgcttc taccccaatg acttgtgggt ggttgtgttc cagtttcagc acatcatggt 60
   tggccttatc ct 72
<210> 1217
   <211> 77
   <212> DNA
   <213> Artificial Sequence
<220>
   <223> Synthetic Oligonucleotide
<400> 1217
   ggtgcctact ccattgtggc gggcgtgttt gtgtgcctgc tggagtaccc ccgggggaag 60
   aggaagaagg gctccac 77
<210> 1218
   <211> 71
   <212> DNA
   <213> Artificial Sequence
<220>
   <223> Synthetic Oligonucleotide
<400> 1218
   ccagctttgt gcctgtcact attcctcatg ccaccactgc caacacctct gtcttgggct 60
   accacattcc c 71
<210> 1219
   <211> 73
   <212> DNA
   <213> Artificial Sequence
<220>
   <223> Synthetic Oligonucleotide
<400> 1219
   ccgtgttcaa gaggaagctc actgccttgt ggaggagttg agaaaaacca aggcttcacc 60
   ctgtgatccc act 73
<210> 1220
   <211> 79
   <212> DNA
   <213> Artificial Sequence
<220>
   <223> Synthetic Oligonucleotide
<400> 1220
   agaacaagga caacatagat ccttacatat acacaccctt tggaagtgga cccagaaact 60
   gcattggcat gaggtttgc 79
<210> 1221
   <211> 70
   <212> DNA
   <213> Artificial Sequence
<220>
   <223> Synthetic Oligonucleotide
<400> 1221
   ccgtgtggct cgctttctgc agtgccgctt cctctttgcg gggccctggt tactcttcag 60
   cgaaatctcc 70
<210> 1222
   <211> 73
   <212> DNA
   <213> Artificial Sequence
<220>
   <223> Synthetic Oligonucleotide
<400> 1222
   cacaatggcg gctctgaaga gttggctgtc gcgcagcgta acttcattct tcaggtacag 60
   acagtgtttg tgt 73
<210> 1223
   <211> 62
   <212> DNA
   <213> Artificial Sequence
<220>
   <223> Synthetic Oligonucleotide
<400> 1223
   caagcagtca aggagaacca gaagcggcgg gagacagaag aaaagatgag gcgagcaaaa 60
   ct 62
<210> 1224
   <211> 68
   <212> DNA
   <213> Artificial Sequence
<220>
   <223> Synthetic Oligonucleotide
<400> 1224
   tccaattcca gcatcactgt ggagaaaagc tgtttgtctc cccagcatac tttatcgcct 60
   tcactgcc 68
<210> 1225
   <211> 75
   <212> DNA
   <213> Artificial Sequence
<220>
   <223> Synthetic Oligonucleotide
<400> 1225
   cagtgcttcc atggacaagt ccttgtcaaa actggcccat gctgatggag atcaaacatc 60
   aatcatccct gtcca 75
<210> 1226
   <211> 83
   <212> DNA
   <213> Artificial Sequence
<220>
   <223> Synthetic Oligonucleotide
<400> 1226
   tgcacagagg gtgtgggtta caccaatgct tccaacaatt tgtttgcttg cctcccatgt 60
   acagcttgta aatcagatga aga 83
<210> 1227
   <211> 84
   <212> DNA
   <213> Artificial Sequence
<220>
   <223> Synthetic Oligonucleotide
<400> 1227
   ctctgagaca gtgcttcgat gactttgcag acttggtgcc ctttgactcc tgggagccgc 60
   tcatgaggaa gttgggcctc atgg 84
<210> 1228
   <211> 76
   <212> DNA
   <213> Artificial Sequence
<220>
   <223> Synthetic Oligonucleotide
<400> 1228
   agacatcagc tcctggttca acgaggccat tgacttcata gactccatca agaatgctgg 60
   aggaagggtg tttgtc 76
<210> 1229
   <211> 74
   <212> DNA
   <213> Artificial Sequence
<220>
   <223> Synthetic Oligonucleotide
<400> 1229
   cagaggatga cgaggatgat gacattgacc tgtttggcag tgacaatgag gaggaggaca 60
   aggaggcggc acag 74
<210> 1230
   <211> 62
   <212> DNA
   <213> Artificial Sequence
<220>
   <223> Synthetic Oligonucleotide
<400> 1230
   tgtcgatgga cttccagaac cacctgggca gctgccaaaa gtgtgatcca agctgtccca 60
   at 62
<210> 1231
   <211> 82
   <212> DNA
   <213> Artificial Sequence
<220>
   <223> Synthetic Oligonucleotide
<400> 1231
   gatctaagat ggcgactgtc gaaccggaaa ccacccctac tcctaatccc ccgactacag 60
   aagaggagaa aacggaatct aa 82
<210> 1232
   <211> 67
   <212> DNA
   <213> Artificial Sequence
<220>
   <223> Synthetic Oligonucleotide
<400> 1232
   aagccgcggt tgaatgtgcc atgaccctct ccctctctgg atggcaccat cattgaagct 60
   ggcgtca 67
<210> 1233
   <211> 67
   <212> DNA
   <213> Artificial Sequence
<220>
   <223> Synthetic Oligonucleotide
<400> 1233
   ctcggatcct agccctcgtg cctccatgga ctcgagtgta ccgagtacag agggatattc 60
   caatgcc 67
<210> 1234
   <211> 76
   <212> DNA
   <213> Artificial Sequence
<220>
   <223> Synthetic Oligonucleotide
<400> 1234
   tggtccatcg ccagttatca catctgtatg cggaacctca aaagagtccc tggtgtgaag 60
   caagatcgct agaaca 76
<210> 1235
   <211> 81
   <212> DNA
   <213> Artificial Sequence
<220>
   <223> Synthetic Oligonucleotide
<400> 1235
   cggttatgtc atgccagata cacacctcaa aggtactccc tcctcccggg aaggcaccct 60
   ttcttcagtg ggtctcagtt c 81
<210> 1236
   <211> 86
   <212> DNA
   <213> Artificial Sequence
<220>
   <223> Synthetic Oligonucleotide
<400> 1236
   tggctcttaa tcagtttcgt tacctgcctc tggagaattt acgcattatt cgtgggacaa 60
   aactttatga ggatcgatat gccttg 86
<210> 1237
   <211> 67
   <212> DNA
   <213> Artificial Sequence
<220>
   <223> Synthetic Oligonucleotide
<400> 1237
   gtccaggtgg atgtgaaaga tccccagcag gccctcaagg agctggctaa gatgtgtatc 60
   ctggccg 67
<210> 1238
   <211> 67
   <212> DNA
   <213> Artificial Sequence
<220>
   <223> Synthetic Oligonucleotide
<400> 1238
   acggatcaca gtggaggaag cgctggctca cccctacctg gagcagtact atgacccgac 60
   ggatgag 67
<210> 1239
   <211> 70
   <212> DNA
   <213> Artificial Sequence
<220>
   <223> Synthetic Oligonucleotide
<400> 1239
   acccccagac cggatcaggc aagctggccc tcatgtcccc ttcacggtgt ttgaggaagt 60
   ctgccctaca 70
<210> 1240
   <211> 68
   <212> DNA
   <213> Artificial Sequence
<220>
   <223> Synthetic Oligonucleotide
<400> 1240
   cgtggtgccc ctctatgacc tgctgctgga gatgctggac gcccaccgcc tacatgcgcc 60
   cactagcc 68
<210> 1241
   <211> 91
   <212> DNA
   <213> Artificial Sequence
<220>
   <223> Synthetic Oligonucleotide
<400> 1241
   ggattgctca acaaccatgc tgggcatctg gaccctccta cctctggttc ttacgtctgt 60
   tgctagatta tcgtccaaaa gtgttaatgc c 91
<210> 1242
   <211> 80
   <212> DNA
   <213> Artificial Sequence
<220>
   <223> Synthetic Oligonucleotide
<400> 1242
   gcactttggg attctttcca ttatgattct ttgttacagg caccgagaat gttgtattca 60
   gtgagggtct tcttacatgc 80
<210> 1243
   <211> 66
   <212> DNA
   <213> Artificial Sequence
<220>
   <223> Synthetic Oligonucleotide
<400> 1243
   gcctcttcct gttcgacggc tcgcccacct acgtactggc ctacacccag agctaccggg 60
   caaagc 66
<210> 1244
   <211> 90
   <212> DNA
   <213> Artificial Sequence
<220>
   <223> Synthetic Oligonucleotide
<400> 1244
   ggctattcct cattttctct acaaagtggc ctcagtgaac atgaagaagg tagcctcctg 60
   gaggagaatt tcggtgacag tctacaatcc 90
<210> 1245
   <211> 67
   <212> DNA
   <213> Artificial Sequence
<220>
   <223> Synthetic Oligonucleotide
<400> 1245
   aaggaaaggg tgcctcatcc cagcaacctg tccttgtggg tgatgatcac tgtgctgctt 60
   gtggctc 67
<210> 1246
   <211> 74
   <212> DNA
   <213> Artificial Sequence
<220>
   <223> Synthetic Oligonucleotide
<400> 1246
   cacgggacat tcaccacatc gactactata aaaagacaac caacggccga ctgcctgtga 60
   agtggatggc accc 74
<210> 1247
   <211> 67
   <212> DNA
   <213> Artificial Sequence
<220>
   <223> Synthetic Oligonucleotide
<400> 1247
   ccagtggagc gcttccatga cctgcgtcct gatgaagtgg ccgatttgtt tcagacgacc 60
   cagagag 67
<210> 1248
   <211> 72
   <212> DNA
   <213> Artificial Sequence
<220>
   <223> Synthetic Oligonucleotide
<400> 1248
   ggttccagct ttctgtagct gtaagcattg gtggccacac cacctcctta caaagcaact 60
   agaacctgcg gc 72
<210> 1249
   <211> 73
   <212> DNA
   <213> Artificial Sequence
<220>
   <223> Synthetic Oligonucleotide
<400> 1249
   gcgtatgatt tcccgaatga gtcaaaatgt tgatatgccc aaacttcatg atgcaatggg 60
   tacgaggtca ctg 73
<210> 1250
   <211> 67
   <212> DNA
   <213> Artificial Sequence
<220>
   <223> Synthetic Oligonucleotide
<400> 1250
   cgagcccttt gatgacttcc tgttcccagc atcatccagg cccagtggct ctgagacagc 60
   ccgctcc 67
<210> 1251
   <211> 82
   <212> DNA
   <213> Artificial Sequence
<220>
   <223> Synthetic Oligonucleotide
<400> 1251
   ccaccccgag caaatctgtc ctccccagaa cccctgaatc ctggaggctc acgcccccag 60
   ccaaagtagg gggactggat tt 82
<210> 1252
   <211> 80
   <212> DNA
   <213> Artificial Sequence
<220>
   <223> Synthetic Oligonucleotide
<400> 1252
   ggataattca gacaacaaca ccatctttgt gcaaggcctg ggtgagaatg ttacaattga 60
   gtctgtggct gattacttca 80
<210> 1253
   <211> 69
   <212> DNA
   <213> Artificial Sequence
<220>
   <223> Synthetic Oligonucleotide
<400> 1253
   gaagcgcaga tcatgaagaa gctgaagcac gacaagctgg tccagctcta tgcagtggtg 60
   tctgaggag 69
<210> 1254
   <211> 67
   <212> DNA
   <213> Artificial Sequence
<220>
   <223> Synthetic Oligonucleotide
<400> 1254
   cctccaactc ctagcctcaa gtgatcctcc tgtctcaacc tcccaagtag gattacaagc 60
   atgcgcc 67
<210> 1255
   <211> 73
   <212> DNA
   <213> Artificial Sequence
<220>
   <223> Synthetic Oligonucleotide
<400> 1255
   gtgctggtga cgaatccaca ttcatctcaa tggaaggatc ctgccttaag tcaacttatt 60
   tgtttttgcc ggg 73
<210> 1256
   <211> 70
   <212> DNA
   <213> Artificial Sequence
<220>
   <223> Synthetic Oligonucleotide
<400> 1256
   accctcgaca agaccacact ttgggacttg ggagctgggg ctgaagttgc tctgtaccca 60
   tgaactccca 70
<210> 1257
   <211> 84
   <212> DNA
   <213> Artificial Sequence
<220>
   <223> Synthetic Oligonucleotide
<400> 1257
   aagggcaatc acagtgttaa aagaagacat gctgaaatgt tgcaggctgc tcctatgttg 60
   gaaaattctt cattgaagtt ctcc 84
<210> 1258
   <211> 74
   <212> DNA
   <213> Artificial Sequence
<220>
   <223> Synthetic Oligonucleotide
<400> 1258
   attccaccca tggcaaattc catggcaccg tcaaggctga gaacgggaag cttgtcatca 60
   atggaaatcc catc 74
<210> 1259
   <211> 75
   <212> DNA
   <213> Artificial Sequence
<220>
   <223> Synthetic Oligonucleotide
<400> 1259
   caaaggagct cactgtggtg tctgtgttcc aaccactgaa tctggacccc atctgtgaat 60
   aagccattct gactc 75
<210> 1260
   <211> 73
   <212> DNA
   <213> Artificial Sequence
<220>
   <223> Synthetic Oligonucleotide
<400> 1260
   ttgggaaata tttgggcatt ggtctggcca agtctacaat gtcccaatat caaggacaac 60
   caccctagct tct 73
<210> 1261
   <211> 83
   <212> DNA
   <213> Artificial Sequence
<220>
   <223> Synthetic Oligonucleotide
<400> 1261
   gcatgggaac catcaaccag caggccatgg accaacttca ctatgtgaca gagctgacag 60
   atcgaatcaa ggcaaactcc tca 83
<210> 1262
   <211> 71
   <212> DNA
   <213> Artificial Sequence
<220>
   <223> Synthetic Oligonucleotide
<400> 1262
   ctgttgcagg aaggcattga tcatctcctg gcccagcatg ttgctcatct ctttattaga 60
   gacccactga c 71
<210> 1263
   <211> 72
   <212> DNA
   <213> Artificial Sequence
<220>
   <223> Synthetic Oligonucleotide
<400> 1263
   cgctccagac ctatgatgac ttgttagcca aagactgcca ctgcatatga gcagtcctgg 60
   tccttccact gt 72
<210> 1264
   <211> 70
   <212> DNA
   <213> Artificial Sequence
<220>
   <223> Synthetic Oligonucleotide
<400> 1264
   ctccgacgtg gctttccagt ggcccacagc atctatggaa tcccatctgt catcaattct 60
   gccaattacg 70
<210> 1265
   <211> 67
   <212> DNA
   <213> Artificial Sequence
<220>
   <223> Synthetic Oligonucleotide
<400> 1265
   ggagctctgc agtaaccaca gaacaggccc catgctgacg tccctcctca agagctggat 60
   ggcattg 67
<210> 1266
   <211> 67
   <212> DNA
   <213> Artificial Sequence
<220>
   <223> Synthetic Oligonucleotide
<400> 1266
   ggtgaaggtt gtctcttccg agggccttct gaagacaggg ctcttgaaca gacaagtgga 60
   agggctg 67
<210> 1267
   <211> 66
   <212> DNA
   <213> Artificial Sequence
<220>
   <223> Synthetic Oligonucleotide
<400> 1267
   tacccttcca tgtgctggat ccgggactcc ctggtcagct acatcaccaa cctgggcctc 60
   ttcagc 66
<210> 1268
   <211> 67
   <212> DNA
   <213> Artificial Sequence
<220>
   <223> Synthetic Oligonucleotide
<400> 1268
   gcttatgacc gaccccaagc tcatcacctg gtctccggtg tgtcgcaacg atgttgcctg 60
   gaacttt 67
<210> 1269
   <211> 67
   <212> DNA
   <213> Artificial Sequence
<220>
   <223> Synthetic Oligonucleotide
<400> 1269
   ccatctgcat ccatcttgtt tgggctcccc acccttgaga agtgcctcag ataataccct 60
   ggtggcc 67
<210> 1270
   <211> 66
   <212> DNA
   <213> Artificial Sequence
<220>
   <223> Synthetic Oligonucleotide
<400> 1270
   gacaaggacg gcagcaaggt gacaacagtg gtggcaactc ctgggcaggg tccagacagg 60
   ccacaa 66
<210> 1271
   <211> 68
   <212> DNA
   <213> Artificial Sequence
<220>
   <223> Synthetic Oligonucleotide
<400> 1271
   gtgatcccaa gcccacaata gaggtcagtg ggaaaaagta caccgcccca cacatcctga 60
   tcgccaca 68
<210> 1272
   <211> 86
   <212> DNA
   <213> Artificial Sequence
<220>
   <223> Synthetic Oligonucleotide
<400> 1272
   aagctatgag gaaaagaagt acacgatggg ggacgctcct gattatgaca gaagccagtg 60
   gctgaatgaa aaattcaagc tgggcc 86
<210> 1273
   <211> 76
   <212> DNA
   <213> Artificial Sequence
<220>
   <223> Synthetic Oligonucleotide
<400> 1273
   gagaccctgc tgtcccagaa ccagggaggc aagaccttca ttgtgggaga ccagatctcc 60
   ttcgctgact acaacc 76
<210> 1274
   <211> 73
   <212> DNA
   <213> Artificial Sequence
<220>
   <223> Synthetic Oligonucleotide
<400> 1274
   cccactcagt agccaagtca caatgtttgg aaaacagccc gtttacttga gcaagactga 60
   taccacctgc gtg 73
<210> 1275
   <211> 66
   <212> DNA
   <213> Artificial Sequence
<220>
   <223> Synthetic Oligonucleotide
<400> 1275
   tcctgtgctc tggaagccct tgagcccttc tgggaggttc ttgtgagatc aactgagacc 60
   gtggag 66
<210> 1276
   <211> 70
   <212> DNA
   <213> Artificial Sequence
<220>
   <223> Synthetic Oligonucleotide
<400> 1276
   cggtgtgaga agtgcagcaa gccctgtgcc cgagtgtgct atggtctggg catggagcac 60
   ttgcgagagg 70
<210> 1277
   <211> 82
   <212> DNA
   <213> Artificial Sequence
<220>
   <223> Synthetic Oligonucleotide
<400> 1277
   tgaacataaa gtctgcaaca tggaaggtat tgcactgcac aggccacatt cacgtatatg 60
   ataccaacag taaccaacct ca 82
<210> 1278
   <211> 73
   <212> DNA
   <213> Artificial Sequence
<220>
   <223> Synthetic Oligonucleotide
<400> 1278
   tccaggatgt taggaactgt gaagatggaa gggcatgaaa ccagcgactg gaacagctac 60
   tacgcagaca cgc 73
<210> 1279
   <211> 67
   <212> DNA
   <213> Artificial Sequence
<220>
   <223> Synthetic Oligonucleotide
<400> 1279
   ggacgaatac gaccccacta tagaggattc ctaccggaag caggtggtca ttgatgggga 60
   gacgtgc 67
<210> 1280
   <211> 70
   <212> DNA
   <213> Artificial Sequence
<220>
   <223> Synthetic Oligonucleotide
<400> 1280
   ctgctgcgac agtccactac ctttttcgag agtgactccc gttgtcccaa ggcttcccag 60
   agcgaacctg 70
<210> 1281
   <211> 63
   <212> DNA
   <213> Artificial Sequence
<220>
   <223> Synthetic Oligonucleotide
<400> 1281
   ggtccgcttc gtctttcgag agtgactccc gcggtcccaa ggctttccag agcgaacctg 60
   tgc 63
<210> 1282
   <211> 72
   <212> DNA
   <213> Artificial Sequence
<220>
   <223> Synthetic Oligonucleotide
<400> 1282
   gcaggtgtga ttgctggact taatgtgcta agaatcatca atgagcccac ggctgctgcc 60
   attgcctatg gt 72
<210> 1283
   <211> 84
   <212> DNA
   <213> Artificial Sequence
<220>
   <223> Synthetic Oligonucleotide
<400> 1283
   ggctagtaga actggatccc aacaccaaac tcttaattag acctaggcct cagctgcact 60
   gcccgaaaag catttgggca gacc 84
<210> 1284
   <211> 70
   <212> DNA
   <213> Artificial Sequence
<220>
   <223> Synthetic Oligonucleotide
<400> 1284
   ggccactaaa gatgctggcc agatatctgg actgaatgtg cttcgggtga ttaatgagcc 60
   cacagctgct 70
<210> 1285
   <211> 84
   <212> DNA
   <213> Artificial Sequence
<220>
   <223> Synthetic Oligonucleotide
<400> 1285
   ccgactggag gagcataaaa gcgcagccga gcccagcgcc ccgcactttt ctgagcagac 60
   gtccagagca gagtcagcca gcat 84
<210> 1286
   <211> 71
   <212> DNA
   <213> Artificial Sequence
<220>
   <223> Synthetic Oligonucleotide
<400> 1286
   caaaaggcag aggctgataa gaacgacaag tctgtgaagg atctggtcat cttgctttat 60
   gaaactgcgc t 71
<210> 1287
   <211> 70
   <212> DNA
   <213> Artificial Sequence
<220>
   <223> Synthetic Oligonucleotide
<400> 1287
   agaaccgcaa ggtgagcaag gtggagattc tccagcacgt catcgactac atcagggacc 60
   ttcagttgga 70
<210> 1288
   <211> 79
   <212> DNA
   <213> Artificial Sequence
<220>
   <223> Synthetic Oligonucleotide
<400> 1288
   cacgcagaaa accacacttc tcaaaccttc actcaacact tccttcccca aagccagaag 60
   atgcacaagg aggaacatg 79
<210> 1289
   <211> 83
   <212> DNA
   <213> Artificial Sequence
<220>
   <223> Synthetic Oligonucleotide
<400> 1289
   gcatggtagc cgaagatttc acagtcaaaa tcggagattt tggtatgacg cgagatatct 60
   atgagacaga ctattaccgg aaa 83
<210> 1290
   <211> 73
   <212> DNA
   <213> Artificial Sequence
<220>
   <223> Synthetic Oligonucleotide
<400> 1290
   gtggacagca ccatgaacat gttgggcggg ggaggcagtg ctggccggaa gcccctcaag 60
   tcgggtatga agg 73
<210> 1291
   <211> 68
   <212> DNA
   <213> Artificial Sequence
<220>
   <223> Synthetic Oligonucleotide
<400> 1291
   acgcaccggg tgtctgatcc caagttccac cccctccatt caaagataat catcatcaag 60
   aaagggca 68
<210> 1292
   <211> 69
   <212> DNA
   <213> Artificial Sequence
<220>
   <223> Synthetic Oligonucleotide
<400> 1292
   tggacaagta cgggatgaag ctgccaggca tggagtacgt tgacggggac tttcagtgcc 60
   acaccttcg 69
<210> 1293
   <211> 74
   <212> DNA
   <213> Artificial Sequence
<220>
   <223> Synthetic Oligonucleotide
<400> 1293
   tctgcgtggt tcctttctca gccgcttctg actgctgatt ctcccgttca cgttgcctaa 60
   taaacatcct tcaa 74
<210> 1294
   <211> 72
   <212> DNA
   <213> Artificial Sequence
<220>
   <223> Synthetic Oligonucleotide
<400> 1294
   cctgaacctt ccaaagatgg ctgaaaaaga tggatgcttc caatctggat tcaatgagga 60
   gacttgcctg gt 72
<210> 1295
   <211> 71
   <212> DNA
   <213> Artificial Sequence
<220>
   <223> Synthetic Oligonucleotide
<400> 1295
   gcggtgattc ggaaattcgc gaattcctct ggtcctcatc caggtgcgcg ggaagcaggt 60
   gcccaggaga g 71
<210> 1296
   <211> 70
   <212> DNA
   <213> Artificial Sequence
<220>
   <223> Synthetic Oligonucleotide
<400> 1296
   aaggaaccat ctcactgtgt gtaaacatga cttccaagct ggccgtggct ctcttggcag 60
   ccttcctgat 70
<210> 1297
   <211> 68
   <212> DNA
   <213> Artificial Sequence
<220>
   <223> Synthetic Oligonucleotide
<400> 1297
   ccgctccgtc actgatgtct acctgctgaa cctagccttg gccgacctac tctttgccct 60
   gaccttgc 68
<210> 1298
   <211> 66
   <212> DNA
   <213> Artificial Sequence
<220>
   <223> Synthetic Oligonucleotide
<400> 1298
   ctcaggattt tctcgcatcc aaatgtgctc ccagtgctag gtgcctgcca gtctccacct 60
   gctcct 66
<210> 1299
   <211> 63
   <212> DNA
   <213> Artificial Sequence
<220>
   <223> Synthetic Oligonucleotide
<400> 1299
   agccatcact ctcagtgcag ccaggtccta tcgtggcccc tgaggagacc ctgactctgc 60
   agt 63
<210> 1300
   <211> 67
   <212> DNA
   <213> Artificial Sequence
<220>
   <223> Synthetic Oligonucleotide
<400> 1300
   gccaggatac tggagtccat cacagtgagc tccctgatgg ctacacccca ggaccccaag 60
   ggtcaag 67
<210> 1301
   <211> 67
   <212> DNA
   <213> Artificial Sequence
<220>
   <223> Synthetic Oligonucleotide
<400> 1301
   ggatggagtt cgcctcctac gtgatcacag acctgaccca gctgcggaag atcaagtcca 60
   tggagcg 67
<210> 1302
   <211> 74
   <212> DNA
   <213> Artificial Sequence
<220>
   <223> Synthetic Oligonucleotide
<400> 1302
   ccacagctca ccttctgtca ggtgtccatc ccagctccag ccagctccca gagaggaaga 60
   gactggcact gagg                                                   74
<210> 1303
   <211> 74
   <212> DNA
   <213> Artificial Sequence
<220>
   <223> Synthetic Oligonucleotide
<400> 1303
   tcagaattgg atttggctca tttgtggaaa agactgtgat gccttacatt agcacaacac 60
   cagctaagct cagg                                                   74
<210> 1304
   <211> 74
   <212> DNA
   <213> Artificial Sequence
<220>
   <223> Synthetic Oligonucleotide
<400> 1304
   tgaggaagaa ggagaggaat actaattatc cattcctttt ggccctgcag catgtcatgc 60
   tcccagaatt tcag                                                   74
<210> 1305
   <211> 68
   <212> DNA
   <213> Artificial Sequence
<220>
   <223> Synthetic Oligonucleotide
<400> 1305
   gaggacgaag gcctctacac ctgccaggca tgcagtgttc ttggctgtgc aaaagtggag 60
   gcattttt                                                          68
<210> 1306
   <211> 80
   <212> DNA
   <213> Artificial Sequence
<220>
   <223> Synthetic Oligonucleotide
<400> 1306
   cggactttgg gtgcgacttg acgagcggtg gttcgacaag tggccttgcg ggccggatcg 60
   tcccagtgga agagttgtaa                                              80
<210> 1307
   <211> 71
   <212> DNA
   <213> Artificial Sequence
<220>
   <223> Synthetic Oligonucleotide
<400> 1307
   tggaggttgt aagccaatgt tgtgaggctt caagttcaga catcactgag aaatcagatg 60
   gacgtaaggc a                                                       71
<210> 1308
   <211> 67
   <212> DNA
   <213> Artificial Sequence
<220>
   <223> Synthetic Oligonucleotide
<400> 1308
   ctaaggcact tgctggaagg gcagaatgcc agtgtgcttg cctatggacc cacaggagct 60
   gggaaga                                                            67
<210> 1309
   <211> 73
   <212> DNA
   <213> Artificial Sequence
<220>
   <223> Synthetic Oligonucleotide
<400> 1309
   aattcctgct ccaaaagaaa gtcttcgaag ccgctccact cgcatgtcca ctgtctcaga 60
   gcttcgcatc acg                                                     73
<210> 1310
   <211> 66
   <212> DNA
   <213> Artificial Sequence
<220>
   <223> Synthetic Oligonucleotide
<400> 1310
   ccacagggtt gaagaaccag aagccagttc ctgctgttcc tgtccagaag tctggcacat 60
   caggtg 66
<210> 1311
   <211> 78
   <212> DNA
   <213> Artificial Sequence
<220>
   <223> Synthetic Oligonucleotide
<400> 1311
   gcccagaggc tccatcgtcc atcctcttcc tccccagtcg gctgaactct ccccttgtct 60
   gcactgttca aacctctg 78
<210> 1312
   <211> 70
   <212> DNA
   <213> Artificial Sequence
<220>
   <223> Synthetic Oligonucleotide
<400> 1312
   caaacagagg gtggcagaag tgctcaatga ccctgagaac atggagaagc gcaggagccg 60
   tgagagcctc 70
<210> 1313
   <211> 72
   <212> DNA
   <213> Artificial Sequence
<220>
   <223> Synthetic Oligonucleotide
<400> 1313
   agccgaggct ttgttgaaga agcttcatat ccagtaccgg cgatcgggca cagaagctgt 60
   gctcatagcc ca 72
<210> 1314
   <211> 71
   <212> DNA
   <213> Artificial Sequence
<220>
   <223> Synthetic Oligonucleotide
<400> 1314
   atgtgccagt gagcttgagt ccttggagaa acacaagcac ctgctagaaa gtactgttaa 60
   ccaggggctc a 71
<210> 1315
   <211> 83
   <212> DNA
   <213> Artificial Sequence
<220>
   <223> Synthetic Oligonucleotide
<400> 1315
   ggcctgctga gatcaaagac tacagtccct acttcaagac cattgaggac ctgaggaaca 60
   agattctcac agccacagtg gac 83
<210> 1316
   <211> 73
   <212> DNA
   <213> Artificial Sequence
<220>
   <223> Synthetic Oligonucleotide
<400> 1316
   cgaggattgg ttcttcagca agacagagga actgaaccgc gaggtggcca ccaacagtga 60
   gctggtgcag agt 73
<210> 1317
   <211> 77
   <212> DNA
   <213> Artificial Sequence
<220>
   <223> Synthetic Oligonucleotide
<400> 1317
   tgagcggcag aatcaggagt accagcggct catggacatc aagtcgcggc tggagcagga 60
   gattgccacc taccgca 77
<210> 1318
   <211> 69
   <212> DNA
   <213> Artificial Sequence
<220>
   <223> Synthetic Oligonucleotide
<400> 1318
   tcagtggaga aggagttgga ccagtcaaca tctctgttgt cacaagcagt gtttcctctg 60
   gatatggca 69
<210> 1319
   <211> 66
   <212> DNA
   <213> Artificial Sequence
<220>
   <223> Synthetic Oligonucleotide
<400> 1319
   cttgctggcc aatgcctaca tctacgttgt ccagctgcca gccaagatcc tgactgcgga 60
   caatca 66
<210> 1320
   <211> 80
   <212> DNA
   <213> Artificial Sequence
<220>
   <223> Synthetic Oligonucleotide
<400> 1320
   actcaagcgg aaattgaagc agataggtct tatcagcaca gtctccgcct cctggattca 60
   gtgtctcggc ttcagggagt 80
<210> 1321
   <211> 67
   <212> DNA
   <213> Artificial Sequence
<220>
   <223> Synthetic Oligonucleotide
<400> 1321
   agcgatgaag atggtcgcgc cctggacgcg gttctactcc aacagctgct gcttgtgctg 60
   ccatgtc 67
<210> 1322
   <211> 67
   <212> DNA
   <213> Artificial Sequence
<220>
   <223> Synthetic Oligonucleotide
<400> 1322
   gcttcaggtg ttgtgactgc agtgcctccc tgtcgcacca gtactatgag aaggatgggc 60
   agctctt 67
<210> 1323
   <211> 66
   <212> DNA
   <213> Artificial Sequence
<220>
   <223> Synthetic Oligonucleotide
<400> 1323
   ctttgggcca ggaggaatct gttactcgaa tccacccagg aactccctgg cagtggattg 60
   tgggag 66
<210> 1324
   <211> 67
   <212> DNA
   <213> Artificial Sequence
<220>
   <223> Synthetic Oligonucleotide
<400> 1324
   agaagctgtc cctgcaagag caggatgcag cgattgtgaa gaacatgaag tctgagctgg 60
   tacggct 67
<210> 1325
   <211> 75
   <212> DNA
   <213> Artificial Sequence
<220>
   <223> Synthetic Oligonucleotide
<400> 1325
   ccgggagcag ggaatcaccc tgcgcgggag cgccgaaatc gtggccgagt tcttctcatt 60
   cggcatcaac agcat 75
<210> 1326
   <211> 76
   <212> DNA
   <213> Artificial Sequence
<220>
   <223> Synthetic Oligonucleotide
<400> 1326
   ctgtcatgtg gcagaccttc catccgaacc acggcttggg aagactacat ttggttccag 60
   gcaccagtga cattta 76
<210> 1327
   <211> 66
   <212> DNA
   <213> Artificial Sequence
<220>
   <223> Synthetic Oligonucleotide
<400> 1327
   gcccagtgga gaaattcgtc tttgagatca cccagcctcc actgctgtcc atcagctcag 60
   actcgc 66
<210> 1328
   <211> 72
   <212> DNA
   <213> Artificial Sequence
<220>
   <223> Synthetic Oligonucleotide
<400> 1328
   cctcagaaat tgccaggact tctttcccgt gatcttcagc aaagcctccg agtacttgca 60
   gctggtcttt gg 72
<210> 1329
   <211> 74
   <212> DNA
   <213> Artificial Sequence
<220>
   <223> Synthetic Oligonucleotide
<400> 1329
   aggactccag caaccaagaa gaggaggggc caagcacctt ccctgacctg gagtctgagt 60
   tccaagcagc actc 74
<210> 1330
   <211> 67
   <212> DNA
   <213> Artificial Sequence
<220>
   <223> Synthetic Oligonucleotide
<400> 1330
   cggaccacca ggtcagagcc aattcgcaga gcagggaaga gtggtacctc aacacccact 60
   acccctg 67
<210> 1331
   <211> 67
   <212> DNA
   <213> Artificial Sequence
<220>
   <223> Synthetic Oligonucleotide
<400> 1331
   gccctccaat gtccttatca acaaggaggg ccatgtgaag atgtgtgact ttggcatcag 60
   tggctac 67
<210> 1332
   <211> 72
   <212> DNA
   <213> Artificial Sequence
<220>
   <223> Synthetic Oligonucleotide
<400> 1332
   gccggtcagg cacacaaggg gcccttggag cgtggactgg ttggttttgc cattttgttg 60
   tgtgtatgct gc 72
<210> 1333
   <211> 77
   <212> DNA
   <213> Artificial Sequence
<220>
   <223> Synthetic Oligonucleotide
<400> 1333
   ccctcaaccg gcaaatccgc gaggaggtgg cgagtgcagt gagcagctcc tacaggaatg 60
   aattcagggc atggacg 77
<210> 1334
   <211> 76
   <212> DNA
   <213> Artificial Sequence
<220>
   <223> Synthetic Oligonucleotide
<400> 1334
   tgagtggaaa agcctgacct atgatgaagt catcagcttt gtgccaccac cccttgacca 60
   agaagagatg gagtcc 76
<210> 1335
   <211> 83
   <212> DNA
   <213> Artificial Sequence
<220>
   <223> Synthetic Oligonucleotide
<400> 1335
   caacacccgt acatcaatgt ctggtatgat ccttctgaag cagaagctcc accaccaaag 60
   atccctgaca agcagttaga tga 83
<210> 1336
   <211> 69
   <212> DNA
   <213> Artificial Sequence
<220>
   <223> Synthetic Oligonucleotide
<400> 1336
   gaccttggaa cctttggaac ctgctgtcaa caggtcttac agggctgctt gaaccctcat 60
   aggcctagg 69
<210> 1337
   <211> 67
   <212> DNA
   <213> Artificial Sequence
<220>
   <223> Synthetic Oligonucleotide
<400> 1337
   cacaagctga ccttccgcga gaacgccaaa gccaagacag accacggggc ggagatcgtg 60
   tacaagt 67
<210> 1338
   <211> 77
   <212> DNA
   <213> Artificial Sequence
<220>
   <223> Synthetic Oligonucleotide
<400> 1338
   cagatggcca ctttgagaac attttagctg acaacagtgt gaacgaccag accaaaatcc 60
   ttgtggttaa tgctgcc 77
<210> 1339
   <211> 75
   <212> DNA
   <213> Artificial Sequence
<220>
   <223> Synthetic Oligonucleotide
<400> 1339
   cttcggaaac tggacatcaa aaacgaagac gatgtgaaat cgttgtctcg agtgatgatc 60
   catgttttca gcgac 75
<210> 1340
   <211> 75
   <212> DNA
   <213> Artificial Sequence
<220>
   <223> Synthetic Oligonucleotide
<400> 1340
   gacttttgcc cgctaccttt cattccggcg tgacaacaat gagctgttgc tcttcatact 60
   gaagcagtta gtggc 75
<210> 1341
   <211> 82
   <212> DNA
   <213> Artificial Sequence
<220>
   <223> Synthetic Oligonucleotide
<400> 1341
   tgatggtcct atgtgtcaca ttcatcacag gtttcatacc aacacaggct tcagcacttc 60
   ctttggtgtg tttcctgtcc ca 82
<210> 1342
   <211> 71
   <212> DNA
   <213> Artificial Sequence
<220>
   <223> Synthetic Oligonucleotide
<400> 1342
   cgctcagcca gatgcaatca atgccccagt cacctgctgt tataacttca ccaataggaa 60
   gatctcagtg c 71
<210> 1343
   <211> 69
   <212> DNA
   <213> Artificial Sequence
<220>
   <223> Synthetic Oligonucleotide
<400> 1343
   gtgaaatgaa acgcaccaca ctggacagcc ctttggggaa gctggagctg tctggttgtg 60
   agcagggtc 69
<210> 1344
   <211> 78
   <212> DNA
   <213> Artificial Sequence
<220>
   <223> Synthetic Oligonucleotide
<400> 1344
   ccaacgcttg ccaaatcctg acaattcaga accagctctc tgtgacccca atttgagttt 60
   tgatgctgtc actaccgt 78
<210> 1345
   <211> 86
   <212> DNA
   <213> Artificial Sequence
<220>
   <223> Synthetic Oligonucleotide
<400> 1345
   ccatgatgga gaggcagaca tcatgatcaa ctttggccgc tgggagcatg gcgatggata 60
   cccctttgac ggtaaggacg gactcc 86
<210> 1346
   <211> 67
   <212> DNA
   <213> Artificial Sequence
<220>
   <223> Synthetic Oligonucleotide
<400> 1346
   gagaaccaat ctcaccgaca ggcagctggc agaggaatac ctgtaccgct atggttacac 60
   tcgggtg 67
<210> 1347
   <211> 78
   <212> DNA
   <213> Artificial Sequence
<220>
   <223> Synthetic Oligonucleotide
<400> 1347
   gccatgctgg ctcagtctga gctgtgggcc acatcagcta gtggctcttc tcatgcatca 60
   gttaggtggg tctgggtg 78
<210> 1348
   <211> 79
   <212> DNA
   <213> Artificial Sequence
<220>
   <223> Synthetic Oligonucleotide
<400> 1348
   tcatggtgcc cgtcaatgct gtgatggcga tgaagaccaa gacgtatcag gtggcccaca 60
   tgaagagcaa agacaatcg 79
<210> 1349
   <211> 65
   <212> DNA
   <213> Artificial Sequence
<220>
   <223> Synthetic Oligonucleotide
<400> 1349
   aggggatgac ttggacacat ctgccattcg acatgactgc aattttgaca aagccatgca 60
   gtttt 65
<210> 1350
   <211> 91
   <212> DNA
   <213> Artificial Sequence
<220>
   <223> Synthetic Oligonucleotide
<400> 1350
   tcatcctggc gatctacttc ctctggcaga acctaggtcc ctctgtcctg gctggagtcg 60
   ctttcatggt cttgctgatt ccactcaacg g 91
<210> 1351
   <211> 82
   <212> DNA
   <213> Artificial Sequence
<220>
   <223> Synthetic Oligonucleotide
<400> 1351
   tgattaccat catggctcag attggctcct atgttcctgc agaagaagcg acaattggga 60
   ttgtggatgg cattttcaca ag 82
<210> 1352
   <211> 71
   <212> DNA
   <213> Artificial Sequence
<220>
   <223> Synthetic Oligonucleotide
<400> 1352
   ggccaggatc tgtggtggta caattgactc tggccttccg agaaggtacc atcaatgtcc 60
   acgacgtgga g 71
<210> 1353
   <211> 78
   <212> DNA
   <213> Artificial Sequence
<220>
   <223> Synthetic Oligonucleotide
<400> 1353
   gaaggaatgg gaatcagtca tgagctaatc accctggaga tcagctcccg agatgtcccg 60
   gatctgactc taatagac 78
<210> 1354
   <211> 74
   <212> DNA
   <213> Artificial Sequence
<220>
   <223> Synthetic Oligonucleotide
<400> 1354
   gccgagatcg ccaagatgtt gccagggagg acagacaatg ctgtgaagaa tcactggaac 60
   tctaccatca aaag 74
<210> 1355
   <211> 85
   <212> DNA
   <213> Artificial Sequence
<220>
   <223> Synthetic Oligonucleotide
<400> 1355
   cggtacttct cagggctaat atatacgtac tctggcctct tctgcgtggt ggtcaacccc 60
   tataaacacc tgcccatcta ctcgg 85
<210> 1356
   <211> 79
   <212> DNA
   <213> Artificial Sequence
<220>
   <223> Synthetic Oligonucleotide
<400> 1356
   gtgaggcagc gcgactctgg cgactggccg gccatgcctt cccgggctga ggactatgaa 60
   gtgttgtaca ccattggca 79
<210> 1357
   <211> 73
   <212> DNA
   <213> Artificial Sequence
<220>
   <223> Synthetic Oligonucleotide
<400> 1357
   cagaccaagg agatggacct cagcgtggtg cggctcatgt ttacagcttt tcttccggat 60
   agcactggca gct 73
<210> 1358
   <211> 68
   <212> DNA
   <213> Artificial Sequence
<220>
   <223> Synthetic Oligonucleotide
<400> 1358
   ctgccgggat ggcttctatg aggctgagct ctgcccggac cgctgcatcc acagtttcca 60
   gaacctgg 68
<210> 1359
   <211> 67
   <212> DNA
   <213> Artificial Sequence
<220>
   <223> Synthetic Oligonucleotide
<400> 1359
   cactgacacc cgcaacacca cccatggttc ggactctgtg gtttcagcca gcagagagat 60
   tgcagcc 67
<210> 1360
   <211> 66
   <212> DNA
   <213> Artificial Sequence
<220>
   <223> Synthetic Oligonucleotide
<400> 1360
   ctgcttcttt cccgagcttg gaacttcgtt atccgcgatg cgtttcctgg cagctacatt 60
   cctgct 66
<210> 1361
   <211> 73
   <212> DNA
   <213> Artificial Sequence
<220>
   <223> Synthetic Oligonucleotide
<400> 1361
   ggctgtggct gaggctgtag catctctgct ggaggtgaga cactctggga actgatttga 60
   cctcgaatgc tcc 73
<210> 1362
   <211> 69
   <212> DNA
   <213> Artificial Sequence
<220>
   <223> Synthetic Oligonucleotide
<400> 1362
   cggacctgaa tgtaatgcaa gaatgaacag aacaagcaaa atgaccagct gcttagtcac 60
   ctggcaaag 69
<210> 1363
   <211> 67
   <212> DNA
   <213> Artificial Sequence
<220>
   <223> Synthetic Oligonucleotide
<400> 1363
   caaaggaaga gcaacggaag aaacgcgagc aagaacgaaa ggagaagaaa gcaaaggttt 60
   tgggaat 67
<210> 1364
   <211> 65
   <212> DNA
   <213> Artificial Sequence
<220>
   <223> Synthetic Oligonucleotide
<400> 1364
   tggagactct cagggtcgaa aacggcggca gaccagcatg acagatttct accactccaa 60
   acgcc 65
<210> 1365
   <211> 66
   <212> DNA
   <213> Artificial Sequence
<220>
   <223> Synthetic Oligonucleotide
<400> 1365
   cggtggacca cgaagagtta acccgggact tggagaagca ctgcagagac atggaagagg 60
   cgagcc 66
<210> 1366
   <211> 67
   <212> DNA
   <213> Artificial Sequence
<220>
   <223> Synthetic Oligonucleotide
<400> 1366
   tcactaccag ctgacatccg gctgcctgag ggctacctgg agaagctgac cctcaatagc 60
   cccatct 67
<210> 1367
   <211> 66
   <212> DNA
   <213> Artificial Sequence
<220>
   <223> Synthetic Oligonucleotide
<400> 1367
   ccagctctcc ttccagctac agatcaatgt ccctgtccga gtgctggagc taagtgagag 60
   ccaccc 66
<210> 1368
   <211> 66
   <212> DNA
   <213> Artificial Sequence
<220>
   <223> Synthetic Oligonucleotide
<400> 1368
   gagaagcacg ccatgaaaca ggccgtcatg gaaatgatga gtcagaagat tcagcagctc 60
   acagcc 66
<210> 1369
   <211> 74
   <212> DNA
   <213> Artificial Sequence
<220>
   <223> Synthetic Oligonucleotide
<400> 1369
   agagccagtt gctgtagaac tcaaatctct gctgggcaag gatgttctgt tcttgaagga 60
   ctgtgtaggc ccag 74
<210> 1370
   <211> 71
   <212> DNA
   <213> Artificial Sequence
<220>
   <223> Synthetic Oligonucleotide
<400> 1370
   gacattgtgg taggggaagg gactcatttt ctcatcccgt gggtacagaa accaattatc 60
   tttgactgcc g 71
<210> 1371
   <211> 83
   <212> DNA
   <213> Artificial Sequence
<220>
   <223> Synthetic Oligonucleotide
<400> 1371
   ataccaatca ccgcacaaac ccaggctatt tgttaagtcc agtcacagcg caaagaaaca 60
   tatgcggaga aaatgctagt gtg 83
<210> 1372
   <211> 67
   <212> DNA
   <213> Artificial Sequence
<220>
   <223> Synthetic Oligonucleotide
<400> 1372
   ctgctcaagg acaccgtcta cacggacttc gatgggaccc gagtgtatag ccctccagag 60
   tggatcc 67
<210> 1373
   <211> 67
   <212> DNA
   <213> Artificial Sequence
<220>
   <223> Synthetic Oligonucleotide
<400> 1373
   tggggacatt ccctttgaga gggaccagga gattctggaa gctgagctcc acttcccagc 60
   ccatgtc 67
<210> 1374
   <211> 76
   <212> DNA
   <213> Artificial Sequence
<220>
   <223> Synthetic Oligonucleotide
<400> 1374
   cccatggatg ctcctctgaa gagactttcc tcattgactg ccgaggcccc atgaatcaat 60
   gtctggtagc caccgg 76
<210> 1375
   <211> 66
   <212> DNA
   <213> Artificial Sequence
<220>
   <223> Synthetic Oligonucleotide
<400> 1375
   ccaagttctg ggtggtggac cagacccact tctacctggg cagtgccaac atggactggc 60
   gttcac 66
<210> 1376
   <211> 69
   <212> DNA
   <213> Artificial Sequence
<220>
   <223> Synthetic Oligonucleotide
<400> 1376
   aatgaataca gtattcccaa gcacatcaac cccgtggccg cctccctcat ccagaagatg 60
   cttcagaca 69
<210> 1377
   <211> 77
   <212> DNA
   <213> Artificial Sequence
<220>
   <223> Synthetic Oligonucleotide
<400> 1377
   cttacggttt tcgtggagaa gccttggggt caatttgttg tatagctgag gttttaatta 60
   caacaagaac ggctgct 77
<210> 1378
   <211> 75
   <212> DNA
   <213> Artificial Sequence
<220>
   <223> Synthetic Oligonucleotide
<400> 1378
   gatgtggact gccattcaaa ccaggaagat accggatgta aatttcgagt tttgcctcag 60
   ccaactaatc tcgca 75
<210> 1379
   <211> 66
   <212> DNA
   <213> Artificial Sequence
<220>
   <223> Synthetic Oligonucleotide
<400> 1379
   ccacataccg tccagcctat ctactggaga acgaagaaga ggagcggaac tcccgcacat 60
   gacctc 66
<210> 1380
   <211> 78
   <212> DNA
   <213> Artificial Sequence
<220>
   <223> Synthetic Oligonucleotide
<400> 1380
   gcaatcatgg aacttgacga tactctaaaa tactctttct tgcagtttga cccagcacct 60
   cgtagaggcg agccacat 78
<210> 1381
   <211> 85
   <212> DNA
   <213> Artificial Sequence
<220>
   <223> Synthetic Oligonucleotide
<400> 1381
   gcatcaggct gtcattatgg tgtccttacc tgtgggagct gtaaggtctt ctttaagagg 60
   gcaatggaag ggcagcacaa ctact 85
<210> 1382
   <211> 67
   <212> DNA
   <213> Artificial Sequence
<220>
   <223> Synthetic Oligonucleotide
<400> 1382
   aggactggga cccatgaaca ttcctttggt atcagacccg aagcgcacca ttgctcagga 60
   ttatggg 67
<210> 1383
   <211> 69
   <212> DNA
   <213> Artificial Sequence
<220>
   <223> Synthetic Oligonucleotide
<400> 1383
   ggtgtccttc gccagatcac tgttaatgat ttgcctgtgg gacgctccgt ggatgaggct 60
   ctgcggctg 69
<210> 1384
   <211> 70
   <212> DNA
   <213> Artificial Sequence
<220>
   <223> Synthetic Oligonucleotide
<400> 1384
   caagcaatgc gtcatcaatg tccccagcct ctgcggaatg gatcacactg agaagagggg 60
   gcggatttac 70
<210> 1385
   <211> 68
   <212> DNA
   <213> Artificial Sequence
<220>
   <223> Synthetic Oligonucleotide
<400> 1385
   ctgacacttg ccgcagagaa tccctttctc acccacctca tctgcacctt ccagaccaag 60
   gaccacct 68
<210> 1386
   <211> 66
   <212> DNA
   <213> Artificial Sequence
<220>
   <223> Synthetic Oligonucleotide
<400> 1386
   gggttctaga cgcccctccc aagcgttcct ggccttctga actccataca gcctctacag 60
   ccgtcc 66
<210> 1387
   <211> 68
   <212> DNA
   <213> Artificial Sequence
<220>
   <223> Synthetic Oligonucleotide
<400> 1387
   ctccacctat gagcgtctgt ctctgtgggc ttgggatgtt aacaggagcc aaaaggaggg 60
   aaagtgtg 68
<210> 1388
   <211> 86
   <212> DNA
   <213> Artificial Sequence
<220>
   <223> Synthetic Oligonucleotide
<400> 1388
   gccctcccag tgtgcaaata agggctgctg tttcgacgac accgttcgtg gggtcccctg 60
   gtgcttctat cctaatacca tcgacg 86
<210> 1389
   <211> 81
   <212> DNA
   <213> Artificial Sequence
<220>
   <223> Synthetic Oligonucleotide
<400> 1389
   tggctaagtg aagatgacaa tcatgttgca gcaattcact gtaaagctgg aaagggacga 60
   actggtgtaa tgatatgtgc a 81
<210> 1390
   <211> 81
   <212> DNA
   <213> Artificial Sequence
<220>
   <223> Synthetic Oligonucleotide
<400> 1390
   cgcttgccta actcatactt tcccgttgac acttgatcca cgcagcgtgg cactgggacg 60
   taagtggcgc agtctgaatg g 81
<210> 1391
   <211> 74
   <212> DNA
   <213> Artificial Sequence
<220>
   <223> Synthetic Oligonucleotide
<400> 1391
   ggctactctg atctatgttg ataaggaaaa tggagaacca ggcacccgtg tggttgctaa 60
   ggatgggctg aagc 74
<210> 1392
   <211> 65
   <212> DNA
   <213> Artificial Sequence
<220>
   <223> Synthetic Oligonucleotide
<400> 1392
   gggacactgc gggacaagag cggttccgga gtctcaccac tgcatttttc agagacgcca 60
   tgggc 65
<210> 1393
   <211> 67
   <212> DNA
   <213> Artificial Sequence
<220>
   <223> Synthetic Oligonucleotide
<400> 1393
   ctgaaggacc ctacgctcgg tggcctggca cctcactttg agaagagtga gcacactggc 60
   tttgcat 67
<210> 1394
   <211> 78
   <212> DNA
   <213> Artificial Sequence
<220>
   <223> Synthetic Oligonucleotide
<400> 1394
   gcgacagctc ctctagttcc accatgtccg cgggcggaga cttcgggaat ccgctgagga 60
   aattcaagct ggtgttcc 78
<210> 1395
   <211> 71
   <212> DNA
   <213> Artificial Sequence
<220>
   <223> Synthetic Oligonucleotide
<400> 1395
   gaggagtggt gacagtctgc aaaatcaata cacaggaacc tgaggagacc ctggactttg 60
   atttctgcag c 71
<210> 1396
   <211> 67
   <212> DNA
   <213> Artificial Sequence
<220>
   <223> Synthetic Oligonucleotide
<400> 1396
   agctagcctc agtgacacac atgacaggtt gcactgccga cgttgtgtca acagccgtca 60
   gatccgg 67
<210> 1397
   <211> 73
   <212> DNA
   <213> Artificial Sequence
<220>
   <223> Synthetic Oligonucleotide
<400> 1397
   cgtcgtatgc gagagtctgt ttccaggatg cctgttagtt ctcagcacag atattctaca 60
   cctcacgcct tca 73
<210> 1398
   <211> 84
   <212> DNA
   <213> Artificial Sequence
<220>
   <223> Synthetic Oligonucleotide
<400> 1398
   ggtgtcagat ataaatgtgc aaatgccttc ttgctgtcct gtcggtctca gtacgttcac 60
   tttatagctg ctggcaatat cgaa 84
<210> 1399
   <211> 67
   <212> DNA
   <213> Artificial Sequence
<220>
   <223> Synthetic Oligonucleotide
<400> 1399
   tgtggatgct ggattgattt caccactggt gcagctgcta aatagcaaag accaggaagt 60
   gctgctt 67
<210> 1400
   <211> 69
   <212> DNA
   <213> Artificial Sequence
<220>
   <223> Synthetic Oligonucleotide
<400> 1400
   agtgggagac acctgacctt tctcaagctg agattgagca gaagatcaag gagtacaatg 60
   cccagatca 69
<210> 1401
   <211> 77
   <212> DNA
   <213> Artificial Sequence
<220>
   <223> Synthetic Oligonucleotide
<400> 1401
   cgaagccctt acaagtttcc tagttcaccc ttacggattc ctggagggaa catctatatt 60
   tcacccctga agagtcc 77
<210> 1402
   <211> 66
   <212> DNA
   <213> Artificial Sequence
<220>
   <223> Synthetic Oligonucleotide
<400> 1402
   cccagtgtac gaggaacaag acagaccgag atctccaacc ggacctagca actccttcct 60
   cgctaa 66
<210> 1403
   <211> 66
   <212> DNA
   <213> Artificial Sequence
<220>
   <223> Synthetic Oligonucleotide
<400> 1403
   gggctgggtg agaatgtgat ggagaggaag aagccggccc tggtatccat tccggaggat 60
   gttgtg 66
<210> 1404
   <211> 68
   <212> DNA
   <213> Artificial Sequence
<220>
   <223> Synthetic Oligonucleotide
<400> 1404
   cctacaagtc ctggggcatt ggagccccaa gcagtgttaa tcctggctac tgtgtgagcc 60
   tgacctca 68
<210> 1405
   <211> 67
   <212> DNA
   <213> Artificial Sequence
<220>
   <223> Synthetic Oligonucleotide
<400> 1405
   gcgaggagct ctacttgctc tgcgacaagg tgcagaaaga ggacatatca gtggtgttca 60
   gcagggc 67
<210> 1406
   <211> 67
   <212> DNA
   <213> Artificial Sequence
<220>
   <223> Synthetic Oligonucleotide
<400> 1406
   aagcatgaac aggacttgac catctttcca acccctgggg aagacatttg caactgactt 60
   ggggagg 67
<210> 1407
   <211> 68
   <212> DNA
   <213> Artificial Sequence
<220>
   <223> Synthetic Oligonucleotide
<400> 1407
   cccgttcggt ctgaggaagg ccgggacatg gcgaaccgga tcagtgcctt tggctacctt 60
   gagtgctc 68
<210> 1408
   <211> 74
   <212> DNA
   <213> Artificial Sequence
<220>
   <223> Synthetic Oligonucleotide
<400> 1408
   ccagacgagc gattagaagc ggcagcttgt gaggtgaatg atttggggga agaggaggag 60
   gaggaagagg agga 74
<210> 1409
   <211> 73
   <212> DNA
   <213> Artificial Sequence
<220>
   <223> Synthetic Oligonucleotide
<400> 1409
   tgtgcacata ggaatgagct tcagatgcag ttggccagca aagagagtga tattgagcaa 60
   ttgcgtgcta aac 73
<210> 1410
   <211> 74
   <212> DNA
   <213> Artificial Sequence
<220>
   <223> Synthetic Oligonucleotide
<400> 1410
   gatctggcac tgtggttcct gcatgaagac agtggctggc ggtgcctgga cgtacaatac 60
   cacttccgct gtca 74
<210> 1411
   <211> 75
   <212> DNA
   <213> Artificial Sequence
<220>
   <223> Synthetic Oligonucleotide
<400> 1411
   ccattctatc atcaacgggt acaaacgagt cctggccttg tctgtggaga cggattacac 60
   cttcccactt gctga 75
<210> 1412
   <211> 67
   <212> DNA
   <213> Artificial Sequence
<220>
   <223> Synthetic Oligonucleotide
<400> 1412
   ctgtcttcct gttggccctg acaatcatag ccagcacctg ggctctgacg cccactcact 60
   acctcac 67
<210> 1413
   <211> 81
   <212> DNA
   <213> Artificial Sequence
<220>
   <223> Synthetic Oligonucleotide
<400> 1413
   gctcattatg aaaaacatcc caaactttaa aatgcgaaat tattggttgg tgtgaagaaa 60
   gccagacaac ttctgtttct t 81
<210> 1414
   <211> 66
   <212> DNA
   <213> Artificial Sequence
<220>
   <223> Synthetic Oligonucleotide
<400> 1414
   gctctgttgt gtcctgttgc cggctctggc cttcctgtga ctgactgtga agtggcttct 60
   ccgtac 66
<210> 1415
   <211> 71
   <212> DNA
   <213> Artificial Sequence
<220>
   <223> Synthetic Oligonucleotide
<400> 1415
   cgaggagatg cctgtggaca ggatcctgga ggcagagctt gctgtggaac agaagagtga 60
   ccagggcgtt g 71
<210> 1416
   <211> 77
   <212> DNA
   <213> Artificial Sequence
<220>
   <223> Synthetic Oligonucleotide
<400> 1416
   acaccaaaat gccatctcaa atggaacacg ccatggaaac catgatgttt acatttcaca 60
   aattcgctgg ggataaa 77
<210> 1417
   <211> 67
   <212> DNA
   <213> Artificial Sequence
<220>
   <223> Synthetic Oligonucleotide
<400> 1417
   cttctgagcc cgtctcccgg acaggttgag gaagctgctc cagaagcgcc tcggaagggg 60
   agctctc 67
<210> 1418
   <211> 86
   <212> DNA
   <213> Artificial Sequence
<220>
   <223> Synthetic Oligonucleotide
<400> 1418
   cgcgagcccc tcattataca ctgggcagcc tccccacagc gcatcgagga atgcgtgctc 60
   tcaggcaagg atgtcaacgg cgagtg 86
<210> 1419
   <211> 70
   <212> DNA
   <213> Artificial Sequence
<220>
   <223> Synthetic Oligonucleotide
<400> 1419
   gagagagcca gtctgatcca gaaggccaag ctggcagagc aggccgaacg ctatgaggac 60
   atggcagcct 70
<210> 1420
   <211> 77
   <212> DNA
   <213> Artificial Sequence
<220>
   <223> Synthetic Oligonucleotide
<400> 1420
   cagtggagac cagttgggta gtggtgactg ggtacgctac aagctctgca tgtgtgctga 60
   tgggacgctc ttcaagg 77
<210> 1421
   <211> 73
   <212> DNA
   <213> Artificial Sequence
<220>
   <223> Synthetic Oligonucleotide
<400> 1421
   tccgcaagac acctcctgga gggcctcctg cagaaggaca ggacaaagcg gctcggggcc 60
   aaggatgact tca 73
<210> 1422
   <211> 71
   <212> DNA
   <213> Artificial Sequence
<220>
   <223> Synthetic Oligonucleotide
<400> 1422
   tgcattcgtt ggtttctctt atgcacctcc ttcagaagac ttatttttgt gagcagtttg 60
   ccattcagaa a 71
<210> 1423
   <211> 71
   <212> DNA
   <213> Artificial Sequence
<220>
   <223> Synthetic Oligonucleotide
<400> 1423
   ccaacacctt cttggcttct gggacctgtg ttcttgctga gcaccctctc cggtttgggt 60
   tgggataaca g 71
<210> 1424
   <211> 72
   <212> DNA
   <213> Artificial Sequence
<220>
   <223> Synthetic Oligonucleotide
<400> 1424
   agctggggtg tctgtttcat gtggaatacc tgacttcagg tcaagggatg gtatttatgc 60
   tcgccttgct gt 72
<210> 1425
   <211> 67
   <212> DNA
   <213> Artificial Sequence
<220>
   <223> Synthetic Oligonucleotide
<400> 1425
   gtggggagcc catcatctcg ccaagccatc acaggctctg catacacatg cactcagtgt 60
   ggactgg 67
<210> 1426
   <211> 64
   <212> DNA
   <213> Artificial Sequence
<220>
   <223> Synthetic Oligonucleotide
<400> 1426
   tctgccagtg ctgaattctt tgctgacatt gccctggctc ctatggaagc tgctaaggtt 60
   cgaa 64
<210> 1427
   <211> 67
   <212> DNA
   <213> Artificial Sequence
<220>
   <223> Synthetic Oligonucleotide
<400> 1427
   cccaactcag gtccttggta aatcctgcaa gccaatccca gtcatgctcc ttggggtgac 60
   cctcttg 67
<210> 1428
   <211> 70
   <212> DNA
   <213> Artificial Sequence
<220>
   <223> Synthetic Oligonucleotide
<400> 1428
   agatgcatac ttggaagcac agtcatatca cactgggagg caatgcaatg tggttacctg 60
   gtcctaggtt 70
<210> 1429
   <211> 70
   <212> DNA
   <213> Artificial Sequence
<220>
   <223> Synthetic Oligonucleotide
<400> 1429
   gcgcagaggc cagttaaagt agatcacctc ctcgaaccca ctccggttcc ccgcaaccca 60
   cagctcagct 70
<210> 1430
   <211> 76
   <212> DNA
   <213> Artificial Sequence
<220>
   <223> Synthetic Oligonucleotide
<400> 1430
   ggctggccaa acataagcag ctgcactgcg atgcccagtc tagaaaatct ttcagctgta 60
   aatactgtga caagga 76
<210> 1431
   <211> 66
   <212> DNA
   <213> Artificial Sequence
<220>
   <223> Synthetic Oligonucleotide
<400> 1431
   agtgacaaat gttggaggag cagtggtgac gggtgtgaca gcagtagccc agaagacagt 60
   ggaggg 66
<210> 1432
   <211> 80
   <212> DNA
   <213> Artificial Sequence
<220>
   <223> Synthetic Oligonucleotide
<400> 1432
   acccaccatg gatgtcttca agaagggctt ctccatcgcc aaggagggcg tggtgggtgc 60
   ggtggaaaag accaagcagg 80
<210> 1433
   <211> 70
   <212> DNA
   <213> Artificial Sequence
<220>
   <223> Synthetic Oligonucleotide
<400> 1433
   tgaagagagg catgttggag acttgggcaa tgtgactgct gacaaagatg gtgtggccga 60
   tgtgtctatt 70
<210> 1434
   <211> 74
   <212> DNA
   <213> Artificial Sequence
<220>
   <223> Synthetic Oligonucleotide
<400> 1434
   tgaggagtgg tattttggca agatcaccag acgggagtca gagcggttac tgctcaatgc 60
   agagaacccg agag 74
<210> 1435
   <211> 74
   <212> DNA
   <213> Artificial Sequence
<220>
   <223> Synthetic Oligonucleotide
<400> 1435
   atacagcacc aatggaaaga tcaccttcga cgactacatc gcctgctgcg tcaaactgag 60
   ggctcttaca gaca 74
<210> 1436
   <211> 81
   <212> DNA
   <213> Artificial Sequence
<220>
   <223> Synthetic Oligonucleotide
<400> 1436
   gggctcagct ttcagaagtg ctgagttggc agttttcttc tgtcaccaaa agaggtctca 60
   atgtggacca gctgaacatg t 81
<210> 1437
   <211> 70
   <212> DNA
   <213> Artificial Sequence
<220>
   <223> Synthetic Oligonucleotide
<400> 1437
   tcacatgcca ctttggtgtt tcataatctc ctgggagaga ttgaccagca gtatagccgc 60
   ttcctgcaag 70
<210> 1438
   <211> 76
   <212> DNA
   <213> Artificial Sequence
<220>
   <223> Synthetic Oligonucleotide
<400> 1438
   caagagggac tcggactgca gcagcctctg cacctctgag agcatggact atggtaccaa 60
   tctctccact gacctg 76
<210> 1439
   <211> 66
   <212> DNA
   <213> Artificial Sequence
<220>
   <223> Synthetic Oligonucleotide
<400> 1439
   ggactcggag acgctgtgca ggagggccgt caagatcctc aagaagaaga agttgcgaag 60
   gatccc 66
<210> 1440
   <211> 69
   <212> DNA
   <213> Artificial Sequence
<220>
   <223> Synthetic Oligonucleotide
<400> 1440
   catcttccag gaggaccact ctctgtggca ccctggacta cctgccccct gaaatgattg 60
   aaggtcgga 69
<210> 1441
   <211> 71
   <212> DNA
   <213> Artificial Sequence
<220>
   <223> Synthetic Oligonucleotide
<400> 1441
   aatacccaac gcacaaatga ccgcacgttc tctgccccgt ttcttgcccc agtgtggttt 60
   gcattgtctc c 71
<210> 1442
   <211> 90
   <212> DNA
   <213> Artificial Sequence
<220>
   <223> Synthetic Oligonucleotide
<400> 1442
   cctggaggct gcaacatacc tcaatcctgt cccaggccgg atcctcctga agcccttttc 60
   gcagcactgc tatcctccaa agccattgta 90
<210> 1443
   <211> 80
   <212> DNA
   <213> Artificial Sequence
<220>
   <223> Synthetic Oligonucleotide
<400> 1443
   tgttttgatt cccgggctta ccaggtgaga agtgagggag gaagaaggca gtgtcccttt 60
   tgctagagct gacagctttg 80
<210> 1444
   <211> 65
   <212> DNA
   <213> Artificial Sequence
<220>
   <223> Synthetic Oligonucleotide
<400> 1444
   cacccttgga ctggaaaact cacacccggt ctggacacag aaagagaacc aacagctcat 60
   caagg 65
<210> 1445
   <211> 66
   <212> DNA
   <213> Artificial Sequence
<220>
   <223> Synthetic Oligonucleotide
<400> 1445
   gatcctcgcg tgagacagat caagatcaag accggcgtgg tgaagcggtt ggtcaaagaa 60
   aaagtg 66
<210> 1446
   <211> 68
   <212> DNA
   <213> Artificial Sequence
<220>
   <223> Synthetic Oligonucleotide
<400> 1446
   ctgttttcct ggaggactgc agtgactgcg tgctggcagt ggcctgccaa cagctccgca 60
   tacacagt 68
<210> 1447
   <211> 66
   <212> DNA
   <213> Artificial Sequence
<220>
   <223> Synthetic Oligonucleotide
<400> 1447
   cagccaggtg tacgagacat tgctcaccta cagtgacgtc gtgggcgcgg atgtgctgga 60
   cgaggt 66
<210> 1448
   <211> 66
   <212> DNA
   <213> Artificial Sequence
<220>
   <223> Synthetic Oligonucleotide
<400> 1448
   tcccgagaga ggaaagcatg atgggagcca cgaagggact gtgtatttta aatgcaggca 60
   cccgac 66
<210> 1449
   <211> 74
   <212> DNA
   <213> Artificial Sequence
<220>
   <223> Synthetic Oligonucleotide
<400> 1449
   cctggttgaa gcctgttaat gctttcaacg tgtggaaaac ccagcgggcc tttagcaaat 60
   atgagaagtc tgca 74
<210> 1450
   <211> 68
   <212> DNA
   <213> Artificial Sequence
<220>
   <223> Synthetic Oligonucleotide
<400> 1450
   ccagtgtgtg taacagggtc acaagaattc gacagccaga tgctccaaga gggtggccca 60
   aggctata 68
<210> 1451
   <211> 68
   <212> DNA
   <213> Artificial Sequence
<220>
   <223> Synthetic Oligonucleotide
<400> 1451
   gccaactgct ttcatttgtg agggatctga accaatacag agcagacata aaggaaatgg 60
   gcctgagt 68
<210> 1452
   <211> 85
   <212> DNA
   <213> Artificial Sequence
<220>
   <223> Synthetic Oligonucleotide
<400> 1452
   catccgcaaa gtgactgaag agaacaaaga gttggccaat gagctgaggc ggcctcccct 60
   atgctatcac aacggagttc agtac 85
<210> 1453
   <211> 84
   <212> DNA
   <213> Artificial Sequence
<220>
   <223> Synthetic Oligonucleotide
<400> 1453
   gccgggaaga ccgtaattgt ggctgcactg gatgggacct tccagaggaa gccatttggg 60
   gccatcctga acctggtgcc gctg 84
<210> 1454
   <211> 72
   <212> DNA
   <213> Artificial Sequence
<220>
   <223> Synthetic Oligonucleotide
<400> 1454
   aatccaaggg ggagagtgat gacttccata tggactttga ctcagctgtg gctcctcggg 60
   caaaatctgt ac                                                      72
<210> 1455
   <211> 66
   <212> DNA
   <213> Artificial Sequence
<220>
   <223> Synthetic Oligonucleotide
<400> 1455
   gtgatgcctt ccctgtgggc gaggtgaaga tgctggagaa gcagacgaac ccacccgact 60
   acctga                                                             66
<210> 1456
   <211> 82
   <212> DNA
   <213> Artificial Sequence
<220>
   <223> Synthetic Oligonucleotide
<400> 1456
   ctatatgcag ccagagatgt gacagccacc gtggacagcc tgccactcat cacagcctcc 60
   attctcagta agaaactcgt gg                                           82
<210> 1457
   <211> 74
   <212> DNA
   <213> Artificial Sequence
<220>
   <223> Synthetic Oligonucleotide
<400> 1457
   tgctgttgct gagtctgttg ccagtcccca gaagaccatg tctgtgttga gctgtatctg 60
   tgaagccagg caag                                                    74
<210> 1458
   <211> 81
   <212> DNA
   <213> Artificial Sequence
<220>
   <223> Synthetic Oligonucleotide
<400> 1458
   ggtgtcgata ttgtcatgaa ccatcacctg caggaaacaa gtttcacaaa agaagcctac 60
   aagaagtaca tcaaagatta c 81
<210> 1459
   <211> 66
   <212> DNA
   <213> Artificial Sequence
<220>
   <223> Synthetic Oligonucleotide
<400> 1459
   gacgctggtc tggtgaagat gtccaggaaa ccacgagcct ccagcccatt gtccaacaac 60
   caccca 66
<210> 1460
   <211> 73
   <212> DNA
   <213> Artificial Sequence
<220>
   <223> Synthetic Oligonucleotide
<400> 1460
   cttcacagtg ctcctgcagt ctctctgtgt ggctgtaact tacgtgtact ttaccaacga 60
   gctgaagcag atg 73
<210> 1461
   <211> 65
   <212> DNA
   <213> Artificial Sequence
<220>
   <223> Synthetic Oligonucleotide
<400> 1461
   gcctcggtgt gcctttcaac atcgccagct acgccctgct cacgtacatg attgcgcaca 60
   tcacg 65
<210> 1462
   <211> 66
   <212> DNA
   <213> Artificial Sequence
<220>
   <223> Synthetic Oligonucleotide
<400> 1462
   ctggtcagcc ttcagggacc ctgagcaccg cctggtctct ttcctgtggc cagcccagaa 60
   ctgaag 66
<210> 1463
   <211> 76
   <212> DNA
   <213> Artificial Sequence
<220>
   <223> Synthetic Oligonucleotide
<400> 1463
   tgcttgtcag ttgtcaacac cttatggcca acctgcctgt ttccagcagc aacagcatca 60
   aatacttgcc actcca 76
<210> 1464
   <211> 68
   <212> DNA
   <213> Artificial Sequence
<220>
   <223> Synthetic Oligonucleotide
<400> 1464
   tgtcaccccg actcaacgtg agacgcaccg cccggactca ccatgcgtga atgcatctca 60
   gtccacgt 68
<210> 1465
   <211> 62
   <212> DNA
   <213> Artificial Sequence
<220>
   <223> Synthetic Oligonucleotide
<400> 1465
   agctcaacat gcgtgagtgt atctctatcc acgtggggca ggcaggagtc cagatcggca 60
   at 62
<210> 1466
   <211> 71
   <212> DNA
   <213> Artificial Sequence
<220>
   <223> Synthetic Oligonucleotide
<400> 1466
   ctcttacatc gaccgcctaa gagtcgcgct gtaagaagca acaacctctc ctcttcgtct 60
   ccgccatcag c 71
<210> 1467
   <211> 71
   <212> DNA
   <213> Artificial Sequence
<220>
   <223> Synthetic Oligonucleotide
<400> 1467
   gaggagggtg agttctccaa ggcccatgag gatatgactg ccctggagaa ggattacaag 60
   gaggtgggca t 71
<210> 1468
   <211> 71
   <212> DNA
   <213> Artificial Sequence
<220>
   <223> Synthetic Oligonucleotide
<400> 1468
   gtcccttcgc ctccttcacc gccgcagacc ccttcaagtt ctagtcatgc gtgagtgcat 60
   ctccatccac g 71
<210> 1469
   <211> 68
   <212> DNA
   <213> Artificial Sequence
<220>
   <223> Synthetic Oligonucleotide
<400> 1469
   cgccctacct ataccaacct caaccgcctc atcagtcaga ttgtgtcctc aatcactgct 60
   tctctccg 68
<210> 1470
   <211> 73
   <212> DNA
   <213> Artificial Sequence
<220>
   <223> Synthetic Oligonucleotide
<400> 1470
   cgaggacgag gcttaaaaac ttctcagatc aatcgtgcat ccttagtgaa cttctgttgt 60
   cctcaagcat ggt 73
<210> 1471
   <211> 70
   <212> DNA
   <213> Artificial Sequence
<220>
   <223> Synthetic Oligonucleotide
<400> 1471
   cgccctcctg cagtatttat ggcctcgtcc tcccccacct aggccacgtg tgagctgctc 60
   ctgtctctgt 70
<210> 1472
   <211> 84
   <212> DNA
   <213> Artificial Sequence
<220>
   <223> Synthetic Oligonucleotide
<400> 1472
   acactgactg gcatcctgct ttccagtgcc tgccagcctc cagaagagcc aggtgcctga 60
   ctagtacatg gggagctaca gagc 84
<210> 1473
   <211> 70
   <212> DNA
   <213> Artificial Sequence
<220>
   <223> Synthetic Oligonucleotide
<400> 1473
   gtggcctaga gccttcagtc actggggaaa gcagggaagc agtgtgaact ctttattcac 60
   tcccagcctg 70
<210> 1474
   <211> 73
   <212> DNA
   <213> Artificial Sequence
<220>
   <223> Synthetic Oligonucleotide
<400> 1474
   acaggcccca tgcatcctcc ctgcctcact cccctcagcc cctgccgacc ttagcttatc 60
   tgggagagaa aca 73
<210> 1475
   <211> 80
   <212> DNA
   <213> Artificial Sequence
<220>
   <223> Synthetic Oligonucleotide
<400> 1475
   ccctatggcc ctgaatggtg cactggttta attgtgttgg tgtcggcccc tcacaaatgc 60
   agccaagtca tgtaattagt 80
<210> 1476
   <211> 75
   <212> DNA
   <213> Artificial Sequence
<220>
   <223> Synthetic Oligonucleotide
<400> 1476
   agtggaatcc ttccctttcc aactctacct ccctcactca gctcctttcc cctgatcaga 60
   gaaagggatc aaggg 75
<210> 1477
   <211> 75
   <212> DNA
   <213> Artificial Sequence
<220>
   <223> Synthetic Oligonucleotide
<400> 1477
   ggaaggaaag aagcatggtc tactttaggt gtgcgctggg tctctggtgc tcttcactgt 60
   tgcctgtcac ttttt 75
<210> 1478
   <211> 67
   <212> DNA
   <213> Artificial Sequence
<220>
   <223> Synthetic Oligonucleotide
<400> 1478
   gatgccgagg gaaatcatca ccctacagtt gggccagtgc ggcaatcaga ttgggttcga 60
   gttctgg 67
<210> 1479
   <211> 64
   <212> DNA
   <213> Artificial Sequence
<220>
   <223> Synthetic Oligonucleotide
<400> 1479
   gcgctgcgga agatcatccc cacgctgccc tcggacaagc tgagcaagat tcagaccctc 60
   aagc 64
<210> 1480
   <211> 67
   <212> DNA
   <213> Artificial Sequence
<220>
   <223> Synthetic Oligonucleotide
<400> 1480
   cagtgtggag gggatggagg agcctgacat ccagtgggtg aaggatgggg ctgtggtcca 60
   gaacttg 67
<210> 1481
   <211> 69
   <212> DNA
   <213> Artificial Sequence
<220>
   <223> Synthetic Oligonucleotide
<400> 1481
   agttgtcgtg tgttctggat tcattccggc accaccatgt cgaaggtttc ctttaagatc 60
   acgctgacg 69
<210> 1482
   <211> 70
   <212> DNA
   <213> Artificial Sequence
<220>
   <223> Synthetic Oligonucleotide
<400> 1482
   gtggatgtgc cctgaaggac aagccaggcg tctacacgag agtctcacac ttcttaccct 60
   ggatccgcag 70
<210> 1483
   <211> 89
   <212> DNA
   <213> Artificial Sequence
<220>
   <223> Synthetic Oligonucleotide
<400> 1483
   tggcttcagg agctgaatac cctcccaggc acacacaggt gggacacaaa taagggtttt 60
   ggaaccacta ttttctcatc acgacagca 89
<210> 1484
   <211> 71
   <212> DNA
   <213> Artificial Sequence
<220>
   <223> Synthetic Oligonucleotide
<400> 1484
   ctgctgtctt gggtgcattg gagccttgcc ttgctgctct acctccacca tgccaagtgg 60
   tcccaggctg c 71
<210> 1485
   <211> 71
   <212> DNA
   <213> Artificial Sequence
<220>
   <223> Synthetic Oligonucleotide
<400> 1485
   tgacgatggc ctggagtgtg tgcccactgg gcagcaccaa gtccggatgc agatcctcat 60
   gatccggtac c 71
<210> 1486
   <211> 83
   <212> DNA
   <213> Artificial Sequence
<220>
   <223> Synthetic Oligonucleotide
<400> 1486
   cctcagcaag acgttatttg aaattacagt gcctctctct caaggcccca aaccagtaac 60
   aatcagtttt gccaatcaca ctt 83
<210> 1487
   <211> 67
   <212> DNA
   <213> Artificial Sequence
<220>
   <223> Synthetic Oligonucleotide
<400> 1487
   cggttggtga cttgtctgcc tcctgctttg ggaagactga ggcatccgtg aggcagggac 60
   aagtctt 67
<210> 1488
   <211> 72
   <212> DNA
   <213> Artificial Sequence
<220>
   <223> Synthetic Oligonucleotide
<400> 1488
   tgcccttaaa ggaaccaatg agtccctgga acgccagatg cgtgaaatgg aagagaactt 60
   tgccgttgaa gc 72
<210> 1489
   <211> 84
   <212> DNA
   <213> Artificial Sequence
<220>
   <223> Synthetic Oligonucleotide
<400> 1489
   ggttgtgctc tacgagctta tgactggctc actgccttac agccacattg gctgccgtga 60
   ccagattatc tttatggtgg gccg 84
<210> 1490
   <211> 68
   <212> DNA
   <213> Artificial Sequence
<220>
   <223> Synthetic Oligonucleotide
<400> 1490
   ctctccagtg tgggcaccag ccggccagaa cagatgcgag cagtccatga ctctgggagc 60
   tacaccgc 68
<210> 1491
   <211> 75
   <212> DNA
   <213> Artificial Sequence
<220>
   <223> Synthetic Oligonucleotide
<400> 1491
   gtatcaggac cacatgcagt acatcggaga aggcgcgaag acaggcatca aagaatgcca 60
   gtatcaattc cgaca 75
<210> 1492
   <211> 77
   <212> DNA
   <213> Artificial Sequence
<220>
   <223> Synthetic Oligonucleotide
<400> 1492
   gcagttggaa gacacaggaa agtatcccca aattgcagat ttatcaacgg cttttatctt 60
   gaaaatagtg ccacgca 77
<210> 1493
   <211> 75
   <212> DNA
   <213> Artificial Sequence
<220>
   <223> Synthetic Oligonucleotide
<400> 1493
   caggtcaggc agaggaagtc atgtgcattg catgagctaa acctatctga atgaattgat 60
   ttggggcttg ttagg 75
<210> 1494
   <211> 66
   <212> DNA
   <213> Artificial Sequence
<220>
   <223> Synthetic Oligonucleotide
<400> 1494
   tggtcagatg ctgctgaagt atatccttag gccgctggca tcttgcccat cccttcatgc 60
   tgtgat                                                            66
<210> 1495
   <211> 66
   <212> DNA
   <213> Artificial Sequence
<220>
   <223> Synthetic Oligonucleotide
<400> 1495
   gagggagcag acagtgggta ccacgatctc cgtaaccatt tgcatgtgac ttagcaaggg 60
   ctctga                                                            66
<210> 1496
   <211> 66
   <212> DNA
   <213> Artificial Sequence
<220>
   <223> Synthetic Oligonucleotide
<400> 1496
   tagaggccat caaaattggc ctcaccaagg ccctgactca gatggaggaa gcccagagga 60
   aacgga                                                            66

## Description

This application claims priority benefit of United States Provisional Application Serial Nos. 61/052,573, filed May 12, 2008, and 61/057,182 filed May 29, 2008.

The present invention provides genes and gene sets, the expression levels of which are useful for predicting response of cancer patients to chemotherapy. The invention further concerns tests using such molecular markers, arrays and kits for use in such methods, and reports comprising the results and/or conclusions of such tests.

For many patients with cancer, treatment may include surgical resection of the tumour, hormonal therapy, and chemotherapy. A range of chemotherapy choices are available. Ideally, the choice for an individual patient takes into account both the risk of cancer recurrence and the likelihood that the patient will respond to the chemotherapy chosen.
One critical issue in treatment of breast cancer is the identification of which patients are likely to respond to a standard chemotherapy (e.g. an anthracycline and a cyclophosphamide) and which patients are less likely to respond to standard chemotherapy and should therefore be considered for more aggressive chemotherapy (e.g., a chemotherapy regimen that includes a taxane). Currently, no satisfactory tests are available for identifying patients more likely to respond to standard chemotherapy as opposed to treatment with a taxane-containing treatment regimen. The present disclosure provides methods and compositions to facilitate prediction of the likelihood of responsiveness of cancer patients to treatment including a taxane and/or a cyclophosphamide.

According to a first aspect of the present invention, there is provided a method of predicting whether a breast cancer patient will exhibit a beneficial response to chemotherapy as specified in claim 1.

According to a second aspect of the present invention, there is provided a method of predicting whether a breast cancer patient will exhibit a beneficial response to chemotherapy as specified in claim 2.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumour sample can be a formalin-fixed and paraffin-embedded (FPE) or a frozen tumour section.

Various aspects and embodiments will be apparent from the following discussion, including the Examples. Such additional embodiments, without limitation, include any and all of the ESR1 gene combinations discussed and/or specifically listed in Example 2.

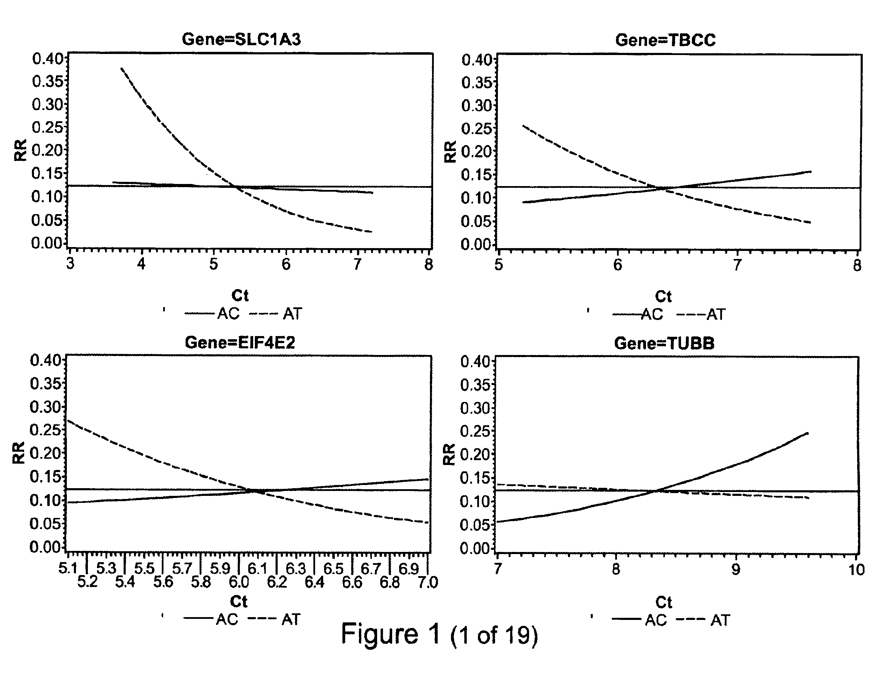

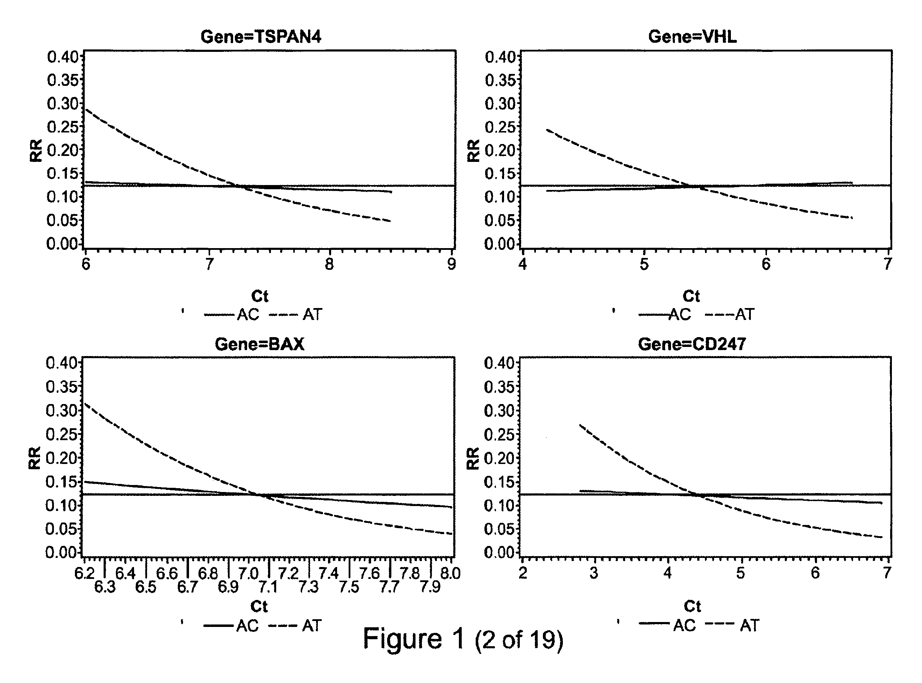

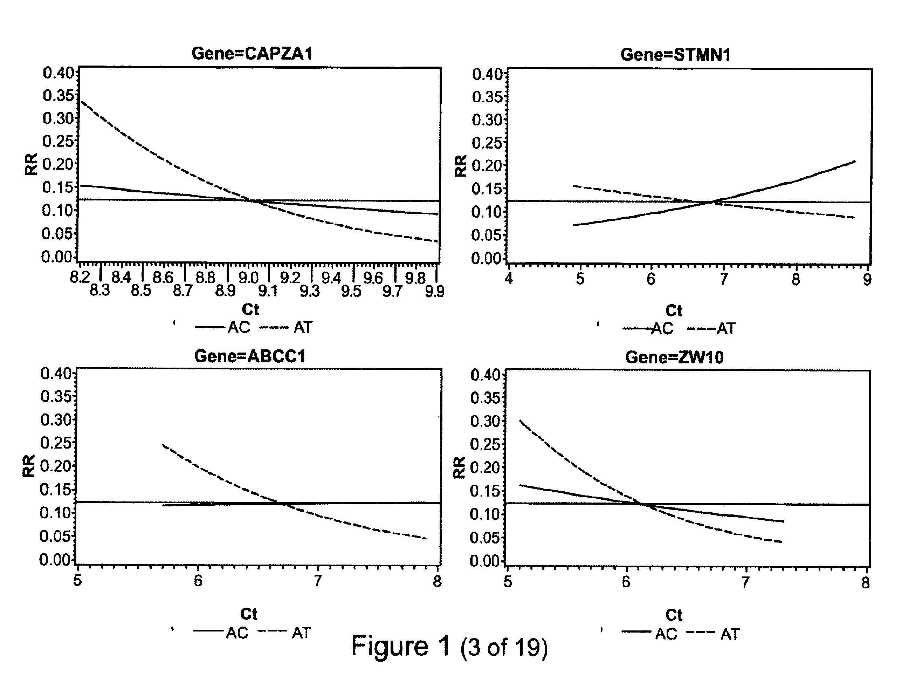

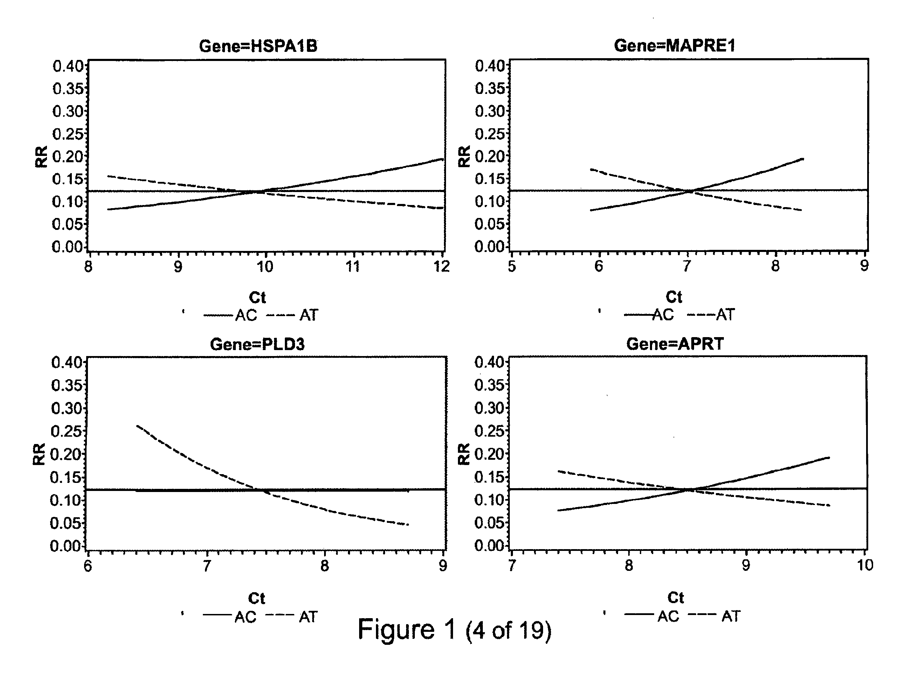

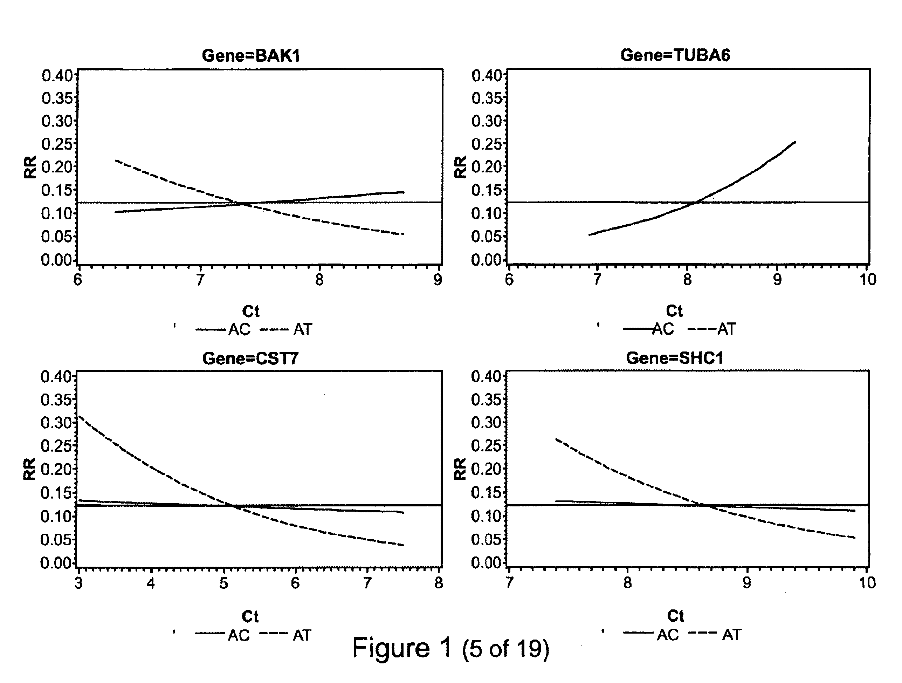

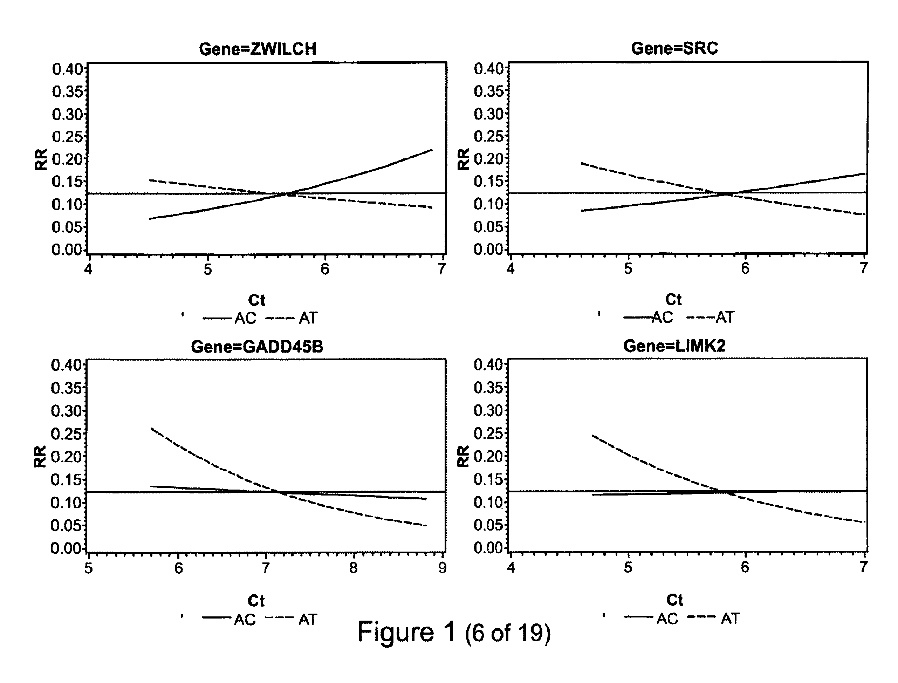

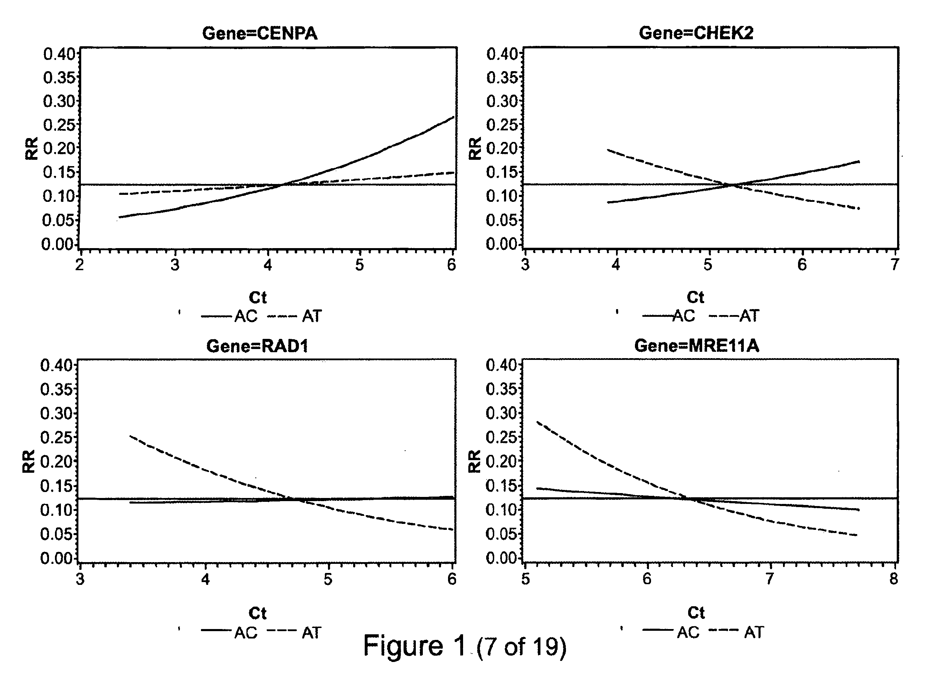

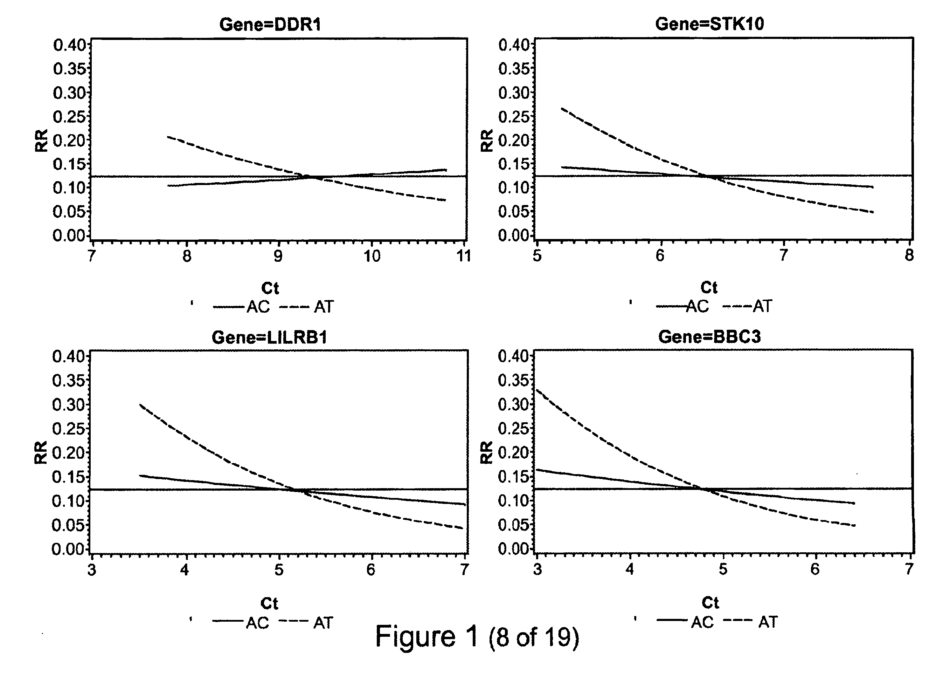

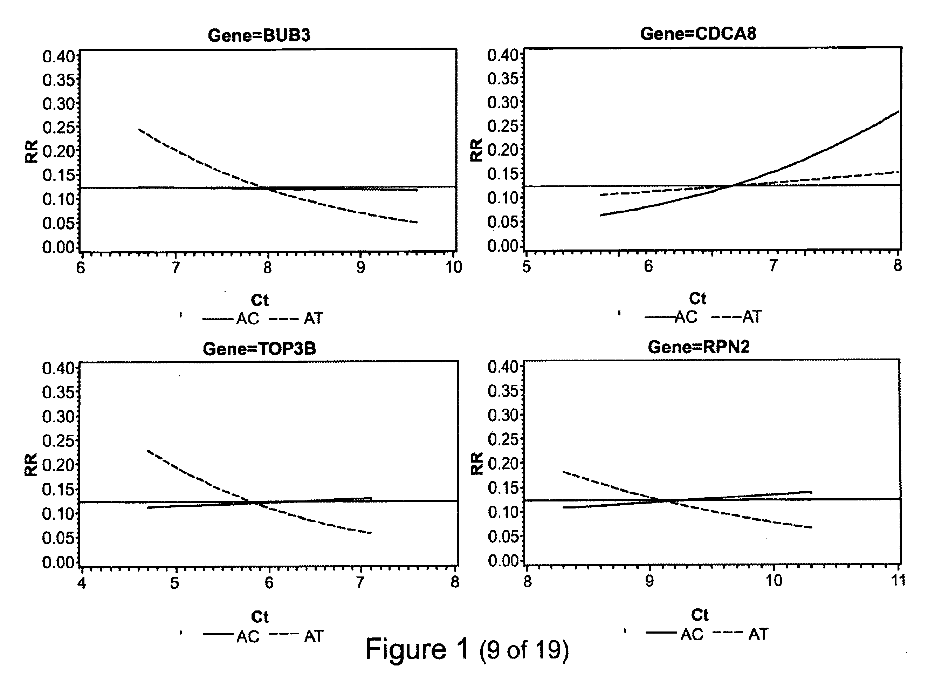

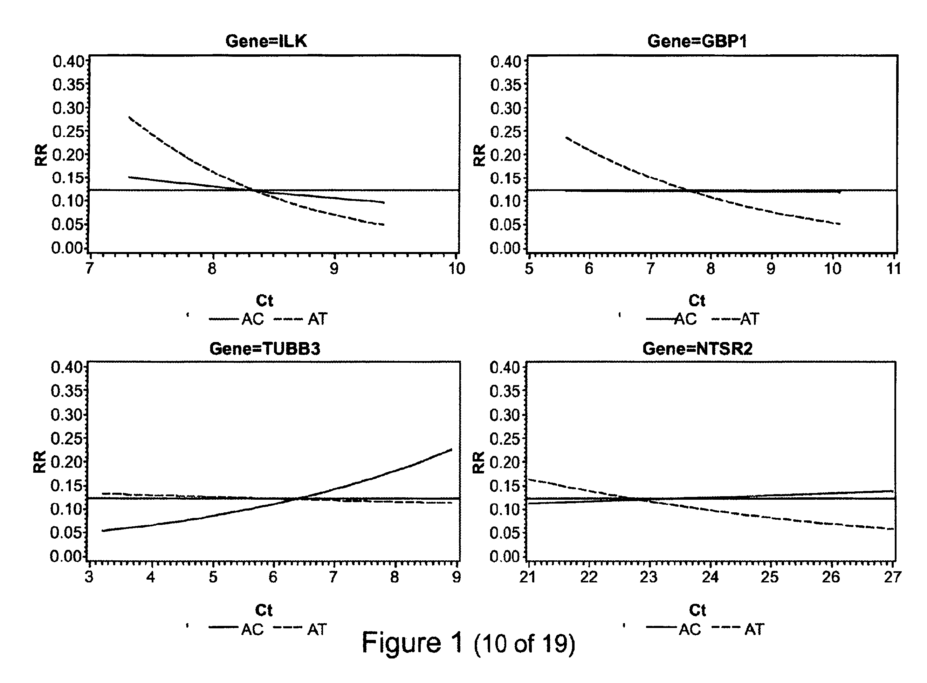

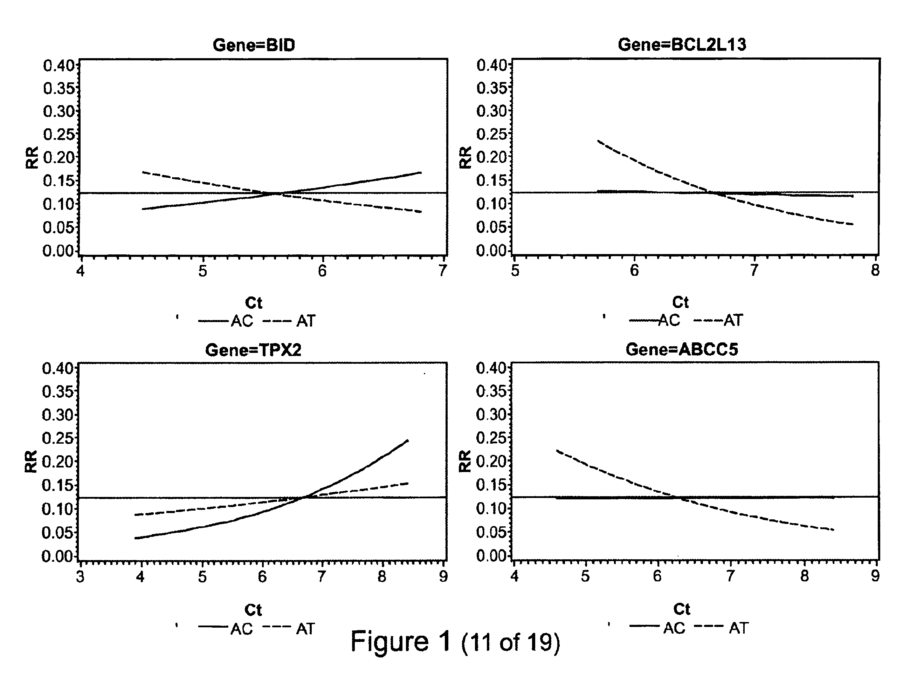

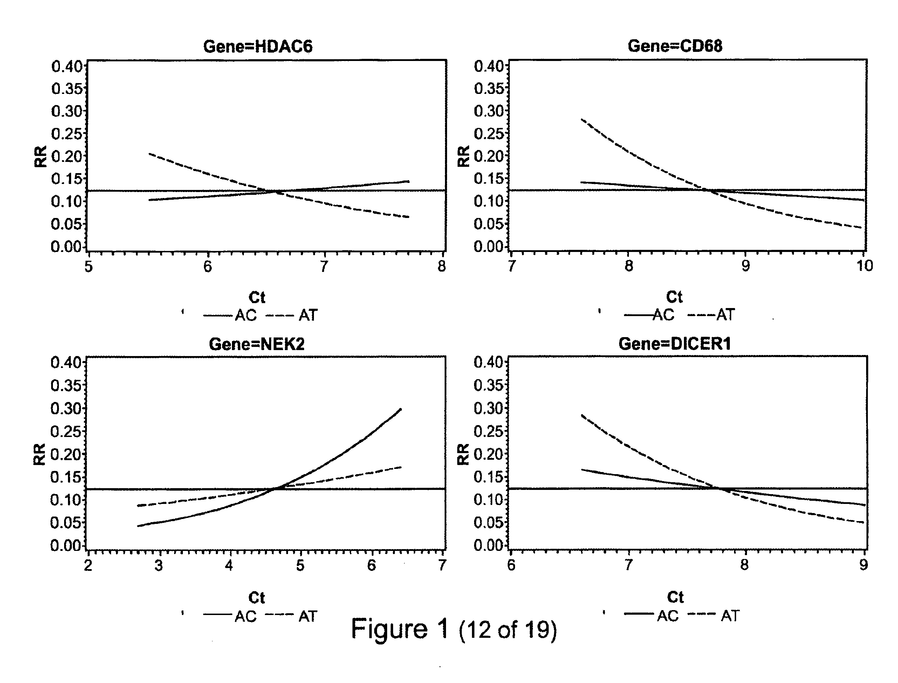

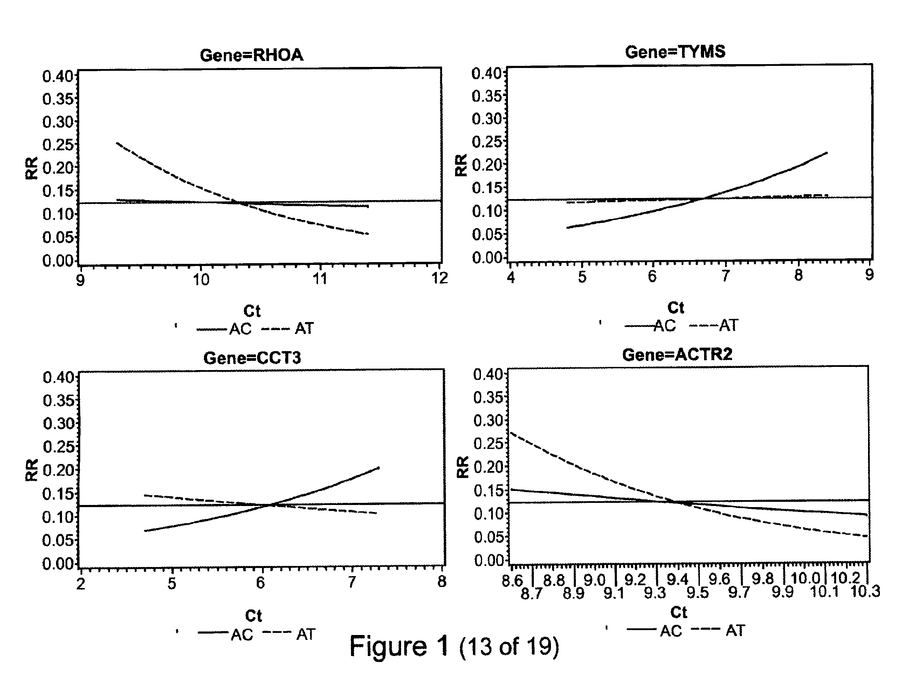

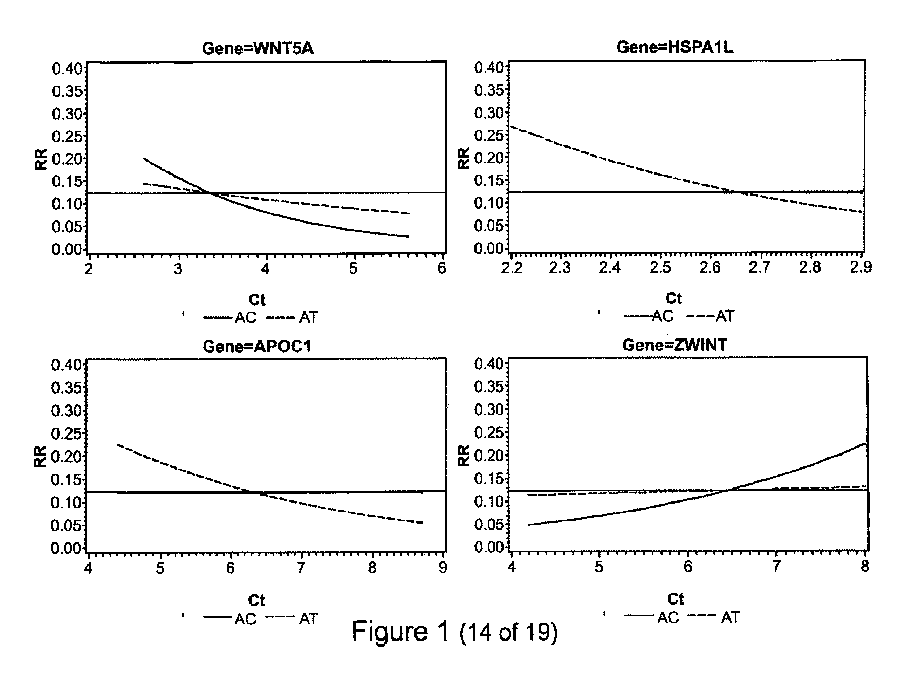

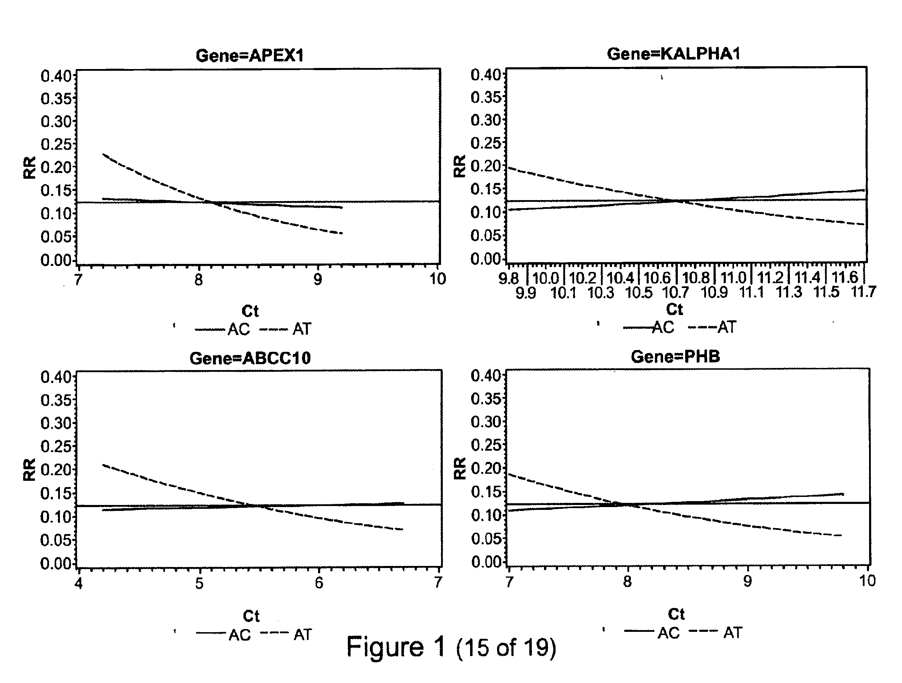

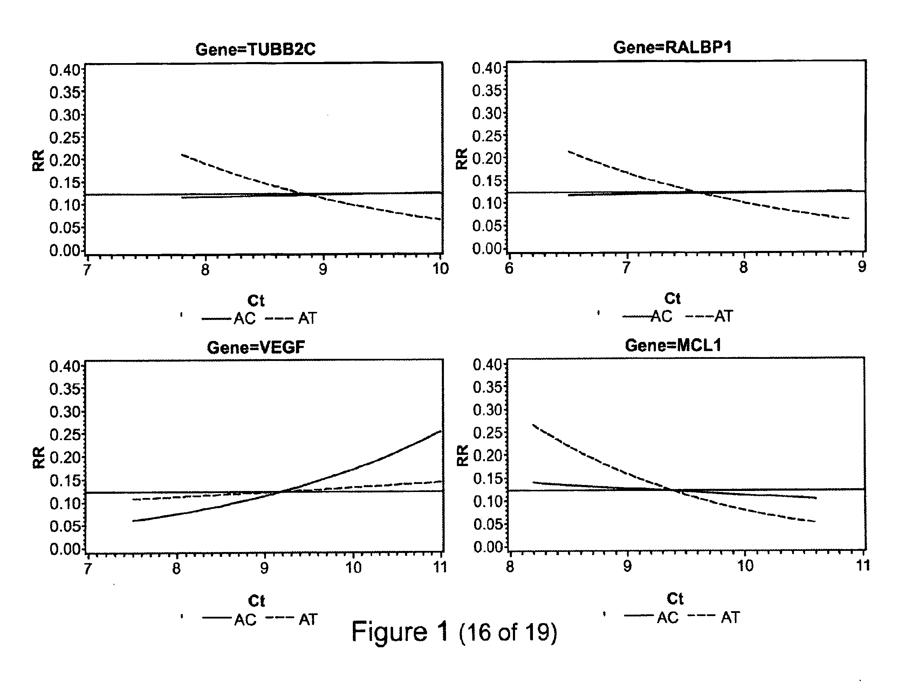

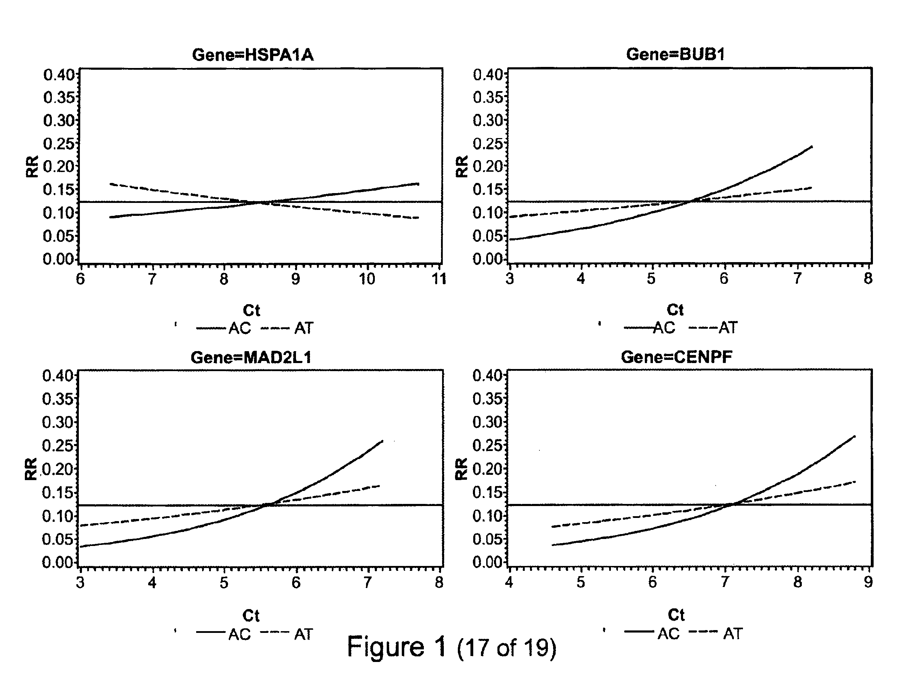

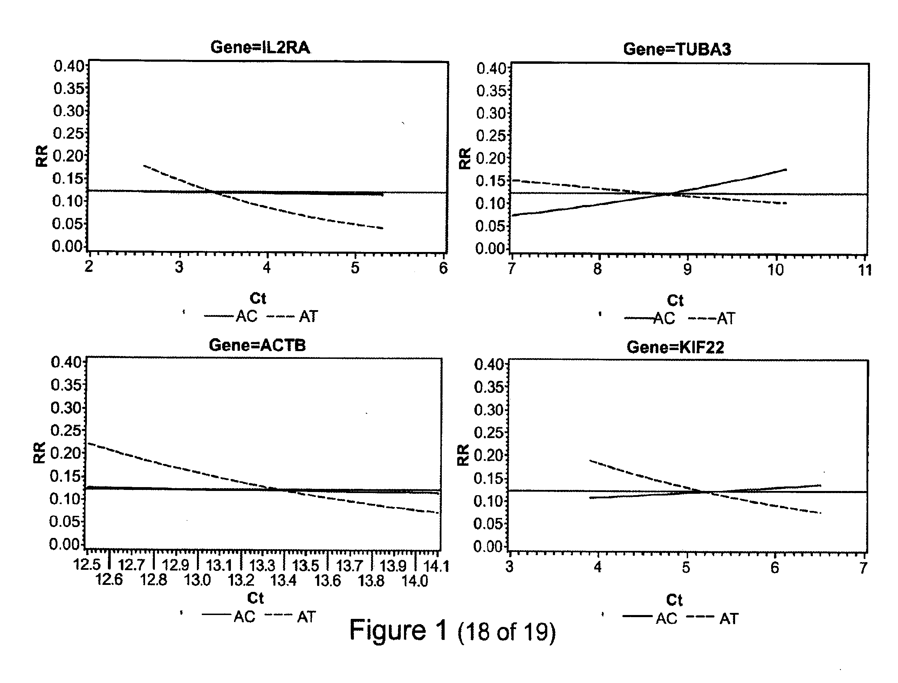

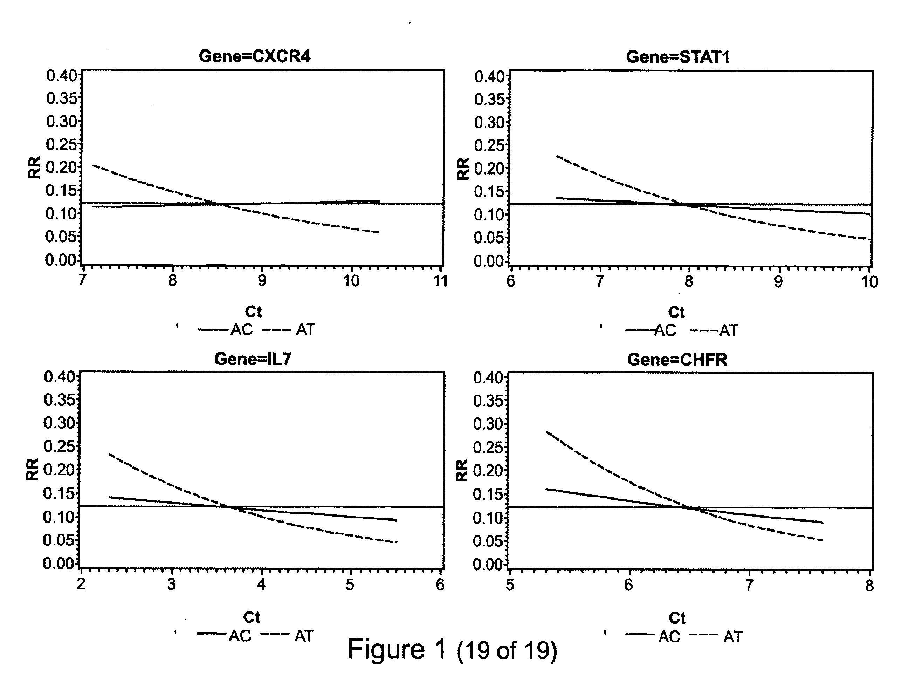

Figure 1 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line). A horizontal dashed line in each graph represents the overall (i.e., not gene expression-specific) 5-year RR in the study population who were randomized to treatment with either AC or AT. In Figure 1 the patients were included without regard to hormone receptor expression status of the tumour.

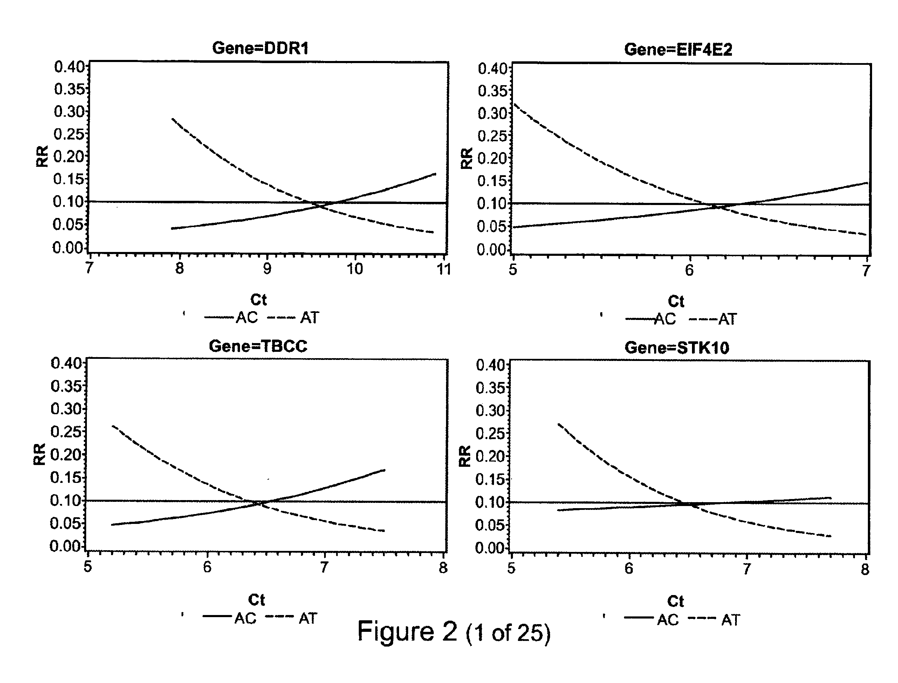

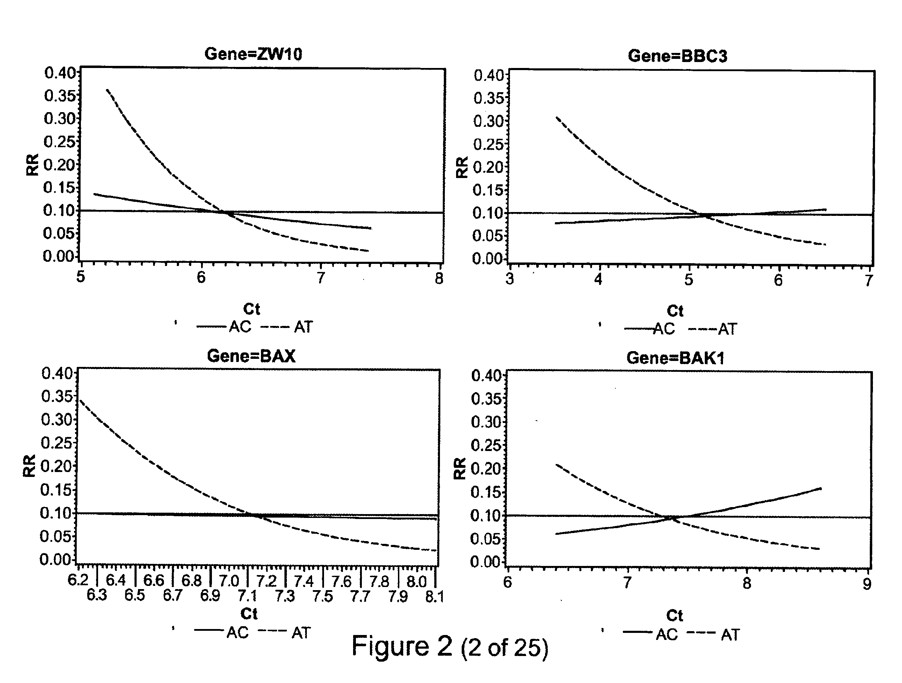

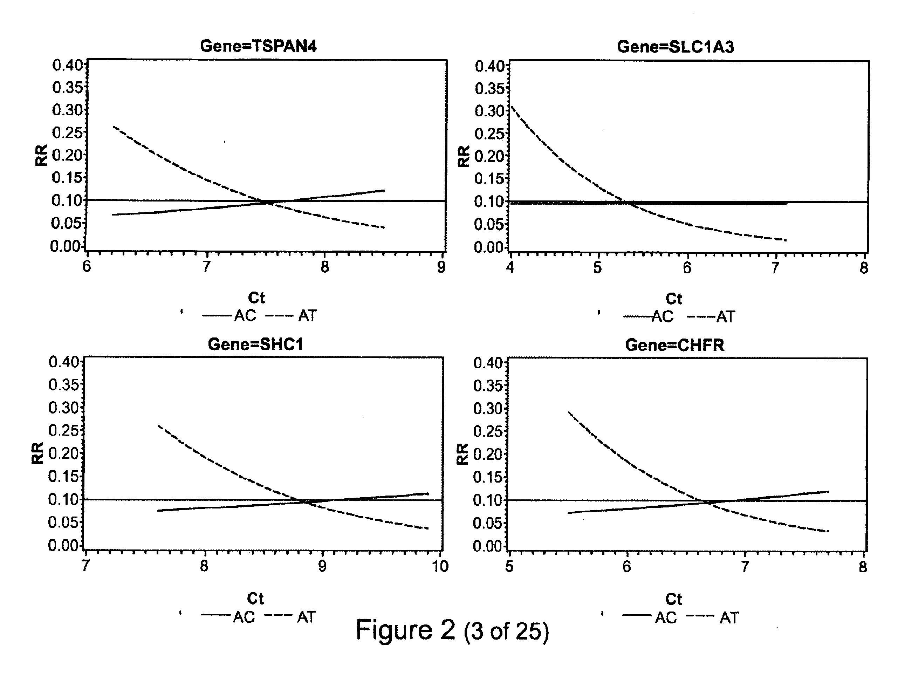

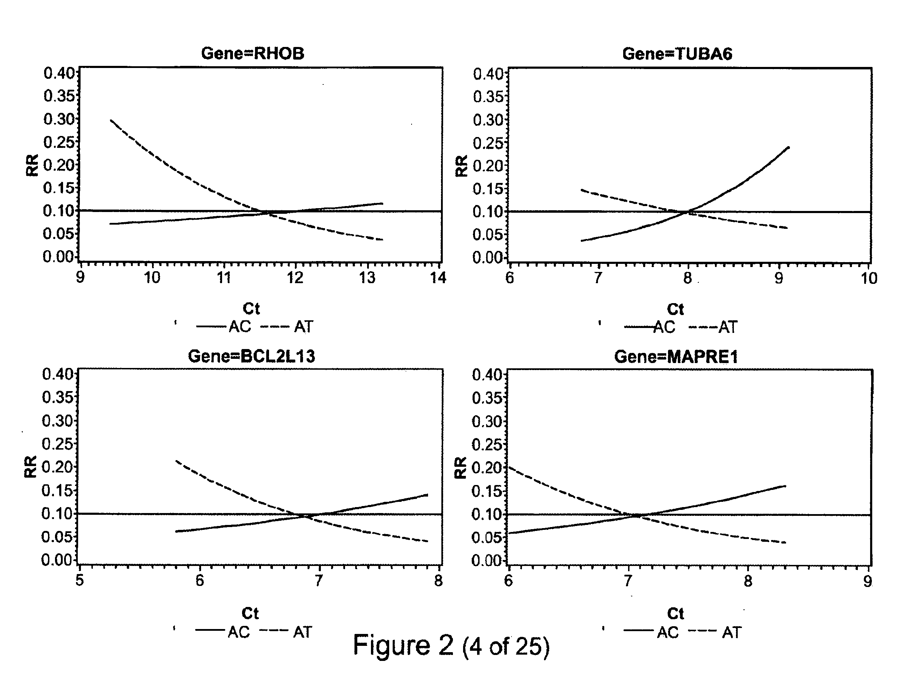

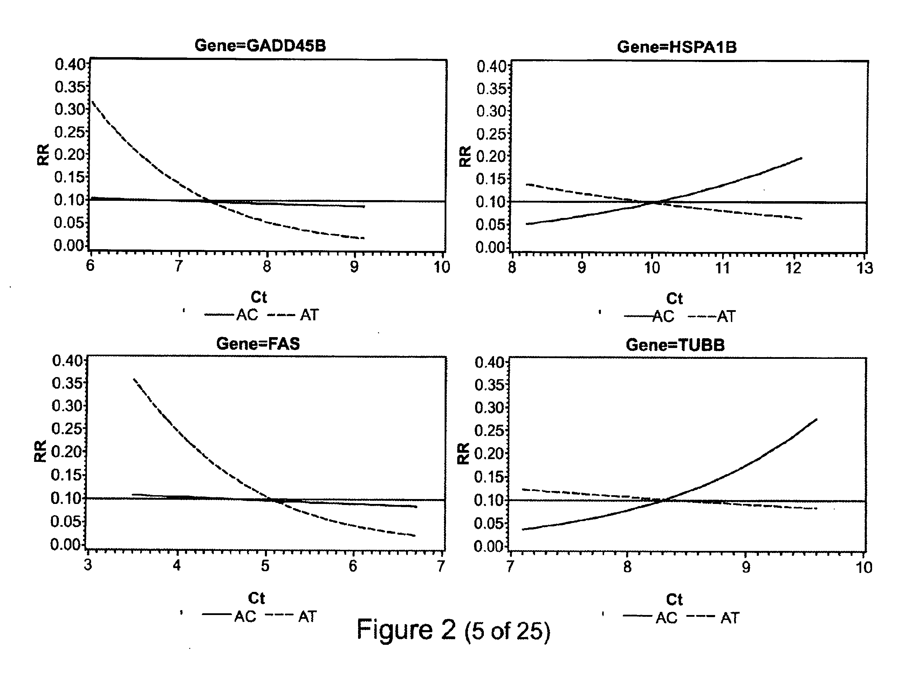

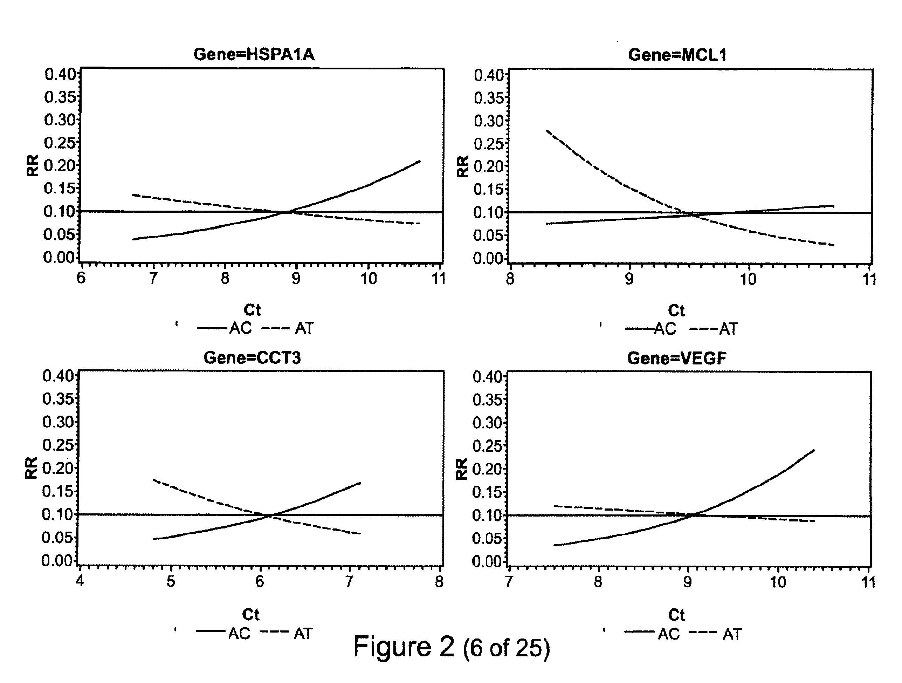

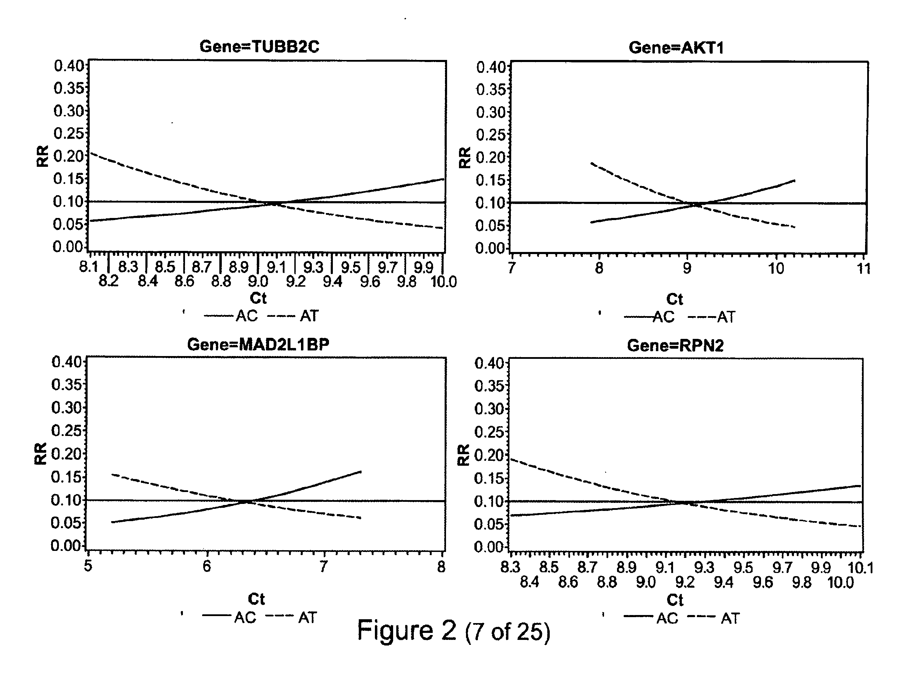

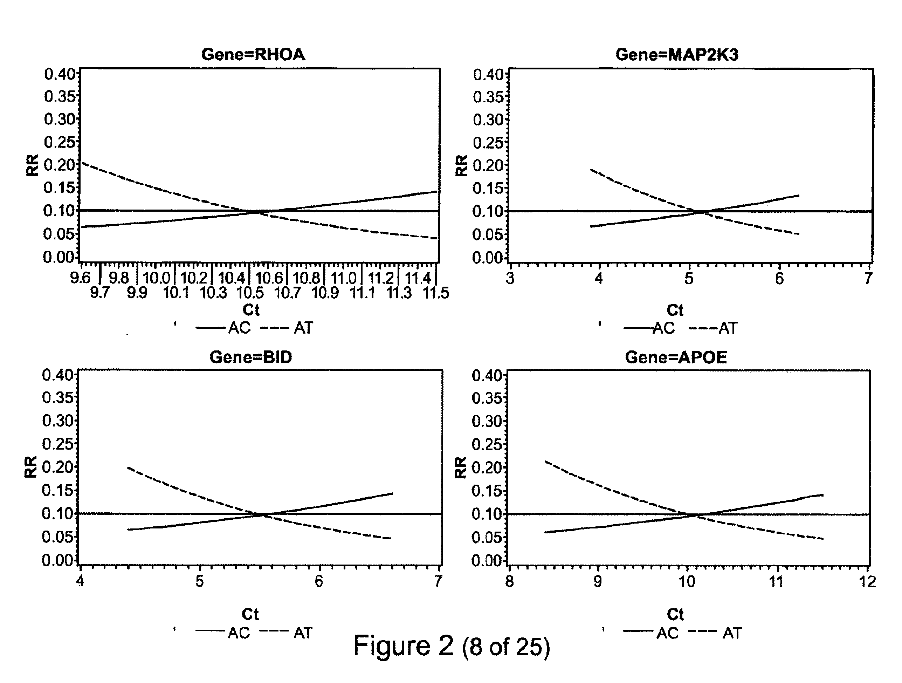

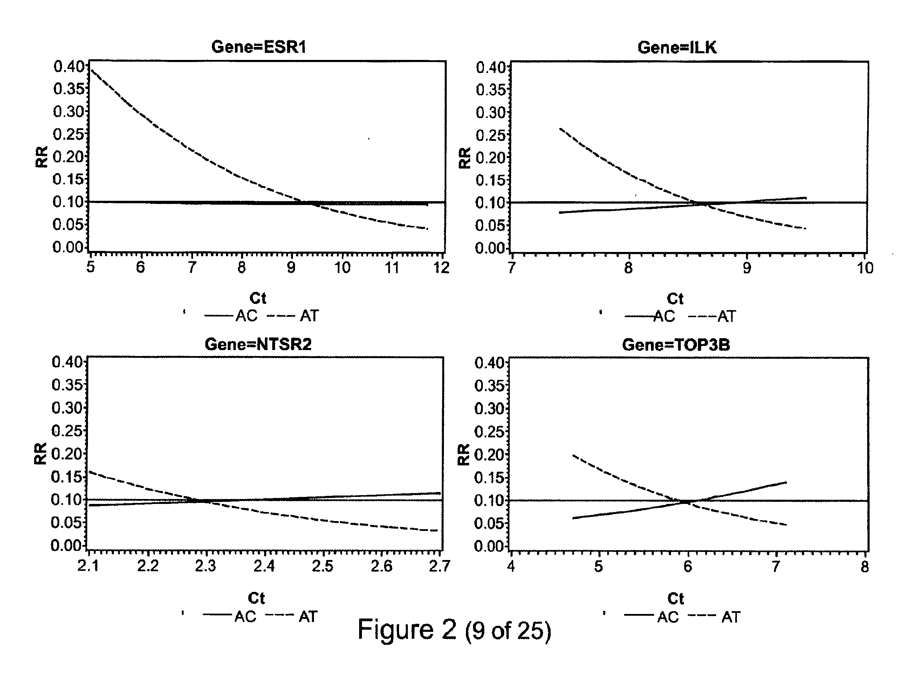

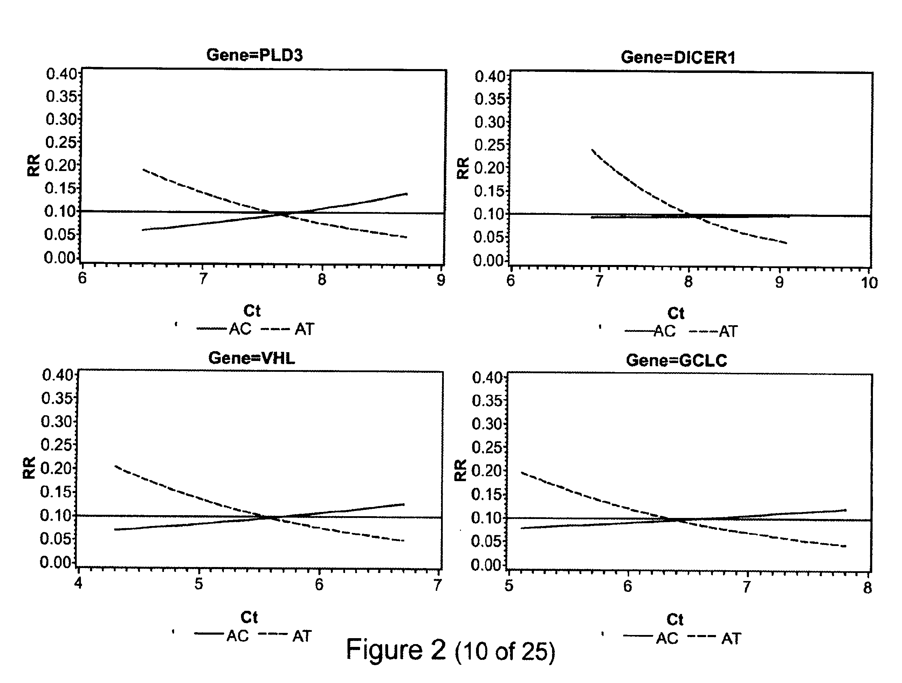

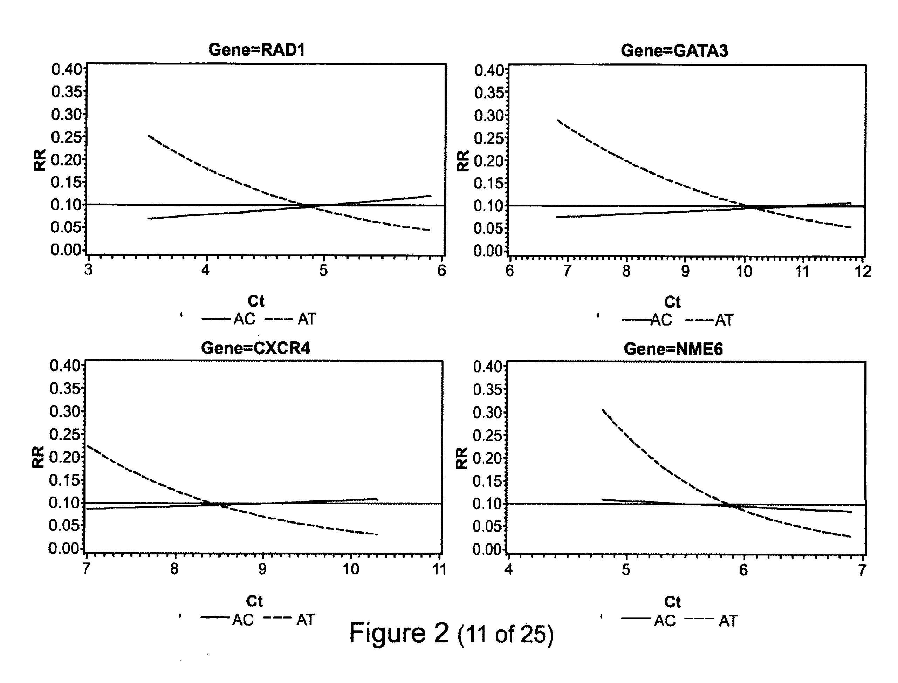

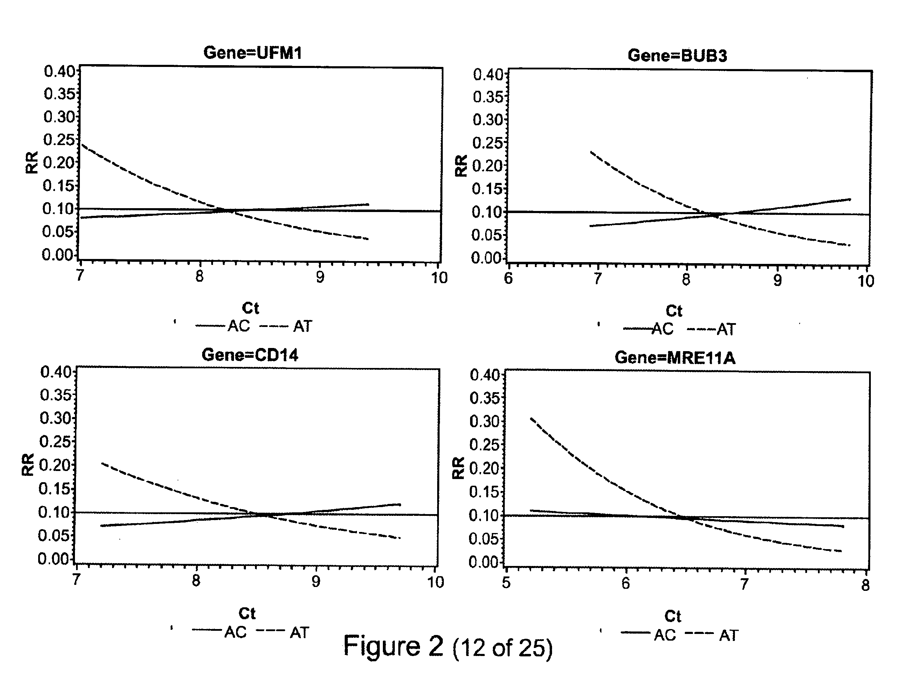

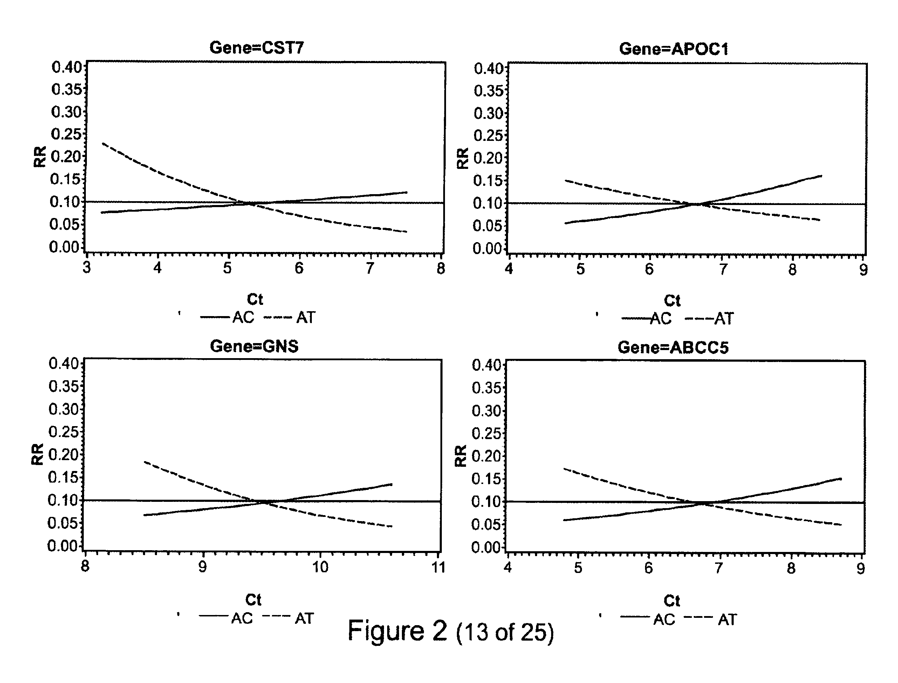

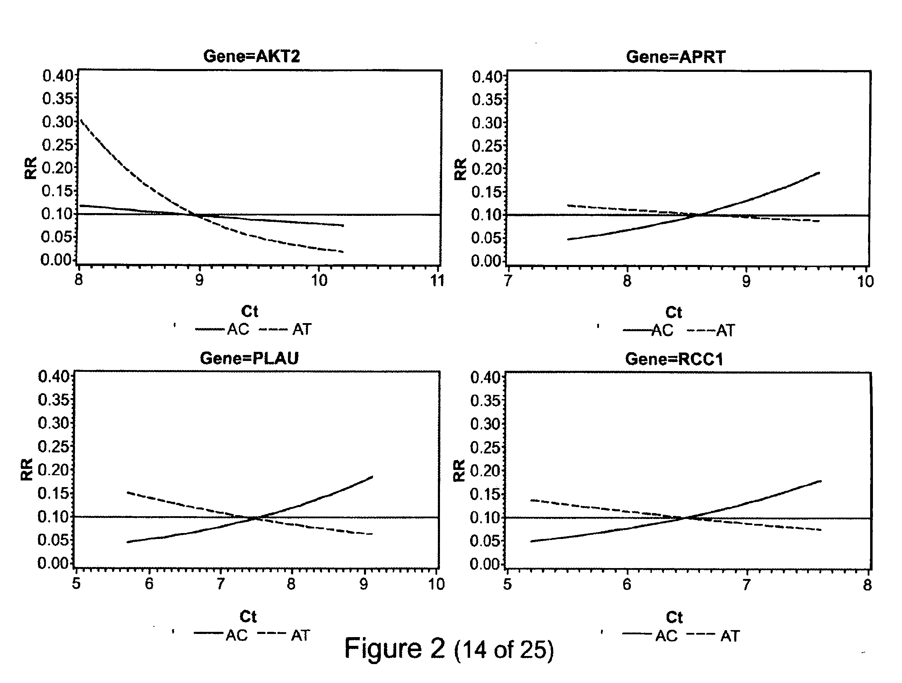

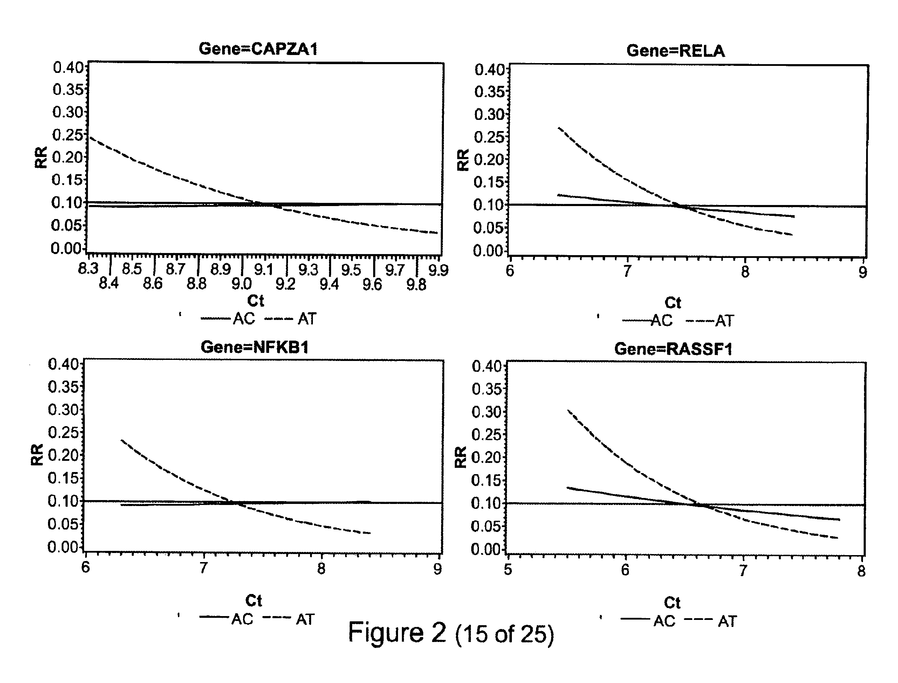

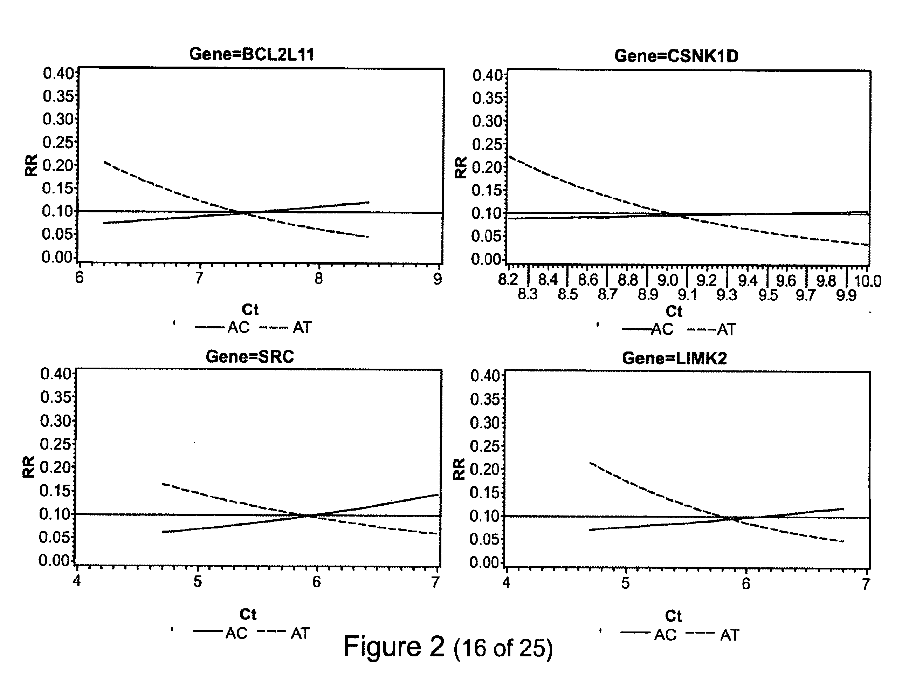

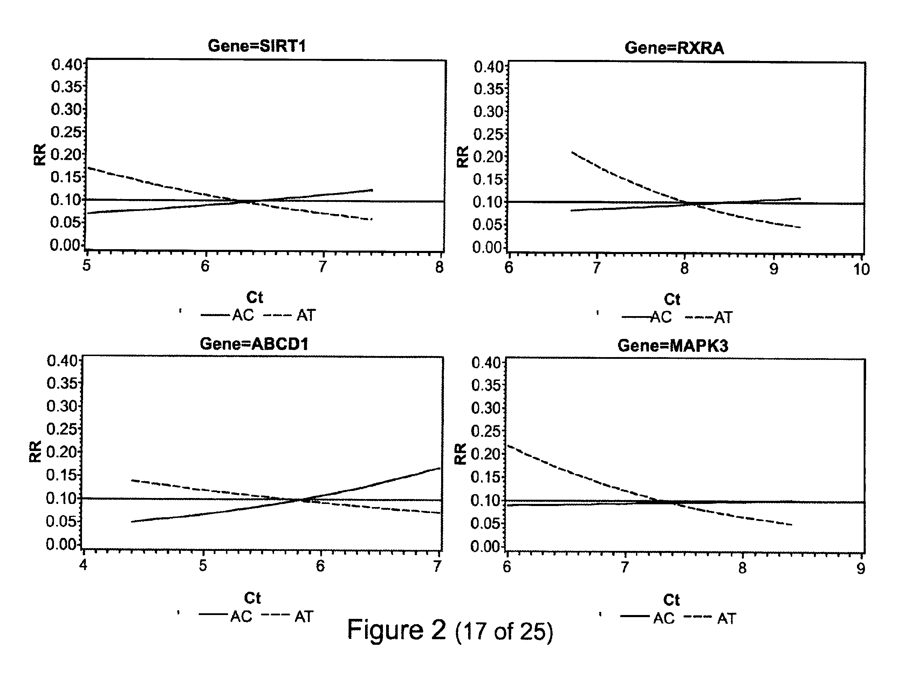

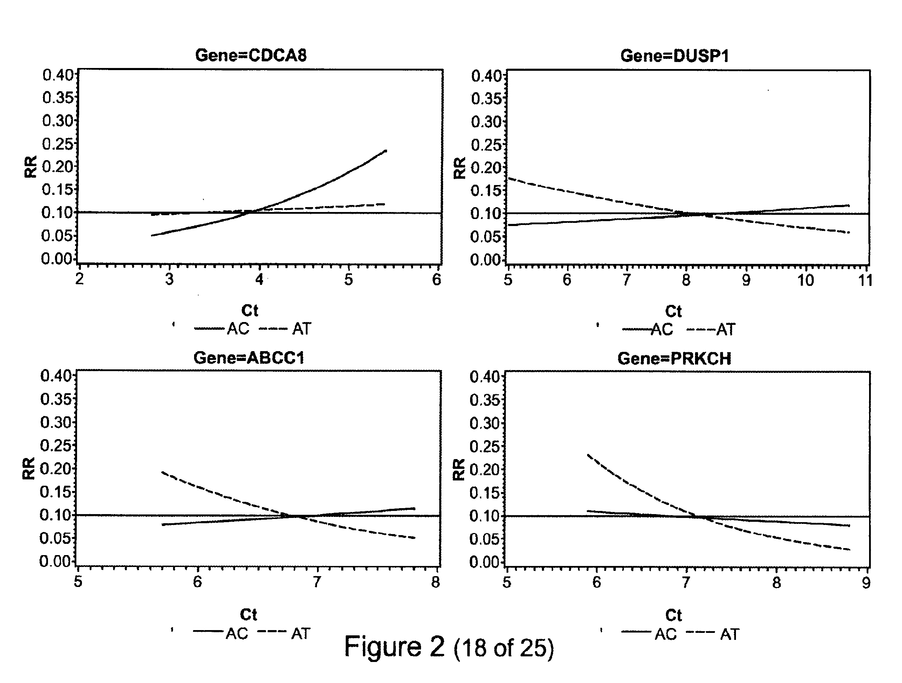

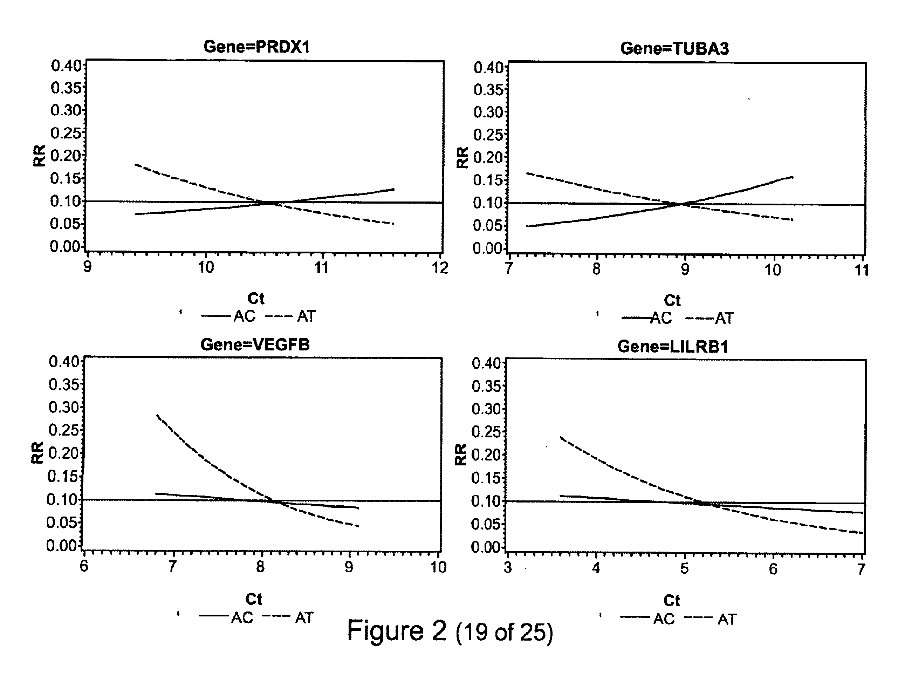

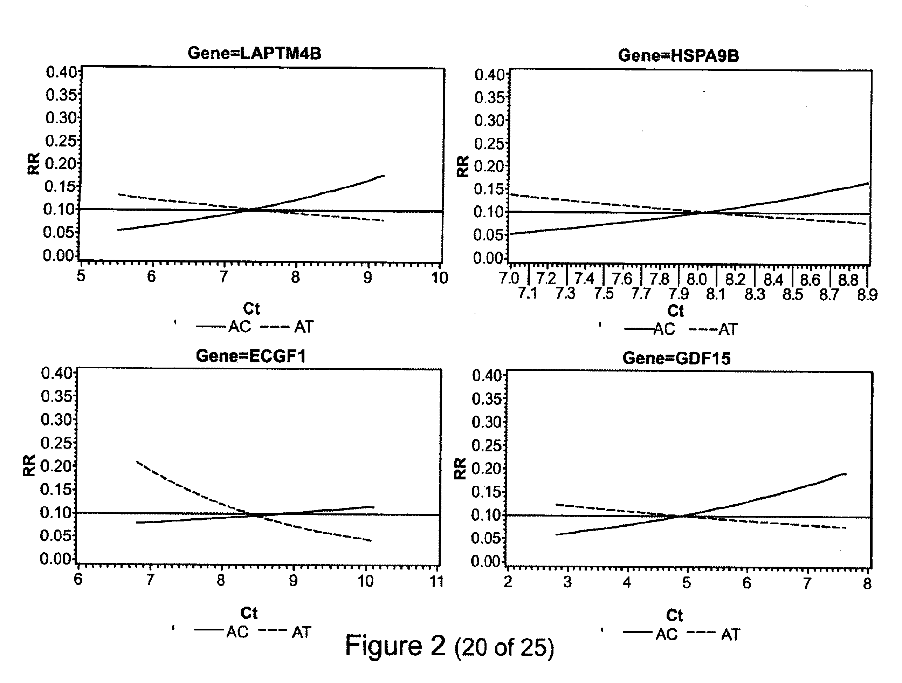

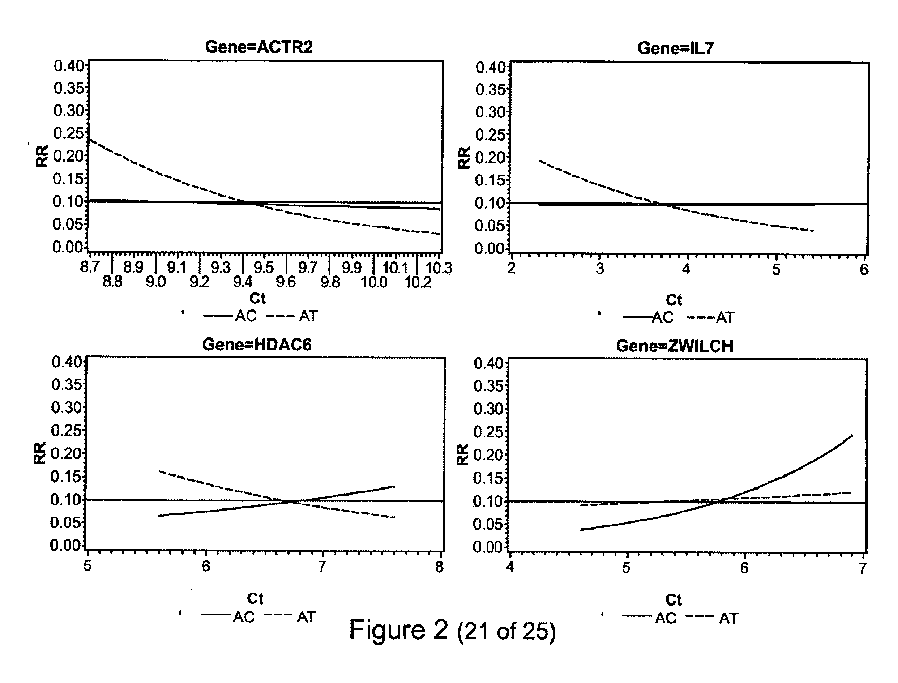

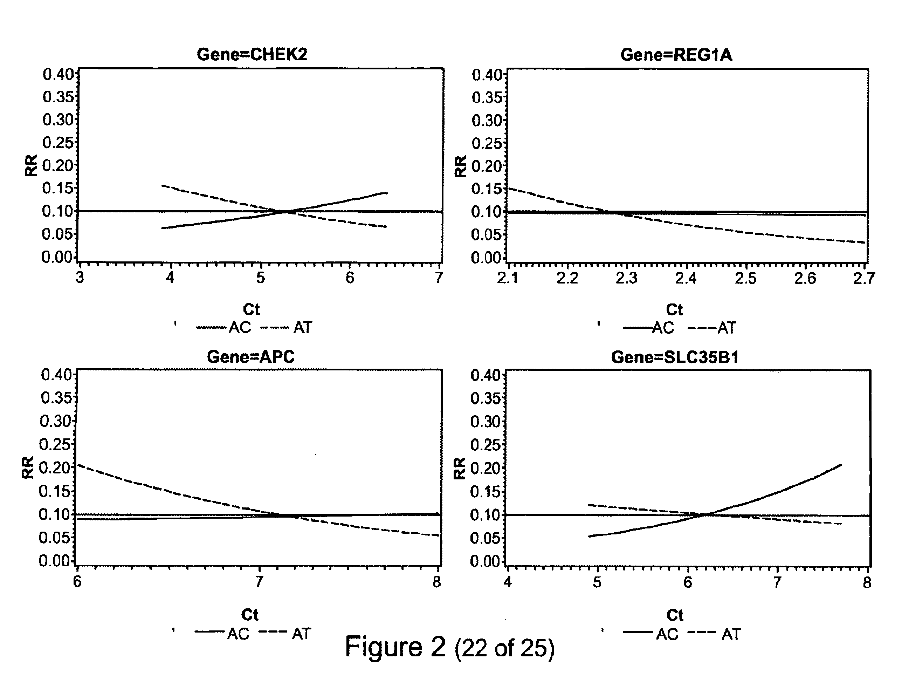

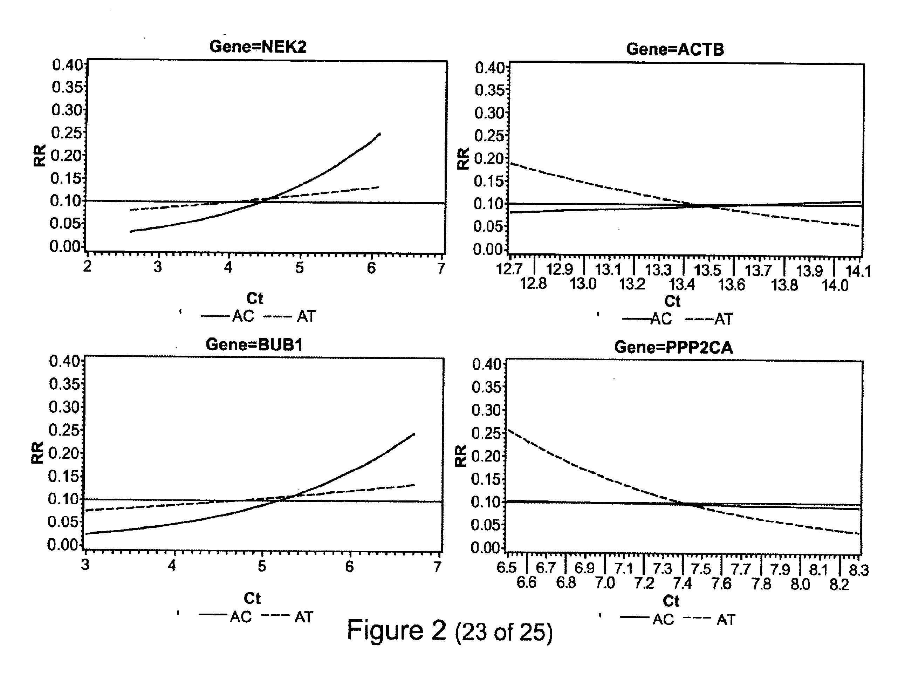

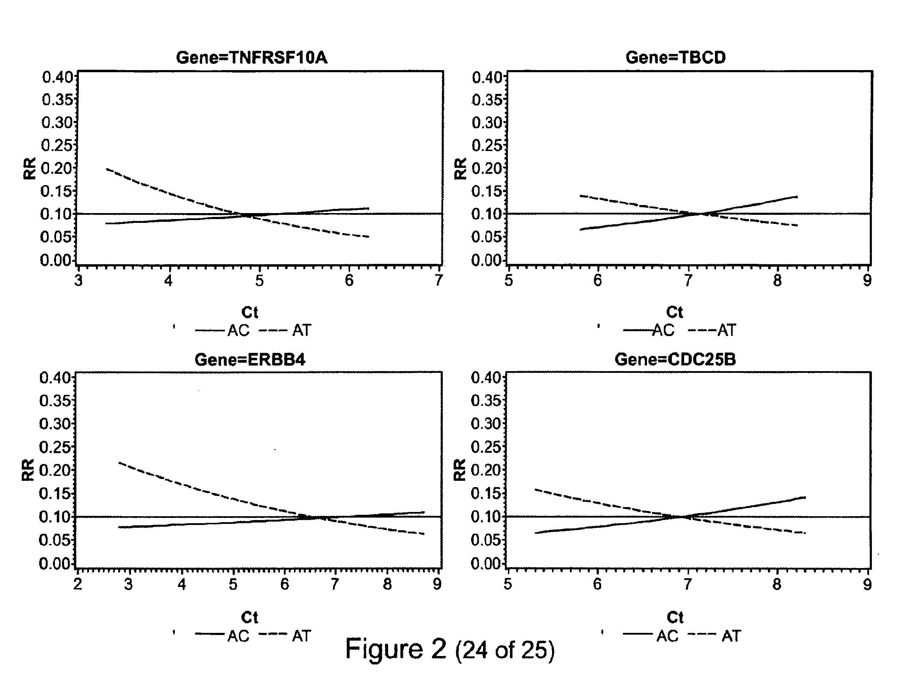

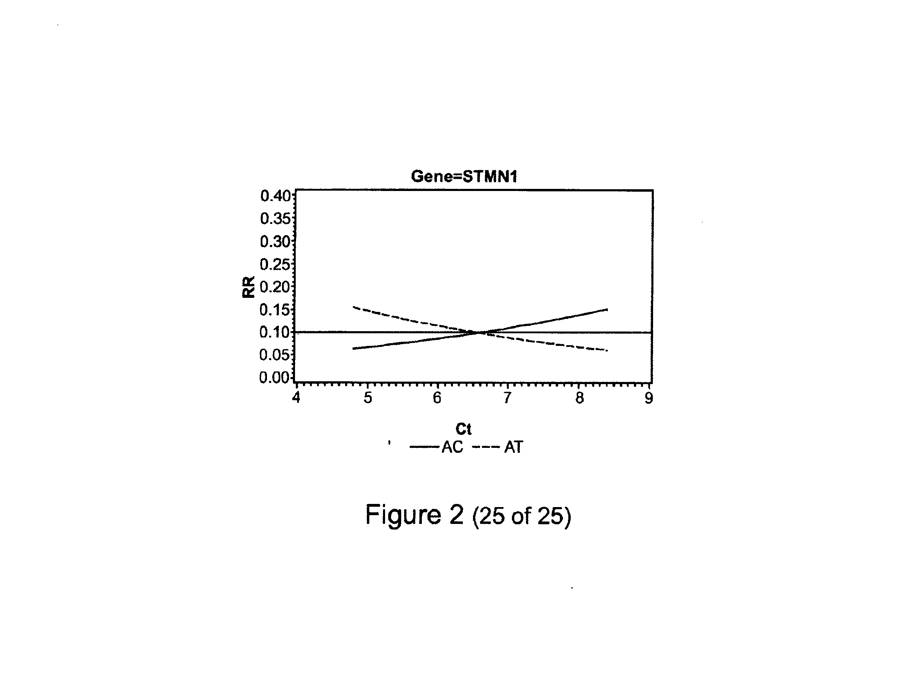

Figure 2 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor positive (HR⁺) breast cancer. A horizontal dashed line in each graph represents the overall (i.e., not gene expression-specific) 5-year RR in patients in the study population having HR+ breast cancer who were randomized to treatment with either AC or AT.

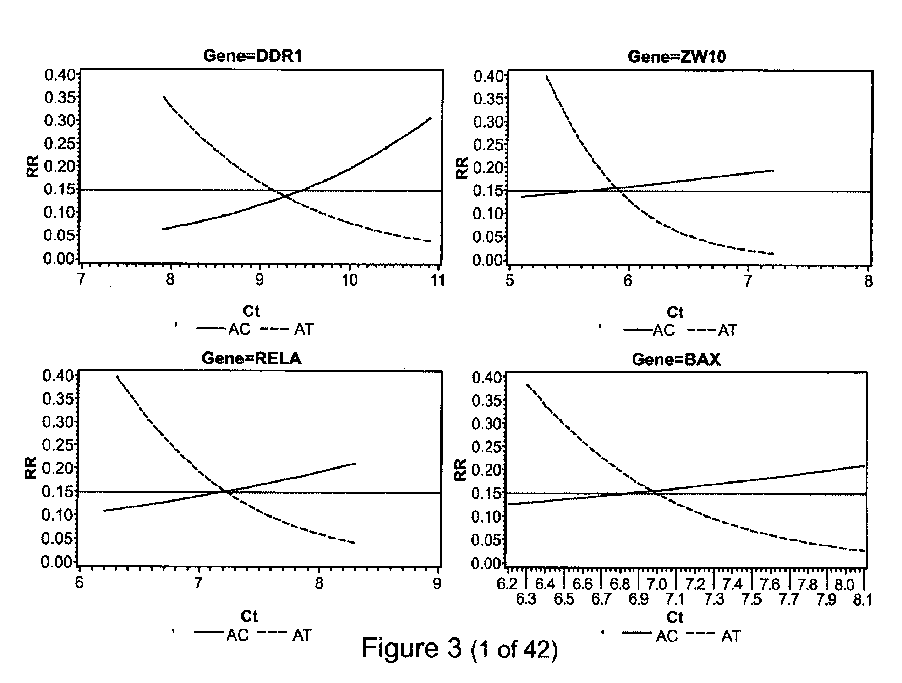

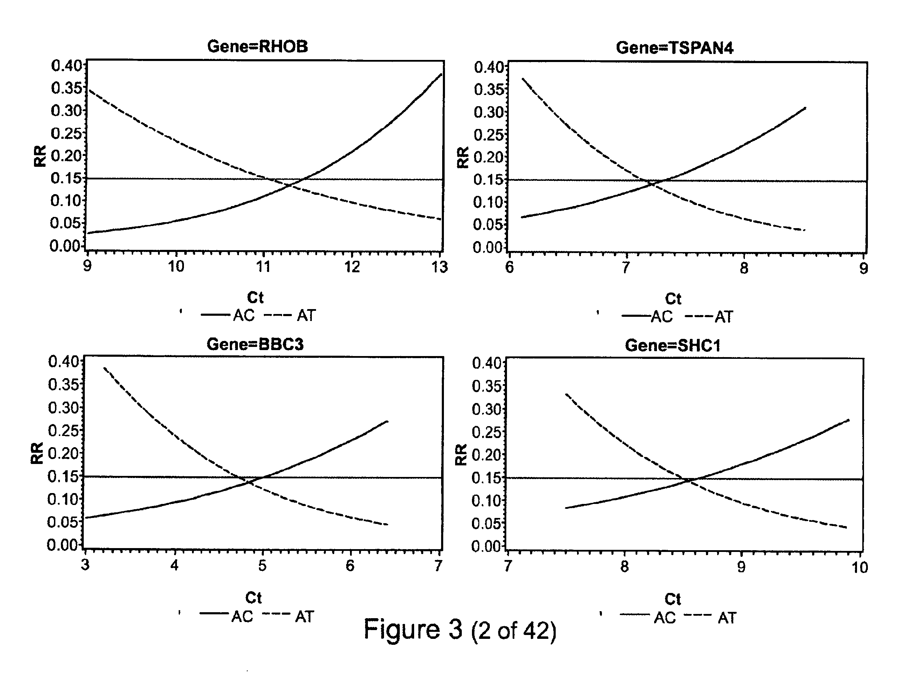

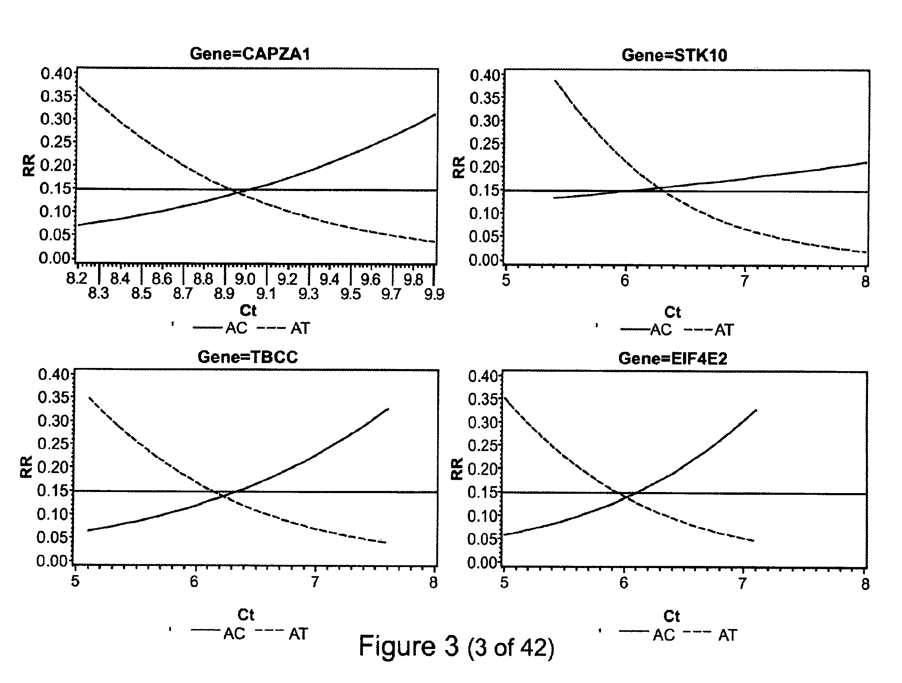

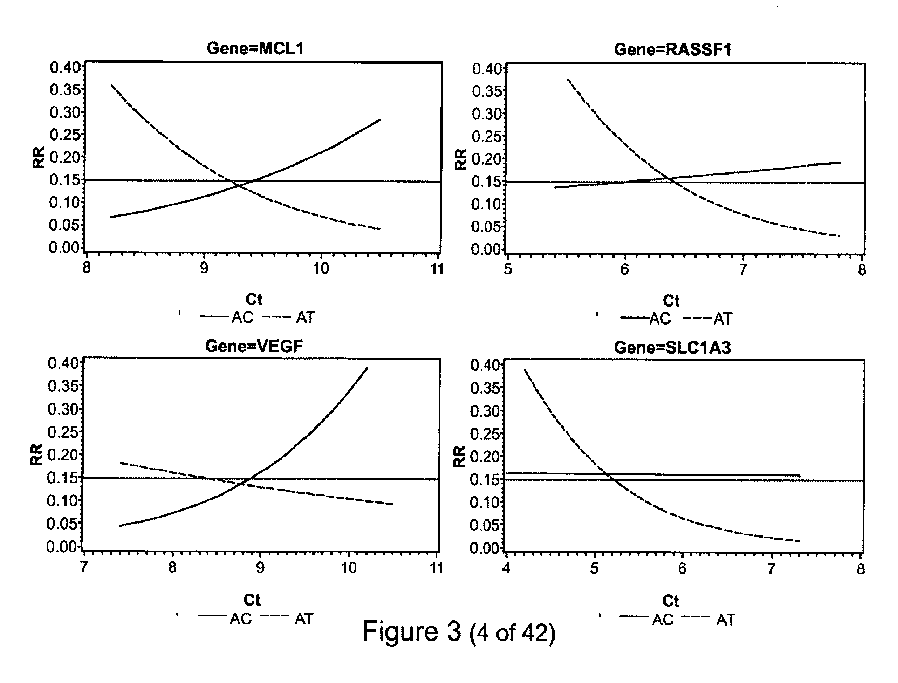

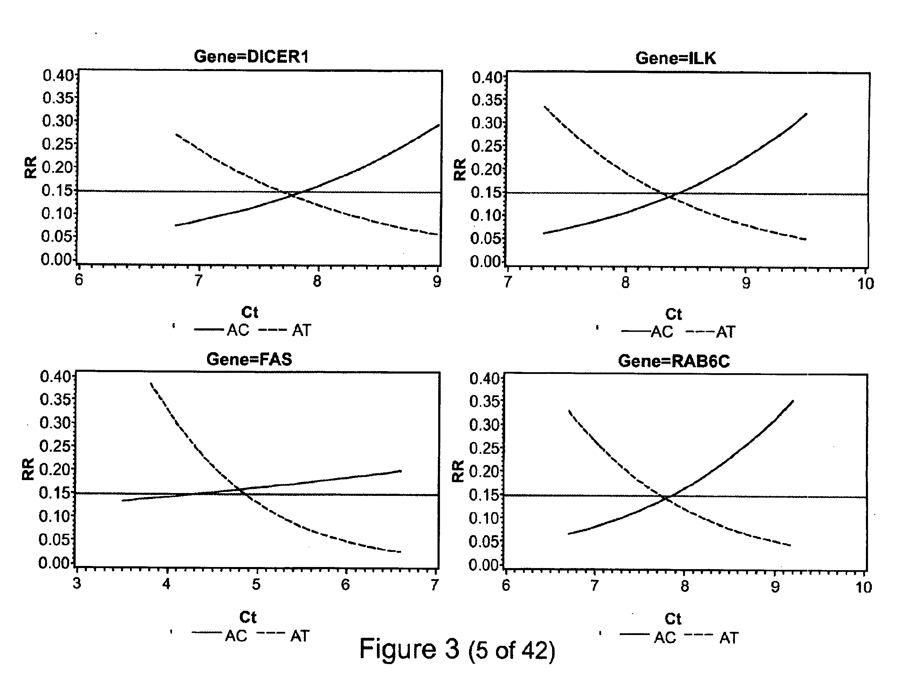

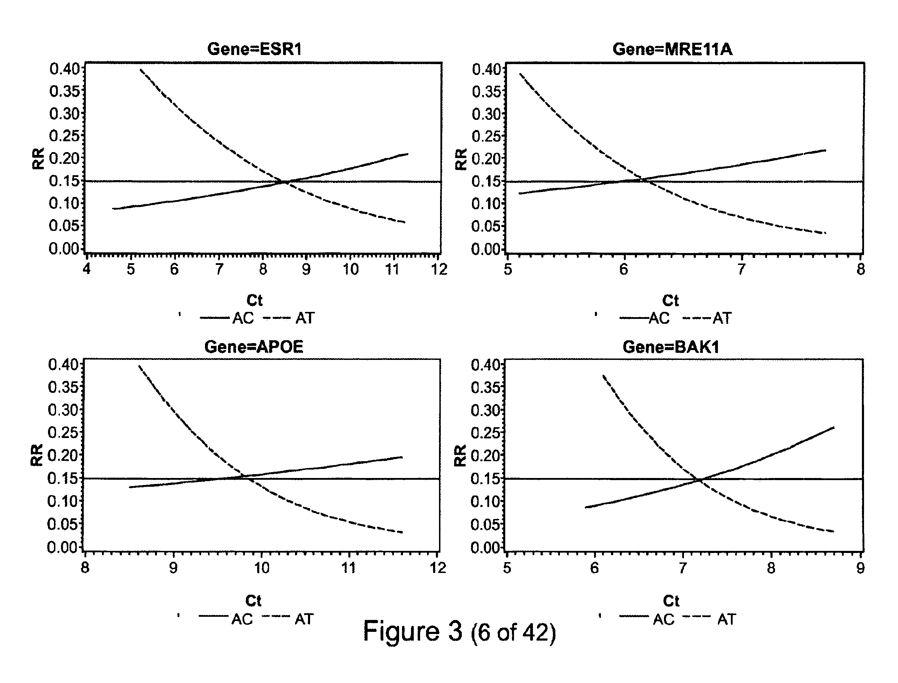

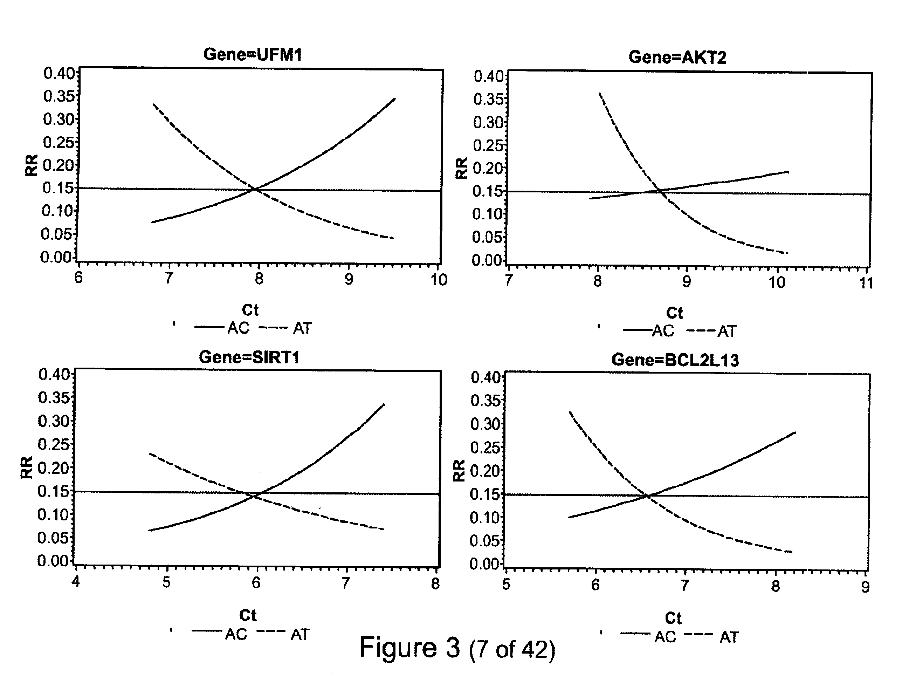

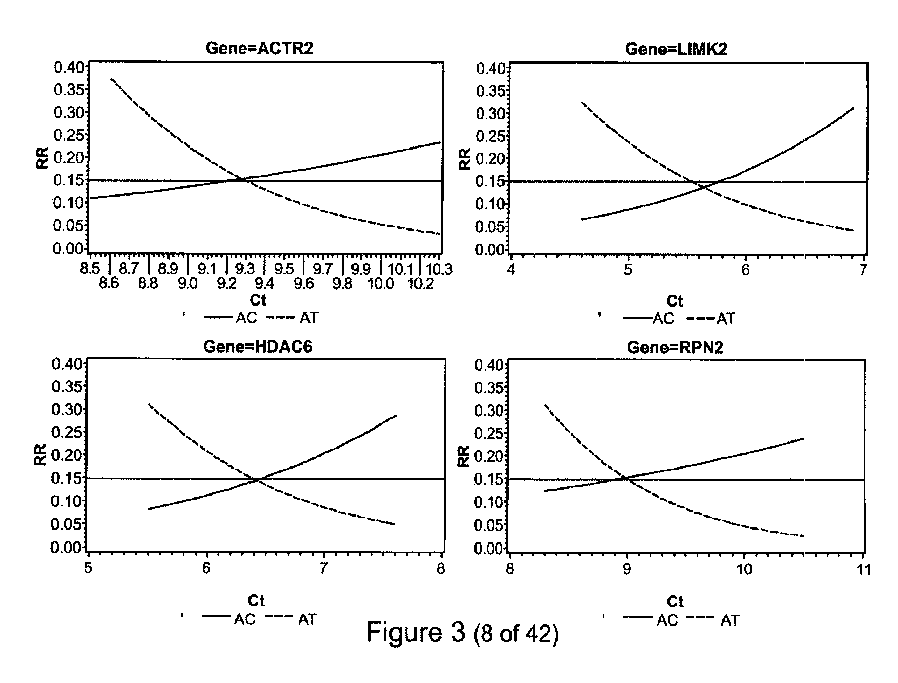

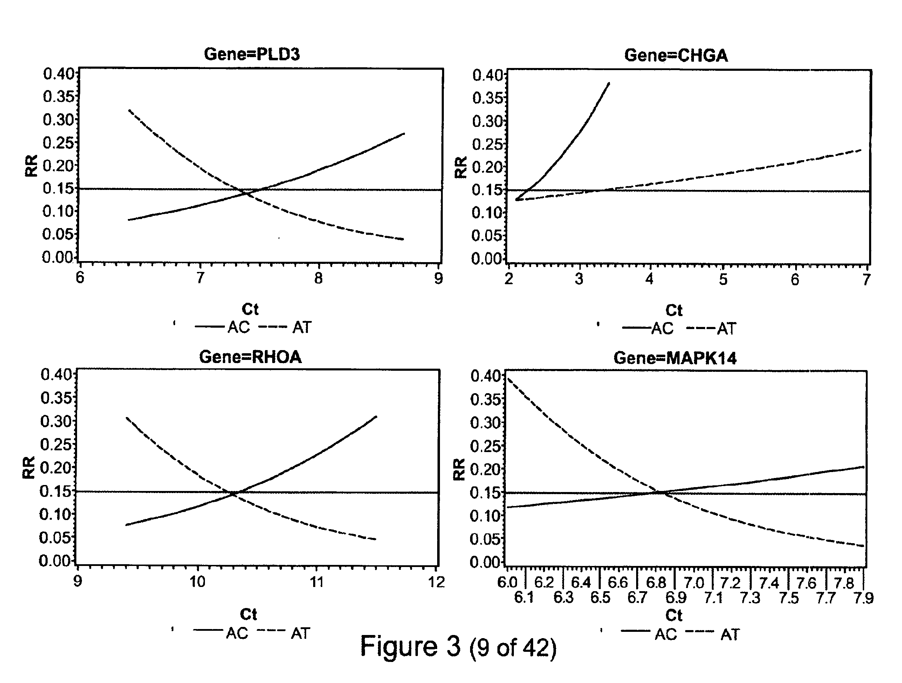

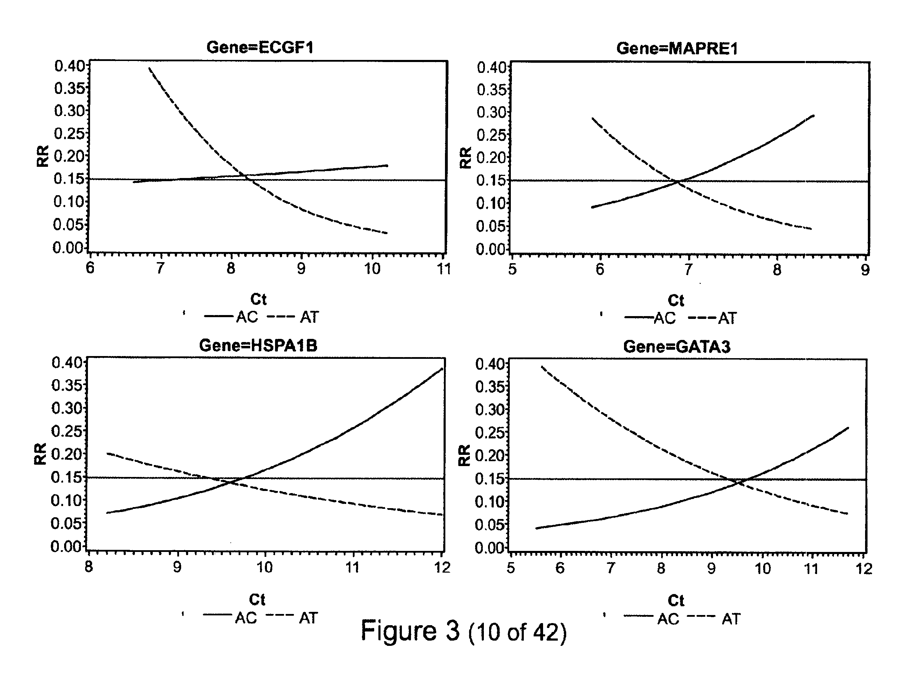

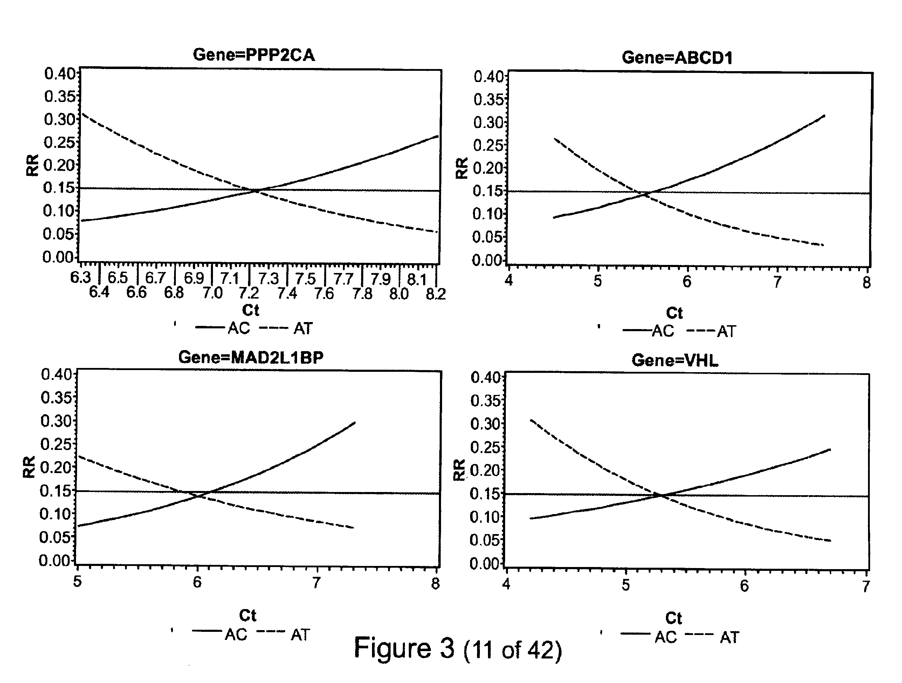

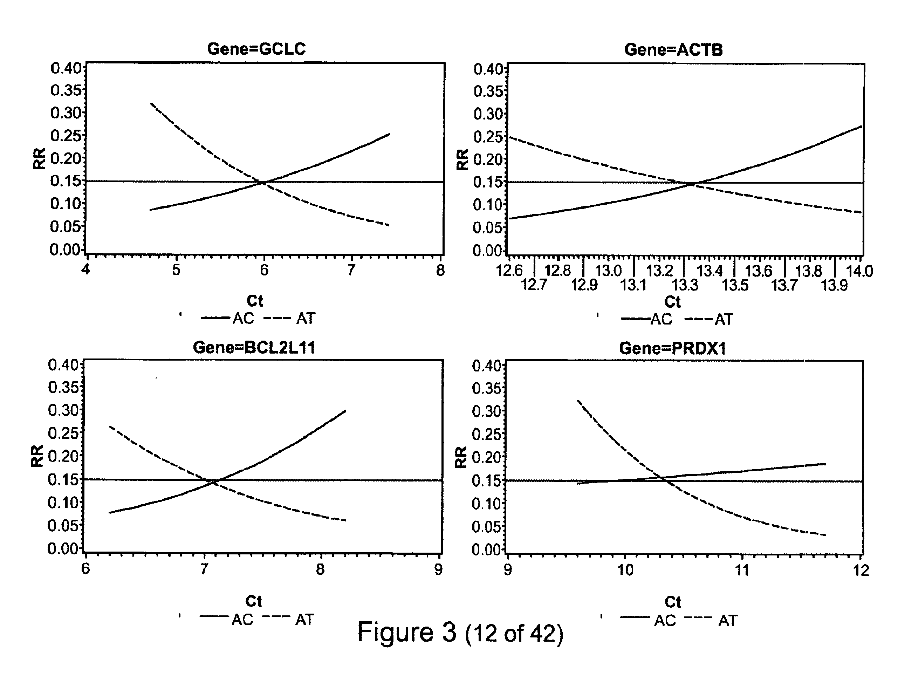

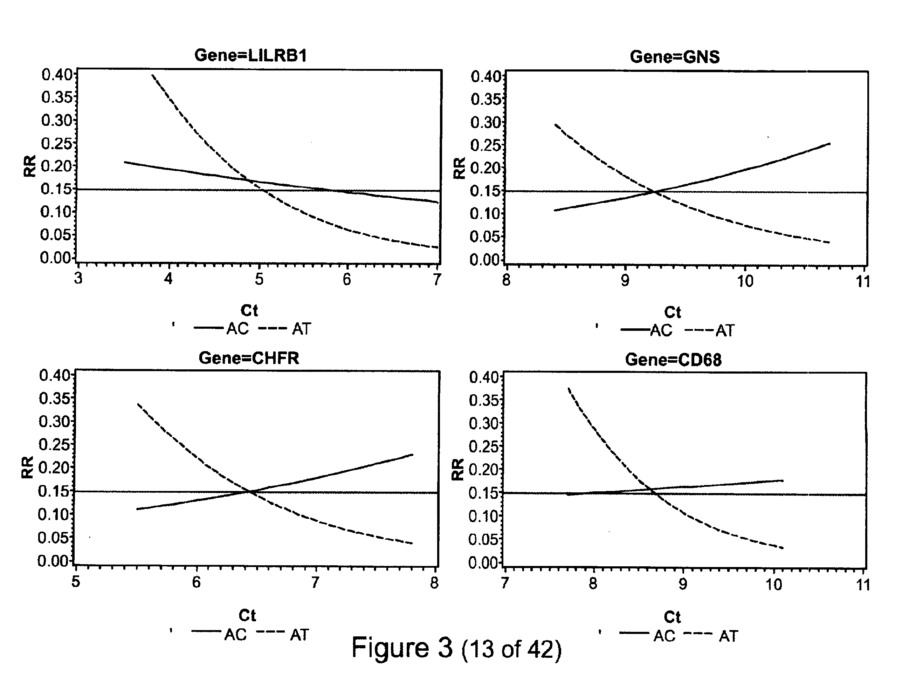

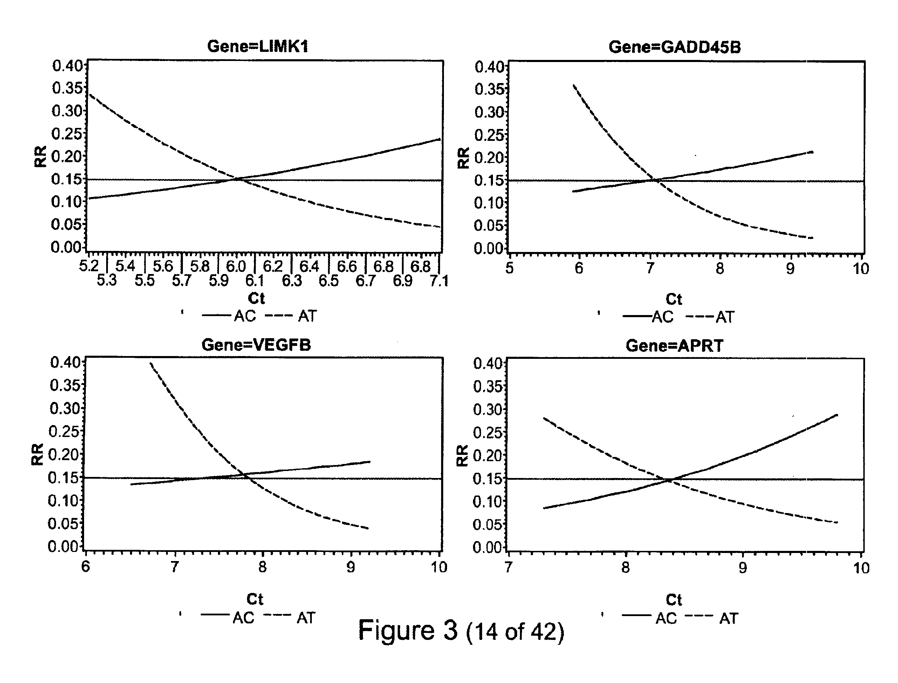

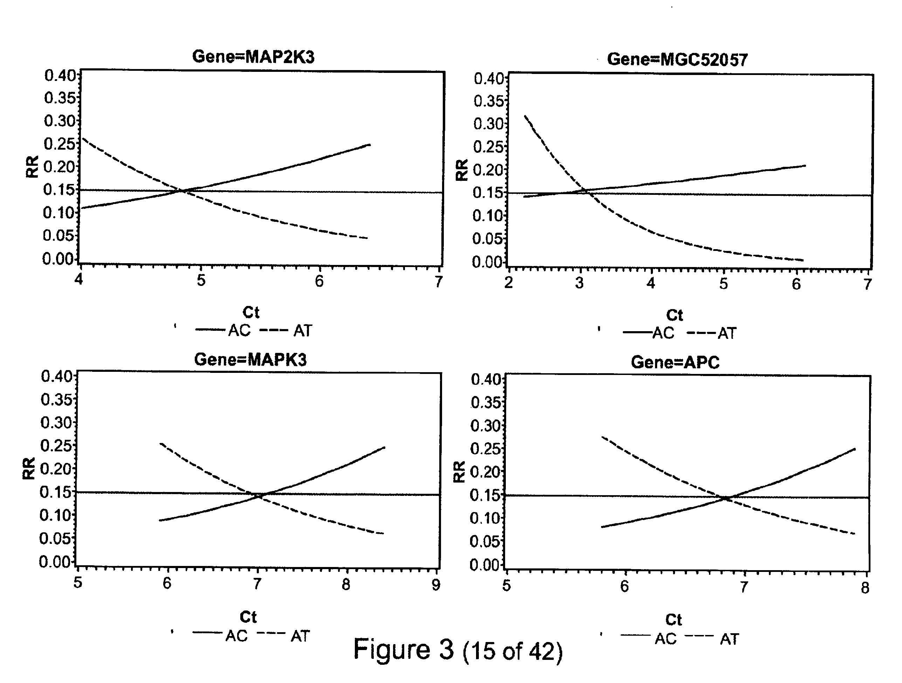

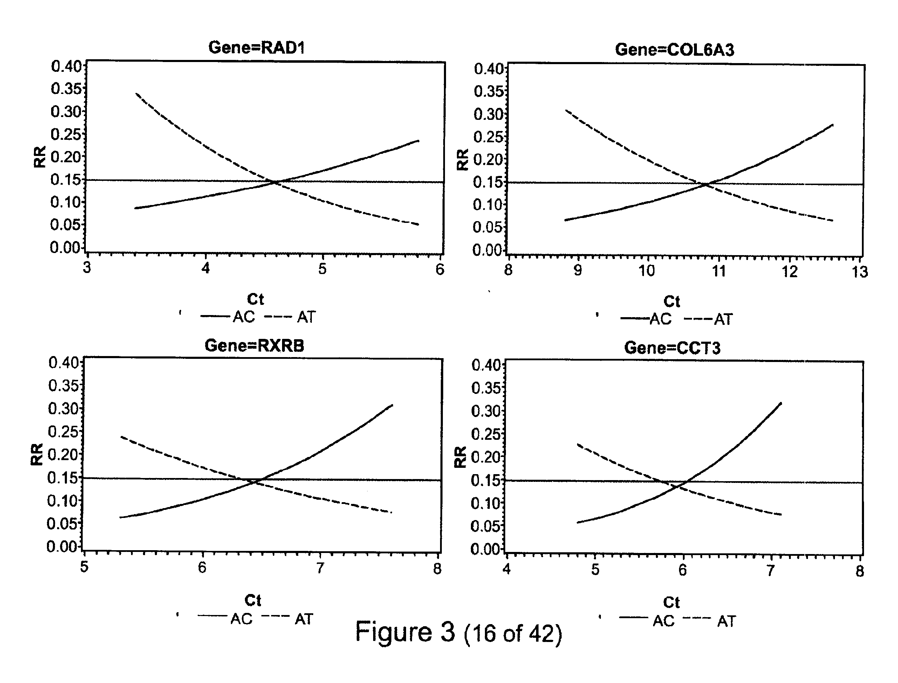

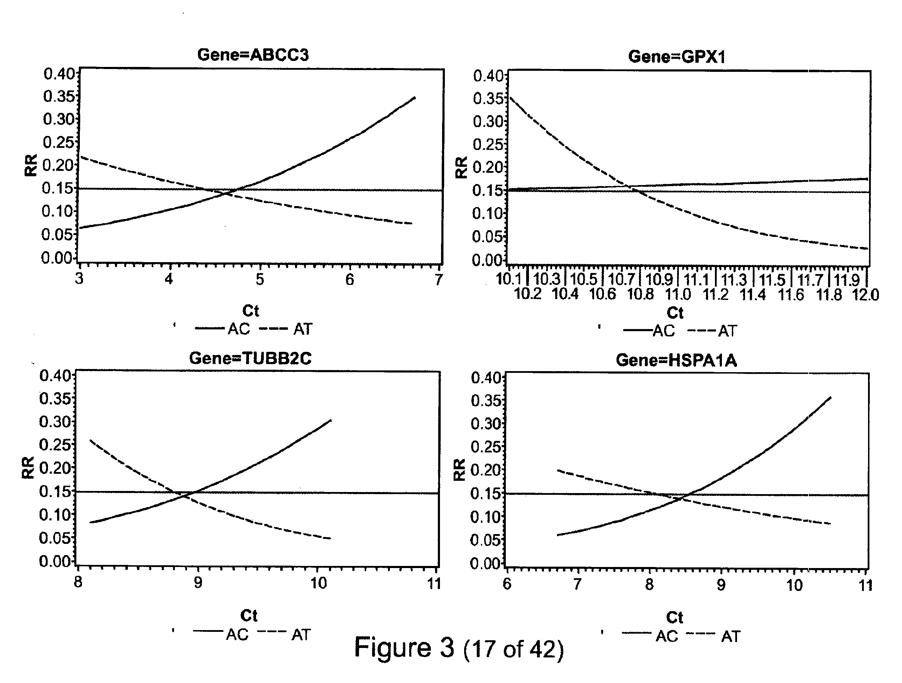

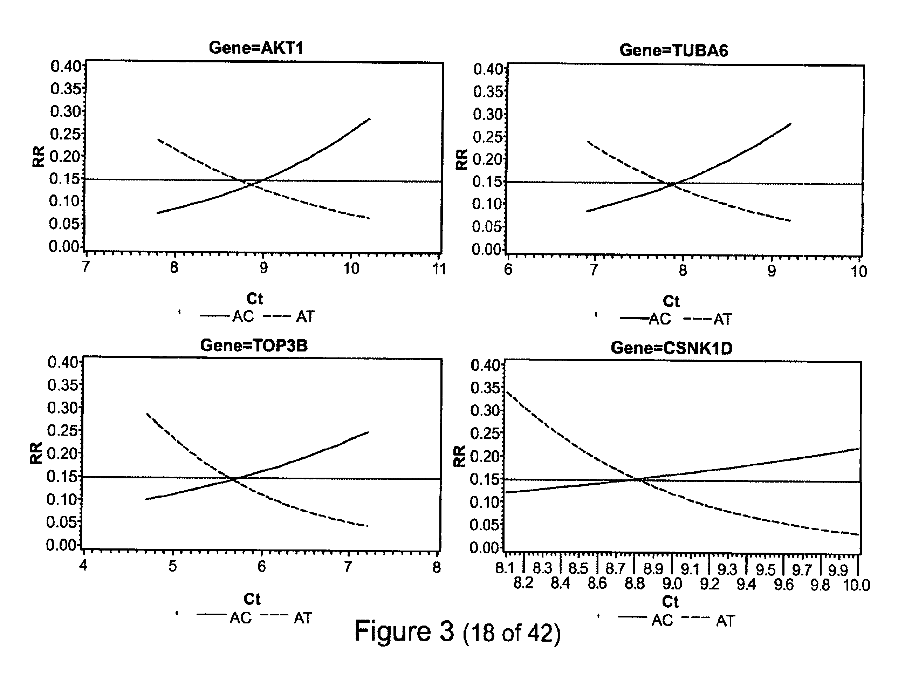

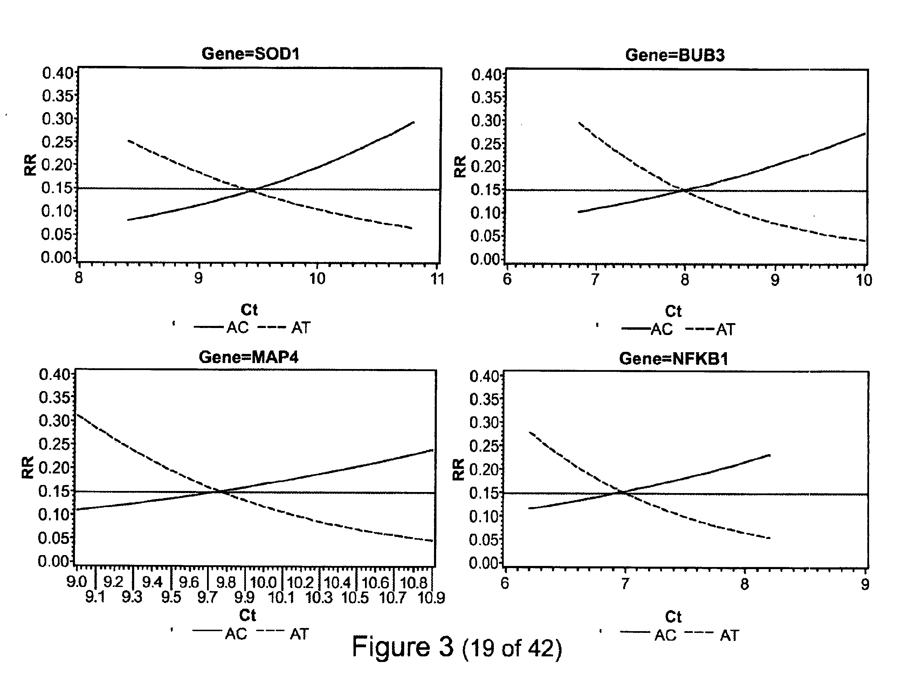

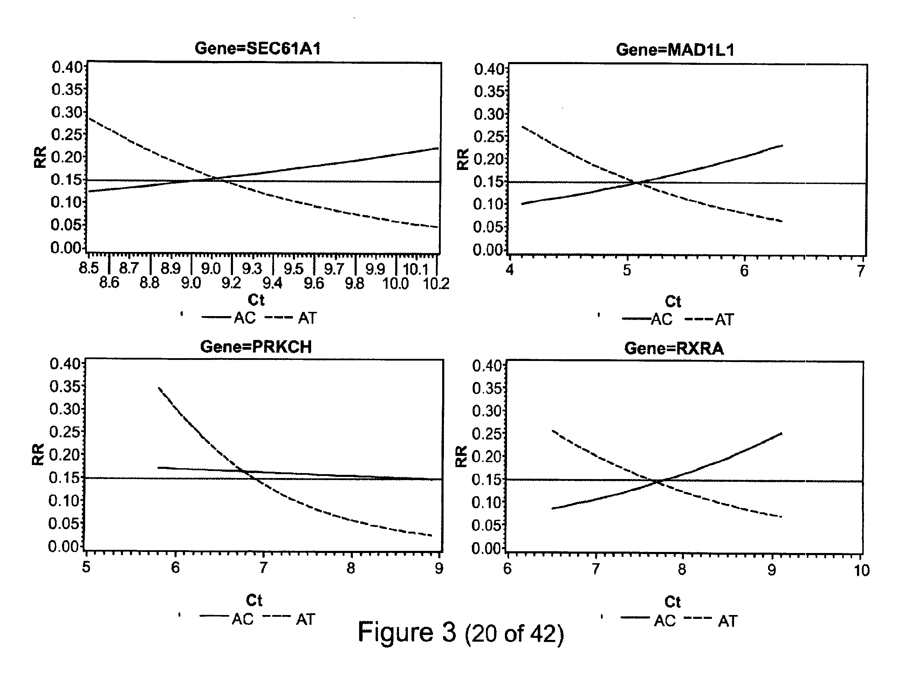

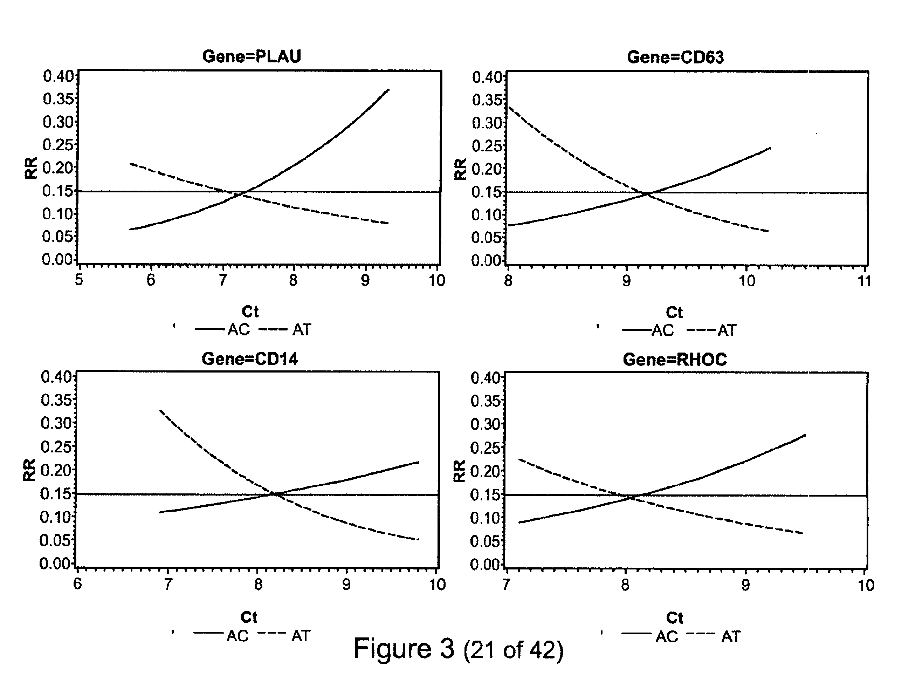

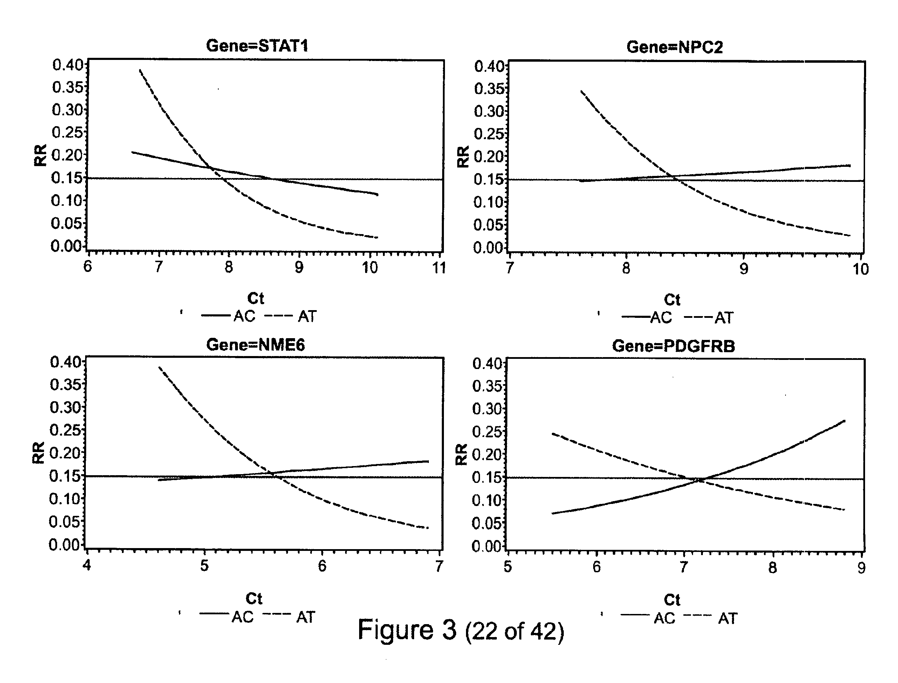

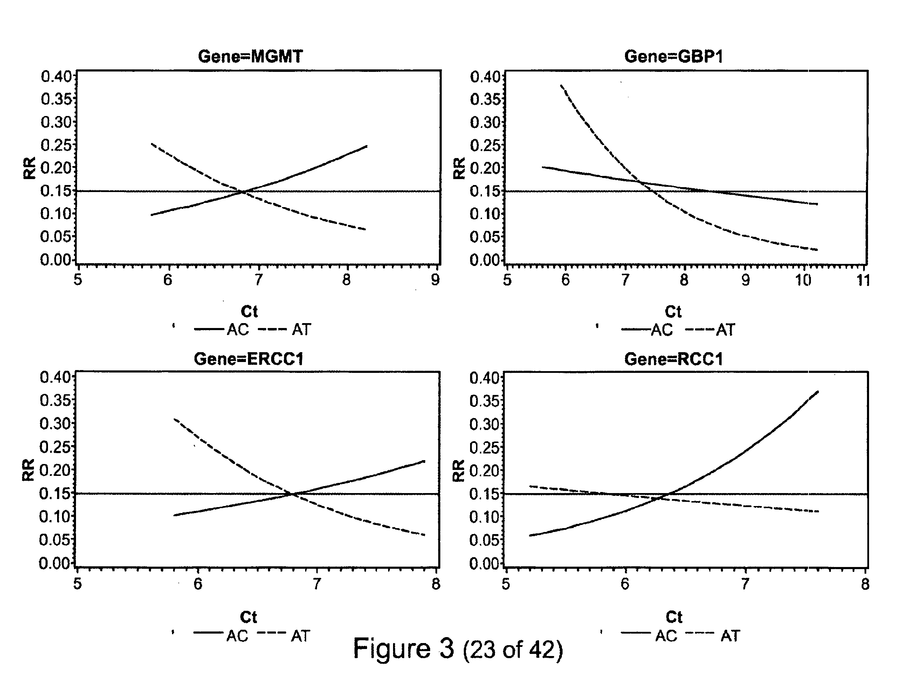

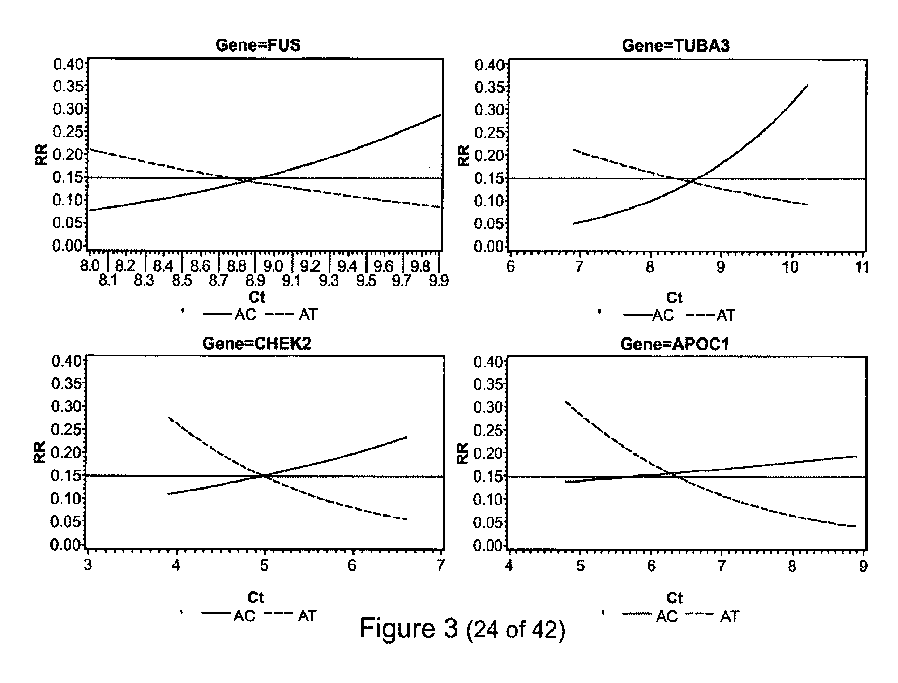

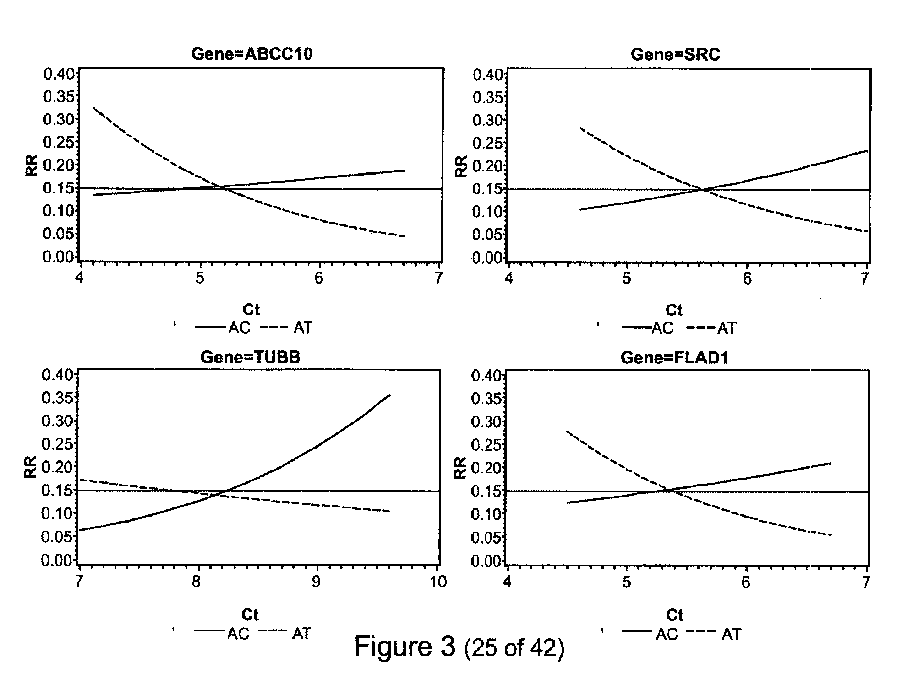

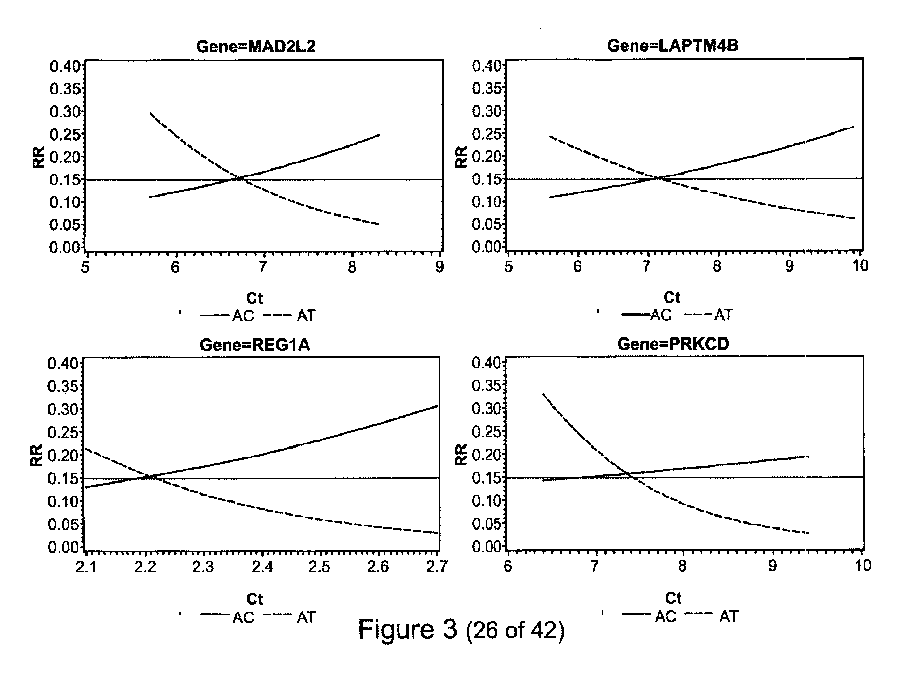

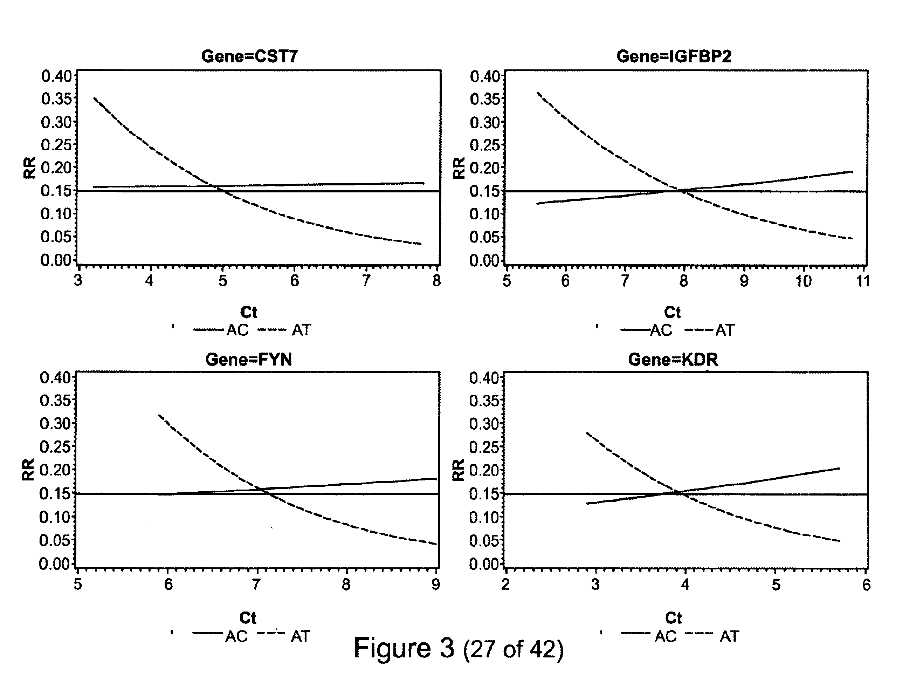

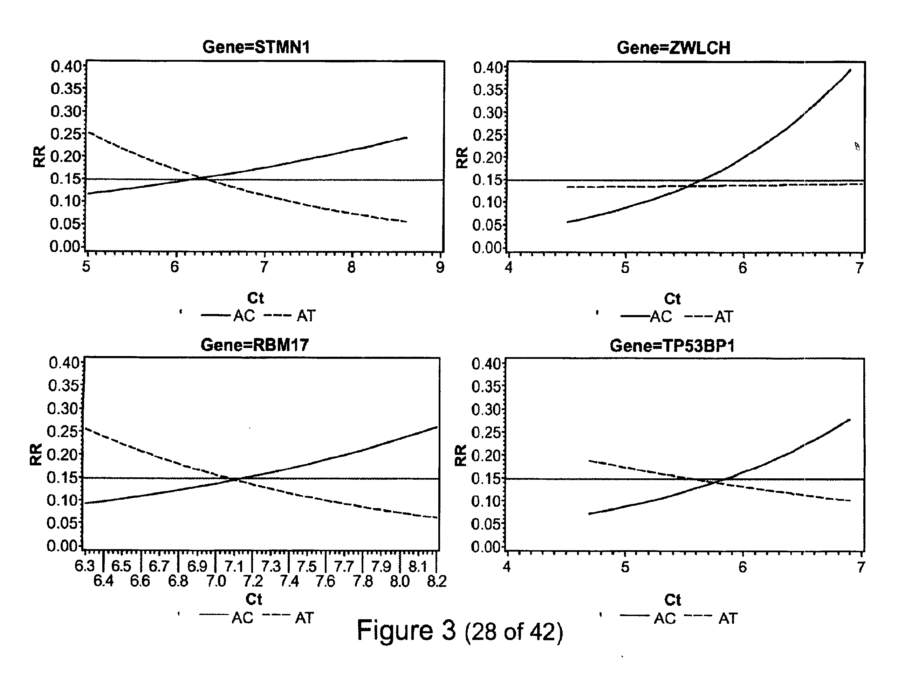

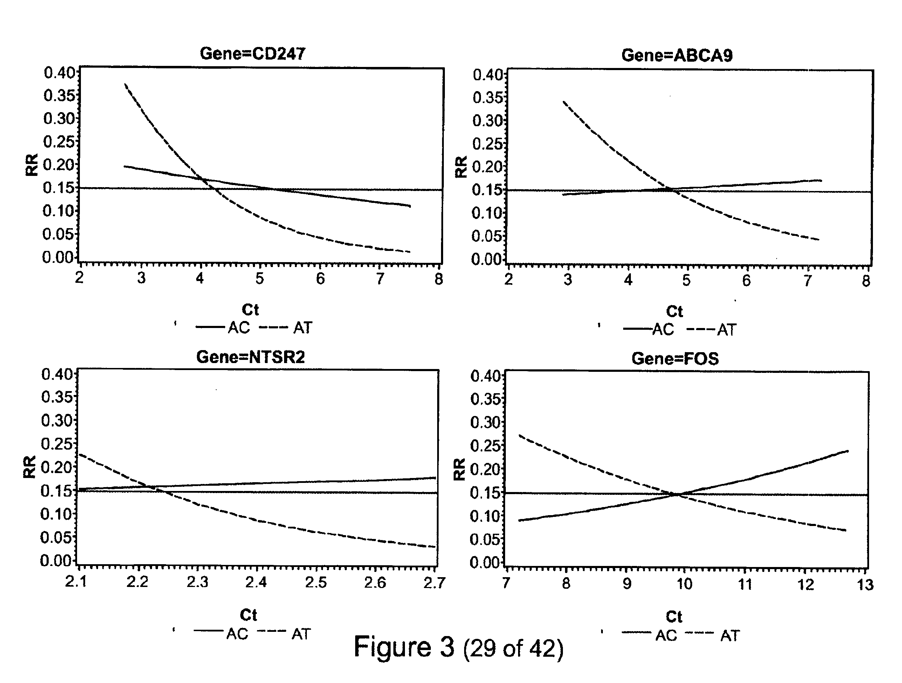

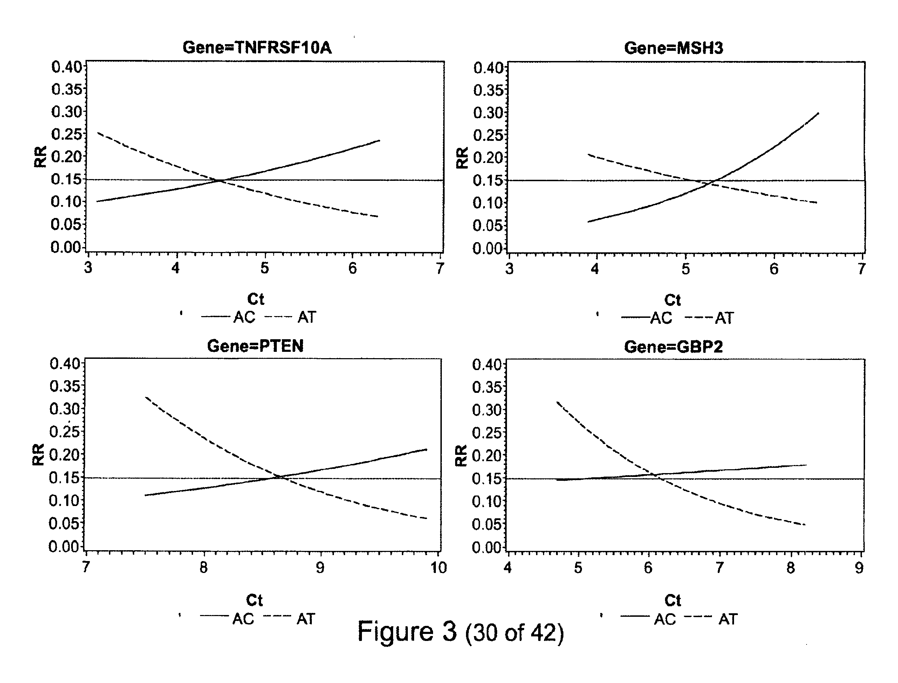

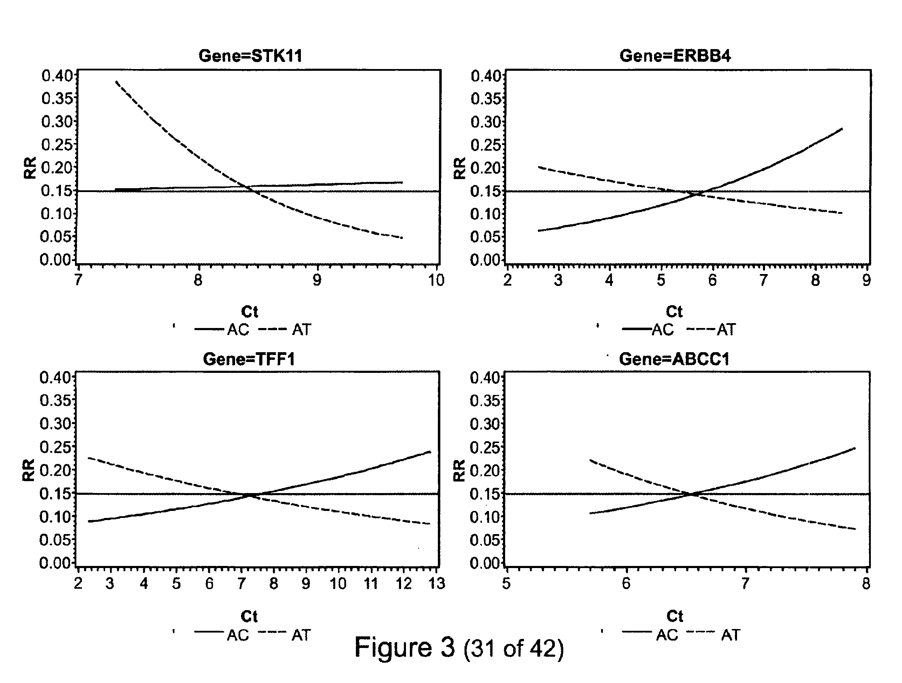

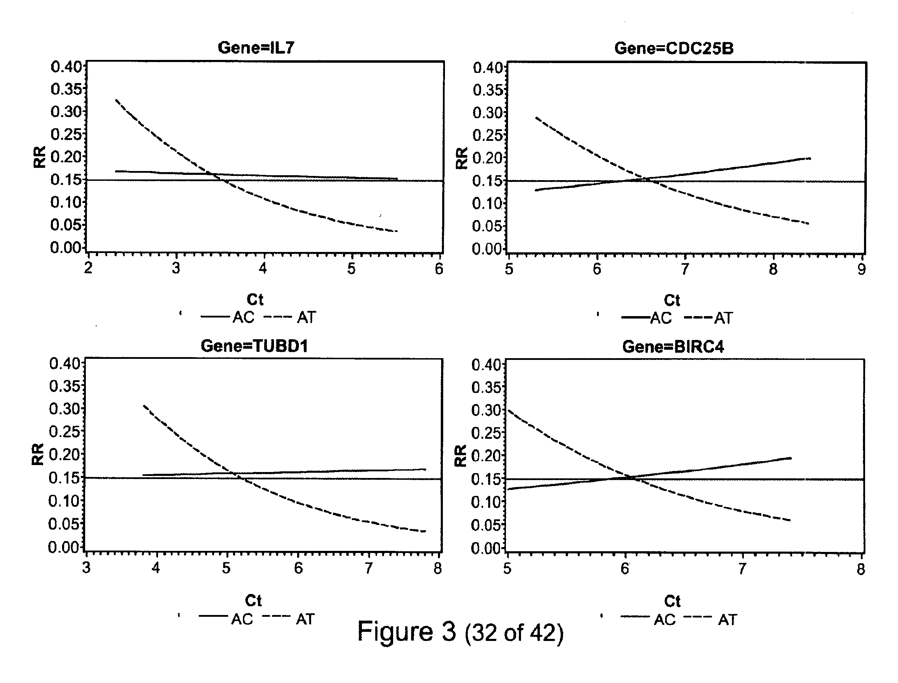

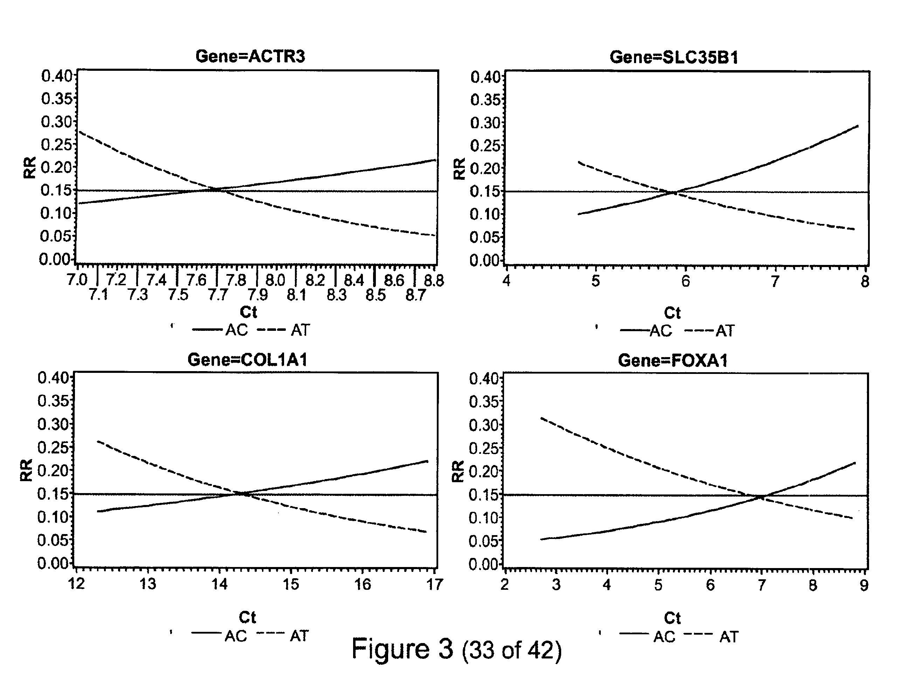

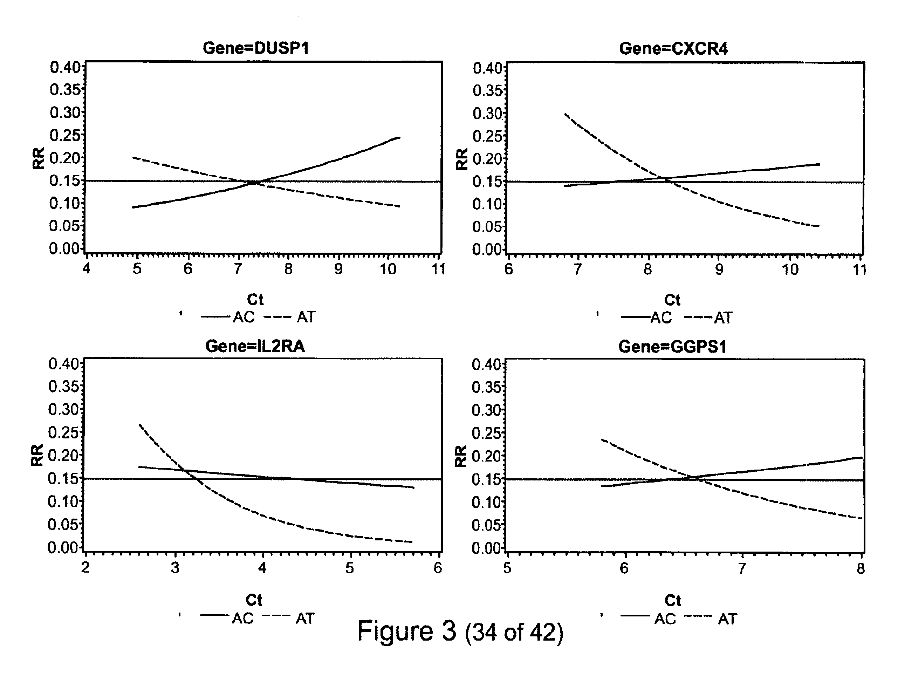

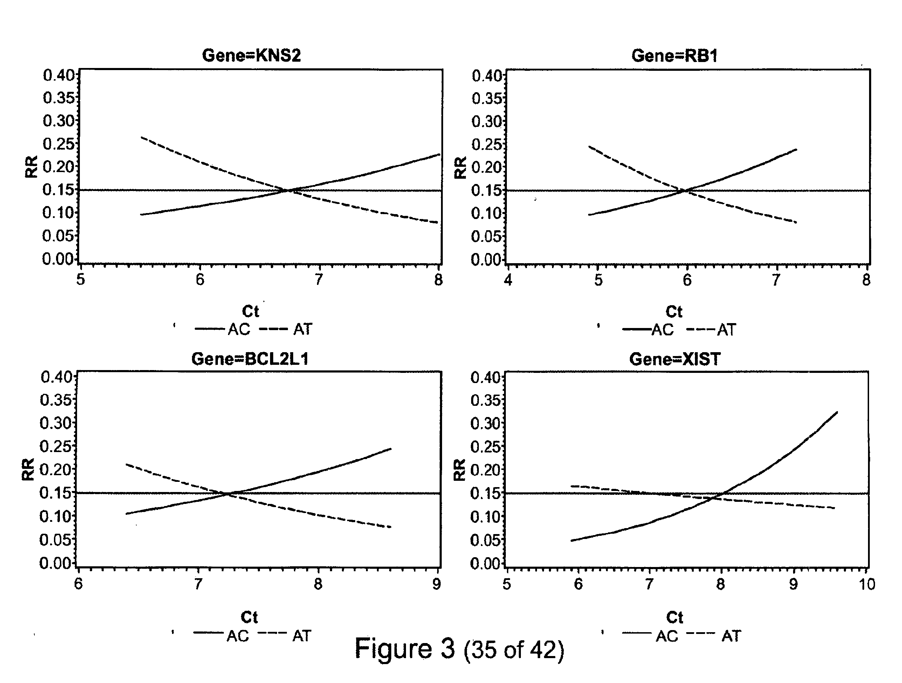

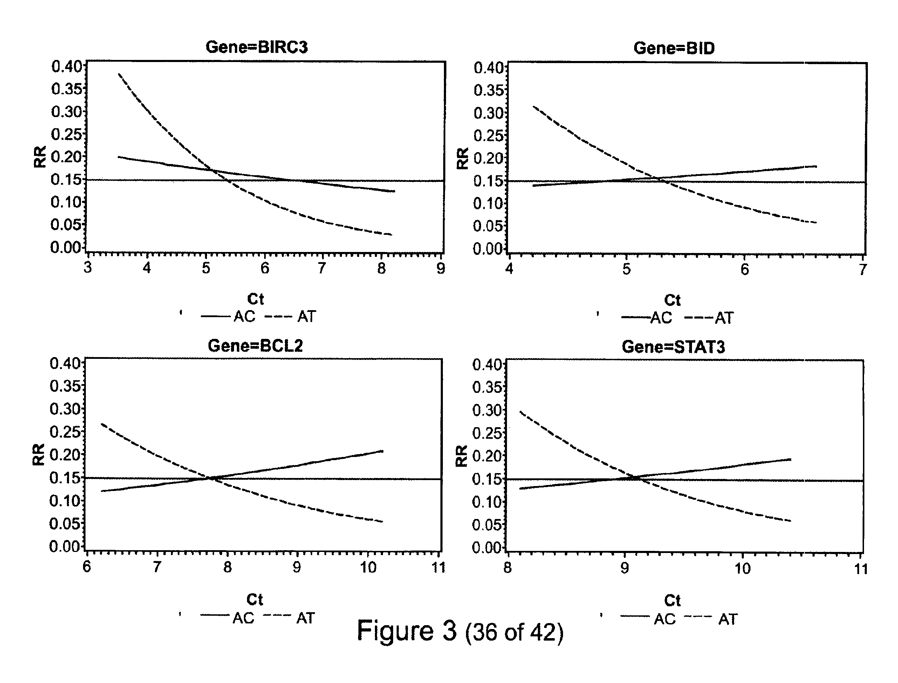

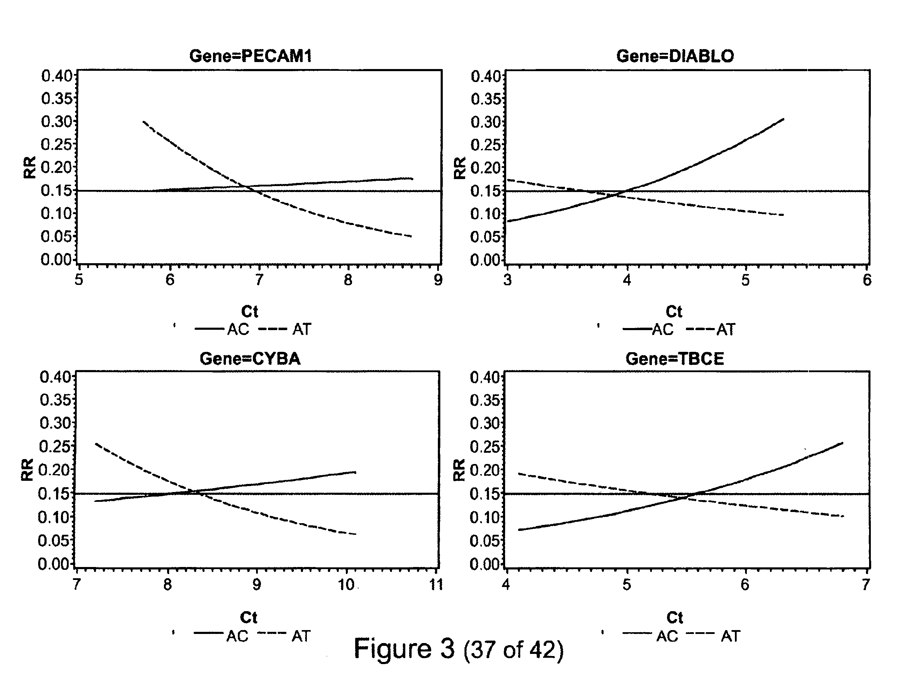

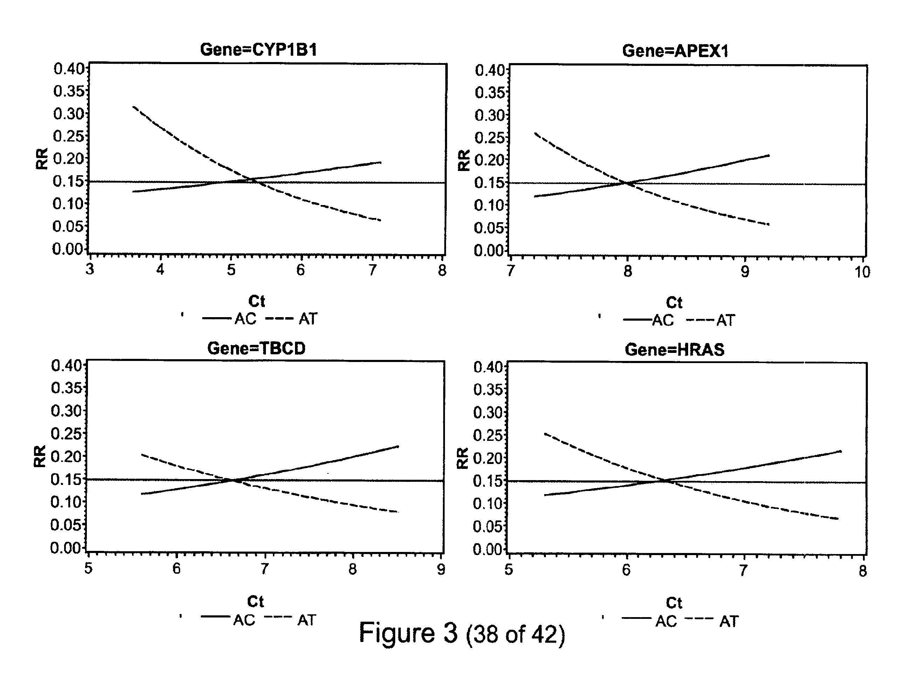

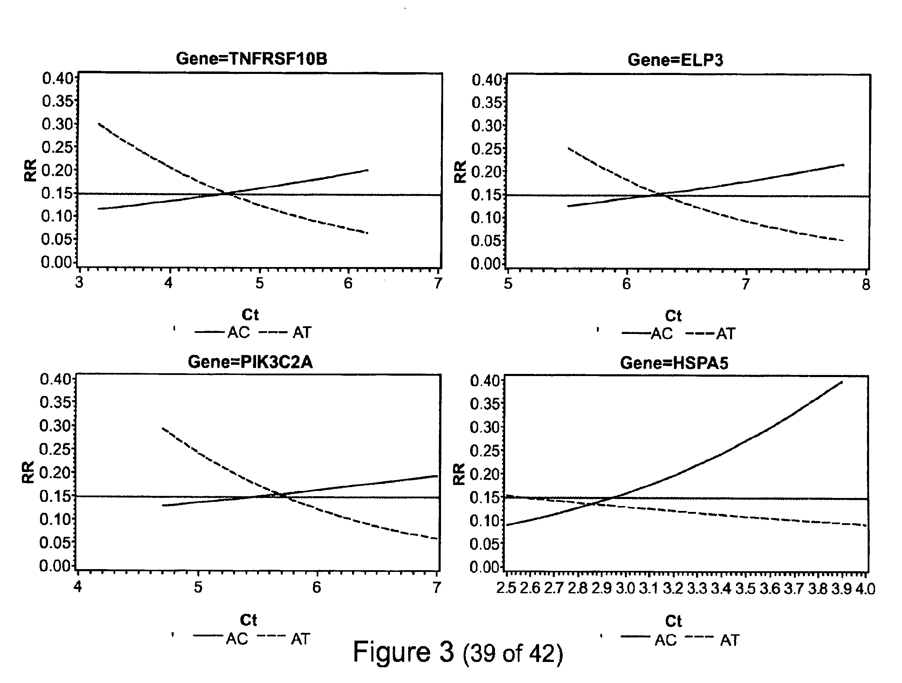

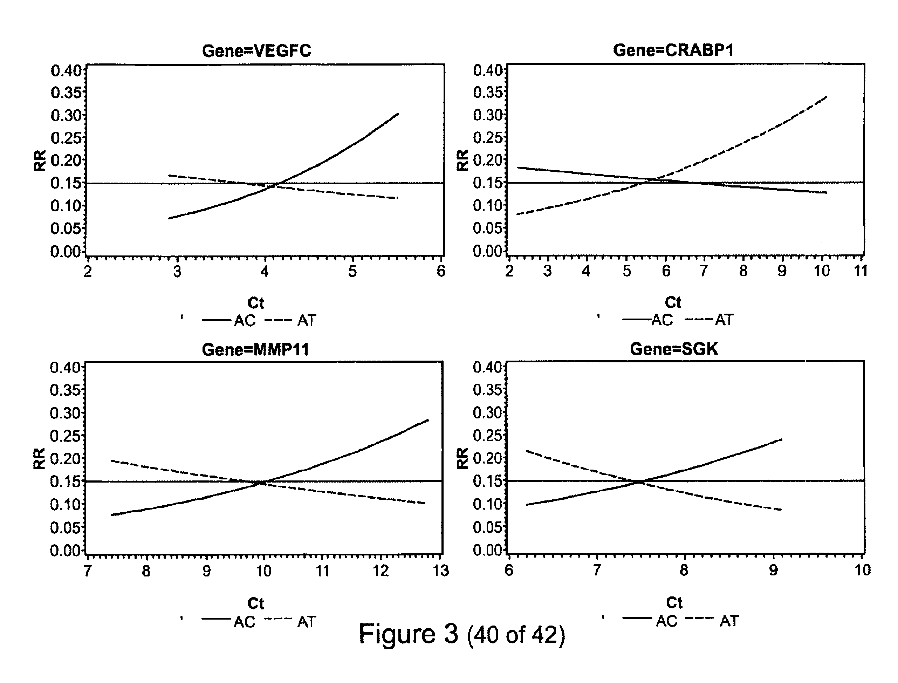

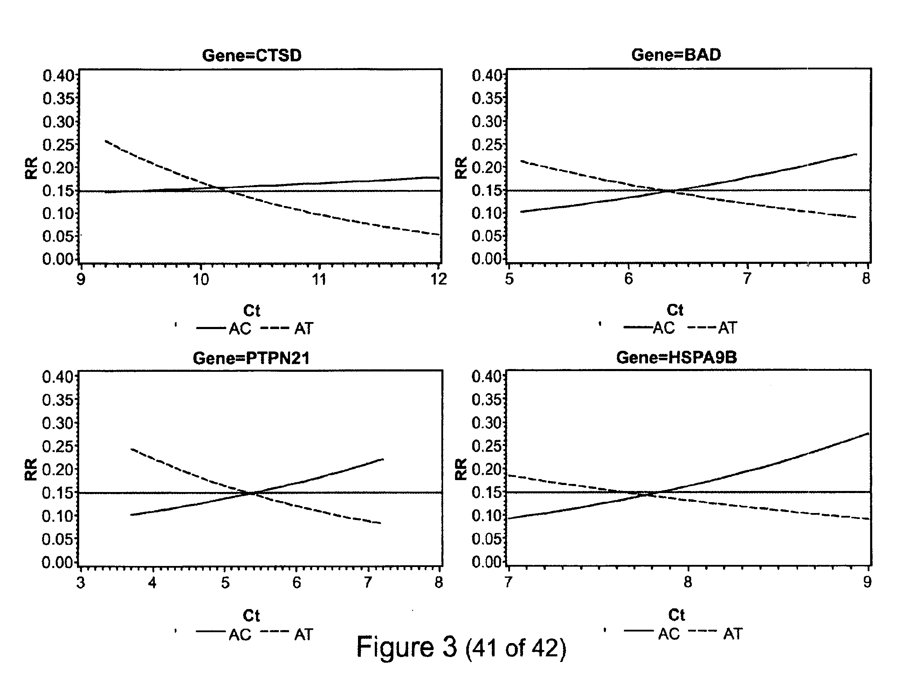

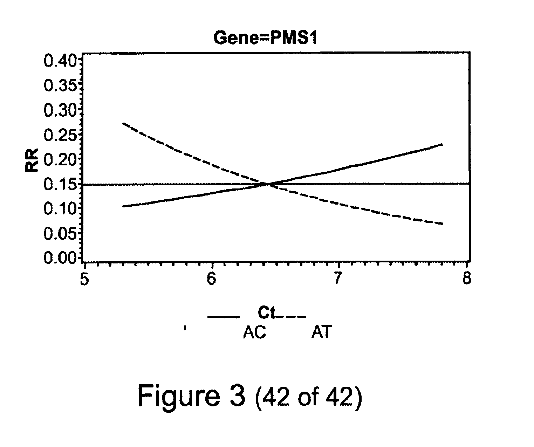

Figure 3 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor positive (HR⁺) breast cancer and an Oncotype Dx Recurrence Score of greater than 18. A horizontal dashed line in each graph represents the overall (i.e., not gene expression-specific) 5-year RR in patients in the study having HR+ breast cancer and an Oncotype Dx Recurrence Score greater than 18 who were randomized to treatment with either AC or AT.

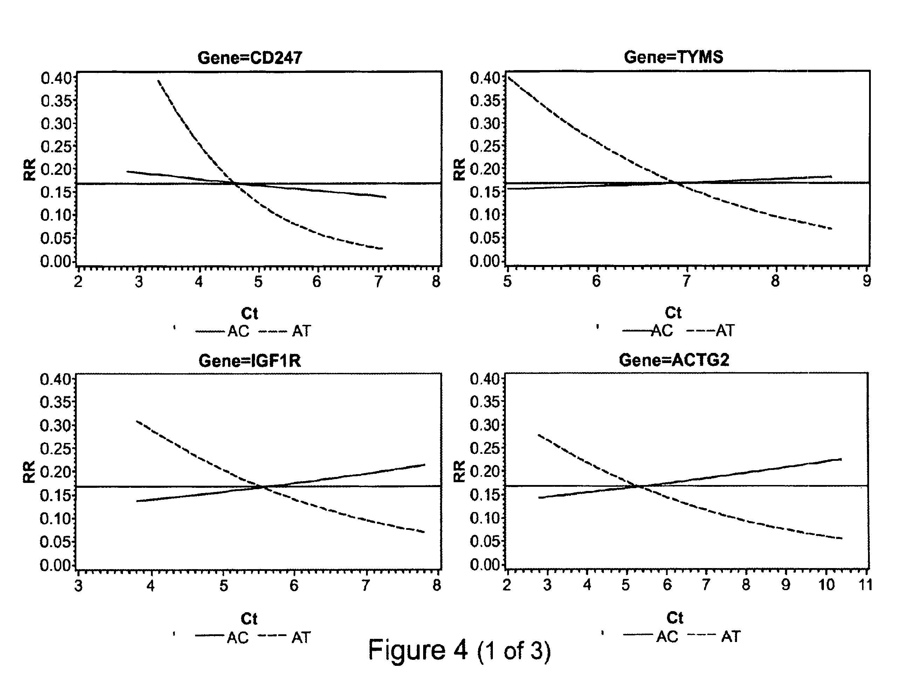

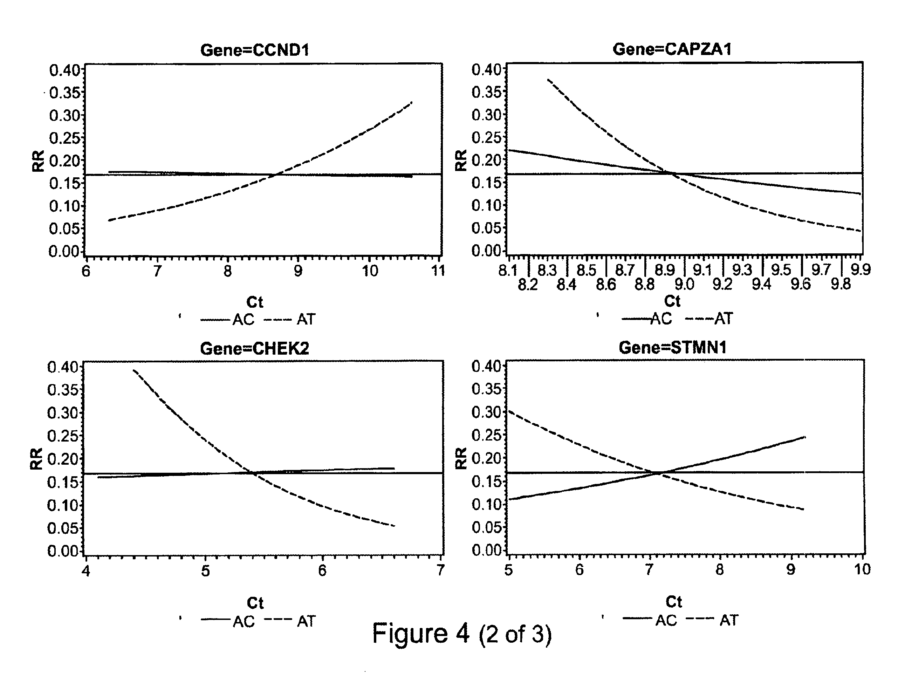

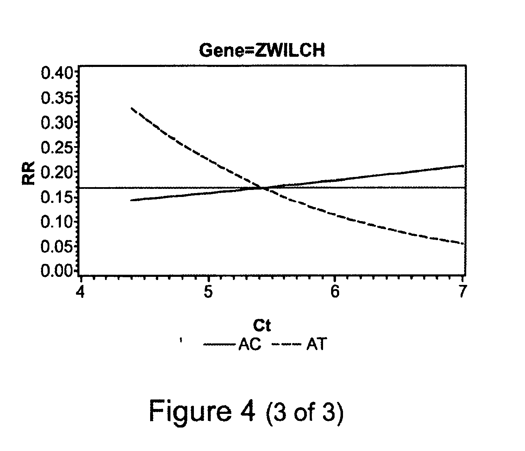

Figure 4 is a set of graphs showing the relationship between normalized expression (represented by "Cₜ") of the indicated gene (gene name provided at top of each graph) and 5-year recurrence rate (RR) of breast cancer in a treatment group receiving an anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor negative (HR⁻) breast. A horizontal dashed line in each graph represents the overall (i.e., not gene expression-specific) 5-year RR in patients in the study having HR⁻ breast cancer who were randomized to treatment with either AC or AT

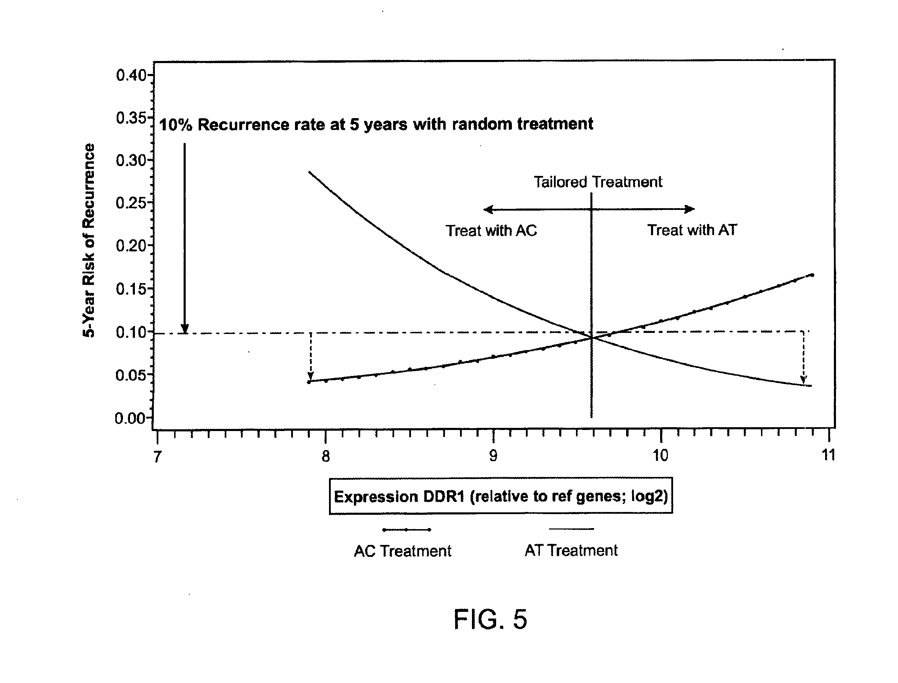

Figure 5 is a graph illustrating the impact of using DDR1 to select HR-positive patients for treatment with AC vs AT. The dotted line depicts the relationship between normalized expression of DDR1 and the 5-year recurrence rate (RR) of breast cancer in the AC treatment group (the AC prediction curve, also referred to as the cyclophosphamide benefit (CB) curve); the solid line depicts the relationship between normalized expression of DDR1 and the 5-year recurrence rate (RR) of breast cancer in the AT treatment group (the AT prediction curve, also referred to as the taxane benefit (TB) curve. Expression is provided on the x-axis as a normalized DDR1 expression level (relative to reference genes; log2). The y-axis provides the risk of cancer recurrence at 5 years.

The following Appendices and Tables are provided in the specification just prior to the claims.
Appendix 1. RT-PCR probe and primer sequences
Appendix 2. RT-PCR amplicon sequences
Table 1.Differential Markers of Response Identified in Breast Cancer Patients, All Patients.
Table 2. Differential Markers of Response Identified in Breast Cancer Patients, HR-Positive Patients
Table 3. Differential Markers of Response Identified in Breast Cancer Patients, HR-Positive Patients, RS > 18
Table 4. Differential Markers of Response Identified in Breast Cancer Patients, HR-Negative Patients.
Table 5. Additional genes involved in NFκB signaling

### DEFINITIONS

Unless defined otherwise, technical and scientific terms used herein have the same meaning as commonly understood by one of ordinary skill in the art to which this invention belongs. See, *e.g*., Singleton P and Sainsbury D., Dictionary of Microbiology and Molecular Biology 3rd ed., J. Wiley & Sons, Chichester, New York, 2001.

As used herein, the term "anthracycline" refers to a class of antineoplastic antibiotics that are typically derived by *Streptomyces* bacteria (e.g., *Streptomyces peucetius* or *Streptomyces coeruleorubidus*). Although the precise mechanism of action is unknown, anthracyclines are believed to derive their chemotherapeutic activity, at least in part, from their ability to damage DNA by intercalation, metal ion chelation, and the generation of free radicals and can inhibit enzyme activity critical to DNA function. Examples of anthracyclines include daunorubicin, doxorubicin, epirubicin, idarubicin, amrubicin, pirarubicin, valrubicin, zorubicin, caminomycin, detorubicin, esorubicin, marcellomycin, quelamycin, rodorubicin, and aclarubicin, as well as pharmaceutically active salts, acids or derivatives of any of these.

As used herein, the term "taxane" refers to a family of antimitotic/antimicrotubule agents that inhibit cancer cell growth by stopping cell division. Examples oftaxanes include paclitaxel, docetaxel, larotaxel, ortataxel, tesetaxel and other related diterpene compounds that have chemotherapeutic activity as well as pharmaceutically active salts, acids or derivatives of any of these. Paclitaxel was originally derived from the Pacific yew tree. Related diterpenes are produced by plants of the genus *Taxus* (yews) and synthetic or semi-synthetic taxanes with chemotherapeutic activity have also been synthesized, e.g., docetaxel, and are encompassed in the term taxane.

As used herein, the term "cyclophosphasmide" refers to a cytotoxic alkylating agent of the nitrogen mustard group, including the chemotherapeutic compound *N,N*-bis(2-chloroethyl)-1,3,2-oxazaphosphinan-2-amine 2-oxide (also known as cytophosphane). It is a highly toxic, immunosuppressive, antineoplastic drug, used in the treatment of Hodgkin's disease, lymphoma, and certain other forms of cancer, such as leukemia and breast cancer.

A "taxane-containing treatment" (also referred to as "taxane-containing regimen" or "taxane-containing treatment regimen") or "cyclophosphamide-containing treatment" (also referred to as "cyclophosphamide-containing regimen" or "cyclophosphamide-containing treatment regimen") is meant to encompass therapies in which a taxane or a cyclophosphamide, respectively, is administered alone or in combination with another therapeutic regimen (e.g., another chemotherapy (e.g., anthracycline), or both). Thus, a taxane-containing treatment can include, for example, administration a taxane in combination with anthracyline, with anthracyline and cyclosphophamide, and the like.

The term "in combination with" such as when used in reference to a therapeutic regimen, refers to administration or two or more therapies over the course of a treatment regimen, where the therapies may be administered together or separately, and, where used in reference to drugs, may be administered in the same or different formulations, by the same or different routes, and in the same or different dosage form type.

The term "prognosis" is used herein to refer to the prediction of the likelihood of cancer-attributable death or progression, including recurrence, of a neoplastic disease, such as breast cancer, in a patient. The concept of prognosis is used in the context of the minimal standard of care. For example, in the context of early stage, ER+ invasive breast care, the minimal standard of care could be surgery plus adjuvant hormonal therapy.

The term "prediction" is used herein to refer to a likelihood that a patient will have a particular clinical outcome following administration of a treatment regimen, e.g., a chemotherapeutic regimen. Clinical benefit may be measured, for example, in terms of clinical outcomes such as disease recurrence, tumour shrinkage, and/or disease progression.

The term "patient" or "subject" as used herein refers to a human patient. The term "long-term" survival is used herein to refer to survival for at least 3 years, more preferably for at least 8 years, most preferably for at least 10 years following surgery or other treatment.

The term "tumour," as used herein, refers to all neoplastic cell growth and proliferation, whether malignant or benign, and all pre-cancerous and cancerous cells and tissues. The terms "cancer" and "cancerous" refer to or describe the physiological condition in mammals that is typically characterized by unregulated cell growth.

The term "breast cancer" is used herein to include all forms and stages of breast cancer, including, without limitation, locally advanced breast cancer, invasive breast cancer, and metastatic breast cancer.

A "tumour sample" as used herein is a sample derived from, or containing tumour cells from, a patient's tumour. Examples of tumour samples herein include, but are not limited to, tumour biopsies, circulating tumour cells, circulating plasma proteins, ascitic fluid, primary cell cultures or cell lines derived from tumours or exhibiting tumour-like properties, as well as preserved tumour samples, such as formalin-fixed, paraffin-embedded tumour samples. The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

As used herein, the term "expression level" as applied to a gene refers to the normalized level of a gene product, e.g. the normalized value determined for the RNA expression level of a gene or for the polypeptide expression level of a gene.

The term "Cₜ" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The terms "threshold" or "thresholding" refer to a procedure used to account for non-linear relationships between gene expression measurements and clinical response as well as to further reduce variation in reported patient scores. When thresholding is applied, all measurements below or above a threshold are set to that threshold value. Non-linear relationship between gene expression and outcome could be examined using smoothers or cubic splines to model gene expression in Cox PH regression on recurrence free interval or logistic regression on recurrence status. Variation in reported patient scores could be examined as a function of variability in gene expression at the limit of quantitation and/or detection for a particular gene.

The term "gene product" or "expression product" are used herein to refer to the RNA transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA.

Unless indicated otherwise, each gene name used herein corresponds to the Official Symbol assigned to the gene and provided by Entrez Gene (URL: http://www.ncbi.nlm.nih.gov/sites/entrez) as of the filing date of this application.

The terms "correlated" and "associated" are used interchangeably herein to refer to a strength of association between two measurements (or measured entities). The disclosure provides genes and gene subsets, the expression levels of which are associated with a particular outcome measure, such as for example between the expression level of a gene and the likelihood of beneficial response to treatment with a drug. For example, the increased expression level of a gene may be positively correlated (positively associated) with an increased likelihood of good clinical outcome for the patient, such as an increased likelihood of long-term survival without recurrence of the cancer and/or beneficial response to a chemotherapy, and the like. Such a positive correlation may be demonstrated statistically in various ways, e.g. by a low hazard ratio. In another example, the increased expression level of a gene may be negatively correlated (negatively associated) with an increased likelihood of good clinical outcome for the patient. In that case, for example, the patient may have a decreased likelihood of long-term survival without recurrence of the cancer and/or beneficial response to a chemotherapy, and the like. Such a negative correlation indicates that the patient likely has a poor prognosis or will respond poorly to a chemotherapy, and this may be demonstrated statistically in various ways, e.g., a high hazard ratio.

A "positive clinical outcome" and "beneficial response" can be assessed using any endpoint indicating a benefit to the patient, including, without limitation, (1) inhibition, to some extent, of tumour growth, including slowing down and complete growth arrest; (2) reduction in the number of tumour cells; (3) reduction in tumour size; (4) inhibition (i.e., reduction, slowing down or complete stopping) of tumour cell infiltration into adjacent peripheral organs and/or tissues; (5) inhibition of metastasis; (6) enhancement of anti-tumour immune response, possibly resulting in regression or rejection of the tumour; (7) relief, to some extent, of one or more symptoms associated with the tumour; (8) increase in the length of survival following treatment; and/or (9) decreased mortality at a given point of time following treatment. Positive clinical response may also be expressed in terms of various measures of clinical outcome. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

The term "risk classification" means a level of risk (or likelihood) that a subject will experience a particular clinical outcome. A subject may be classified into a risk group or classified at a level of risk based on the methods of the present disclosure, e.g. high, medium, or low risk. A "risk group" is a group of subjects or individuals with a similar level of risk for a particular clinical outcome.

The term "normalized expression" with regard to a gene or an RNA transcript or other expression product (e.g., protein) is used to refer to the level of the transcript (or fragmented RNA) determined by normalization to the level of reference mRNAs, which might be all measured transcripts in the specimen or a particular reference set of mRNAs. A gene exhibits "increased expression" or "increased normalized expression" in a subpopulation of subjects when the normalized expression level of an RNA transcript (or its gene product) is higher in one clinically relevant subpopulation of patients (e.g., patients who are responsive to chemotherapy treatment) than in a related subpopulation (e.g., patients who are not responsive to said chemotherapy). In the context of an analysis of a normalized expression level of a gene in tissue obtained from an individual subject, a gene is exhibits "increased expression" when the normalized expression level of the gene trends toward or more closely approximates the normalized expression level characteristic of such a clinically relevant subpopulation of patients. Thus, for example, when the gene analyzed is a gene that shows increased expression in responsive subjects as compared to non-responsive subjects, then if the expression level of the gene in the patient sample trends toward a level of expression characteristic of a responsive subject, then the gene expression level supports a determination that the individual patient is likely to be a responder. Similarly, where the gene analyzed is a gene that is increased in expression in non-responsive patients as compared to responsive patients, then if the expression level of the gene in the patient sample trends toward a level of expression characteristic of a non-responsive subject, then the gene expression level supports a determination that the individual patient will be nonresponsive. Thus normalized expression of a given gene as disclosed herein can be described as being positively correlated with an increased likelihood of positive clinical response to chemotherapy or as being positively correlated with a decreased likelihood of a positive clinical response to chemotherapy.

The term "recurrence score" or "RS" refers to an algorithm-based indicator useful in determining the likelihood of an event of interest, such as a likelihood of cancer recurrence and/or the likelihood that a patient will respond to a treatment modality as may be assessed by cancer recurrence following therapy with the treatment modality.

The term "hormone receptor positive (HR+) tumour" means a tumour expressing either estrogen receptor (ER+) or progesterone receptor (PR+) above a certain threshold as determined by standard methods, including immunohistochemical staining of nuclei and polymerase chain reaction (PCR) in a biological sample obtained from a patient. The term "hormone receptor negative (HR-) tumour" means a tumour that does not express either estrogen receptor (ER-) or progesterone receptor (PR-) above a certain threshold. The threshold may be measured, for example, using an Allred score or gene expression. See, e.g., J. Harvey, et al., J Clin Oncol 17:1474-1481 (1999); S. Badve, et al., J Clin Oncol 26(15):2473-2481 (2008). "Overall survival (OS)" refers to the patient remaining alive for a defined period of time, such as 1 year, 5 years, etc, e.g., from the time of diagnosis or treatment.

"Progression-free survival (PFS)" refers to the patient remaining alive, without the cancer getting worse.

"Neoadjuvant therapy" is adjunctive or adjuvant therapy given prior to the primary (main) therapy. Neoadjuvant therapy includes, for example, chemotherapy, radiation therapy, and hormone therapy. Thus, chemotherapy may be administered prior to surgery to shrink the tumour, so that surgery can be more effective, or, in the case of previously unoperable tumours, possible.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1 % bovine serum albumin/0.1% Ficoll/0.1% polyvinylpyrrolidone/50 mM sodium phosphate buffer at pH 6.5 with 750 mM sodium chloride, 75 mM sodium citrate at 42 °C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42 °C, with washes at 42 °C in 0.2 x SSC (sodium chloride/sodium citrate) and 50% formamide at 55 °C, followed by a high-stringency wash consisting of 0.1 x SSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37 °C. in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50 °C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

Herein, numerical ranges or amounts prefaced by the term "about" expressly include the exact range or exact numerical amount.

### GENERAL DESCRIPTION

The disclosed methods are useful to facilitate treatment decisions by providing an assessment of the likelihood of clinical benefit to a treatment that includes a taxane, a treatment that includes a cyclophosphamide, or both. Because taxanes and cyclophosphamide have different mechanisms of action, it is possible that tumours of certain patients exhibit molecular pathology that makes them more likely to respond to one drug type than the other. For example, the methods disclosed herein can be used to facilitate treatment decisions by providing an assessment of the likelihood of clinical benefit to an anthracycline-based treatment that includes a taxane, an anthracycline-based treatment that includes a cyclophosphamide, or an anthracycline-based treatment that includes both a cyclophosphamide and a taxane. Accordingly, such predictive methods are useful to facilitate chemotherapy treatment decisions that are tailored to individual patients. For example, the methods disclosed herein can be used to assess whether there is clinical benefit to addition of a taxane to a chemotherapeutic regimen. Genes for which expression is correlated either positively or negatively with increased likelihood of response to a treatment that includes a taxane, a treatment that includes a cyclophosphamide, or both are provided in Figures 1-4 and Tables 1-4.

The relationships between expression level of a marker gene of the present disclosure and a positive or negative correlation with likelihood of recurrence of cancer (e.g., breast cancer) following treatment with a taxane-containing regimen or a cyclophosphamide-containing regimen are exemplified in Figures 1-4. The hatched line in each graph represents the relationship between expression of the gene in patients treated with a taxane-containing regimen (e.g., anthracycline plus a taxane) and the 5-year recurrence rate (RR) of cancer (the taxane benefit (TB) prediction curve). The TB prediction line thus represents the correlation of expression of the gene and the likelihood of clinical benefit of a taxane in a treatment regimen. The smooth line in each graph represents the relationship between expression of the gene in patients treated with a cyclophosphamide-containing regimen (e.g., anthracycline plus cyclophosphamide) and the 5-year recurrence rate (RR) of cancer (the cyclophosphamide benefit (CB) prediction curve). The CB prediction curve thus represents the correlation of expression of the gene and the likelihood of clinical benefit of a cyclophosphamide in a treatment regimen. Because the patients in the study also received an anthracycline, the TB prediction curve and CB prediction curve can also be considered an anthracycline plus a taxane (AT) benefit prediction curve and an anthracycline plus a cyclophosphamide (AC) benefit prediction curve, respectively. Each of the graphs in Figures 1-4 include a horizontal dashed line that represents the overall (i.e., not gene expression-specific) recurrence rate at 5-years in the relevant population who were randomized to treatment with AC or AT. The difference between the TB and CB prediction curves and this horizontal line depicts the extent to which clinical benefit may be improved by a gene expression-guided treatment decision.

Other characteristics of the tumour can be taken into account when assessing likelihood of taxane and/or cyclophosphamide benefit by analysis of expression level of a marker gene disclosed herein. For example, hormone receptor expression status (e.g., ER⁺, ER⁻, PR⁺, PR⁻) can be assessed for the tumour sample, and taken into consideration when evaluating expression levels of the marker gene, e.g., the expression level is compared to expression level correlations to TB and/or CB in a population sharing the same characteristics. For example, Figure 1 provides TB (AT) and CB (AC) prediction curves in all patients in the study discussed in the Examples below without regard to hormone expression status or likelihood of cancer recurrence as predicted by the Oncotype DX RS. Figure 2 provides TB (AT) and CB (AC) prediction curves in hormone receptor positive patients. Figure 3 provides TB (AT) and CB (AC) prediction curves in hormone receptor positive patients having an Oncotype DX RS score of about 18 or greater, which indicates a significant risk of cancer recurrence within 10 years following surgery and tamoxifen therapy. Figure 4 provides TB (AT) and CB (AC) prediction curves in hormone receptor negative patients.

The prediction curves can be used to assess information provided by an expression level of a marker gene disclosed herein and in turn facilitate a treatment decision with respect to selection of a taxane-containing and/or a cyclophosphamide-containing regimen. For example, where a gene exhibits an expression level having a TB (AT) prediction curve having a negative slope as exemplified in Figures 1-4, then increasing normalized expression levels ofthe gene are positively correlated with a likelihood of clinical benefit of including a taxane in the treatment regimen (since patients who exhibited this expression pattern of the particular gene had lower recurrence rates following a taxane-containing regimen). Conversely, where a gene exhibits an expression level having a TB (AT) prediction curve having a positive slope as exemplified in Figures 1-4, then increasing normalized expression levels of the gene are negatively correlated with a likelihood of clinical benefit of including a taxane in the treatment regimen. Similarly, where a gene exhibits an expression level having a CB (AC) prediction curve having a negative slope as exemplified in Figures 1-4, then increasing normalized expression levels ofthe gene are positively correlated with a likelihood of clinical benefit of including a cyclophosphamide in the treatment regimen (since patients who exhibited this expression pattern of the particular gene had lower recurrence rates following cyclophosphamide-containing regimen). Conversely, where a gene exhibits an expression level having a CB (AC) prediction curve having a positive slope as exemplified in Figures 1-4, then increasing normalized expression levels of the gene are negatively correlated with a likelihood of clinical benefit of including a cyclophosphamide in the treatment regimen.

Accordingly, the expression levels of the marker genes can be used to facilitate a decision as to whether a taxane should be included or excluded in a treatment regimen, and to facilitate a decision as to whether a cyclophosphamide should be included or excluded in a treatment regimen. The marker genes can be used to facilitate selection of a treatment regimen that includes, a taxane and/or a cyclophosphamide, or neither a taxane nor a cyclophosphamide. In some instances the marker gene expression level may suggest clinical benefit for both a taxane and a cyclophosphamide, e.g., where increasing expression levels are associated with a recurrence risk below a selected recurrence risk. For example, as illustrated in Fig. 2 for the gene ZW10, increased expression of ZW10 in HR-positive cancer patients is associated with increased likelihood of clinical benefit for both a taxane and for a cyclophosphamide. In addition, because the magnitudes of the slopes are significantly different, patients with increased expression of ZW10 are predicted to have lower risks of recurrence if treated with AT instead of AC, and patients with decreased expression of ZW10 are predicted to have lower risks of recurrence if treated with AC instead of AT. Thus, the marker genes that are associated with TB (AT) and CT (AC) prediction curves that differ in slope can facilitate a decision in selecting between a taxane-containing regimen and a cyclophosphamide-containing regimen, even where there may be clinical benefit with either or both treatment regimen.

The methods of the present disclosure also can facilitate selection between a taxane-containing regimen and a cyclophosphamide-containing regimen (e.g., between and AT and AC therapy). For example, where the curves in Figures 1-4 have significantly different slopes in the Cox regression model and the TB (AT) and CB (AC) prediction curves cross, expression levels of the marker gene can be used to assess the likelihood the patient will respond to a taxane-containing regimen (e.g., AT) or to a cyclophosphamide-containing regimen (e.g., AC).

For example, Fig. 5 illustrates a plot of the 5-year risk of relapse versus gene expression, presented for an exemplary gene, DDR1. As illustrated in Fig. 5, the expression level of DDR1 can be used to facilitate selection of therapy where treatment with a cyclophosphamide is favored over treatment with a taxane at lower expression levels of DDR1, with a "switch" of the relative clinical benefit of these therapies occurring at a point where the recurrence risk associated with taxane treatment is lower than that associated with cyclophosphamide treatment, thus favoring a treatment regimen including a taxane over a cyclophosphamide.

There are many types of systemic treatment regimens available for patients diagnosed with cancer. For example, the table below lists various chemotherapeutic and hormonal therapies for breast cancer.

### Single Agents Useful in Breast Cancer

| GENERIC NAME | COMMON TRADE NAME | CLASS |
|---|---|---|
| Cyclophosphamide (C) | Cytoxan® | Nitrogen mustards |
| Doxorubicin | Adriamycin® | Anthracyclines |
| Epirubicin | Pharmorubicin® | Anthracyclines |
| Fluorouracil | | Pyrimidine analogs |
| Methotrexate | Rheumatrex® | Folic acid analogs |
| Paclitaxel | Taxol® | Taxanes (T) |
| Docetaxel | Taxotere® | Taxanes (T) |
| Capecitabine | Xeloda® | Pyrimidine analogs |
| Trastuzumab | Herceptin® | Monoclonal Antibodies |
| Bevacizumab | Avastin® | Monoclonal Antibodies |

### Combinations Useful in Breast Cancer

| | | |
|---|---|---|
| CAF | Cyclophosphamide, Adriamycin, Fluorouracil | US |
| CMF | Cyclophosphamide, Methotrexate, Fluorouracil | US |
| AC | Adriamycin, Cyclophosphamide | US |
| AT | Adriamycin, Taxane | US |
| ACT | Adriamycin, Cyclophosphamide, Taxane | US |
| TAC | Taxane, Adriamycin, Cyclophosphamide | US |
| TC | Taxane, Cyclophosphamide | US |
| | Fluorouracil, Epirubicin, Cyclophosphamide | Europe |

### GENE EXPRESSION PROFILING

The practice of the methods and compositions of the present disclosure will employ, unless otherwise indicated, conventional techniques of molecular biology (including recombinant techniques), microbiology, cell biology, and biochemistry, which are within the skill ofthe art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

Methods of gene expression profiling include methods based on hybridization analysis of polynucleotides, methods based on sequencing of polynucleotides, and proteomics- based methods. Exemplary methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription PCT (RT-PCR) (Weis et al., Trends in Genetics 8:263-264 (1992)). Antibodies may be employed that can recognize sequence-specific duplexes, including DNA duplexes, RNA duplexes, and DNA-RNA hybrid duplexes or DNA-protein duplexes. Representative methods for nucleic acid sequencing analysis include Serial Analysis of Gene Expression (SAGE), and Digital Gene Expression (DGE). Representative methods of gene expression profiling are disclosed, for example, in U.S. Patent Nos. 7,056,674 and 7,081,340, and in U.S. Patent Publication Nos. 20020095585; 20050095634; 20050260646; and 20060008809. Representative scientific publications including methods of gene expression profiling, including data analysis, include Gianni et al., J Clin Oncol. 2005 Oct 10;23(29):7265-77; Paik et al., N Engl J Med. 2004 Dec 30;351(27):2817-26; and Cronin et al., Am J Pathol. 2004 Jan;164(1):35-42.

### Reverse Transcriptase PCR (RT-PCR)

Typically, mRNA is isolated from a test sample. The starting material is typically total RNA isolated from a human tumour, usually from a primary tumour. Optionally, normal tissues from the same patient can be used as an internal control. mRNA can be extracted from a tissue sample, e.g., from a sample that is fresh, frozen (e.g. fresh frozen), or paraffin-embedded and fixed (e.g. formalin-fixed).

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumour can be isolated, for example, by cesium chloride density gradient centrifugation.

The sample containing the RNA is then subjected to reverse transcription to produce cDNA from the RNA template, followed by exponential amplification in a PCR reaction. The two most commonly used reverse transcriptase enzymes are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

PCR-based methods use a thermostable DNA-dependent DNA polymerase, such as a Taq DNA polymerase. For example, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction product. A third oligonucleotide, or probe, can be designed to facilitate detection of a nucleotide sequence of the amplicon located between the hybridization sites the two PCR primers. The probe can be detectably labeled, e.g., with a reporter dye, and can further be provided with both a fluorescent dye, and a quencher fluorescent dye, as in a Taqman® probe configuration. Where a Tasman® probe is used, during the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data. TaqMan® RT-PCR can be performed using commercially available equipment, such as, for example, ABI PRISM 7700^{™} Sequence Detection System^{™} (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700^{™} Sequence Detection System^{™}. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as a threshold cycle ("Cₜ"). Fluorescence values are recorded during every cycle and represent the amount of product amplified to that point in the amplification reaction. The threshold cycle (Cₜ) is generally described as the point when the fluorescent signal is first recorded as statistically significant.

It is desirable to correct for (normalize away) both differences in the amount of RNA assayed and variability in the quality of the RNA used. Therefore, the assay typically measures, and expression analysis of a marker gene incorporates analysis of, the expression of certain reference genes (or "normalizing genes"), including well known housekeeping genes, such as GAPDH. Alternatively, normalization can be based on the mean or median signal (Ct) of all of the assayed genes or a large subset thereof (often referred to as a "global normalization" approach). On a gene-by-gene basis, measured normalized amount of a patient tumour mRNA may be compared to the amount found in a colon cancer tissue reference set. See M. Cronin, et al., Am. Soc. Investigative Pathology 164:35-42 (2004).

Gene expression measurements can be normalized relative to the mean of one or more (e.g., 2, 3, 4, 5, or more) reference genes. Reference-normalized expression measurements can range from 0 to 15, where a one unit increase generally reflects a 2-fold increase in RNA quantity.

RT- PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for use in the methods of the present disclosure use fixed, paraffin-embedded tissues as the RNA source mRNA isolation, purification, primer extension and amplification can be preformed according to methods available in the art. (see, e.g., Godfrey et al. J. Molec. Diagnostics 2: 84-91 (2000); Specht et al., Am. J. Pathol. 158: 419-29 (2001)). Briefly, a representative process starts with cutting about 10 µm thick sections of paraffin-embedded tumour tissue samples. The RNA is then extracted, and protein and DNA depleted from the RNA-containing sample. After analysis of the RNA concentration, RNA is reverse transcribed using gene specific primers followed by RT-PCR to provide for cDNA amplification products.

### Design of Intron-Based PCR Primers and Probes

PCR primers and probes can be designed based upon exon or intron sequences present in the mRNA transcript of the gene of interest. Primer/probe design can be performed using publicly available software, such as the DNA BLAT software developed by Kent, W.J., Genome Res. 12(4):656-64 (2002), or by the BLAST software including its variations.

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz et al. (eds.) Bioinformatics Methods and Protocols: Methods in Molecular Biology. Humana Press, Totowa, NJ, pp 365-386). Other factors that can influence PCR primer design include primer length, melting temperature (Tm), and G/C content, specificity, complementary primer sequences, and 3 '-end sequence. In general, optimal PCR primers are generally 17-30 bases in length, and contain about 20-80%, such as, for example, about 50-60% G+C bases, and exhibit Tm's between 50 and 80 °C, e.g. about 50 to 70 °C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997).

### Quantitative PCR for Gene Expression Analysis

Per VanGuilder et al., BioTechniques 44: 619 (2008), quantitative PCR (qPCR) now represents the method of choice for analyzing gene expression of numerous genes in anywhere from a small number to thousands of samples. For investigators studying gene expression, there is a multitiered technological approach depending on the number of genes and samples being examined. Gene expression microarrays are still the preferred method for large-scale (e.g., whole-genome) discovery experiments. Due to the logistics, sensitivity, and costs of whole-genome micorarrays, there is also a niche for focused microarrays that allow for analysis of a smaller number of genes in a larger number of samples. Nonetheless, for validation of microarray discovery, reverse-transcription quantitative PCR (RT-qPCR) remains the gold standard. The current maturation of real-time qPCR with fluorescent probes allows for rapid and easy confirmation of microarray results in a large number of samples. Often, a whole-genome discovery experiment is not required, as the gene or pathway of interest is already known. In that case, the data collection can begin with qPCR. Finally, qPCR has also shown great utility in biomarker monitoring. In this scenario, previously developed identified targets can be assayed in very large numbers of samples (1000s).

Data Analysis. Analysis of real-time qPCR data has also reached a mature stage of development. Analyses can be either of absolute levels (i.e., numbers of copies of a specific RNA per sample) or relative levels (i.e., sample 1 has twice as much mRNA of a specific gene as sample 2). By far, the majority of analyses use relative quantitation as this is easier to measure and is of primary interest to researchers examining disease states. For absolute quantitation, an RNA standard curve of the gene of interest is required in order to calculate the number of copies. In this case, a serial dilution of a known amount (number of copies) of pure RNA is diluted and subjected to amplification. Like a protein assay, the unknown signal is compared with the curve so as to extrapolate the starting concentration.

The most common method for relative quantitation is the 2^{-ΔΔCT} method. This method relies on two assumptions. The first is that the reaction is occurring with 100% efficiency; in other words, with each cycle of PCR, the amount of product doubles. This can be ascertained through simple experiments as described in the scientific literature. This assumption is also one of the reasons for using a low cycle number when the reaction is still in the exponential phase. In the initial exponential phase of PCR, substrates are not limiting and there is no degradation of products. In practice, this requires setting the crossing threshold or cycle threshold (Cₜ) at the earliest cycle possible. The Cₜ is the number of cycles that it takes each reaction to reach an arbitrary amount of fluorescence. The second assumption of the 2^{-ΔΔCT} method is that there is a gene (or genes) that is expressed at a constant level between the samples. This endogenous control will be used to correct for any difference in sample loading.

Once the Cₜ value is collected for each reaction, it can be used to generate a relative expression level. One 2^{-ΔΔCT} method is now described. In this example, there are two samples (Control and Treated) and we have measured the levels of (i) a gene of interest (Target Gene (TG)) and (ii) an endogenous control gene (Control Gene (CG)). For each sample, the difference in Cₜ values for the gene of interest and the endogenous control is calculated (the ΔCₜ). Next, subtraction of the control-condition Δ Cₜ from the treated-condition ΔCₜ yields the ΔΔCₜ. The negative value of this subtraction, the -ΔΔCₜ, is used as the exponent of 2 in the equation and represents the difference in "corrected" number of cycles to threshold. The exponent conversion comes from the fact that the reaction doubles the amount of product per cycle. For example, if the control sample ΔCₜ is 2 and the treated sample ΔCₜ is 4, computing the 2^{-ΔΔCT} (which becomes 2 ⁻⁽⁴⁻²⁾) yields 0.25. This value is often referred to as the RQ, or relative quantity value. This means that the level of the gene of interest in the treated sample is only 25% of the level of that gene in the control sample. This becomes evident because the treated sample took two more cycles of PCR to reach the same amount of product as the control sample and therefore there was less of that cDNA to begin with in the treated sample. The 2^{-ΔΔCT} method is the most common quantitation strategy, but it should be noted that there are other valid methods for analyzing qPCR Cₜ values. Several investigators have proposed alternative analysis methods.

### MassARRAY® System

In MassARRAY-based methods, such as the exemplary method developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### Other PCR-based Methods

Further PCR-based techniques that can find use in the methods disclosed herein include, for example, BeadArray® technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression® (BADGE), using the commercially available Luminex 100 LabMAP® system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003).

### Microarrays

Expression levels of a gene of interest can also be assessed using the microarray technique. In this method, polynucleotide sequences of interest (including cDNAs and oligonucleotides) are arrayed on a substrate. The arrayed sequences are then contacted under conditions suitable for specific hybridization with detectably labeled cDNA generated from mRNA of a test sample. As in the RT-PCR method, the source ofmRNA typically is total RNA isolated from a tumour sample, and optionally from normal tissue of the same patient as an internal control or cell lines. mRNA can be extracted, for example, from frozen or archived paraffin-embedded and fixed (e.g. formalin-fixed) tissue samples.

For example, PCR amplified inserts of cDNA clones of a gene to be assayed are applied to a substrate in a dense array. Usually at least 10,000 nucleotide sequences are applied to the substrate. For example, the microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After washing under stringent conditions to remove non-specifically bound probes, the chip is scanned by confocal laser microscopy or by another detection method, such as a CCD camera. Quantitation of hybridization of each arrayed element allows for assessment of corresponding mRNA abundance.

With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et at, Proc. Natl. Acad. Sci. USA 93(2):106-149 (1996)). Microarray analysis can be performed by commercially available equipment, following manufacturer's protocols, such as by using the Affymetrix GenChip® technology.

### Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### Gene Expression Analysis by Nucleic Acid Sequencing

Nucleic acid sequencing technologies are suitable methods for analysis of gene expression. The principle underlying these methods is that the number of times a cDNA sequence is detected in a sample is directly related to the relative expression of the mRNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

### Isolating RNA from Body Fluids

Methods of isolating RNA for expression analysis from tissue (e.g., breast tissue), blood, plasma and serum (See for example, Tsui NB et al. (2002) 48,1647-53 and references cited therein) and from urine (See for example, Boom R et al. (1990) J Clin Microbiol. 28, 495-503 and reference cited therein) have been described.

### Immunonological methods

Immunological methods (e.g., immunohistochemistry methods)are also suitable for detecting the expression levels of genes and applied to the method disclosed herein. Antibodies (e.g., monoclonal antibodies) that specifically bind a gene product of a gene of interest can be used in such methods. The antibodies can be detected by direct labeling of the antibodies themselves, for example, with radioactive labels, fluorescent labels, haptene labels such as, biotin, or an enzyme such as horse radish peroxidase or alkaline phosphatase. Alternatively, unlabeled primary antibody can be used in conjunction with a labeled secondary antibody specific for the primary antibody. Immunological methods protocols and kits are well known in the art and are commercially available.

### Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N- terminal sequencing, and (3) analysis of the data using bioinformatics.

### GENERAL DESCRIPTION OF EXEMPLARY PROTOCOL

The steps of a representative protocol for profiling gene expression using fixed, paraffin-embedded tissues as the RNA source, including mRNA isolation, purification, primer extension and amplification are provided in various published journal articles. (See, e.g., T.E. Godfrey et al,. J. Molec. Diagnostics 2: 84-91 (2000); K. Specht et al., Am. J. Pathol. 158: 419-29 (2001), M. Cronin, et al., Am J Pathol 164:35-42 (2004)). Briefly, a representative process starts with cutting a tissue sample section (e.g. about 10 µm thick sections of a paraffin-embedded tumour tissue sample). The RNA is then extracted, and protein and DNA are removed. After analysis of the RNA concentration, RNA repair is performed if desired. The sample can then be subjected to analysis, e.g., by reverse transcribed using gene specific promoters followed by RT-PCR. **KITS**

The materials for use in the methods of the present disclosure are suited for preparation of kits produced in accordance with well known procedures. The present disclosure thus provides kits comprising agents, which may include gene-specific or gene-selective probes and/or primers, for quantitating the expression of the disclosed genes for predicting clinical outcome or response to treatment. Such kits may optionally contain reagents for the extraction of RNA from tumour samples, in particular fixed paraffin-embedded tissue samples and/or reagents for RNA amplification. In addition, the kits may optionally comprise the reagent(s) with an identifying description or label or instructions relating to their use in the methods of the present disclosure. The kits may comprise containers (including microtiter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present disclosure (e.g., appropriate length poly(T) or random primers linked to a promoter reactive with the RNA polymerase). Mathematical algorithms used to estimate or quantify prognostic and/or predictive information are also properly potential components of kits.

The methods provided by the present disclosure may also be automated in whole or in part.

### REPORTS

The methods of the present disclosure are suited for the preparation of reports summarizing the predictions resulting from the methods of the present disclosure. A "report," as described herein, is an electronic or tangible document which includes report elements that provide information of interest relating to a likelihood assessment and its results. A subject report includes at least a likelihood assessment, e.g., an indication as to the likelihood that a cancer patient will exhibit a beneficial clinical response to a treatment regimen of interest. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more genes of interest, and 6) other features.

The present disclosure thus provides for methods of creating reports and the reports resulting therefrom. The report may include a summary of the expression levels of the RNA transcripts, or the expression products of such RNA transcripts, for certain genes in the cells obtained from the patients tumour tissue. The report may include a prediction that said subject has an increased likelihood of response to treatment with a particular chemotherapy or the report may include a prediction that the subject has a decreased likelihood of response to the chemotherapy. The report may include a recommendation for treatment modality such as surgery alone or surgery in combination with chemotherapy. The report may be presented in electronic format or on paper.

Thus, in some embodiments, the methods of the present disclosure further includes generating a report that includes information regarding the patient's likelihood of response to chemotherapy, particularly a therapy including cyclophophamide and/or a taxane. For example, the methods disclosed herein can further include a step of generating or outputting a report providing the results of a subject response likelihood assessment, which report can be provided in the form of an electronic medium (e.g., an electronic display on a computer monitor), or in the form of a tangible medium (e.g., a report printed on paper or other tangible medium).

A report that includes information regarding the likelihood that a patient will respond to treatment with chemotherapy, particularly a including cyclophophamide and/or a taxane, is provided to a user. An assessment as to the likelihood that a cancer patient will respond to treatment with chemotherapy, or predicted comparative response to two therapy options, is referred to below as a "response likelihood assessment" or, simply, "likelihood assessment." A person or entity who prepares a report ("report generator") will also perform the likelihood assessment. The report generator may also perform one or more of sample gathering, sample processing, and data generation, e.g., the report generator may also perform one or more of: a) sample gathering; b) sample processing; c) measuring a level of an indicator response gene product(s); d) measuring a level of a reference gene product(s); and e) determining a normalized level of a response indicator gene product(s). Alternatively, an entity other than the report generator can perform one or more sample gathering, sample processing, and data generation. For clarity, it should be noted that the term "user," which is used interchangeably with "client," is meant to refer to a person or entity to whom a report is transmitted, and may be the same person or entity who does one or more of the following: a) collects a sample; b) processes a sample; c) provides a sample or a processed sample; and d) generates data (e.g., level of a response indicator gene product(s); level of a reference gene product(s); normalized level of a response indicator gene product(s)) for use in the likelihood assessment. In some cases, the person(s) or entity(ies) who provides sample collection and/or sample processing and/or data generation, and the person who receives the results and/or report may be different persons, but are both referred to as "users" or "clients" herein to avoid confusion. In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods of the invention, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### COMPUTER-BASED SYSTEMS AND METHODS

The methods and systems described herein can be implemented in numerous ways. In one embodiment of particular interest, the methods involve use of a communications infrastructure, for example the internet. Several embodiments of the invention are discussed below. It is also to be understood that the present invention may be implemented in various forms of hardware, software, firmware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site associated (e.g., at a service provider's facility).

For example, during or after data input by the user, portions of the data processing can be performed in the user-side computing environment. For example, the user-side computing environment can be programmed to provide for defined test codes to denote a likelihood "score," where the score is transmitted as processed or partially processed responses to the reviewer's computing environment in the form of test code for subsequent execution of one or more algorithms to provide a results and/or generate a report in the reviewer's computing environment. The score can be a numerical score (representative of a numerical value) or a non-numerical score representative of a numerical value or range of numerical values (e.g., "A' representative of a 90-95% likelihood of an outcome; "high" representative of a greater than 50% chance of response (or some other selected threshold of likelihood); "low" representative of a less than 50% chance of response (or some other selected threshold of likelihood); and the like.

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) which is executed via the operating system. In addition, various other peripheral devices may be connected to the computer platform such as an additional data storage device and a printing device.

As a computer system, the system generally includes a processor unit. The processor unit operates to receive information, which can include test data (e.g., level of a response indicator gene product(s); level of a reference gene product(s); normalized level of a response indicator gene product(s)); and may also include other data such as patient data. This information received can be stored at least temporarily in a database, and data analyzed to generate a report as described above.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In an embodiment of particular interest, all or a portion of the input data and/or all or a portion of the output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record which may exist in a confidential database at the healthcare facility.

A system for use in the methods described herein generally includes at least one computer processor (e.g., where the method is carried out in its entirety at a single site) or at least two networked computer processors (e.g., where data is to be input by a user (also referred to herein as a "client") and transmitted to a remote site to a second computer processor for analysis, where the first and second computer processors are connected by a network, e.g., via an intranet or internet). The system can also include a user component(s) for input; and a reviewer component(s) for review of data, generated reports, and manual intervention. Additional components of the system can include a server component(s); and a database(s) for storing data (e.g., as in a database of report elements, e.g., interpretive report elements, or a relational database (RDB) which can include data input by the user and data output. The computer processors can be processors that are typically found in personal desktop computers (e.g., IBM, Dell, Macintosh), portable computers, mainframes, minicomputers, or other computing devices.

The networked client/server architecture can be selected as desired, and can be, for example, a classic two or three tier client server model. A relational database management system (RDMS), either as part of an application server component or as a separate component (RDB machine) provides the interface to the database.

In one example, the architecture is provided as a database-centric client/server architecture, in which the client application generally requests services from the application server which makes requests to the database (or the database server) to populate the report with the various report elements as required, particularly the interpretive report elements, especially the interpretation text and alerts. The server(s) (e.g., either as part of the application server machine or a separate RDB/relational database machine) responds to the client's requests.

The input client components can be complete, stand-alone personal computers offering a full range of power and features to run applications. The client component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe and offers data management, information sharing between clients, network administration and security. The application and any databases used can be on the same or different servers. Other computing arrangements for the client and server(s), including processing on a single machine such as a mainframe, a collection of machines, or other suitable configuration are contemplated. In general, the client and server machines work together to accomplish the processing of the present invention.

Where used, the database(s) is usually connected to the database server component and can be any device which will hold data. For example, the database can be a any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### Computer-readable storage media

The present disclosure also contemplates a computer-readable storage medium (e.g. CDROM, memory key, flash memory card, diskette, etc.) having stored thereon a program which, when executed in a computing environment, provides for implementation of algorithms to carry out all or a portion of the results of a response likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium provides a program which provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g., fax or mailed paper report). The storage medium containing a program according to the invention can be packaged with instructions (e.g., for program installation, use, etc.) recorded on a suitable substrate or a web address where such instructions may be obtained. The computer-readable storage medium can also be provided in combination with one or more reagents for carrying out response likelihood assessment (e.g., primers, probes, arrays, or other such kit components).

All aspects of the present disclosure may also be practiced such that a limited number of additional genes that are co-expressed with the disclosed genes, for example as evidenced by high Pearson correlation coefficients, are included in a prognostic and/or predictive test in addition to and/or in place of disclosed genes.
Having described exemplary embodiments of the invention, the same will be more readily understood through reference to the following Examples, which are provided by way of illustration, and are not intended to limit the invention in any way.

### EXAMPLES

The following examples are offered by way of illustration and not by way of limitation.

### EXAMPLE 1: IDENTIFICATION OF DIFFERENTIAL MARKERS OF RESPONSE IN BREAST CANCER PATIENTS

The data from intergroup trial E2197 (Goldstein L, O'Neill A, Sparano J, et al. E2197: phase III AT (doxorubicin/docetaxel) vs. AC (doxorubicin/cyclophosphamide) in the adjuvant treatment of node positive and high risk node negative breast cancer. *Proc Am Soc Clin Oncol.* 2005; 23:7s. [Abstract 512]) was used to evaluate the relative efficacy of adjuvant treatment of breast cancer patients with an anthracycline (doxorubicin) + a taxane (AT) compared to an anthracycline (doxorubicin) + cyclophosphamide(AC). The trial compared 4 cycles of a standard doxorubicin-cyclophosphamide (AC) combination given every 3 weeks with 4 cycles of doxorubicin plus docetaxel (AT) in patients with 0-3 positive lymph nodes. The trial was powered to detect a 25% reduction in the disease-free survival (DFS) hazard rate (from an anticipated 5-year DFS of 78% for the AC arm to 83% for the AT arm). Tamoxifen (20 mg daily for 5 years) was recommended for hormone receptor-positive patients following completion of chemotherapy, although approximately 40% of patients eventually took an aromatase inhibitor at some point before or after 5 years. The treatment arms were well balanced with regard to median age (51 years), proportion of lymph node-negative disease (65%), and estrogen receptor (ER)-positive disease (64%).

When single genes by treatment (taxane (T) vs cyclophosphamide (C); or AT vs AC) interactions were evaluated, large numbers of genes with significant interaction effects were observed, in all subjects analyzed; in hormone receptor (HR) positive subjects; in HR positive, Oncotype DX Recurrence Score® value > about 18 subjects; and in HR negative subjects. Most of these interactions are in the same "direction", *i.e*., higher expression is associated with greater T benefit and/or less C benefit. Where Oncotype DX Recurrence Score® (RS) was used, the RS was calculated according to the algorithm described in Paik et al., N Engl J Med. 2004 Dec 30;351(27):2817-26 and in U.S. application publication No. 20050048542, published March 3, 2005.
The predictive utility of PR protein expression was evaluated by immunohistochemistry in a central lab and quantitative RNA expression by RT-PCR for 371 genes (including the 21-gene Recurrence Score [RS]) in a representative sample of 734 patients who received at least 3-4 treatment cycles.

### Methods

*Patient Selection:* All recurrences with available tissue and randomly selected patients without recurrence were identified by an ECOG statistician (ratio 3.5 without recurrence to 1 with recurrence).
*Central Immunohistochemistry (IHC) for ER and PR:* IHC was performed on two 1.0-mm tissue microarrays (TMAs), using 4 µm sections, DakoCytomation En Vision+ System® (Dako Corporation, Carpinteria, CA), and standard methodology using anti-ER antibody (clone 1D5, dilution 1:100) and anti-PR antibody 636 (1:200).
TMAs were reviewed centrally and scored by two pathologists who were blinded to outcomes and local laboratory ER/PR status.

Scoring was performed using the Allred method (see, e. g. Harvey JM, Clark GM, Osborne CK et al. J Clin Oncol 1999; 17:1474-1481) scoring the proportion of positive cells (scored on a 0-5 scale) and staining intensity (scored on a 0-3 scale); proportion and intensity scores were added to yield Allred Score of 0 or 2 through 8 with Allred scores > 2 considered positive.

*Genes and RT-PCR analysis:* Candidate genes were selected to represent multiple biological processes. Quantitative RT-PCR analysis was performed by methods known in the art. For each gene, the appropriate mRNA reference sequence (REFSEQ) accession number was identified and the consensus sequence was accessed through the NCBI Entrez nucleotide database. Appendix 1. Besides the REFSEQ, RT-PCR probe and primer sequences are provided in Appendix 1. Sequences for the amplicons that result from the use of these primer sets are listed in Appendix 2.

*Statistical methods:* Single Gene by Treatment Interaction Analysis. The objective of this evaluation was to identify genes whose expression, treated as a continuous variable, is differentially associated with the risk of relapse between patients treated with AC versus those treated with AT. A gene expression by treatment interaction model was employed for this purpose and statistical analyses were performed by using Cox Regression models (SAS version 9.1.3). The Cox regression model that was employed for these analyses includes terms for the main effect of treatment, the main effect of gene expression, and the interaction of treatment and gene expression. This model enables prediction of the association between gene expression and the risk of recurrence for patients treated with AC, and of the association between gene expression and the risk of recurrence for patients treated with AT. The point at which these two curves cross is the level of gene expression at which the predicted risk of recurrence is identical if the patient is treated with AC or with AT. This crossover point is easily calculated from the parameter estimates from this model as the negative of the estimated treatment effect, divided by the estimate of the interaction effect.

All hypothesis tests were reported using two-sided p-values, and p-values of < 0.05 was considered statistically significant. Relapse-Free Interval was defined as the time from study entry to the first evidence of breast cancer relapse, defined as invasive breast cancer in local, regional or distant sites, including the ipsilateral breast, but excluding new primary breast cancers in the opposite breast. Follow-up for relapse was censored at the time of death without relapse, new primary cancer in the opposite breast, or at the time of the patient was last evaluated for relapse.

The variance of the partial likelihood estimators was estimated with a weighted estimate. See R. Gray, Lifetime Data Anal. 15(1):24-40 (2009); K. Chen K, S-H Lo, Biometrika 86:755-764 (1999).

Individual genes by treatment interactions were tested in Cox models for relapse-free interval (RFI) for the HR+ and HR- patients combined and separately. Since there is little chemotherapy benefit for RS<18, the HR+, RS>18 subset was also analyzed.
The interaction between gene expression and treatment for genes could be depicted graphically. As example we present treatment group-specific plots of the 5-year risk of relapse versus DDR1 gene expression.

Supervised principal components (SPC) was used to combine genes into a multigene predictor of differential treatment benefit, and was evaluated via cross-validation (CV). Pre-validation (PV) inference (Tibshirani and Efron, Stat Appl Genet and Mol Biol 2002;1:Articlel. Epub 2002 Aug 22), based on 20 replicates of 5 fold cross-validation, was used to estimate and test (via permutations) the utility of the SPC predictors.

### Results

Tables 1-4 include an Estimated Coefficient for each response indicator gene listed in the tables in all subjects analyzed (Table 1); in HR+ subjects (Table 2); in HR⁺ subjects having an Oncotype DX Recurrence Score® value greater than about 18 (Table 3); and in HR negative subjects (Table 4). Figures 1-4 represent graphically the results for each gene summarized in Tables 1-4, respectively. Each graph of Figures 1-4 shows a smooth line representing the model-predicted relationship between expression of the gene and 5-year recurrence rate (RR) in an AC treatment group (the AC prediction curve) and a hatched line representing the model-predicted relationship between gene expression and RR in an AT treatment group (the AT prediction curve). Each of the graphs in Figures 1-4 are presented with 5-year risk of recurrence on the y-axis and normalized expression (Cₜ) on the x-axis, where increasing normalized Cₜ values indicate increasing expression levels.

The Estimated Coefficient referred to in Tables 1-4 is a reflection of the difference between the slopes in the Cox regression model of the AC prediction curve and the AT prediction curve. The magnitude of the Estimated Coefficient is related to the difference between the slopes of the AC prediction curve and the AT prediction curve; the sign of the Estimated Coefficient is an indication of which treatment (AT or AC) becomes the favored treatment as expression of the gene increases. For example, in Table 1, the Estimated Coefficient for SLClA3 is -0.7577. The magnitude (absolute value = 0.7577) is related to the difference between the slopes of the AC prediction curve and the AT prediction curve (shown in the first panel of Figure 1) for SLClA3 in this population (all patients, i.e. not stratified by hormone receptor status or by RS). The negative sign indicates that higher expression levels of SLClA3 favor treatment with AT while lower expression levels of SLClA3 favor treatment with AC.

The p-value given in Table 1 is a measure of the statistical significance of the difference between the slope of the AC prediction curve and the slope of the AT prediction curve in the Cox regression model, i.e. the probability that the observed difference in slopes is due to chance. Smaller p-values indicate greater statistical significance.

### Analysis of gene expression in all patients in study population (irrespective of HR status and Oncotype Dx® RS score)

Table 1 shows a list of 76 genes whose normalized expression level is differentially associated with response to AT vs. AC treatment in all patients. When the estimated coefficient is <0, high expression of that gene is indicative that AT treatment is more effective than AC treatment; low gene expression of that gene is indicative that AC treatment is more effective than AT treatment. When the estimated coefficient is >0, high expression of that gene is indicative that AC treatment is more effective than AT treatment; low expression of that gene is indicative that AT treatment is more effective than AC treatment.

As noted above, Figure 1 shows a graph for each gene in Table 1. Each graph shows a smooth line representing the model-predicted relationship between expression of the gene and 5-year recurrence rate (RR) in an AC treatment group (the AC prediction curve) and a hatched line representing the model-predicted relationship between gene expression and RR in an AT treatment group (the AT prediction curve). For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line, represents the 12.3% recurrence rate at 5-year RR in all patients analyzed (i.e., without regard to HR status or Oncotype Dx RS).

The first panel of Figure 1, for example, shows the AC-prediction curve and the AT prediction curve for SLClA3. The curves have significantly different slopes in the Cox regression model and the lines cross, resulting in the ability to discriminate, based on the expression level of SLC1A3, patients who are more likely to respond to AT (or to AC). For SLC1A3, patients with higher expression levels are more likely to respond to AT than AC, while patients with lower expression levels are more likely to respond to AC than AT.

### Analysis of gene expression in HR+ patients in study population

Table 2 shows a list of 97 genes having a normalized expression level that is differently correlated with response to AT vs. AC in hormone receptor (HR)-positive patients (without regard to Oncotype Dx RS value). When the estimated coefficient is <0, high expression of that gene is indicative that AT treatment is more effective than AC treatment; low expression of that gene is indicative that AC treatment is more effective than AT treatment. When the estimated coefficient is >0, high expression of that gene is indicative than AC treatment is more effective than AT treatment; low expression of that gene is indicative that AT treatment is more effective than AC treatment.

The data summarized in Table 2 are provided in graph form for each gene in Figure 2. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 10.0% recurrence rate at 5-year RR in HR-positive patients.

### Analysis of gene expression in HR+ patients in the study population having an Oncotype Dx RS of about 18 or greater

Table 3 shows a list of 165 genes whose normalized expression level is differentially associated with response to AT vs. AC in HR-positive patients having a Recurrence Score (RS) > 18. These patients have an increased likelihood of cancer recurrence. When the estimated coefficient is <0, high expression of that gene is indicative that AT treatment is more effective than AC treatment; low expression of that gene is indicative that AC treatment is more effective than AT treatment. When the estimated coefficient is > 0, high expression of that gene is indicative that AC treatment is more effective than AT treatment; low expression ofthat gene is indicative that AT treatment is more effective than AC treatment.

The data summarized in Table 3 are provided in graph form for each gene in Figure 3. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 14.9% recurrence rate at 5-year RR in the HR-positive patient group having an Oncotype Dx RS of about 18 or greater.

### Analysis of gene expression in HR- patients in study population

Table 4 shows a list of 9 genes whose normalized expression level is differentially associated with response to AT vs. AC treatment in HR-negative patients.
The data summarized in Table 4 is provided in graph form for each gene in Figure 4. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 16.9% recurrence rate at 5-year RR in the HR-negative patient group.

### Discussion

PR Analysis. There was a weak benefit for AT in PR-negative (AT vs AC hazard ratio [RR]=0.75; p=0.06) and AC in PR-positive disease (RR=1.37; p=0.05) by central immunhistochemistry (Allred score > 2 positive) but not when genomic PR was evaluated by RT-PCR (>5.5 units positive).

RS and Genes Analyzed. Table 1 illustrates genes that can be used as markers of benefit of taxane therapy irrespective of hormone receptor expression status, and facilitate selection of AC vs AT therapy. (Table 1). Several genes strongly predicted taxane benefit when assessed in the context of AT vs AC therapy in the HR-positive subset (Table 2), and especially in the HR-positive, Oncotype Dx RS > 18 subset (Table 3).

Nine genes were identified for which gene expression can be used as markers of benefit of taxane therapy in hormone receptor (HR)-negative breast cancer, and could be used to assess AT vs. AC benefit in the hormone receptor (HR)-negative patients (Table 4).
Of the genes listed in Table 1, SLC1A3 (glial high affinity glutamase transporter 3) is a member of a large family of solute transport proteins, located within the multiple sclerosis locus on 5p. Of the genes identified in the HR-positive subset (Table 2), DDR1 (discoidin domain receptor 1) is a transmembrane receptor TK the aberrant expression and signaling of which has been linked to accelerated matrix degradation and remodeling, including tumour invasion. Collagen-induced DDR1 activation is believed to be involved in normal mammary cell adhesion, and may distinguish between invasive ductal carcinoma (IDC) and invasive lobular carcinoma (ILC), and further may induce cyclooxygenase-2 and promoter chemoresistance through the NF-κB pathway. EIF4E2 (human transcription initiation factor 4) is an mRNA cap-binding protein. When differential response markers in HR-positive, RS > 18 patients (Table 3) are ranked in ascending order by p-value, DDR1, RELA, ZW10, and RhoB are four of the top five genes. RELA is an NF-κB subunit, which plays a role in inflammation, innate immunity, cancer and anti-apoptosis. This gene has also been associated with chemoresistance, and may be necessary for IL-6 induction, which is involved in immune cell homeostasis. ZW10 is a kinetochore protein involved in mitotic spindle formation. It is part of the ROD-ZW10-Zwilch complex, and binds tubulin. RhoB is a low molecular weight GPTase belonging to the RAS superfamily. The Rho protein is pivotal in regulation of actin cytoskeleton. RhoB acts as tumour suppressor gene and inhibits tumour growth and metastases in vitro and in vivo, and activates NF-κB. KO mice for RhoB show increased sensitivity to chemical carcinogenesis and resistance to radiation and cytotoxic induced apoptosis.

DDR1, RELA and RhoB are key elements in the NFκB signaling pathway. Based on these findings, it is expected that other genes in the NFκB pathway are likely to be differentially associated with response to AT vs. AC treatment in HR-positive patients at high risk for cancer recurrence, and such can be used as differential response markers for AT vs. AC treatment. Some additional genes that are known to be involved in NFκB signaling are shown in Table 5.

In the HR-negative subset, CD247 exhibited a correlation of expression with AT vs. AC therapy (p-value < 0.01) and exhibited a strong correlation indicating that expression was positively correlated with increased likelihood of benefit of treatment including a taxane (Fig. 4). The estimated coefficient <0 indicates that high gene expression favors AT treatment, while low gene expression favors AC treatment (see also Fig. 4). CD247, also known as T cell receptor zeta (TCRzeta) functions as an amplification module of the TCR signaling cascade. This gene is downregulated in many chronic infectious and inflammatory processes, such as systemic lupus erythematosus (SLE).

Fig. 5 illustrates an exemplary treatment group-specific plot of the 5-year risk of relapse versus gene expression presented for an exemplary gene, DDR1.

### EXAMPLE 2:ESR1 GENE COMBINATIONS

Using the differential response markers identified in Table 2, supervised principle component analysis was carried out in HR+ RS>18 patients treated with AT vs AC according the methods of Bair E, Hastie T, Paul D, Tibshirani R. Prediction by supervised principal components. J. Amer. Stat. Assoc. 101:119-137, 2006.

Principal Components can be used in regression problems for dimensionality reduction in a data set by keeping the most important principal components and ignoring the other ones. Supervised principal components (Bair et al. *supra*) is similar to conventional principal components analysis except that it uses a subset of the predictors (i.e. individual genes) that are selected based on their association with relapse-free interval (assessed using Cox regression). In the present example, only the first component was utilized to obtain a score from a weighted combination of genes.

In this patient group, the most heavily weighted gene by supervised principle components analysis was ESR1, indicating that ESR1 is particularly useful when used in combinations with any of the other genes listed in Table 3 in predicting differential response to taxane vs. cyclophosphamide in HR+ high recurrence risk patients. Exemplary combinations of genes include, without limitation:
DDR1 + ESR1, ZW10 + ESR1, RELA + ESR1, BAX + ESR1, RHOB + ESR1, TSPAN4 + ESR1, BBC3 + ESR1, SHC1 + ESR1, CAPZA1 + ESR1, STK10 + ESR1, TBCC + ESR1, EIF4E2 + ESR1, MCL1 + ESR1, RASSF1 + ESR1, VEGF + ESR1, SLC1A3 + ESR1, DICER1 + ESR1, ILK + ESR1, FAS + ESR1, RAB6C + ESR1, ESR1 + ESR1, MRE11A + ESR1, APOE + ESR1, BAK1 + ESR1, UFM1 + ESR1, AKT2 + ESR1, SIRT1 + ESR1, BCL2L13 + ESR1, ACTR2 + ESR1, LIMK2 + ESR1, HDAC6 + ESR1, RPN2 + ESR1, PLD3 + ESR1, CHGA + ESR1, RHOA + ESR1, MAPK14 + ESR1, ECGF1 + ESR1, MAPRE1 + ESR1, HSPA1B + ESR1, GATA3 + ESR1, PPP2CA + ESR1, ABCD1 + ESR1, MAD2L1BP + ESR1, VHL + ESR1, GCLC + ESR1, ACTB + ESR1, BCL2L11 + ESR1, PRDX1 + ESR1, LILRB1 + ESR1, GNS + ESR1, CHFR + ESR1, CD68 + ESR1, LIMK1 + ESR1, GADD45B + ESR1, VEGFB + ESR1, APRT + ESR1, MAP2K3 + ESR1, MGC52057 + ESR1, MAPK3 + ESR1, APC + ESR1, RAD1 + ESR1, COL6A3 + ESR1, RXRB + ESR1, CCT3 + ESR1, ABCC3 + ESR1, GPX1 + ESR1, TUBB2C + ESR1, HSPA1A + ESR1, AKT1 + ESR1, TUBA6 + ESR1, TOP3B + ESR1, CSNK1D + ESR1, SOD1 + ESR1, BUB3 + ESR1, MAP4 +ESR1, NFKB1 + ESR1, SEC61A1 + ESR1, MAD1L1 + ESR1, PRKCH + ESR1, RXRA + ESR1, PLAU + ESR1, CD63 + ESR1, CD14 + ESR1, RHOC + ESR1, STAT1 + ESR1, NPC2 + ESR1, NME6 + ESR1, PDGFRB + ESR1, MGMT + ESR1, GBP1 + ESR1, ERCC1 + ESR1, RCC1 + ESR1, FUS + ESR1, TUBA3 + ESR1, CHEK2 + ESR1, APOC1 + ESR1, ABCC10 + ESR1, SRC + ESR1, TUBB + ESR1, FLAD1 + ESR1, MAD2L2 + ESR1, LAPTM4B + ESR1, REG1A + ESR1, PRKCD + ESR1, CST7 + ESR1, IGFBP2 + ESR1, FYN + ESR1, KDR + ESR1, STMN1 + ESR1, ZWILCH + ESR1, RBM17 + ESR1, TP53BP1 + ESR1, CD247 + ESR1, ABCA9 + ESR1, NTSR2 + ESR1, FOS + ESR1, TNFRSF10A + ESR1, MSH3 + ESR1, PTEN + ESR1, GBP2 + ESR1, STK11 + ESR1, ERBB4 + ESR1, TFF1 + ESR1, ABCC1 + ESR1, IL7 + ESR1, CDC25B + ESR1, TUBD1 + ESR1, BIRC4 + ESR1, ACTR3 + ESR1, SLC35B1 + ESR1, COL1A1 + ESR1, FOXA1 + ESR1, DUSP1 + ESR1, CXCR4 + ESR1, IL2RA + ESR1, GGPS1 + ESR1, KNS2 + ESR1, RB1 + ESR1, BCL2L1 + ESR1, XIST + ESR1, BIRC3 + ESR1, BID + ESR1, BCL2 + ESR1, STAT3 + ESR1, PECAM1 + ESR1, DIABLO + ESR1, CYBA + ESR1, TBCE + ESR1, CYP1B1 + ESR1, APEX1 + ESR1, TBCD + ESR1, HRAS + ESR1, TNFRSF10B + ESR1, ELP3 + ESR1, PIK3C2A + ESR1, HSPA5 + ESR1, VEGFC + ESR1, CRABP1 + ESR1, MMP11 + ESR1, SGK + ESR1, CTSD + ESR1, BAD + ESR1, PTPN21 + ESR1, HSPA9B + ESR1, and PMS1 + ESR1

Any combination of two or more genes from Table 3, said combination not comprising ESR1 is also expected to be useful in predicting differential response to taxane vs. cyclophosphamide in HR+ high recurrence risk patients.

Similarly it is expected that ESR1 is particularly useful when used in combinations with any of the other genes listed in Table 2 in predicting differential response to taxane vs. cyclophosphamide in HR+ patients. Exemplary combinations of genes include:
DDR1 + ESR1, EIF4E2 + ESR1, TBCC + ESR1, STK10 + ESR1, ZW10 + ESR1, BBC3 + ESR1, BAX + ESR1, BAK1 + ESR1, TSPAN4 +ESR1, SLC1A3 + ESR1, SHC1 + ESR1, CHFR + ESR1, RHOB + ESR1, TUBA6 + ESR1, BCL2L13 + ESR1, MAPRE1 + ESR1, GADD45B + ESR1, HSPA1B + ESR1, FAS + ESR1, TUBB + ESR1, HSPA1A + ESR1, MCL1 + ESR1, CCT3 + ESR1, VEGF + ESR1, TUBB2C + ESR1, AKT1 + ESR1, MAD2L1BP + ESR1, RPN2 + ESR1, RHOA + ESR1, MAP2K3 + ESR1, BID + ESR1, APOE + ESR1, ESR1 + ESR1, ILK + ESR1, NTSR2 + ESR1, TOP3B + ESR1, PLD3 + ESR1, DICER1 + ESR1, VHL + ESR1, GCLC + ESR1, RAD1 + ESR1, GATA3 +ESR1, CXCR4 + ESR1, NME6 + ESR1, UFM1 + ESR1, BUB3 + ESR1, CD14 + ESR1, MRE11A + ESR1, CST7 + ESR1, APOC1 + ESR1, GNS + ESR1, ABCC5 + ESR1, AKT2 + ESR1, APRT + ESR1, PLAU + ESR1, RCC1 + ESR1, CAPZA1 + ESR1, RELA + ESR1, NFKB1 + ESR1, RASSF1 + ESR1, BCL2L11 + ESR1, CSNK1D + ESR1, SRC + ESR1, LIMK2 + ESR1, SIRT1 + ESR1, RXRA + ESR1, ABCD1 + ESR1, MAPK3 + ESR1, CDCA8 + ESR1, DUSP1 + ESR1, ABCC1 + ESR1, PRKCH + ESR1, PRDX1 + ESR1, TUBA3 + ESR1, VEGFB + ESR1, LILRB1 + ESR1, LAPTM4B + ESR1, HSPA9B + ESR1, ECGF1 + ESR1, GDF15 + ESR1, ACTR2 + ESR1, IL7 + ESR1, HDAC6 + ESR1, ZWILCH + ESR1, CHEK2 + ESR1, REG1A + ESR1, APC + ESR1, SLC35B1 + ESR1, NEK2 + ESR1, ACTB + ESR1, BUB1 + ESR1, PPP2CA + ESR1, TNFRSF10A + ESR1, TBCD + ESR1, ERBB4 + ESR1, CDC25B + ESR1, and STMN1+ ESR1.

A combination of two or more genes from Table 2, said combination not comprising ESR1 is also expected to be useful in predicting differential response to taxane vs. cyclophosphamide in HR+ patients at high recurrence risk for cancer.

### EXAMPLE 3:GENES OF THE NFκB PATHWAY

When the differential response markers in HR-positive, RS > 18 patients are ranked in ascending order of p-value, three of the top five revealed genes are DDR1, RELA and RHOB. The RELA gene encodes one of the principle subunits of the NFκB transcription factor. Therefore, it is notable that both the DDR1 gene and the RHOB gene stimulate the NFκB signaling pathway. These results indicate that additional genes that stimulate the activity of the NFκB pathway, given in Table 5, also predict increased likelihood of response to AT vs. AC chemotherapy.

### EXAMPLE 4: GENE EXPRESSION PROFILING PROTOCOL

Breast tumour formalin-fixed and paraffin-embedded (FPE) blocks or frozen tumour sections are provided. Fixed tissues are incubated for 5 to 10 hours in 10% neutral-buffered formalin before being alcohol-dehydrated and embedded in paraffin.

RNA is extracted from three 10-µm FPE sections per each patient case. Paraffin is removed by xylene extraction followed by ethanol wash. RNA is isolated from sectioned tissue blocks using the MasterPure Purification kit (Epicenter, Madison, WI); a DNase I treatment step is included. RNA is extracted from frozen samples using Trizol reagent according to the supplier's instructions (Invitrogen Life Technologies, Carlsbad, CA). Residual genomic DNA contamination is assayed by a TaqMan® (Applied Biosystems, Foster City, CA) quantitative PCR assay (no RT control) for ß-actin DNA. Samples with measurable residual genomic DNA are resubjected to DNase I treatment, and assayed again for DNA contamination. TaqMan is a registered trademark of Roche Molecular Systems.

RNA is quantitated using the RiboGreen® fluorescence method (Molecular Probes, Eugene, OR), and RNA size is analyzed by microcapillary electrophoresis using an Agilent 2100 Bioanalyzer (Agilent Technologies, Palo Alto, CA).

Reverse transcription (RT) is performed using a SuperScript® First-Strand Synthesis kit for RT-PCR (Invitrogen Corp., Carlsbad, CA). Total FPE RNA and pooled gene-specific primers are present at 10 to 50 ng/µl and 100 nmol/L (each), respectively.

TaqMan reactions are performed in 384-well plates according to instructions of the manufacturer, using Applied Biosystems Prism 7900HT TaqMan instruments. Expression of each gene is measured either in duplicate 5-µl reactions using cDNA synthesized from 1 ng of total RNA per reaction well, or in single reactions using cDNA synthesized from 2 ng of total RNA. Final primer and probe concentrations are 0.9 µmol/L (each primer) and 0.2 µmol/L, respectively. PCR cycling is performed as follows: 95°C for 10 minutes for one cycle, 95°C for 20 seconds, and 60°C for 45 seconds for 40 cycles. To verify that the RT-PCR signals derives from RNA rather than genomic DNA, for each gene tested a control identical to the test assay but omitting the RT reaction (no RT control) is included. The threshold cycle for a given amplification curve during RT-PCR occurs at the point the fluorescent signal from probe cleavage grows beyond a specified fluorescence threshold setting. Test samples with greater initial template exceed the threshold value at earlier amplification cycle numbers than those with lower initial template quantities.

For normalization of extraneous effects, cycle threshold (C_{T}) measurements obtained by RT-PCR were normalized relative to the mean expression of a set of five reference genes: ATP5E, PGK1, UBB, VDAC2, and GPX1. A one unit increase in reference normalized expression measurements generally reflects a 2-fold increase in RNA quantity.
While the present invention has been described with reference to what are considered to be the specific embodiments, it is to be understood that the invention is not limited to such embodiments.

| Appendix 1 | | | | |
|---|---|---|---|---|
| Gene Name | Accession # | Oligo Name | Oligo Sequence | SEQ ID NO |
| ABCA9 | NM_172386 | T2132/ABCA9.f1 | TTACCCGTGGGAACTGTCTC | 1 |
| ABCA9 | NM_172386 | T2133/ABCA9.r1 | GACCAGTAAATGGGTCAGAGGA | 2 |
| ABCA9 | NM_172386 | T2134/ABCA9.p1 | | 3 |
| ABCB1 | NM_000927 | S8730/ABCB1.f5 | AAACACCACTGGAGCATTGA | 4 |
| ABCB1 | NM_000927 | S8731/ABCB1.r5 | CAAGCCTGGAACCTATAGCC | 5 |
| ABCB1 | NM_000927 | S8732/ABCB1.p5 | CTCGCCAATGATGCTGCTCAAGTT | 6 |
| ABCB5 | NM_178559 | T2072/ABCB5.f1 | AGACAGTCGCCTTGGTCG | 7 |
| ABCB5 | NM_178559 | T2073/ABCB5.r1 | AACCTCTGCAGAAGCTGGAC | 8 |
| ABCB5 | NM_178559 | T2074/ABCB5.p1 | CCGTACTCTTCCCACTGCCATTGA | 9 |
| ABCC10 | NM_033450 | S9064/ABCC10.f1 | ACCAGTGCCACAATGCAG | 10 |
| ABCC10 | NM_033450 | S9065/ABCC10.r1 | ATAGCGCTGACCACTGCC | 11 |
| ABCC10 | NM_033450 | S9066/ABCC10.p1 | CCATGAGCTGTAGCCGAATGTCCA | 12 |
| ABCC11 | NM_032583 | T2066/ABCC11.f1 | AAGCCACAGCCTCCATTG | 13 |
| ABCC11 | NM_032583 | T2067/ABCC11.r1 | GGAAGGCTTCACGGATTGT | 14 |
| ABCC11 | NM_032583 | T2068/ABCC11.p1 | TGGAGACAGACACCCTGATCCAGC | 15 |
| ABCC5 | NM_005688 | S5605/ABCC5.f1 | TGCAGACTGTACCATGCTGA | 16 |
| ABCC5 | NM_005688 | S5606/ABCC5.r1 | GGCCAGCACCATAATCCTAT | 17 |
| ABCC5 | NM_005688 | S5607/ABCC5.p1 | CTGCACACGGTTCTAGGCTCCG | 18 |
| ABCD1 | NM_000033 | T1991/ABCD1.f1 | TCTGTGGCCCACCTCTACTC | 19 |
| ABCD1 | NM_000033 | T1992/ABCD1.r1 | GGGTGTAGGAAGTCACAGCC | 20 |
| ABCD1 | NM_000033 | T1993/ABCD1.p1 | AACCTGACCAAGCCACTCCTGGAC | 21 |
| ACTG2 | NM_001615 | S4543/ACTG2.f3 | ATGTACGTCGCCATTCAAGCT | 22 |
| ACTG2 | NM_001615 | S4544/ACTG2.r3 | ACGCCATCACCTGAATCCA | 23 |
| ACTG2 | NM_001615 | S4545/ACTG2.p3 | CTGGCCGCACGACAGGCATC | 24 |
| ACTR2 | NM_005722 | T2380/ACTR2.f1 | ATCCGCATTGAAGACCCA | 25 |
| ACTR2 | NM_005722 | T2381/ACTR2.r1 | ATCCGCTAGAACTGCACCAC | 26 |
| ACTR2 | NM_005722 | T2382/ACTR2.p1 | CCCGCAGAAAGCACATGGTATTCC | 27 |
| ACTR3 | NM_005721 | T2383/ACTR3.f1 | CAACTGCTGAGAGACCGAGA | 28 |
| ACTR3 | NM_005721 | T2384/ACTR3.r1 | CGCTCCTTTACTGCCTTAGC | 29 |
| ACTR3 | NM_005721 | T2385/ACTR3.p1 | | 30 |
| AK055699 | NM_194317 | S2097/AK0556.f1 | | 31 |
| AK055699 | NM_194317 | S2098/AK0556.r1 | TGTGGACCTGATCCCTGTACAC | 32 |
| AK055699 | MM_194317 | S5057/AK0556.p1 | TGACCACACCAAAGCCTCCCTGG | 33 |
| AKT1 | NM_005163 | S0010/AKT1.f3 | CGCTTCTATGGCGCTGAGAT | 34 |
| AKT1 | NM_005163 | S0012/AKT1.r3 | TCCCGGTACACCACGTTCTT | 35 |
| AKT1 | NM_005163 | S4776/AKT1.p3 | CAGCCCTGGACTACCTGCACTCGG | 36 |
| AKT2 | NM_001626 | S0828/AKT2.f3 | TCCTGCCACCCTTCAAACC | 37 |
| AKT2 | NM_001626 | S0829/AKT2.r3 | GGCGGTAAATTCATCATCGAA | 38 |
| AKT2 | NM_001626 | S4727/AKT2.p3 | CAGGTCACGTCCGAGGTCGACACA | 39 |
| AKT3 | NM_005465 | S0013/AKT3.f2 | TTGTCTCTGCCTTGGACTATCTACA | 40 |
| AKT3 | NM_005465 | S0015/AKT3.r2 | CCAGCATTAGATTCTCCAACTTGA | 41 |
| AKT3 | NM_005465 | S4884/AKT3.p2 | TCACGGTACACAATCTTTCCGGA | 42 |
| ANXA4 | NM_001153 | T1017/ANXA4.f1 | TGGGAGGGATGAAGGAAAT | 43 |
| ANXA4 | NM_001153 | T1018/ANXA4.r1 | CTCATACAGGTCCTGGGCA | 44 |
| ANXA4 | NM_001153 | T1019/ANXA4.p1 | TGTCTCACGAGAGCATCGTCCAGA | 45 |
| APC | NM_000038 | S0022/APC.f4 | GGACAGCAGGAATGTGTTTC | 46 |
| APC | NM_000038 | S0024/APC. r4 | ACCCACTCGATTTGTTTCTG | 47 |
| APC | NM_000038 | S4888/APC.p4 | CATTGGCTCCCCGTGACCTGTA | 48 |
| APEX-1 | NM_001641 | S9947/APEX-1.f1 | GATGAAGCCTTTCGCAAGTT | 49 |
| APEX-1 | NM_001641 | S9948/APEX-1.r1 | AGGTCTCCACACAGCACAAG | 50 |
| APEX-1 | NM_001641 | S9949/APEX-1.p1 | CTTTCGGGAAGCCAGGCCCTT | 51 |
| APOC1 | NM_001645 | S9667/APOC1.f2 | GGAAACACACTGGAGGACAAG | 52 |
| APOC1 | NM_001645 | S9668/APOC1.r2 | CGCATCTTGGCAGAAAGTT | 53 |
| APOC1 | NM_001645 | S9669/APOC1.p2 | TCATCAGCCGCATCAAACAGAGTG | 54 |
| APOD | NM_001647 | T0536/APOD.f1 | GTTTATGCCATCGGCACC | 55 |
| APOD | NM_001647 | T0537/APOD.r1 | GGAATACACGAGGGCATAGTTC | 56 |
| APOD | NM_001647 | T0538/APOD.p1 | ACTGGATCCTGGCCACCGACTATG | 57 |
| APOE | NM_000041 | T1994/APOE.f1 | GCCTCAAGAGCTGGTTCG | 58 |
| APOE | NM_000041 | T1995/APOE.r1 | CCTGCACCTTCTCCACCA | 59 |
| APOE | NM_000041 | T1996/APOE.p1 | ACTGGCGCTGCATGTCTTCCAC | 60 |
| APRT | NM_000485 | T1023/APRT.f1 | GAGGTCCTGGAGTGCGTG | 61 |
| APRT | NM_000485 | T1024/APRT.r1 | AGGTGCCAGCTTCTCCCT | 62 |
| APRT | NM_000485 | T1025/APRT.p1 | CCTTAAGCGAGGTCAGCTCCACCA | 63 |
| ARHA | NM_001664 | S8372/ARHA.f1 | GGTCCTCCGTCGGTTCTC | 64 |
| ARHA | MM_001664 | S8373/ARHA.r1 | GTCGCAAACTCGGAGACG | 65 |
| ARHA | NM_001664 | S8374/ARHA.p1 | CCACGGTCTGGTCTTCAGCTACCC | 66 |
| AURKB | NM_004217 | S7250/AURKB.f1 | AGCTGCAGAAGAGCTGCACAT | 67 |
| AURKB | NM_004217 | S7251/AURKB.r1 | GCATCTGCCAACTCCTCCAT | 68 |
| AURKB | NM_004217 | S7252/AURKB.p1 | TGACGAGCAGCGAACAGCCACG | 69 |
| B-actin | NM_001101 | S0034/B-acti.f2 | CAGCAGATGTGGATCAGCAAG | 70 |
| B-actin | NM_001101 | S0036/B-acti.r2 | GCATTTGCGGTGGACGAT | 71 |
| B-actin | NM_001101 | S4730/B-acti.p2 | AGGAGTATGACGAGTCCGGCCCC | 72 |
| B-Catenin | NM_001904 | S2150/B-Cate.f3 | GGCTCTTGTGCGTACTGTCCTT | 73 |
| B-Catenin | NM_001904 | S2151/B-Cate.r3 | TCAGATGACGAAGAGCACAGATG | 74 |
| B-Catenin | NM_001904 | S5046/B-Cate.p3 | | 75 |
| BAD | NM_032989 | S2011/BAD.f1 | GGGTCAGGTGCCTCGAGAT | 76 |
| BAD | NM_032989 | S2012/BAD.r1 | CTGCTCACTCGGCTCAAACTC | 77 |
| BAD | NM_032989 | S5058/BAD.p1 | TGGGCCCAGAGCATGTTCCAGATC | 78 |
| BAG1 | NM_004323 | S13861BAG1.f2 | CGTTGTCAGCACTTGGAATACAA | 79 |
| BAG1 | NM_004323 | S1387/BAG1.r2 | GTTCAACCTCTTCCTGTGGACTGT | 80 |
| BAG1 | MM_004323 | S4731/BAG1.p2 | | 81 |
| Bak | NM_001188 | S0037/Bak.f2 | CCATTCCCACCATTCTACCT | 82 |
| Bak | NM_001188 | S0039/Bak.r2 | GGGAACATAGACCCACCAAT | 83 |
| Bak | NM_001188 | S4724/Bak.p2 | ACACCCCAGACGTCCTGGCCT | 84 |
| Bax | NM_004324 | S0040/Bax.f1 | CCGCCGTGGACACAGACT | 85 |
| Bax | NM_004324 | S0042/Bax.r1 | TTGCCGTCAGAAAACATGTCA | 86 |
| Bax | NM_004324 | S4897/Bax.p1 | | 87 |
| BBC3 | NM_014417 | S1584/BBC3.f2 | CCTGGAGGGTCCTGTACAAT | 88 |
| BBC3 | NM_014417 | S1585/BBC3.r2 | CTAATTGGGCTCCATCTCG | 89 |
| BBC3 | NM_014417 | S4890/BBC3.p2 | CATCATGGGACTCCTGCCCTTACC | 90 |
| Bcl2 | NM_000633 | S0043/Bcl2.f2 | | 91 |
| Bcl2 | NM_000633 | S0045/Bcl2.r2 | CCTATGATTTAAGGGCATTTTTCC | 92 |
| Bcl2 | NM_000633 | S4732/Bcl2.p2 | TTCCACGCCGAAGGACAGCGAT | 93 |
| BCL2L11 | NM_138621 | S7139/BCL2L1.f1 | AATTACCAAGCAGCCGAAGA | 94 |
| BCL2L11 | NM_138621 | S7140/BCL2L1.r1 | CAGGCGGACAATGTAACGTA | 95 |
| BCL2L11 | NM_138621 | S7141/BCL2L1.p1 | | 96 |
| BCL2L13 | NM_015367 | S9025/BCL2L1.f1 | CAGCGACAACTCTGGACAAG | 97 |
| BCL2L13 | NM_015367 | S9026/BCL2L1.r1 | GCTCTCAGACTGCCAGGAA | 98 |
| BCL2L13 | NM_015367 | S9027/BCL2L1.p1 | CCCCAGAGTCTCCAACTGTGACCA | 99 |
| Bclx | NM_001191 | S0046/Bclx.f2 | CTTTTGTGGAACTCTATGGGAACA | 100 |
| Bclx | NM_001191 | S0048/Bclx.r2 | CAGCGGTTGAAGCGTTCCT | 101 |
| Bclx | NM_001191 | S4898/Bclx.p2 | TTCGGCTCTCGGCTGCTGCA | 102 |
| BCRP | NM_004827 | S0840/BCRP.f1 | | 103 |
| BCRP | NM_004827 | S0841/BCRP.r1 | GCCACGTGATTCTTCCACAA | 104 |
| BCRP | NM_004827 | S4836/BCRP.p1 | CAGGGCATCGATCTCTCACCCTGG | 105 |
| BID | NM_001196 | S6273/BID.f3 | GGACTGTGAGGTCAACAACG | 106 |
| BID | NM_001196 | S6274/BID.r3 | GGAAGCCAAACACCAGTAGG | 107 |
| BID | NM_001196 | S6275/BID.p3 | TGTGATGCACTCATCCCTGAGGCT | 108 |
| BIN1 | NM_004305 | S2651/BIN1.f3 | CCTGCAAAAGGGAACAAGAG | 109 |
| BIN1 | NM_004305 | S2652/BIN1.r3 | CGTGGTTGACTCTGATCTCG | 110 |
| BIN1 | NM_004305 | S4954/BIN1.p3 | CTTCGCCTCCAGATGGCTCCC | 111 |
| BRCA1 | NM_007295 | S0049/BRCA1.f2 | TCAGGGGGCTAGAAATCTGT | 112 |
| BRCA1 | NM_007295 | S0051/BRCA1.r2 | CCATTCCAGTTGATCTGTGG | 113 |
| BRCA1 | NM_007295 | S4905/BRCA1.p2 | CTATGGGCCCTTCACCAACATGC | 114 |
| BRCA2 | NM_000059 | S0052/BRCA2.f2 | AGTTCGTGCTTTGCAAGATG | 115 |
| BRCA2 | NM_000059 | S0054/BRCA2.r2 | AAGGTAAGCTGGGTCTGCTG | 116 |
| BRCA2 | NM_000059 | S4985/BRCA2.p2 | | 117 |
| BUB1 | NM_004336 | S4294/BUB1.f1 | CCGAGGTTAATCCAGCACGTA | 118 |
| BUB1 | NM_004336 | S4295/BUB1.r1 | AAGACATGGCGCTCTCAGTTC | 119 |
| BUB1 | NM_004336 | S4296/BUB1.p1 | TGCTGGGAGCCTACACTTGGCCC | 120 |
| BUB1B | NM_001211 | S8060/BUB1B.f1 | TCAACAGAAGGCTGAACCACTAGA | 121 |
| BUB1B | NM_001211 | S8061/BUB1B.r1 | CAACAGAGTTTGCCGAGACACT | 122 |
| BUB1B | NM_001211 | S8062/BUB1B.p1 | TACAGTCCCAGCACCGACAATTCC | 123 |
| BUB3 | NM_004725 | S8475/BUB3.f1 | CTGAAGCAGATGGTTCATCATT | 124 |
| BUB3 | NM_004725 | S8476/BUB3.r1 | GCTGATTCCCAAGAGTCTAACC | 125 |
| BUB3 | NM_004725 | S8477/BUB3.p1 | CCTCGCTTTGTTTAACAGCCCAGG | 126 |
| c-Src | NM_005417 | S7320/c-Src.f1 | TGAGGAGTGGTATTTTGGCAAGA | 127 |
| c-Src | NM_005417 | S7321/c-Src.r1 | CTCTCGGGTTCTCTGCATTGA | 128 |
| c-Src | NM_005417 | S7322/c-Src.p1 | AACCGCTCTGACTCCCGTCTGGTG | 129 |
| C14orf10 | NM_017917 | T2054/C14orf.f1 | GTCAGCGTGGTAGCGGTATT | 130 |
| C14orf10 | NM_017917 | T2055/C14orf.r1 | GGAAGTCTTGGCTAAAGAGGC | 131 |
| C14orf10 | NM_017917 | T2056/C14orf.p1 | AACAATTACTGTCACTGCCGCGGA | 132 |
| C20 orf1 | NM_012112 | S3560/C20 or.f1 | TCAGCTGTGAGCTGCGGATA | 133 |
| C20 orf1 | NM_012112 | S3561/C20 or.r1 | ACGGTCCTAGGTTTGAGGTTAAGA | 134 |
| C20 orf1 | NM_012112 | S3562/C20 or.p1 | CAGGTCCCATTGCCGGGCG | 135 |
| CA9 | NM_001216 | S1398/CA9.f3 | ATCCTAGCCCTGGTTTTTGG | 136 |
| CA9 | NM_001216 | S1399/CA9.r3 | CTGCCTTCTCATCTGCACAA | 137 |
| CA9 | NM_001216 | S4938/CA9.p3 | TTTGCTGTCACCAGCGTCGC | 138 |
| CALD1 | NM_004342 | S4683/CALD1.f2 | | 139 |
| CALD1 | NM_004342 | S4684/CALD1.r2 | GCGAATTAGCCCTCTACAACTGA | 140 |
| CALD1 | NM_004342 | S4685/CALD1.p2 | | 141 |
| CAPZA1 | NM_006135 | T2228/CAPZA1.f1 | TCGTTGGAGATCAGAGTGGA | 142 |
| CAPZA1 | NM_006135 | T2229/CAPZA1.r1 | TTAAGCACGCCAACCACC | 143 |
| CAPZA1 | NM_006135 | T2230/CAPZA1.p1 | TCACCATCACACCACCTACAGCCC | 144 |
| CAV1 | NM_001753 | S7151/CAV1.f1 | GTGGCTCAACATTGTGTTCC | 145 |
| CAV1 | NM_001753 | S7152/CAV1.r1 | CAATGGCCTCCATTTTACAG | 146 |
| CAV1 | NM_001753 | S7153/CAV1.p1 | ATTTCAGCTGATCAGTGGGCCTCC | 147 |
| CCNB1 | NM_031966 | S1720/CCNB1.f2 | TTCAGGTTGTTGCAGGAGAC | 148 |
| CCNB1 | NM_031966 | S1721/CCNB1.r2 | CATCTTCTTGGGCACACAAT | 149 |
| CCNB1 | NM_031966 | S4733/CCNB1.p2 | | 150 |
| CCND1 | NM_053056 | S0058/CCND1.f3 | GCATGTTCGTGGCCTCTAAGA | 151 |
| CCND1 | NM_053056 | S0060/CCND1.r3 | CGGTGTAGATGCACAGCTTCTC | 152 |
| CCND1 | NM_053056 | S4986/CCND1.p3 | AAGGAGACCATCCCCCTGACGGC | 153 |
| CCNE2 | NM_057749 | S1458/CCNE2.f2 | ATGCTGTGGCTCCTTCCTAACT | 154 |
| CCNE2 | NM_057749 | S1459/CCN E2.r2 | | 155 |
| CCNE2 | NM_057749 | S4945/CCNE2.p2 | | 156 |
| CCT3 | NM_001008800 | T1053/CCT3.f1 | ATCCAAGGCCATGACTGG | 157 |
| CCT3 | NM_001008800 | T1054/CCT3.r1 | GGAATGACCTCTAGGGCCTG | 158 |
| CCT3 | NM_001008800 | T1055/CCT3.p1 | ACAGCCCTGTATGGCCATTGTTCC | 159 |
| CD14 | NM_000591 | T1997/CD14.f1 | GTGTGCTAGCGTACTCCCG | 160 |
| CD14 | NM_000591 | T1998/CD14.r1 | GCATGGTGCCGGTTATCT | 161 |
| CD14 | NM_000591 | T1999/CD14.p1 | CAAGGAACTGACGCTCGAGGACCT | 162 |
| CD31 | NM_000442 | S1407/CD31.f3 | TGTATTTCAAGACCTCTGTGCACTT | 163 |
| CD31 | NM_000442 | S1408/CD31.r3 | TTAGCCTGAGGAATTGCTGTGTT | 164 |
| CD31 | NM_000442 | S4939/CD31.p3 | | 165 |
| CD3z | NM_000734 | S0064/CD3z.f1 | AGATGAAGTGGAAGGCGCTT | 166 |
| CD3z | NM_000734 | S0066/CD3z.r1 | TGCCTCTGTAATCGGCAACTG | 167 |
| CD3z | NM_000734 | S4988/CD3z.p1 | CACCGCGGCCATCCTGCA | 168 |
| CD63 | NM_001780 | T1988/CD63.f1 | AGTGGGACTGATTGCCGT | 169 |
| CD63 | NM_001780 | T1989/CD63.r1 | GGGTAGCCCCCTGGATTAT | 170 |
| CD63 | NM_001780 | T1990/CD63.p1 | TCTGACTCAGGACAAGCTGTGCCC | 171 |
| CD68 | NM_001251 | S0067/CD68.f2 | TGGTTCCCAGCCCTGTGT | 172 |
| CD68 | NM_001251 | S0069/CD68.r2 | CTCCTCCACCCTGGGTTGT | 173 |
| CD68 | NM_001251 | S4734/CD68.p2 | | 174 |
| CDC2 | NM_001786 | S7238/CDC2.f1 | GAGAGCGACGCGGTTGTT | 175 |
| CDC2 | NM_001786 | S7239/CDC2.r1 | | 176 |
| CDC2 | NM_001786 | S7240/CDC2.p1 | TAGCTGCCGCTGCGGCCG | 177 |
| CDC20 | NM_001255 | S4447/CDC20.f1 | TGGATTGGAGTTCTGGGAATG | 178 |
| CDC20 | NM_001255 | S4448/CDC20.r1 | GCTTGCACTCCACAGGTACACA | 179 |
| CDC20 | NM_001255 | S4449/CDC20.p1 | ACTGGCCGTGGCACTGGACAACA | 180 |
| CDC25B | NM_021873 | S1160/CDC25B.f1 | AAACGAGCAGTTTGCCATCAG | 181 |
| CDC25B | NM_021873 | S1161/CDC25B.r1 | GTTGGTGATGTTCCGAAGCA | 182 |
| CDC25B | NM_021873 | S4842/CDC25B.p1 | CCTCACCGGCATAGACTGGAAGCG | 183 |
| CDCA8 | NM_018101 | T2060/CDCA8.f1 | GAGGCACAGTATTGCCCAG | 184 |
| CDCA8 | NM_018101 | T2061/CDCA8.r1 | GAGACGGTTGGAGAGCTTCTT | 185 |
| CDCA8 | NM_018101 | T2062/CDCA8.p1 | ATGTTTCCCAAGGCCTCTGGATCC | 186 |
| CDH1 | NM_004360 | S0073/CDH1.f3 | TGAGTGTCCCCCGGTATCTTC | 187 |
| CDH1 | NM_004360 | S0075/CDH1.r3 | CAGCCGCTTTCAGATTTTCAT | 188 |
| CDH1 | NM_004360 | S4990/CDH1.p3 | | 189 |
| CDK5 | NM_004935 | T2000/CDK5.f1 | AAGCCCTATCCGATGTACCC | 190 |
| CDK5 | NM_004935 | T2001/CDK5.r1 | CTGTGGCATTGAGTTTGGG | 191 |
| CDK5 | NM_004935 | T2002/CDK5.p1 | CACAACATCCCTGGTGAACGTCGT | 192 |
| CDKN1C | NM_000076 | T2003/CDKN1C.f1 | CGGCGATCAAGAAGCTGT | 193 |
| CDKN1C | NM_000076 | T2004/CDKN1C.r1 | CAGGCGCTGATCTCTTGC | 194 |
| CDKN1C | NM_000076 | T2005/CDKN1C.p1 | CGGGCCTCTGATCTCCGATTTCTT | 195 |
| CEGP1 | NM_020974 | S1494/CEGP1.f2 | TGACAATCAGCACACCTGCAT | 196 |
| CEGP1 | NM_020974 | S1495/CEGP1.r2 | TGTGACTACAGCCGTGATCCTTA | 197 |
| CEGP1 | NM_020974 | S4735/CEGP1.p2 | CAGGCCCTCTTCCGAGCGGT | 198 |
| CENPA | NM_001809 | S7082/CENPA.f1 | TAAATTCACTCGTGGTGTGGA | 199 |
| CENPA | NM_001809 | S7083/CENPA.r1 | GCCTCTTGTAGGGCCAATAG | 200 |
| CENPA | NM_001809 | S7084/CENPA.p1 | CTTCAATTGGCAAGCCCAGGC | 201 |
| CENPE | NM_001813 | S5496/CENPE.f3 | GGATGCTGGTGACCTCTTCT | 202 |
| CENPE | NM_001813 | S5497/CENPE.r3 | GCCAAGGCACCAAGTAACTC | 203 |
| CENPE | NM_001813 | S5498/CENPE.p3 | | 204 |
| CENPF | NM_016343 | S9200/CENPF.f1 | CTCCCGTCAACAGCGTTC | 205 |
| CENPF | NM_016343 | S9201/CENPF.r1 | GGGTGAGTCTGGCCTTCA | 206 |
| CENPF | NM_016343 | S9202/CENPF.p1 | ACACTGGACCAGGAGTGCATCCAG | 207 |
| CGA(CHGA official) | NM_001275 | S3221/CGA (C.f3 | CTGAAGGAGCTCCAAGACCT | 208 |
| CGA (CHGA official) | NM_001275 | S3222/CGA (C.r3 | CAAAACCGCTGTGTTTCTTC | 209 |
| CGA(CHGA official) | NM_001275 | S3254/CGA (C.p3 | TGCTGATGTGCCCTCTCCTTGG | 210 |
| CHFR | NM_018223 | S7085/CHFR.f1 | AAGGAAGTGGTCCCTCTGTG | 211 |
| CHFR | NM_018223 | S7086/CHFR.r1 | GACGCAGTCTTTCTGTCTGG | 212 |
| CHFR | NM_018223 | S7087/CHFR.p1 | TGAAGTCTCCAGCTTTGCCTCAGC | 213 |
| Chk1 | NM_001274 | S1422/Chk1.f2 | | 214 |
| Chk1 | NM_001274 | S1423/Chk1.r2 | GGGTGCCAAGTAACTGACTATTCA | 215 |
| Chk1 | NM_001274 | S4941/Chk1.p2 | | 216 |
| Chk2 | NM_007194 | S1434/Chk2.f3 | ATGTGGAACCCCCACCTACTT | 217 |
| Chk2 | NM_007194 | S1435/Chk2.r3 | CAGTCCACAGCACGGTTATACC | 218 |
| Chk2 | NM_007194 | S4942/Chk2.p3 | | 219 |
| clAP2 | NM_001165 | S0076/clAP2.f2 | GGATATTTCCGTGGCTCTTATTCA | 220 |
| clAP2 | NM_001165 | S0078/clAP2.r2 | CTTCTCATCAAGGCAGAAAAATCTT | 221 |
| clAP2 | NM_001165 | S4991/clAP2.p2 | | 222 |
| CKAP1 | NM_001281 | T2293/CKAP1.f1 | TCATTGACCACAGTGGCG | 223 |
| CKAP1 | NM_001281 | T2294/CKAP1.r1 | TCGTGTACTTCTCCACCCG | 224 |
| CKAP1 | NM_001281 | T2295/CKAP1.p1 | CACGTCCTCATACTCACCAAGGCG | 225 |
| CLU | NM_001831 | S5666/CLU.f3 | CCCCAGGATACCTACCACTACCT | 226 |
| CLU | NM_001831 | S5667/CLU.r3 | TGCGGGACTTGGGAAAGA | 227 |
| CLU | NM_001831 | S5668/CLU.p3 | CCCTTCAGCCTGCCCCACCG | 228 |
| Met | NM_000245 | S0082/cMet.f2 | GACATTTCCAGTCCTGCAGTCA | 229 |
| Met | NM_000245 | S0084/cMet.r2 | CTCCGATCGCACACATTTGT | 230 |
| cMet | NM_000245 | S4993/cMet.p2 | TGCCTCTCTGCCCCACCCTTTGT | 231 |
| cMYC | NM_002467 | S0085/cMYC.f3 | TCCCTCCACTCGGAAGGACTA | 232 |
| cMYC | NM_002467 | S0087/cMYC.r3 | CGGTTGTTGCTGATCTGTCTCA | 233 |
| cMYC | NM_002467 | S4994/cMYC.p3 | | 234 |
| CNN | NM_001299 | S4564/CNN.f1 | TCCACCCTCCTGGCTTTG | 235 |
| CNN | NM_001299 | S4565/CNN.r1 | TCACTCCCACGTTCACCTTGT | 236 |
| CNN | NM_001299 | S4566/CNN.p1 | TCCTTTCGTCTTCGCCATGCTGG | 237 |
| COL1A1 | NM_000088 | S4531/COL1A1.f1 | GTGGCCATCCAGCTGACC | 238 |
| COL1A1 | NM_000088 | S4532/COL1A1.r1 | CAGTGGTAGGTGATGTTCTGGGA | 239 |
| COL1A1 | NM_000088 | S4533/COL1A1.p1 | TCCTGCGCCTGATGTCCACCG | 240 |
| COL1A2 | NM_000089 | S4534/COL1A2.f1 | CAGCCAAGAACTGGTATAGGAGCT | 241 |
| COL1A2 | NM_000089 | S4535/COL1A2.r1 | AAACTGGCTGCCAGCATTG | 242 |
| COL1A2 | NM_000089 | S4536/COL1A2.p1 | | 243 |
| COL6A3 | NM_004369 | T1062/COL6A3.f1 | GAGAGCAAGCGAGACATTCTG | 244 |
| COL6A3 | NM_004369 | T1063/COL6A3.r1 | AACAGGGAACTGGCCCAC | 245 |
| COL6A3 | NM_004369 | T1064/COL6A3.p1 | CCTCTTTGACGGCTCAGCCAATCT | 246 |
| Contig 51037 | NM_198477 | S2070/Contig.f1 | CGACAGTTGCGATGAAAGTTCTAA | 247 |
| Contig 51037 | NM_198477 | S2071/Contig.r1 | GGCTGCTAGAGACCATGGACAT | 248 |
| Contig 51037 | NM_198477 | S5059/Contig.p1 | | 249 |
| COX2 | NM_000963 | S0088/COX2.f1 | TCTGCAGAGTTGGAAGCACTCTA | 250 |
| COX2 | NM_000963 | S0090/COX2.r1 | GCCGAGGCTTTTCTACCAGAA | 251 |
| COX2 | NM_000963 | S4995/COX2.p1 | | 252 |
| COX7C | NM_001867 | T0219/COX7C.f1 | ACCTCTGTGGTCCGTAGGAG | 253 |
| COX7C | NM_001867 | T0220/COX7C.r1 | CGACCACTTGTTTTCCACTG | 254 |
| COX7C | NM_001867 | T0221/COX7C.p1 | TCTTCCCAGGGCCCTCCTCATAGT | 255 |
| CRABP1 | NM_004378 | S5441/CRABP1.f3 | AACTTCAAGGTCGGAGAAGG | 256 |
| CRABP1 | NM_004378 | S5442/CRABP1.r3 | TGGCTAAACTCCTGCACTTG | 257 |
| CRABP1 | NM_004378 | S5443/CRABP1.p3 | CCGTCCACGGTCTCCTCCTCA | 258 |
| CRIP2 | NM_001312 | S5676/CRIP2.f3 | GTGCTACGCCACCCTGTT | 259 |
| CRIP2 | NM_001312 | S5677/CRIP2.r3 | CAGGGGCTTCTCGTAGATGT | 260 |
| CRIP2 | NM_001312 | S5678/CRIP2.p3 | CCGATGTTCACGCCTTTGGGTC | 261 |
| CRYAB | NM_001885 | S8302/CRYAB.f1 | GATGTGATTGAGGTGCATGG | 262 |
| CRYAB | NM_001885 | S8303/CRYAB.r1 | GAACTCCCTGGAGATGAAACC | 263 |
| CRYAB | NM_001885 | S8304/CRYAB.p1 | TGTTCATCCTGGCGCTCTTCATGT | 264 |
| CSF1 | NM_000757 | S1482/CSF1.f1 | TGCAGCGGCTGATTGACA | 265 |
| CSF1 | NM_000757 | S1483/CSF1.r1 | CAACTGTTCCTGGTCTACAAACTCA | 266 |
| CSF1 | NM_000757 | S4948/CSF1.p1 | | 267 |
| CSNK1D | NM_001893 | S2332/CSNK1D.f3 | AGCTTTTCCGGAATCTGTTC | 268 |
| CSNK1D | NM_001893 | S2333/CSNK1D.r3 | ATTTGAGCATGTTCCAGTCG | 269 |
| CSNK1D | NM_001893 | S4850/CSNK1D.p3 | CATCGCCAGGGCTTCTCCTATGAC | 270 |
| CST7 | NM_003650 | T2108/CST7.f1 | TGGCAGAACTACCTGCAAGA | 271 |
| CST7 | NM_003650 | T2109/CST7.r1 | TGCTTCAAGGTGTGGTTGG | 272 |
| CST7 | NM_003650 | T2110/CST7.p1 | CACCTGCGTCTGGATGACTGTGAC | 273 |
| CTSD | NM_001909 | S1152/CTSD.f2 | GTACATGATCCCCTGTGAGAAGGT | 274 |
| CTSD | NM_001909 | S1153/CTSD.r2 | GGGACAGCTTGTAGCCTTTGC | 275 |
| CTSD | NM_001909 | S4841/CTSD.p2 | ACCCTGCCCGCGATCACACTGA | 276 |
| CTSL | NM_001912 | S1303/CTSL.f2 | GGGAGGCTTATCTCACTGAGTGA | 277 |
| CTSL | NM_001912 | S1304/CTSL.r2 | CCATTGCAGCCTTCATTGC | 278 |
| CTSL | NM_001912 | S4899/CTSL.p2 | | 279 |
| CTSL2 | NM_001333 | S4354/CTSL2.f1 | TGTCTCACTGAGCGAGCAGAA | 280 |
| CTSL2 | NM_001333 | S4355/CTSL2.r1 | ACCATTGCAGCCCTGATTG | 281 |
| CTSL2 | NM_001333 | S4356/CTSL2.p1 | CTTGAGGACGCGAACAGTCCACCA | 282 |
| CXCR4 | NM_003467 | S5966/CXCR4.f3 | TGACCGCTTCTACCCCAATG | 283 |
| CXCR4 | NM_003467 | S5967/CXCR4.r3 | AGGATAAGGCCAACCATGATGT | 284 |
| CXCR4 | NM_003467 | S5968/CXCR4.p3 | | 285 |
| CYBA | NM_000101 | S5300/CYBA.f1 | GGTGCCTACTCCATTGTGG | 286 |
| CYBA | NM_000101 | S5301/CYBA.r1 | GTGGAGCCCTTCTTCCTCTT | 287 |
| CYBA | NM_000101 | S5302/CYBA.p1 | TACTCCAGCAGGCACACAAACACG | 288 |
| CYP1B1 | NM_000104 | S0094/CYP1B1.f3 | CCAGCTTTGTGCCTGTCACTAT | 289 |
| CYP1B1 | NM 000104 | S0096/CYP1B1.r3 | GGGAATGTGGTAGCCCAAGA | 290 |
| CYP1B1 | NM_000104 | S4996/CYP1B1.p3 | | 291 |
| CYP2C8 | NM_000770 | S1470/CYP2C8.f2 | CCGTGTTCAAGAGGAAGCTC | 292 |
| CYP2C8 | NM_000770 | S1471/CYP2C8.r2 | AGTGGGATCACAGGGTGAAG | 293 |
| CYP2C8 | NM_000770 | S4946/CYP2C8.p2 | TTTTCTCAACTCCTCCACAAGGCA | 294 |
| CYP3A4 | NM_017460 | S1620/CYP3A4.f2 | | 295 |
| CYP3A4 | NM 017460 | S1621/CYP3A4.r2 | GCAAACCTCATGCCAATGC | 296 |
| CYP3A4 | NM_017460 | S4906/CYP3A4.p2 | | 297 |
| DDR1 | NM_001954 | T2156/DDR1.f1 | CCGTGTGGCTCGCTTTCT | 298 |
| DDR1 | NM_001954 | T2157/DDR1.r1 | GGAGATTTCGCTGAAGAGTAACCA | 299 |
| DDR1 | NM_001954 | T21581DDR1.p1 | TGCCGCTTCCTCTTTGCGGG | 300 |
| DIABLO | NM_019887 | S0808/DIABLO.f1 | CACAATGGCGGCTCTGAAG | 301 |
| DIABLO | NM_019887 | S0809/DIABLO.r1 | | 302 |
| DIABLO | NM_019887 | S4813/DIABLO.p1 | AAGTTACGCTGCGCGACAGCCAA | 303 |
| DIAPH1 | NM_005219 | S7608/DIAPH1.f1 | CAAGCAGTCAAGGAGAACCA | 304 |
| DIAPH1 | NM_005219 | S7609/DIAPH 1.r1 | AGTTTTGCTCGCCTCATCTT | 305 |
| DIAPH1 | NM_005219 | S7610/DIAPH1.p1 | TTCTTCTGTCTCCCGCCGCTTC | 306 |
| DICER1 | NM_177438 | S5294/DICER1.f2 | TCCAATTCCAGCATCACTGT | 307 |
| DICER1 | NM_177438 | S5295/DICER1.r2 | GGCAGTGAAGGCGATAAAGT | 308 |
| DICER1 | NM_177438 | S5296/DICER1.p2 | | 309 |
| DKFZp564D0462; | NM_198569 | S4405/DKFZp5.f2 | CAGTGCTTCCATGGACAAGT | 310 |
| DKFZp564D0462; | NM_198569 | S4406/DKFZp5.r2 | TGGACAGGGATGATTGATGT | 311 |
| DKFZp564D0462; | NM_198569 | S4407/DKFZp5.p2 | ATCTCCATCAGCATGGGCCAGTTT | 312 |
| DR4 | NM_003844 | S2532/DR4.f2 | TGCACAGAGGGTGTGGGTTAC | 313 |
| DR4 | NM_003844 | S2533/DR4.r2 | | 314 |
| DR4 | NM_003844 | S4981/DR4.p2 | | 315 |
| DR5 | NM_003842 | S2551/DR5.f2 | CTCTGAGACAGTGCTTCGATGACT | 316 |
| DR5 | NM_003842 | S2552/DR5.r2 | CCATGAGGCCCAACTTCCT | 317 |
| DR5 | NM_003842 | S4979/DR5.p2 | CAGACTTGGTGCCCTTTGACTCC | 318 |
| DUSP1 | NM_004417 | S7476/DUSP1.f1 | AGACATCAGCTCCTGGTTCA | 319 |
| DUSP1 | NM_004417 | S7477/DUSP1.r1 | GACAAACACCCTTCCTCCAG | 320 |
| DUSP1 | NM_004417 | S7478/DUSP1.p1 | | 321 |
| EEF1D | NM_001960 | T2159/EEF1D.f1 | CAGAGGATGACGAGGATGATGA | 322 |
| EEF1D | NM_001960 | T2160/EEF1D.r1 | CTGTGCCGCCTCCTTGTC | 323 |
| EEF1D | NM_001960 | T2161/EEF1D.p1 | | 324 |
| EGFR | NM_005228 | S0103/EGFR.f2 | TGTCGATGGACTTCCAGAAC | 325 |
| EGFR | NM_005228 | S0105/EGFR.r2 | ATTGGGACAGCTTGGATCA | 326 |
| EGFR | NM_005228 | S4999/EGFR.p2 | CACCTGGGCAGCTGCCAA | 327 |
| EIF4E | NM_001968 | S0106/EIF4E.f1 | GATCTAAGATGGCGACTGTCGAA | 328 |
| EIF4E | NM_001968 | S0108/EIF4E.r1 | TTAGATTCCGTTTTCTCCTCTTCTG | 329 |
| EIF4E | NM_001968 | S5000/EIF4E.p1 | | 330 |
| EIF4EL3 | NM_004846 | S4495/EIF4EL.f1 | AAGCCGCGGTTGAATGTG | 331 |
| EIF4EL3 | NM_004846 | S4496/EIF4EL.r1 | TGACGCCAGCTTCAATGATG | 332 |
| EIF4EL3 | NM_004846 | S4497/EIF4EL.p1 | | 333 |
| ELP3 | NM_018091 | T2234/ELP3.f1 | CTCGGATCCTAGCCCTCG | 334 |
| ELP3 | NM_018091 | T2235/ELP3.r1 | GGCATTGGAATATCCCTCTGTA | 335 |
| ELP3 | NM_018091 | T2236/ELP3.p1 | CCTCCATGGACTCGAGTGTACCGA | 336 |
| ER2 | NM_001437 | S0109/ER2.f2 | TGGTCCATCGCCAGTTATCA | 337 |
| ER2 | NM_001437 | S0111/ER2.r2 | TGTTCTAGCGATCTTGCTTCACA | 338 |
| ER2 | NM_001437 | S5001/ER2.p2 | | 339 |
| ErbB3 | NM_001982 | S0112/ErbB3.f1 | CGGTTATGTCATGCCAGATACAC | 340 |
| ErbB3 | NM_001982 | S0114/ErbB3.r1 | GAACTGAGACCCACTGAAGAAAGG | 341 |
| ErbB3 | NM_001982 | S5002/ErbB3.p1 | | 342 |
| ERBB4 | NM_005235 | S1231/ERBB4.f3 | TGGCTCTTAATCAGTTTCGTTACCT | 343 |
| ERBB4 | NM_005235 | S1232/ERBB4.r3 | CAAGGCATATCGATCCTCATAAAGT | 344 |
| ERBB4 | NM_005235 | S4891/ERBB4.p3 | | 345 |
| ERCC1 | NM_001983 | S2437/ERCC1.f2 | GTCCAGGTGGATGTGAAAGA | 346 |
| ERCC1 | NM_001983 | S2438/ERCC1.r2 | CGGCCAGGATACACATCTTA | 347 |
| ERCC1 | NM_001983 | S4920/ERCC1.p2 | CAGCAGGCCCTCAAGGAGCTG | 348 |
| ERK1 | NM_002746 | S1560/ERK1.f3 | ACGGATCACAGTGGAGGAAG | 349 |
| ERK1 | NM_002746 | S1561/ERK1.r3 | CTCATCCGTCGGGTCATAGT | 350 |
| ERK1 | NM_002746 | S4882/ERK1.p3 | CGCTGGCTCACCCCTACCTG | 351 |
| ESPL1 | NM_012291 | S5686/ESPL1.f3 | ACCCCCAGACCGGATCAG | 352 |
| ESPL1 | NM_012291 | S5687/ESPL1.r3 | TGTAGGGCAGACTTCCTCAAACA | 353 |
| ESPL1 | NM_012291 | S5688/ESPL1.p3 | CTGGCCCTCATGTCCCCTTCACG | 354 |
| EstR1 | NM_000125 | S0115/EstR1.f1 | CGTGGTGCCCCTCTATGAC | 355 |
| EstR1 | NM_000125 | S0117/EstR1.r1 | GGCTAGTGGGCGCATGTAG | 356 |
| EstR1 | NM_000125 | S4737/EstR1.p1 | CTGGAGATGCTGGACGCCC | 357 |
| fas | NM_000043 | S0118/fas.f1 | GGATTGCTCAACAACCATGCT | 358 |
| fas | NM_000043 | S0120/fas.r1 | GGCATTAACACTTTTGGACGATAA | 359 |
| fas | NM_000043 | S5003/fas.p1 | | 360 |
| fasI | NM_000639 | S0121/fasl.f2 | GCACTTTGGGATTCTTTCCATTAT | 361 |
| fasI | NM_000639 | S0123/fasl.r2 | GCATGTAAGAAGACCCTCACTGAA | 362 |
| fasI | NM_000639 | S5004/fasl.p2 | | 363 |
| FASN | NM_004104 | S8287/FASN.f1 | GCCTCTTCCTGTTCGACG | 364 |
| FASN | NM_004104 | S8288/FASN.r1 | GCTTTGCCCGGTAGCTCT | 365 |
| FASN | NM_004104 | S8289/FASN.p1 | TCGCCCACCTACGTACTGGCCTAC | 366 |
| FBXO5 | NM_012177 | S2017/FBX05.r1 | | 367 |
| FBXO5 | NM_012177 | S2018/FBXO5.f1 | | 368 |
| FBXO5 | NM_012177 | S5061/FBXO5.p1 | | 369 |
| FDFT1 | NM_004462 | T2006/FDFT1.f1 | AAGGAAAGGGTGCCTCATC | 370 |
| FDFT1 | NM_004462 | T2007/FDFT1.r1 | GAGCCACAAGCAGCACAGT | 371 |
| FDFT1 | NM_004462 | T2008/FDFT1.p1 | CATCACCCACAAGGACAGGTTGCT | 372 |
| FGFR1 | NM_023109 | S0818/FGFR1.f3 | CACGGGACATTCACCACATC | 373 |
| FGFR1 | NM_023109 | S0819/FGFR1.r3 | GGGTGCCATCCACTTCACA | 374 |
| FGFR1 | NM_023109 | S4816/FGFR1.p3 | | 375 |
| FHIT | NM_002012 | S2443/FHIT.f1 | CCAGTGGAGCGCTTCCAT | 376 |
| FHIT | NM_002012 | S2444/FHIT.r1 | CTCTCTGGGTCGTCTGAAACAA | 377 |
| FHIT | NM_002012 | S4921/FHIT.p1 | TCGGCCACTTCATCAGGACGCAG | 378 |
| FIGF | NM_004469 | S8941/FIGF.f1 | GGTTCCAGCTTTCTGTAGCTGT | 379 |
| FIGF | NM_004469 | S8942/FIGF.r1 | GCCGCAGGTTCTAGTTGCT | 380 |
| FIGF | NM_004469 | S8943/FIGF.p1 | ATTGGTGGCCACACCACCTCCTTA | 381 |
| FLJ20354 (DEPDC1 official) | NM_017779 | S4309/FLJ203.f1 | GCGTATGATTTCCCGAATGAG | 382 |
| FLJ20354 (DEPDC1 official) | NM_017779 | S4310/FLJ203.r1 | CAGTGACCTCGTACCCATTGC | 383 |
| FLJ20354 (DEPDC1 official) | NM_017779 | S4311/FLJ203.p1 | ATGTTGATATGCCCAAACTTCATGA | 384 |
| FOS | NM_005252 | S6726/FOS.f1 | CGAGCCCTTTGATGACTTCCT | 385 |
| FOS | NM_005252 | S6727/FOS.r1 | GGAGCGGGCTGTCTCAGA | 386 |
| FOS | NM_005252 | S6728/FOS.p1 | TCCCAGCATCATCCAGGCCCAG | 387 |
| FOXM1 | NM_021953 | S2006/FOXM1.f1 | CCACCCCGAGCAAATCTGT | 388 |
| FOXM1 | NM_021953 | S2007/FOXM1.r1 | AAATCCAGTCCCCCTACTTTGG | 389 |
| FOXM1 | NM_021953 | S4757/FOXM1.p1 | CCTGAATCCTGGAGGCTCACGCC | 390 |
| FUS | NM_004960 | S2936/FUS.f1 | | 391 |
| FUS | NM_004960 | S2937/FUS.r1 | | 392 |
| FUS | NM_004960 | S4801/FUS.p1 | | 393 |
| FYN | NM_002037 | S5695/FYN.f3 | GAAGCGCAGATCATGAAGAA | 394 |
| FYN | NM_002037 | S5696/FYN.r3 | CTCCTCAGACACCACTGCAT | 395 |
| FYN | NM_002037 | S5697/FYN.p3 | CTGAAGCACGACAAGCTGGTCCAG | 396 |
| G1P3 | NM_002038 | T1086/G1P3.f1 | CCTCCAACTCCTAGCCTCAA | 397 |
| G1P3 | MM_002038 | T1087/G1P3.r1 | GGCGCATGCTTGTAATCC | 398 |
| G1P3 | NM_002038 | T1088/G1P3.p1 | TGATCCTCCTGTCTCAACCTCCCA | 399 |
| GADD45 | NM_001924 | S5835/GADD45.f3 | GTGCTGGTGACGAATCCA | 400 |
| GADD45 | NM_001924 | S5836/GADD45.r3 | CCCGGCAAAAACAAATAAGT | 401 |
| GADD45 | NM_001924 | S5837/GADD45.p3 | 402 | 402 |
| GADD45B | NM_015675 | S6929/GADD45.f1 | ACCCTCGACAAGACCACACT | 403 |

| Gene Name | Accession # | Oligo Name | Oigo Sequence | SEQ ID NO |
|---|---|---|---|---|
| GADD45B | NM_015675 | S6930/GADD45.r1 | TGGGAGTTCATGGGTACAGA | 404 |
| GADD45B | NM_015675 | S6931/GADD45.p1 | AACTTCAGCCCCAGCTCCCAAGTC | 405 |
| GAGE1 | NM_001468 | T2162/GAGE1.f1 | AAGGGCAATCACAGTGTTAAAAGA A | 406 |
| GAGE1 | NM_001468 | T2163/GAGE1.r1 | | 407 |
| GAGE1 | NM_001468 | T2164/GAGE1.p1 | | 408 |
| GAPDH | NM_002046 | S0374/GAPDH.f1 | ATTCCACCCATGGCAAATTC | 409 |
| GAPDH | NM_002046 | S0375/GAPDH.r1 | GATGGGATTTCCATTGATGACA | 410 |
| GAPDH | NM_002046 | S4738/GAPDH.p1 | CCGTTCTCAGCCTTGACGGTGC | 411 |
| GATA3 | NM_002051 | S0127/GATA3.f3 | CAAAGGAGCTCACTGTGGTGTCT | 412 |
| GATA3 | NM_002051 | S0129/GATA3.r3 | | 413 |
| GATA3 | NM_002051 | S5005/GATA3.p3 | TGTTCCAACCACTGAATCTGGACC | 414 |
| GBP1 | NM_002053 | S5698/GBP1.f1 | TTGGGAAATATTTGGGCATT | 415 |
| GBP1 | NM_002053 | S5699/GBP1.r1 | AGAAGCTAGGGTGGTTGTCC | 416 |
| GBP1 | NM_002053 | S5700/GBP1.p1 | | 417 |
| GBP2 | NM_004120 | S5707/GBP2.f2 | GCATGGGAACCATCAACCA | 418 |
| GBP2 | NM_004120 | S5708/GBP2.r2 | TGAGGAGTTTGCCTTGATTCG | 419 |
| GBP2 | NM_004120 | S5709/GBP2.p2 | | 420 |
| GCLC | NM_001498 | S0772/GCLC.f3 | CTGTTGCAGGAAGGCATTGA | 421 |
| GCLC | NM_001498 | S0773/GCLC.r3 | | 422 |
| GCLC | NM_001498 | S4803/GCLC.p3 | CATCTCCTGGCCCAGCATGTT | 423 |
| GDF15 | NM_004864 | S7806/GDF15.f1 | CGCTCCAGACCTATGATGACT | 424 |
| GDF15 | NM_004864 | S7807/GDF15.r1 | ACAGTGGAAGGACCAGGACT | 425 |
| GDF15 | NM_004864 | S7808/GDF15.p1 | TGTTAGCCAAAGACTGCCACTGCA | 426 |
| GGPS1 | NM_004837 | S1590/GGPS1.f1 | CTCCGACGTGGCTTTCCA | 427 |
| GGPS1 | NM_004837 | S1591/GGPS1.r1 | CGTAATTGGCAGAATTGATGACA | 428 |
| GGPS1 | NM_004837 | S4896/GGPS1.p1 | 429 | 429 |
| GLRX | NM_002064 | T2165/GLRX.f1 | GGAGCTCTGCAGTAACCACAGAA | 430 |
| GLRX | NM_002064 | T2166/GLRX.r1 | CAATGCCATCCAGCTCTTGA | 431 |
| GLRX | NM_002064 | T2167/GLRX.p1 | AGGCCCCATGCTGACGTCCCTC | 432 |
| GNS | NM_002076 | T2009/GNS.f1 | GGTGAAGGTTGTCTCTTCCG | 433 |
| GNS | NM_002076 | T2010/GNS.r1 | CAGCCCTTCCACTTGTCTG | 434 |
| GNS | NM_002076 | T2011/GNS.p1 | AAGAGCCCTGTCTTCAGAAGGCCC | 435 |
| GPR56 | NM_005682 | T2120/GPR56.f1 | TACCCTTCCATGTGCTGGAT | 436 |
| GPR56 | NM_005682 | T2121/GPR56.r1 | GCTGAAGAGGCCCAGGTT | 437 |
| GPR56 | NM_005682 | T2122/GPR56.p1 | CGGGACTCCCTGGTCAGCTACATC | 438 |
| GPX1 | NM_000581 | S8296/GPX1.f2 | GCTTATGACCGACCCCAA | 439 |
| GPX1 | NM_000581 | S8297/GPX1.r2 | AAAGTTCCAGGCAACATCGT | 440 |
| GPX1 | NM_000581 | S8298/GPX1.p2 | CTCATCACCTGGTCTCCGGTGTGT | 441 |
| GRB7 | NM_005310 | S0130/GRB7.f2 | CCATCTGCATCCATCTTGTT | 442 |

| Gene Name | Accession # | Oligo Name | Oligo Sequence | SEQ ID NO |
|---|---|---|---|---|
| GRB7 | NM_005310 | S0132/GRB7.r2 | GGCCACCAGGGTATTATCTG | 443 |
| GRB7 | NM_005310 | S4726/GRB7.p2 | CTCCCCACCCTTGAGAAGTGCCT | 444 |
| GSK3B | NM_002093 | T0408/GSK3B.f2 | GACAAGGACGGCAGCAAG | 445 |
| GSK3B | NM_002093 | T0409/GSK3B.r2 | TTGTGGCCTGTCTGGACC | 446 |
| GSK3B | NM_002093 | T0410/GSK3B.p2 | CCAGGAGTTGCCACCACTGTTGTC | 447 |
| GSR | NM_000637 | S8633/GSR.f1 | GTGATCCCAAGCCCACAATA | 448 |
| GSR | NM_000637 | S8634/GSR.r1 | TGTGGCGATCAGGATGTG | 449 |
| GSR | NM_000637 | S8635/GSR.p1 | TCAGTGGGAAAAAGTACACCGCCC | 450 |
| GSTM1 | NM_000561 | S2026/GSTM1.r1 | GGCCCAGCTTGAATTTTCA | 451 |
| GSTM1 | NM_000561 | S2027/GSTM1.f1 | | 452 |
| GSTM1 | NM_000561 | S4739/GSTM1.p1 | | 453 |
| GSTp | NM_000852 | S0136/GSTp.f3 | GAGACCCTGCTGTCCCAGAA | 454 |
| GSTp | NM_000852 | S0138/GSTp.r3 | GGTTGTAGTCAGCGAAGGAGATC | 455 |
| GSTp | NM_000852 | S5007/GSTp.p3 | | 456 |
| GUS | NM_000181 | S0139/GUS.f1 | CCCACTCAGTAGCCAAGTCA | 457 |
| GUS | NM_000181 | S0141/GUS.r1 | CACGCAGGTGGTATCAGTCT | 458 |
| GUS | NM_000181 | S4740/GUS.p1 | | 459 |
| HDAC6 | NM_006044 | S9451/HDAC6.f1 | TCCTGTGCTCTGGAAGCC | 460 |
| HDAC6 | NM_006044 | S9452/HDAC6.r1 | CTCCACGGTCTCAGTTGATCT | 461 |
| HDAC6 | NM_006044 | S9453/HDAC6.p1 | CAAGAACCTCCCAGAAGGGCTCAA | 462 |
| HER2 | NM_004448 | S0142/HER2.f3 | CGGTGTGAGAAGTGCAGCAA | 463 |
| HER2 | NM_004448 | S0144/HER2.r3 | CCTCTCGCAAGTGCTCCAT | 464 |
| HER2 | NM_004448 | S4729/HER2.p3 | CCAGACCATAGCACACTCGGGCAC | 465 |
| HIF1A | NM_001530 | S1207/HIF1A.f3 | TGAACATAAAGTCTGCAACATGGA | 466 |
| HIF1A | NM_001530 | S1208/HIF1A.r3 | | 467 |
| HIF1A | NM_001530 | S4753/HIF1A.p3 | TTGCACTGCACAGGCCACATTCAC | 468 |
| HNF3A | NM_004496 | S0148/HNF3A.f1 | TCCAGGATGTTAGGAACTGTGAAG | 469 |
| HNF3A | NM_004496 | S0150/HNF3A.r1 | GCGTGTCTGCGTAGTAGCTGTT | 470 |
| HNF3A | NM_004496 | S5008/HNF3A.p1 | AGTCGCTGGTTTCATGCCCTTCCA | 471 |
| HRAS | NM_005343 | S8427/HRAS.f1 | GGACGAATACGACCCCACT | 472 |
| HRAS | NM_005343 | S8428/HRAS.r1 | GCACGTCTCCCCATCAAT | 473 |
| HRAS | NM_005343 | S8429/HRAS.p1 | ACCACCTGCTTCCGGTAGGAATCC | 474 |
| HSPA1A | NM_005345 | S6708/HSPA1A.f1 | CTGCTGCGACAGTCCACTA | 475 |
| HSPA1A | NM_005345 | S6709/HSPA1A.r1 | CAGGTTCGCTCTGGGAAG | 476 |
| HSPA1A | NM_005345 | S6710/HSPA1A.p1 | AGAGTGACTCCCGTTGTCCCAAGG | 477 |
| HSPA1B | NM_005346 | S6714/HSPA1B.f1 | GGTCCGCTTCGTCTTTCGA | 478 |
| HSPA1B | NM_005346 | S6715/HSPA1B.r1 | GCACAGGTTCGCTCTGGAA | 479 |
| HSPA1B | NM_005346 | S6716/HSPA1B.p1 | TGACTCCCGCGGTCCCAAGG | 480 |
| HSPA1L | NM_005527 | T2015/HSPA1L.f1 | GCAGGTGTGATTGCTGGAC | 481 |
| HSPA1L | NM_005527 | T2016/HSPA1L.r1 | ACCATAGGCAATGGCAGC | 482 |
| HSPA1L | NM_005527 | T2017/HSPA1L.p1 | AAGAATCATCAATGAGCCCACGGC | 483 |
| HSPA5 | NM_005347 | S7166/HSPA5.f1 | GGCTAGTAGAACTGGATCCCAACA | 484 |
| HSPA5 | NM_005347 | S7167/HSPA5.r1 | GGTCTGCCCAAATGCTTTTC | 485 |
| HSPA5 | NM_005347 | S7168/HSPA5.p1 | | 486 |
| HSPA9B | NM_004134 | T2018/HSPA9B.f1 | GGCCACTAAAGATGCTGGC | 487 |
| HSPA9B | NM_004134 | T2019/HSPA9B.r1 | AGCAGCTGTGGGCTCATT | 488 |
| HSPA9B | NM_004134 | T2020/HSPA9B.p1 | ATCACCCGAAGCACATTCAGTCCA | 489 |
| HSPB1 | NM_001540 | S6720/HSPB1.f1 | CCGACTGGAGGAGCATAAA | 490 |
| HSPB1 | NM_001540 | S6721/HSPB1.r1 | ATGCTGGCTGACTCTGCTC | 491 |
| HSPB1 | NM_001540 | S6722/HSPB1.p1 | CGCACTTTTCTGAGCAGACGTCCA | 492 |
| HSPCA | NM_005348 | S7097/HSPCA.f1 | CAAAAGGCAGAGGCTGATAA | 493 |
| HSPCA | NM_005348 | S7098/HSPCA.r1 | AGCGCAGTTTCATAAAGCAA | 494 |
| HSPCA | NM_005348 | S7099/HSPCA.p1 | | 495 |
| ID1 | NM_002165 | S0820/ID1.f1 | AGAACCGCAAGGTGAGCAA | 496 |
| ID1 | NM_002165 | S0821/ID1.r1 | TCCAACTGAAGGTCCCTGATG | 497 |
| ID1 | NM_002165 | S4832/ID1.p1 | | 498 |
| IFITM1 | NM_003641 | S7768/IFITM1.f1 | CACGCAGAAAACCACACTTC | 499 |
| IFITM1 | NM_003641 | S7769/IFITM1.r1 | CATGTTCCTCCTTGTGCATC | 500 |
| IFITM1 | NM_003641 | S7770/IFITM1.p1 | CAACACTTCCTTCCCCAAAGCCAG | 501 |
| IGF1R | NM_000875 | S1249/IGF1R.f3 | GCATGGTAGCCGAAGATTTCA | 502 |
| IGF1R | NM_000875 | S1250/IGF1R.r3 | | 503 |
| IGF1R | NM_000875 | S4895/IGF1R.p3 | | 504 |
| IGFBP2 | NM_000597 | S1128/IGFBP2.f1 | GTGGACAGCACCATGAACA | 505 |
| IGFBP2 | NM_000597 | S1129/IGFBP2.r1 | CCTTCATACCCGACTTGAGG | 506 |
| IGFBP2 | NM_000597 | S4837/IGFBP2.p1 | CTTCCGGCCAGCACTGCCTC | 507 |
| IGFBP3 | NM_000598 | S0157/IGFBP3.f3 | ACGCACCGGGTGTCTGA | 508 |
| IGFBP3 | NM_000598 | S0159/IGFBP3.r3 | TGCCCTTTCTTGATGATGATTATC | 509 |
| IGFBP3 | MM_000598 | S5011/IGFBP3.p3 | CCCAAGTTCCACCCCCTCCATTCA | 510 |
| IGFBP5 | NM_000599 | S1644/IGFBP5.f1 | TGGACAAGTACGGGATGAAGCT | 511 |
| IGFBP5 | NM_000599 | S1645/IGFBP5.r1 | CGAAGGTGTGGCACTGAAAGT | 512 |
| IGFBP5 | NM_000599 | S4908/IGFBP5.p1 | CCCGTCAACGTACTCCATGCCTGG | 513 |
| IL-7 | NM_000880 | S5781/IL-7.f1 | GCGGTGATTCGGAAATTCG | 514 |
| IL-7 | NM_000880 | S5782/IL-7.r1 | CTCTCCTGGGCACCTGCTT | 515 |
| IL-7 | NM_000880 | S5783/IL-7.p1 | CTCTGGTCCTCATCCAGGTGCGC | 516 |
| IL-8 | NM_000584 | S5790/IL-8.f1 | | 517 |
| IL-8 | NM_000584 | S5791/IL-8.r1 | ATCAGGAAGGCTGCCAAGAG | 518 |
| IL-8 | NM_000584 | S5792/IL-8.p1 | TGACTTCCAAGCTGGCCGTGGC | 519 |
| IL2RA | NM_000417 | T2147/IL2RA.f1 | TCTGCGTGGTTCCTTTCTCA | 520 |
| IL2RA | NM_000417 | T2148/IL2RA.r1 | TTGAAGGATGTTTATTAGGCAACGT | 521 |
| IL2RA | NM_000417 | T2149/IL2RA.p1 | | 522 |
| IL6 | NM_000600 | S0760/IL6.f3 | CCTGAACCTTCCAAAGATGG | 523 |
| IL6 | NM_000600 | S0761/IL6.r3 | ACCAGGCAAGTCTCCTCATT | 524 |
| IL6 | NM_000600 | S4800/IL6.p3 | | 525 |
| ILBRB | NM_001557 | T2168/IL8RB.f1 | CCGCTCCGTCACTGATGTCT | 526 |
| IL8RB | NM_001557 | T2169/ILBRB.r1 | GCAAGGTCAGGGCAAAGAGTA | 527 |
| IL8RB | NM_001557 | T2170/IL8RB.p1 | CCTGCTGAACCTAGCCTTGGCCGA | 528 |
| ILK | NM_001014794 | T0618/ILK.f1 | CTCAGGATTTTCTCGCATCC | 529 |
| ILK | NM_001014794 | T0619/ILK.r1 | AGGAGCAGGTGGAGACTGG | 530 |
| ILK | NM_001014794 | T0620/ILK.p1 | ATGTGCTCCCAGTGCTAGGTGCCT | 531 |
| ILT-2 | NM_006669 | S1611/ILT-2.f2 | AGCCATCACTCTCAGTGCAG | 532 |
| ILT-2 | NM_006669 | S1612/ILT-2.r2 | ACTGCAGAGTCAGGGTCTCC | 533 |
| ILT-2 | NM_006669 | S4904/ILT-2.p2 | CAGGTCCTATCGTGGCCCCTGA | 534 |
| INCENP | NM_020238 | T2024/INCENP.f1 | GCCAGGATACTGGAGTCCATC | 535 |
| INCENP | NM_020238 | T2025/INCENP.r1 | CTTGACCCTTGGGGTCCT | 536 |
| INCENP | NM_020238 | T2026/INCENP.p1 | TGAGCTCCCTGATGGCTACACCC | 537 |
| IRAK2 | NM_001570 | T2027/IRAK2.f1 | GGATGGAGTTCGCCTCCT | 538 |
| IRAK2 | NM_001570 | T2028/IRAK2.r1 | CGCTCCATGGACTTGATCTT | 539 |
| IRAK2 | NM_001570 | T2029/IRAK2.p1 | CGTGATCACAGACCTGACCCAGCT | 540 |
| IRS1 | NM_005544 | S1943/IRS1.f3 | CCACAGCTCACCTTCTGTCA | 541 |
| IRS1 | NM_005544 | S1944/IRS1.r3 | CCTCAGTGCCAGTCTCTTCC | 542 |
| IRS1 | NM_005544 | S5050/IRS1.p3 | TCCATCCCAGCTCCAGCCAG | 543 |
| ITGB1 | NM_002211 | S7497/ITGB1.f1 | TCAGAATTGGATTTGGCTCA | 544 |
| ITGB1 | NM_002211 | S7498/ITGB1.r1 | CCTGAGCTTAGCTGGTGTTG | 545 |
| ITGB1 | NM_002211 | S7499/ITGB1.p1 | | 546 |
| K-Alpha-1 | NM_006082 | S8706/K-Alph.f2 | | 547 |
| K-Alpha-1 | NM_006082 | S8707/K-Alph.r2 | CTGAAATTCTGGGAGCATGAC | 548 |
| K-Alpha-1 | NM_006082 | S8708/K-Alph.p2 | TATCCATTCCTTTTGGCCCTGCAG | 549 |
| KDR | NM_002253 | S1343/KDR.f6 | GAGGACGAAGGCCTCTACAC | 550 |
| KDR | NM_002253 | S1344/KDR.r6 | AAAAATGCCTCCACTTTTGC | 551 |
| KDR | NM_002253 | S4903/KDR.p6 | CAGGCATGCAGTGTTCTTGGCTGT | 552 |
| Ki-67 | NM_002417 | S0436/Ki-67.f2 | CGGACTTTGGGTGCGACTT | 553 |
| Ki-67 | NM_002417 | S0437/Ki-67.r2 | TTACAACTCTTCCACTGGGACGAT | 554 |
| Ki-67 | NM_002417 | S4741/Ki-67.p2 | CCACTTGTCGAACCACCGCTCGT | 555 |
| KIF11 | NM_004523 | T2409/KIF11.f2 | TGGAGGTTGTAAGCCAATGT | 556 |
| KIF11 | NM_004523 | T2410/KIF11.r2 | TGCCTTACGTCCATCTGATT | 557 |
| KIF11 | NM_004523 | T2411/KIF11.p2 | | 558 |
| KIF22 | NM_007317 | S8505/KIF22.f1 | CTAAGGCACTTGCTGGAAGG | 559 |
| KIF22 | NM_007317 | S8506/KIF22.r1 | TCTTCCCAGCTCCTGTGG | 560 |
| KIF22 | NM_007317 | S8507/KIF22.p1 | TCCATAGGCAAGCACACTGGCATT | 561 |
| KIF2C | NM_006845 | S7262/KIF2C.f1 | AATTCCTGCTCCAAAAGAAAGTCTT | 562 |
| KIF2C | NM_006845 | S7263/KIF2C.r1 | CGTGATGCGAAGCTCTGAGA | 563 |
| KIF2C | NM_006845 | S7264/KIF2C.p1 | AAGCCGCTCCACTCGCATGTCC | 564 |
| KIFC1 | NM_002263 | S8517/KIFC1.f1 | CCACAGGGTTGAAGAACCAG | 565 |
| KIFC1 | MM_002263 | S8519/KIFC1.r1 | CACCTGATGTGCCAGACTTC | 566 |
| KIFC1 | NM_002263 | S8520/KIFC1.p1 | AGCCAGTTCCTGCTGTTCCTGTCC | 567 |
| KLK10 | NM_002776 | S2624/KLK10.f3 | GCCCAGAGGCTCCATCGT | 568 |
| KLK10 | NM_002776 | S2625/KLK10.r3 | CAGAGGTTTGAACAGTGCAGACA | 569 |
| KLK10 | NM_002776 | S4978/KLK10.p3 | CCTCTTCCTCCCCAGTCGGCTGA | 570 |
| KNS2 | NM_005552 | T2030/KNS2.f1 | CAAACAGAGGGTGGCAGAAG | 571 |
| KNS2 | NM_005552 | T2031/KNS2.r1 | GAGGCTCTCACGGCTCCT | 572 |
| KNS2 | NM_005552 | T2032/KNS2.p1 | CGCTTCTCCATGTTCTCAGGGTCA | 573 |
| KNTC1 | NM_014708 | T2126/KNTC1.f1 | AGCCGAGGCTTTGTTGAA | 574 |
| KNTC1 | NM_014708 | T2127/KNTC1.r1 | TGGGCTATGAGCACAGCTT | 575 |
| KNTC1 | NM_014708 | T2128/KNTC1.p1 | TTCATATCCAGTACCGGCGATCGG | 576 |
| KNTC2 | NM_006101 | S7296/KNTC2.f1 | ATGTGCCAGTGAGCTTGAGT | 577 |
| KNTC2 | NM_006101 | S7297/KNTC2.r1 | TGAGCCCCTGGTTAACAGTA | 578 |
| KNTC2 | NM_006101 | S7298/KNTC2.p1 | CCTTGGAGAAACACAAGCACCTGC | 579 |
| KRT14 | NM_000526 | S1853/KRT14.f1 | GGCCTGCTGAGATCAAAGAC | 580 |
| KRT14 | NM_000526 | S1854/KRT14.r1 | GTCCACTGTGGCTGTGAGAA | 581 |
| KRT14 | NM_000526 | S5037/KRT14.p1 | | 582 |
| KRT17 | NM_000422 | S0172/KRT17.f2 | CGAGGATTGGTTCTTCAGCAA | 583 |
| KRT17 | NM_000422 | S0173/KRT17.p2 | CACCTCGCGGTTCAGTTCCTCTGT | 584 |
| KRT17 | NM_000422 | S0174/KRT17.r2 | ACTCTGCACCAGCTCACTGTTG | 585 |
| KRT19 | NM_002276 | S1515/KRT19.f3 | TGAGCGGCAGAATCAGGAGTA | 586 |
| KRT19 | NM_002276 | S1516/KRT19.r3 | TGCGGTAGGTGGCAATCTC | 587 |
| KRT19 | NM_002276 | S4866/KRT19.p3 | CTCATGGACATCAAGTCGCGGCTG | 588 |
| KRT5 | NM_000424 | S0175/KRT5.f3 | TCAGTGGAGAAGGAGTTGGA | 589 |
| KRT5 | NM_000424 | S0177/KRT5.r3 | TGCCATATCCAGAGGAAACA | 590 |
| KRT5 | NM_000424 | S5015/KRT5.p3 | | 591 |
| L1CAM | NM_000425 | T1341/L1CAM.f1 | CTTGCTGGCCAATGCCTA | 592 |
| L1CAM | NM_000425 | T1342/L1CAM.r1 | TGATTGTCCGCAGTCAGG | 593 |
| L1CAM | NM_000425 | T1343/L1CAM.p1 | ATCTACGTTGTCCAGCTGCCAGCC | 594 |
| LAMC2 | NM_005562 | S2826/LAMC2.f2 | ACTCAAGCGGAAATTGAAGCA | 595 |
| LAMC2 | NM_005562 | S2827/LAMC2.r2 | ACTCCCTGAAGCCGAGACACT | 596 |
| LAMC2 | NM_005562 | S4969/LAMC2.p2 | | 597 |
| LAPTM4B | NM_018407 | T2063/LAPTM4.f1 | AGCGATGAAGATGGTCGC | 598 |
| LAPTM4B | NM_018407 | T2064/LAPTM4.r1 | GACATGGCAGCACAAGCA | 599 |
| LAPTM4B | NM_018407 | T2065/LAPTM4.p1 | CTGGACGCGGTTCTACTCCAACAG | 600 |
| LIMK1 | NM_016735 | T0759/LIMKI.f1 | GCTTCAGGTGTTGTGACTGC | 601 |
| LIMK1 | NM_016735 | T0760/LIMK1.r1 | AAGAGCTGCCCATCCTTCTC | 602 |
| LIMK1 | NM_016735 | T0761/LIMK1.p1 | TGCCTCCCTGTCGCACCAGTACTA | 603 |
| LIMK2 | NM_005569 | T2033/LIMK2.f1 | CTTTGGGCCAGGAGGAAT | 604 |
| LIMK2 | NM_005569 | T2034/LIMK2.r1 | CTCCCACAATCCACTGCC | 605 |
| LIMK2 | NM_005569 | T2035/LIMK2.p1 | ACTCGAATCCACCCAGGAACTCCC | 606 |
| MAD1L1 | NM_003550 | S7299/MAD1L1.f1 | AGAAGCTGTCCCTGCAAGAG | 607 |
| MAD1L1 | NM_003550 | S7300/MAD1L1.r1 | AGCCGTACCAGCTCAGACTT | 608 |
| MAD1L1 | NM_003550 | S7301/MAD1L1.p1 | CATGTTCTTCACAATCGCTGCATCC | 609 |
| MAD2L1 | NM_002358 | S7302/MAD2L1.f1 | CCGGGAGCAGGGAATCAC | 610 |
| MAD2L1 | NM_002358 | S7303/MAD2L1.r1 | ATGCTGTTGATGCCGAATGA | 611 |
| MAD2L1 | NM_002358 | S7304/MAD2L1.p1 | CGGCCACGATTTCGGCGCT | 612 |
| MAD2L1 BP | NM_014628 | T2123/MAD2L1.f1 | CTGTCATGTGGCAGACCTTC | 613 |
| MAD2L1 BP | NM_014628 | T2124/MAD2L1.r1 | TAAATGTCACTGGTGCCTGG | 614 |
| MAD2L1BP | NM_014628 | T2125/MAD2L1.p1 | CGAACCACGGCTTGGGAAGACTAC | 615 |
| MAD2L2 | NM_006341 | T1125/MAD2L2.f1 | GCCCAGTGGAGAAATTCGT | 616 |
| MAD2L2 | NM_006341 | T1126/MAD2L2.r1 | GCGAGTCTGAGCTGATGGA | 617 |
| MAD2L2 | NM_006341 | T1127/MAD2L2.p1 | TTTGAGATCACCCAGCCTCCACTG | 618 |
| MAGE2 | NM_005361 | S5623/MAGE2.f1 | CCTCAGAAATTGCCAGGACT | 619 |
| MAGE2 | NM_005361 | S5625/MAGE2.p1 | TTCCCGTGATCTTCAGCAAAGCCT | 620 |
| MAGE2 | NM_005361 | S5626/MAGE2.r1 | CCAAAGACCAGCTGCAAGTA | 621 |
| MAGE6 | NM_005363 | S5639/MAGE6.f3 | AGGACTCCAGCAACCAAGAA | 622 |
| MAGE6 | NM_005363 | S5640/MAGE6.r3 | GAGTGCTGCTTGGAACTCAG | 623 |
| MAGE6 | NM_005363 | S5641/MAGE6.p3 | CAAGCACCTTCCCTGACCTGGAGT | 624 |
| MAP2 | NM_031846 | S8493/MAP2.f1 | CGGACCACCAGGTCAGAG | 625 |
| MAP2 | NM_031846 | S8494/MAP2.r1 | CAGGGGTAGTGGGTGTTGAG | 626 |
| MAP2 | NM_031846 | S8495/MAP2.p1 | CCACTCTTCCCTGCTCTGCGAATT | 627 |
| MAP2K3 | NM_002756 | T2090/MAP2K3.f1 | GCCCTCCAATGTCCTTATCA | 628 |
| MAP2K3 | NM_002756 | T2091/MAP2K3.r1 | GTAGCCACTGATGCCAAAGTC | 629 |
| MAP2K3 | NM_002756 | T2092/MAP2K3.p1 | CACATCTTCACATGGCCCTCCTTG | 630 |
| MAP4 | NM_002375 | S5724/MAP4.f1 | GCCGGTCAGGCACACAAG | 631 |
| MAP4 | NM_002375 | S5725/MAP4.r1 | GCAGCATACACACAACAAAATGG | 632 |
| MAP4 | NM_002375 | S5726/MAP4.p1 | ACCAACCAGTCCACGCTCCAAGGG | 633 |
| MAP6 | NM_033063 | T2341/MAP6.f2 | CCCTCAACCGGCAAATCC | 634 |
| MAP6 | NM_033063 | T2342/MAP6.r2 | CGTCCATGCCCTGAATTCA | 635 |
| MAP6 | NM_033063 | T2343/MAP6.p2 | TGGCGAGTGCAGTGAGCAGCTCC | 636 |
| MAPK14 | NM_139012 | S5557/MAPK14.f2 | TGAGTGGAAAAGCCTGACCTATG | 637 |
| MAPK14 | NM_139012 | S5558/MAPK14.r2 | GGACTCCATCTCTTCTTGGTCAA | 638 |
| MAPK14 | NM_139012 | S5559/MAPK14.p2 | | 639 |
| MAPK8 | NM_002750 | T2087/MAPK8.f1 | CAACACCCGTACATCAATGTCT | 640 |
| MAPK8 | NM_002750 | T2088/MAPK8.r1 | TCATCTAACTGCTTGTCAGGGA | 641 |
| MAPK8 | NM_002750 | T2089/MAPK8.p1 | CTGAAGCAGAAGCTCCACCACCAA | 642 |
| MAPRE1 | NM_012325 | T2180/MAPRE1.f1 | GACCTTGGAACCTTTGGAAC | 643 |
| MAPRE1 | NM_012325 | T2181/MAPRE1.r1 | CCTAGGCCTATGAGGGTTCA | 644 |
| MAPRE1 | NM_012325 | T2182/MAPRE1.p1 | | 645 |
| MAPT | NM_016835 | S8502/MAPT.f1 | CACAAGCTGACCTTCCGC | 646 |
| MAPT | NM_016835 | S8503/MAPT.r1 | ACTTGTACACGATCTCCGCC | 647 |
| MAPT | NM_016835 | S8504/MAPT.p1 | AGAACGCCAAAGCCAAGACAGACC | 648 |
| Maspin | NM_002639 | S0836/Maspin.f2 | CAGATGGCCACTTTGAGAACATT | 649 |
| Maspin | NM_002639 | S0837/Maspin.r2 | GGCAGCATTAACCACAAGGATT | 650 |
| Maspin | NM_002639 | S4835/Maspin.p2 | | 651 |
| MCL1 | NM_021960 | S5545/MCL1.f1 | CTTCGGAAACTGGACATCAA | 652 |
| MCL1 | NM_021960 | S5546/MCL1.r1 | GTCGCTGAAAACATGGATCA | 653 |
| MCL1 | NM_021960 | S5547/MCL1.p1 | | 654 |
| MCM2 | NM_004526 | S1602/MCM2.f2 | GACTTTTGCCCGCTACCTTTC | 655 |
| MCM2 | NM_004526 | S1603/MCM2.r2 | | 656 |
| MCM2 | NM_004526 | S4900/MCM2.p2 | ACAGCTCATTGTTGTCACGCCGGA | 657 |
| MCM6 | NM_005915 | S1704/MCM6.f3 | TGATGGTCCTATGTGTCACATTCA | 658 |
| MCM6 | NM_005915 | S1705/MCM6.r3 | TGGGACAGGAAACACACCAA | 659 |
| MCM6 | NM_005915 | S4919/MCM6.p3 | | 660 |
| MCP1 | NM_002982 | S1955/MCP1.f1 | CGCTCAGCCAGATGCAATC | 661 |
| MCP1 | NM_002982 | S1956/MCP1.r1 | | 662 |
| MCP1 | NM_002982 | S5052/MCP1.p1 | TGCCCCAGTCACCTGCTGTTA | 663 |
| MGMT | NM_002412 | S1922/MGMT.f1 | GTGAAATGAAACGCACCACA | 664 |
| MGMT | NM_002412 | S1923/MGMT.r1 | GACCCTGCTCACAACCAGAC | 665 |
| MGMT | NM_002412 | S5045/MGMT.p1 | CAGCCCTTTGGGGAAGCTGG | 666 |
| MMP12 | NM_002426 | S4381/MMP12.f2 | CCAACGCTTGCCAAATCCT | 667 |
| MMP12 | NM_002426 | S4382/MMP12.r2 | ACGGTAGTGACAGCATCAAAACTC | 668 |
| MMP12 | NM_002426 | S4383/MMP12.p2 | AACCAGCTCTCTGTGACCCCAATT | 669 |
| MMP2 | NM_004530 | S1874/MMP2.f2 | CCATGATGGAGAGGCAGACA | 670 |
| MMP2 | NM_004530 | S1875/MMP2.r2 | GGAGTCCGTCCTTACCGTCAA | 671 |
| MMP2 | NM_004530 | S5039/MMP2.p2 | CTGGGAGCATGGCGATGGATACCC | 672 |
| MMP9 | NM_004994 | S0656/MMP9.f1 | GAGAACCAATCTCACCGACA | 673 |
| MMP9 | NM_004994 | S0657/MMP9.r1 | CACCCGAGTGTAACCATAGC | 674 |
| MMP9 | NM_004994 | S4760/MMP9.p1 | ACAGGTATTCCTCTGCCAGCTGCC | 675 |
| MRE11A | NM_005590 | T2039/MRE11A.f1 | GCCATGCTGGCTCAGTCT | 676 |
| MRE11A | NM_005590 | T2040/MRE11A.r1 | CACCCAGACCCACCTAACTG | 677 |
| MRE11A | NM_005590 | T2041/MRE11A.p1 | CACTAGCTGATGTGGCCCACAGCT | 678 |
| MRP1 | NM_004996 | S0181/MRP1.f1 | TCATGGTGCCCGTCAATG | 679 |
| MRP1 | NM_004996 | S0183/MRP1.r1 | CGATTGTCTTTGCTCTTCATGTG | 680 |
| MRP1 | NM_004996 | S5019/MRP1.p1 | | 681 |
| MRP2 | NM_000392 | S0184/MRP2.f3 | AGGGGATGACTTGGACACAT | 682 |
| MRP2 | NM_000392 | S0186/MRP2.r3 | AAAACTGCATGGCTTTGTCA | 683 |
| MRP2 | NM_000392 | S5021/MRP2.p3 | CTGCCATTCGACATGACTGCAATTT | 684 |
| MRP3 | NM_003786 | S0187/MRP3.f1 | TCATCCTGGCGATCTACTTCCT | 685 |
| MRP3 | NM_003786 | S0189/MRP3.r1 | CCGTTGAGTGGAATCAGCAA | 686 |
| MRP3 | NM_003786 | S5023/MRP3.p1 | | 687 |
| MSH3 | NM_002439 | S5940/MSH3.f2 | TGATTACCATCATGGCTCAGA | 688 |
| MSH3 | NM_002439 | S5941/MSH3.r2 | CTTGTGAAAATGCCATCCAC | 689 |
| MSH3 | NM_002439 | S5942/MSH3.p2 | TCCCAATTGTCGCTTCTTCTGCAG | 690 |
| MUC1 | NM_002456 | S0782/MUC1.f2 | GGCCAGGATCTGTGGTGGTA | 691 |
| MUC1 | NM_002456 | S0783/MUC1.r2 | CTCCACGTCGTGGACATTGA | 692 |
| MUC1 | NM_002456 | S4807/MUC1.p2 | CTCTGGCCTTCCGAGAAGGTACC | 693 |
| MX1 | NM_002462 | S7611/MX1.f1 | GAAGGAATGGGAATCAGTCATGA | 694 |
| MX1 | NM_002462 | S7612/MX1.r1 | | 595 |
| MX1 | NM_002462 | S7613/MX1.p1 | TCACCCTGGAGATCAGCTCCCGA | 696 |
| MYBL2 | NM_002466 | S3270/MYBL2.f1 | GCCGAGATCGCCAAGATG | 697 |
| MYBL2 | NM_002466 | S3271/MYBL2.r1 | | 698 |
| MYBL2 | NM_002466 | S4742/MYBL2.p1 | CAGCATTGTCTGTCCTCCCTGGCA | 699 |
| MYH11 | NM_002474 | S4555/MYH11.f1 | | 700 |
| MYH11 | NM_002474 | S4556/MYH11.r1 | CCGAGTAGATGGGCAGGTGTT | 701 |
| MYH11 | NM_002474 | S4557/MYH11.p1 | | 702 |
| NEK2 | NM_002497 | S4327/NEK2.f1 | GTGAGGCAGCGCGACTCT | 703 |
| NEK2 | NM_002497 | S4328/NEK2.r1 | TGCCAATGGTGTACAACACTTCA | 704 |
| NEK2 | NM_002497 | S4329/NEK2.p1 | TGCCTTCCCGGGCTGAGGACT | 705 |
| NFKBp50 | NM_003998 | S9661/NFKBp5.f3 | CAGACCAAGGAGATGGACCT | 706 |
| NFKBp50 | NM_003998 | S9662/NFKBp5.r3 | AGCTGCCAGTGCTATCCG | 707 |
| NFKBp50 | NM_003998 | S9663/NFKBp5.p3 | AAGCTGTAAACATGAGCCGCACCA | 708 |
| NFKBp65 | NM_021975 | S0196/NFKBp6.f3 | CTGCCGGGATGGCTTCTAT | 709 |
| NFKBp65 | NM_021975 | S0198/NFKBp6.r3 | CCAGGTTCTGGAAACTGTGGAT | 710 |
| NFKBp65 | NM_021975 | S5030/NFKBp6.p3 | CTGAGCTCTGCCCGGACCGCT | 711 |
| NME6 | NM_005793 | T2129/NME6.f1 | CACTGACACCCGCAACAC | 712 |
| NME6 | NM_005793 | T2130/NME6.r1 | GGCTGCAATCTCTCTGCTG | 713 |
| NME6 | NM_005793 | T2131/NME6.p1 | AACCACAGAGTCCGAACCATGGGT | 714 |
| NPC2 | NM_006432 | T2141/NPC2.f1 | CTGCTTCTTTCCCGAGCTT | 715 |
| NPC2 | NM_006432 | T2142/NPC2.r1 | AGCAGGAATGTAGCTGCCA | 716 |
| NPC2 | NM_006432 | T2143/NPC2.p1 | ACTTCGTTATCCGCGATGCGTTTC | 717 |
| NPD009 (ABAT official) | NM_020686 | S4474/NPD009.f3 | GGCTGTGGCTGAGGCTGTAG | 718 |
| NPD009 (ABAT official) | NM_020686 | S4475/NPD009.r3 | GGAGCATTCGAGGTCAAATCA | 719 |
| NPD009 (ABAT official) | NM_020686 | S4476/NPD009.p3 | | 720 |
| NTSR2 | NM_012344 | T2332/NTSR2.f2 | CGGACCTGAATGTAATGCAA | 721 |
| NTSR2 | NM_012344 | T2333/NTSR2.r2 | CTTTGCCAGGTGACTAAGCA | 722 |
| NTSR2 | NM_012344 | T2334/NTSR2.p2 | | 723 |
| NUSAP1 | NM_016359 | S7106/NUSAP1.f1 | CAAAGGAAGAGCAACGGAAG | 724 |
| NUSAP1 | NM_016359 | S7107/NUSAP1.r1 | ATTCCCAAAACCTTTGCTT | 725 |
| NUSAP1 | NM_016359 | S7108/NUSAP1.p1 | TTCTCCTTTCGTTCTTGCTCGCGT | 726 |
| p21 | NM_000389 | S0202/p21.f3 | TGGAGACTCTCAGGGTCGAAA | 727 |
| p21 | NM_000389 | S0204/p21.r3 | GGCGTTTGGAGTGGTAGAAATC | 728 |
| p21 | NM_000389 | S5047/p21.p3 | CGGCGGCAGACCAGCATGAC | 729 |
| p27 | NM_004064 | S0205/p27.f3 | CGGTGGACCACGAAGAGTTAA | 730 |
| p27 | NM_004064 | S0207/p27.r3 | GGCTCGCCTCTTCCATGTC | 731 |
| p27 | NM_004064 | S4750/p27.p3 | CCGGGACTTGGAGAAGCACTGCA | 732 |
| PCTK1 | NM_006201 | T2075/PCTK1.f1 | TCACTACCAGCTGACATCCG | 733 |
| PCTK1 | NM_006201 | T2076/PCTK1.r1 | AGATGGGGCTATTGAGGGTC | 734 |
| PCTK1 | NM_006201 | T2077/PCTK1.p1 | CTTCTCCAGGTAGCCCTCAGGCAG | 735 |
| PDGFRb | NM_002609 | S1346/PDGFRb.f3 | CCAGCTCTCCTTCCAGCTAC | 736 |
| PDGFRb | NM_002609 | S1347/PDGFRb.r3 | GGGTGGCTCTCACTTAGCTC | 737 |
| PDGFRb | NM_002609 | S4931/PDGFRb.p3 | ATCAATGTCCCTGTCCGAGTGCTG | 738 |
| PFDN5 | NM_145897 | T2078/PFDN5.f1 | GAGAAGCACGCCATGAAAC | 739 |
| PFDN5 | NM_145897 | T2079/PFDN5.r1 | GGCTGTGAGCTGCTGAATCT | 740 |
| PFDN5 | NM_145897 | T2080/PFDN5.p1 | TGACTCATCATTTCCATGACGGCC | 741 |
| PGK1 | NM_000291 | S0232/PGK1.f1 | AGAGCCAGTTGCTGTAGAACTCAA | 742 |
| PGK1 | NM_000291 | S0234/PGK1.r1 | CTGGGCCTACACAGTCCTTCA | 743 |
| PGK1 | NM_000291 | S5022/PGK1.p1 | | 744 |
| PHB | NM_002634 | T2171/PHB.f1 | GACATTGTGGTAGGGGAAGG | 745 |
| PHB | NM_002634 | T2172/PHB.r1 | CGGCAGTCAAAGATAATTGG | 746 |
| PHB | NM_002634 | T2173/PHB.p1 | | 747 |
| PI3KC2A | NM_002645 | S2020/PI3KC2.r1 | CACACTAGCATTTTCTCCGCATA | 748 |
| PI3KC2A | NM_002645 | S2021/PI3KC2.f1 | ATACCAATCACCGCACAAACC | 749 |
| PI3KC2A | NM_002645 | S5062/PI3KC2.p1 | | 750 |
| PIM1 | NM_002648 | S7858/PIM1.f3 | CTGCTCAAGGACACCGTCTA | 751 |
| PIM1 | NM_002648 | S7859/PIM1.r3 | GGATCCACTCTGGAGGGC | 752 |
| PIM1 | NM_002648 | S7860/PIM1.p3 | TACACTCGGGTCCCATCGAAGTCC | 753 |
| PIM2 | NM_006875 | T2144/PIM2.f1 | TGGGGACATTCCCTTTGAG | 754 |
| PIM2 | NM_006875 | T2145/PIM2.r1 | GACATGGGCTGGGAAGTG | 755 |
| PIM2 | NM_006875 | T2146/PIM2.p1 | CAGCTTCCAGAATCTCCTGGTCCC | 756 |
| PLAUR | NM_002659 | S1976/PLAUR.f3 | CCCATGGATGCTCCTCTGAA | 757 |
| PLAUR | NM_002659 | S1977/PLAUR.r3 | CCGGTGGCTACCAGACATTG | 758 |
| PLAUR | NM_002659 | S5054/PLAUR.p3 | CATTGACTGCCGAGGCCCCATG | 759 |
| PLD3 | NM_012268 | S8645/PLD3.f1 | CCAAGTTCTGGGTGGTGG | 760 |
| PLD3 | NM_012268 | S8646/PLD3.r1 | GTGAACGCCAGTCCATGTT | 761 |
| PLD3 | NM_012268 | S8647/PLD3.p1 | CCAGACCCACTTCTACCTGGGCAG | 762 |
| PLK | NM_005030 | S3099/PLK.f3 | | 763 |
| PLK | NM_005030 | S3100/PLK.r3 | TGTCTGAAGCATCTTCTGGATGA | 764 |
| PLK | NM_005030 | S4825/PLK.p3 | AACCCCGTGGCCGCCTCC | 765 |
| PMS1 | NM_000534 | S5894/PMS1.f2 | CTTACGGTTTTCGTGGAGAAG | 766 |
| PMS1 | NM_000534 | S5895/PMS1.r2 | AGCAGCCGTTCTTGTTGTAA | 767 |
| PMS1 | NM_000534 | S5896/PMS1.p2 | | 768 |
| PMS2 | NM_000535 | S5878/PMS2.f3 | GATGTGGACTGCCATTCAAA | 769 |
| PMS2 | NM_000535 | S5879/PMS2.r3 | TGCGAGATTAGTTGGCTGAG | 770 |
| PMS2 | NM_000535 | S5880/PMS2.p3 | | 771 |
| PP591 | NM_025207 | S8657/PP591.f1 | CCACATACCGTCCAGCCTA | 772 |
| PP591 | NM_025207 | S8658/PP591.r1 | GAGGTCATGTGCGGGAGT | 773 |
| PP591 | NM_025207 | S8659/PP591.p1 | CCGCTCCTCTTCTTCGTTCTCCAG | 774 |
| PPP2CA | NM_002715 | T0732/PPP2CA.f1 | GCAATCATGGAACTTGACGA | 775 |
| PPP2CA | NM_002715 | T0733/PPP2CA.r1 | ATGTGGCTCGCCTCTACG | 776 |
| PPP2CA | NM_002715 | T0734/PPP2CA.p1 | TTTCTTGCAGTTTGACCCAGCACC | 777 |
| PR | NM_000926 | S1336/PR.f6 | GCATCAGGCTGTCATTATGG | 778 |
| PR | NM_000926 | S1337/PR.r6 | AGTAGTTGTGCTGCCCTTCC | 779 |
| PR | NM_000926 | S4743/PR.p6 | | 780 |
| PRDX1 | NM_002574 | T1241/PRDX1.f1 | AGGACTGGGACCCATGAAC | 781 |
| PRDX1 | NM_002574 | T1242/PRDX1.r1 | CCCATAATCCTGAGCAATGG | 782 |
| PRDX1 | NM_002574 | T1243/PRDX1.p1 | TCCTTTGGTATCAGACCCGAAGCG | 783 |
| PRDX2 | NM_005809 | S8761/PRDX2.f1 | GGTGTCCTTCGCCAGATCAC | 784 |
| PRDX2 | NM_005809 | S8762/PRDX2.r1 | CAGCCGCAGAGCCTCATC | 785 |
| PRDX2 | NM_005809 | S8763/PRDX2.p1 | | 786 |
| PRKCA | NM_002737 | S7369/PRKCA.f1 | CAAGCAATGCGTCATCAATGT | 787 |
| PRKCA | NM_002737 | S7370/PRKCA.r1 | GTAAATCCGCCCCCTCTTCT | 788 |
| PRKCA | NM_002737 | S7371/PRKCA.p1 | | 789 |
| PRKCD | NM_006254 | S1738/PRKCD.f2 | CTGACACTTGCCGCAGAGAA | 790 |
| PRKCD | NM_006254 | S1739/PRKCD.r2 | AGGTGGTCCTTGGTCTGGAA | 791 |
| PRKCD | NM_006254 | S4923/PRKCD.p2 | | 792 |
| PRKCG | NM_002739 | T2081/PRKCG.f1 | GGGTTCTAGACGCCCCTC | 793 |
| PRKCG | NM_002739 | T2082/PRKCG.r1 | GGACGGCTGTAGAGGCTGTAT | 794 |
| PRKCG | NM_002739 | T2083/PRKCG.p1 | CAAGCGTTCCTGGCCTTCTGAACT | 795 |
| PRKCH | NM_006255 | T2084/PRKCH.f1 | CTCCACCTATGAGCGTCTGTC | 796 |
| PRKCH | NM_006255 | T2085/PRKCH.r1 | CACACTTTCCCTCCTTTTGG | 797 |
| PRKCH | NM_006255 | T2086/PRKCH.p1 | TCCTGTTAACATCCCAAGCCCACA | 798 |
| pS2 | NM_003225 | S0241/pS2.f2 | GCCCTCCCAGTGTGCAAAT | 799 |
| pS2 | NM_003225 | S0243/pS2.r2 | | 800 |
| pS2 | NM_003225 | S5026/pS2.p2 | TGCTGTTTCGACGACACCGTTCG | 801 |
| PTEN | NM_000314 | S0244/PTEN.f2 | TGGCTAAGTGAAGATGACAATCAT G | 802 |
| PTEN | NM_000314 | S0246/PTEN.r2 | TGCACATATCATTACACCAGTTCGTI | 803 |
| PTEN | NM_000314 | S5027/PTEN.p2 | | 804 |
| PTPD1 | NM_007039 | S3069/PTPD1.f2. | CGCTTGCCTAACTCATACTTTCC | 805 |
| PTPD1 | NM_007039 | S3070/PTPD1.r2 | CCATTCAGACTGCGCCACTT | 806 |
| PTPD1 | NM_007039 | S4822/PTPD1.p2 | TCCACGCAGCGTGGCACTG | 807 |
| PTTG1 | NM_004219 | S4525/PTTG1.f2 | | 808 |
| PTTG1 | NM_004219 | S4526/PTTG1.r2 | GCTTCAGCCCATCCTTAGCA | 809 |
| PTTG1 | NM_004219 | S4527/PTTG1.p2 | CACACGGGTGCCTGGTTCTCCA | 810 |
| RAB27B | NM_004163 | S4336/RAB27B.f1 | GGGACACTGCGGGACAAG | 811 |
| RAB27B | NM_004163 | S4337/RAB27B.r1 | GCCCATGGCGTCTCTGAA | 812 |
| RAB27B | NM_004163 | S4338/RAB27B.p1 | | 313 |
| RAB31 | NM_006868 | S9306/RAB31.f1 | CTGAAGGACCCTACGCTCG | 814 |
| RAB31 | NM_006868 | S9307/RAB31.r1 | ATGCAAAGCCAGTGTGCTC | 815 |
| RAB31 | NM_006868 | S9308/RAB31.p1 | CTTCTCAAAGTGAGGTGCCAGGCC | 816 |
| RAB6C | NM_032144 | S5535/RAB6C.f1 | GCGACAGCTCCTCTAGTTCCA | 817 |
| RAB6C | NM_032144 | S5537/RAB6C.p1 | TTCCCGAAGTCTCCGCCCG | 818 |
| RAB6C | NM_032144 | S5538/RAB6C.r1 | GGAACACCAGCTTGAATTTCCT | 819 |
| RAD1 | NM_002853 | T2174/RAD1.f1 | GAGGAGTGGTGACAGTCTGC | 820 |
| RAD1 | NM_002853 | T2175/RAD1.r1 | GCTGCAGAAATCAAAGTCCA | 821 |
| RAD1 | NM_002853 | T2176/RAD1.p1 | | 822 |
| RAD54L | NM_003579 | S4369/RAD54L.f1 | AGCTAGCCTCAGTGACACACATG | 823 |
| RAD54L | NM_003579 | S4370/RAD54L.r1 | CCGGATCTGACGGCTGTT | 824 |
| RAD54L | NM_003579 | S4371/RAD54L.p1 | ACACAACGTCGGCAGTGCAACCTG | 825 |
| RAF1 | NM_002880 | S5933/RAF1.f3 | CGTCGTATGCGAGAGTCTGT | 826 |
| RAF1 | NM_002880 | S5934/RAF1.r3 | TGAAGGCGTGAGGTGTAGAA | 827 |
| RAF1 | NM_002880 | S5935/RAF1.p3 | | 828 |
| RALBP1 | NM_006788 | S5853/RALBP1.f1 | | 829 |
| RALBP1 | NM_006788 | S5854/RALBP1.r1 | TTCGATATTGCCAGCAGCTATAAA | 830 |
| RALBP1 | NM_006788 | S5855/RALBP1.p1 | | 831 |
| RAP1GDS1 | NM_021159 | S5306/RAP1GD.f2 | TGTGGATGCTGGATTGATTT | 832 |
| RAP1GDS1 | NM_021159 | S5307/RAP1GD.r2 | AAGCAGCACTTCCTGGTCTT | 833 |
| RAP1GDS1 | NM_021159 | S5308/RAP1GD.p2 | | 834 |
| RASSF1 | NM_007182 | S2393/RASSF1.f3 | AGTGGGAGACACCTGACCTT | 835 |
| RASSF1 | NM_007182 | S2394/RASSF1.r3 | TGATCTGGGCATTGTACTCC | 836 |
| RASSF1 | NM_007182 | S4909/RASSF1.p3 | | 837 |
| RB1 | NM_000321 | S2700/RB1.f1 | CGAAGCCCTTACAAGTTTCC | 838 |
| RB1 | NM_000321 | S2701/RB1.r1 | GGACTCTTCAGGGGTGAAAT | 839 |
| RB1 | NM_000321 | S4765/RB1.p1 | CCCTTACGGATTCCTGGAGGGAAC | 840 |
| RBM17 | NM_032905 | T2186/RBM17.f1 | CCCAGTGTACGAGGAACAAG | 841 |
| RBM17 | NM_032905 | T2187/RBM17.r1 | TTAGCGAGGAAGGAGTTGCT | 842 |
| RBM17 | NM_032905 | T2188/RBM17.p1 | ACAGACCGAGATCTCCAACCGGAC | 843 |
| RCC1 | NM_001269 | S8854/RCC1.f1 | GGGCTGGGTGAGAATGTG | 844 |
| RCC1 | NM_001269 | S8855/RCC1.r1 | CACAACATCCTCCGGAATG | 845 |
| RCC1 | NM_001269 | S8856/RCC1.p1 | ATACCAGGGCCGGCTTCTTCCTCT | 846 |
| REG1A | NM_002909 | T2093/REG1A.f1 | CCTACAAGTCCTGGGGCA | 847 |
| REG1A | NM_002909 | T2094/REG1A.r1 | TGAGGTCAGGCTCACACAGT | 848 |
| REG1A | NM_002909 | T2095/REG1A.p1 | TGGAGCCCCAAGCAGTGTTAATCC | 849 |
| RELB | NM_006509 | T2096/RELB.f1 | GCGAGGAGCTCTACTTGCTC | 850 |
| RELB | NM_006509 | T2097/RELB.r1 | GCCCTGCTGAACACCACT | 851 |
| RELB | NM_006509 | T2098/RELB.p1 | TGTCCTCTTTCTGCACCTTGTCGC | 852 |
| RhoB | NM_004040 | S8284/RhoB.f1 | AAGCATGAACAGGACTTGACC | 853 |
| RhoB | NM_004040 | S8285/RhoB.r1 | CCTCCCCAAGTCAGTTGC | 854 |
| RhoB | NM_004040 | S8286/RhoB.p1 | CTTTCCAACCCCTGGGGAAGACAT | 855 |
| rhoC | NM_175744 | S2162/rhoC.f1 | CCCGTTCGGTCTGAGGAA | 856 |
| rhoC | NM_175744 | S2163/rhoC.r1 | GAGCACTCAAGGTAGCCAAAGG | 857 |
| rhoC | NM_175744 | S5042/rhoC.p1 | TCCGGTTCGCCATGTCCCG | 858 |
| RIZ1 | NM_012231 | S1320/RIZ1.f2 | CCAGACGAGCGATTAGAAGC | 859 |
| RIZ1 | NM_012231 | S1321/RIZ1.r2 | TCCTCCTCTTCCTCCTCCTC | 860 |
| RIZ1 | NM_012231 | S4761/RIZ1.p2 | TGTGAGGTGAATGATTTGGGGGA | 861 |
| ROCK1 | NM_005406 | S8305/ROCK1.f1 | TGTGCACATAGGAATGAGCTTC | 862 |
| ROCK1 | NM_005406 | S8306/ROCK1.r1 | GTTTAGCACGCAATTGCTCA | 863 |
| ROCK1 | NM_005406 | S8307/ROCK1.p1 | TCACTCTCTTTGCTGGCCAACTGC | 864 |
| RPL37A | NM_000998 | T2418/RPL37A.f2 | GATCTGGCACTGTGGTTCC | 865 |
| RPL37A | NM_000998 | T2419/RPL37A.r2 | TGACAGCGGAAGTGGTATTG | 866 |
| RPL37A | NM_000998 | T2420/RPL37A.p2 | CACCGCCAGCCACTGTCTTCAT | 867 |
| RPLPO | NM_001002 | S0256/RPLPO.f2 | CCATTCTATCATCAACGGGTACAA | 868 |
| RPLPO | NM_001002 | S0258/RPLPO.r2 | TCAGCAAGTGGGAAGGTGTAATC | 869 |
| RPLPO | NM_001002 | S4744/RPLPO.p2 | | 870 |
| RPN2 | NM_002951 | T1158/RPN2.f1 | CTGTCTTCCTGTTGGCCCT | 871 |
| RPN2 | NM_002951 | T1159/RPN2.r1 | GTGAGGTAGTGAGTGGGCGT | 872 |
| RPN2 | NM_002951 | T1160/RPN2.p1 | ACAATCATAGCCAGCACCTGGGCT | 873 |
| RPS6KB1 | NM_003161 | S2615/RPS6KB.f3 | GCTCATTATGAAAAACATCCCAAAC | 874 |
| RPS6KB1 | NM_003161 | S2616/RPS6KB.r3 | | 875 |
| RPS6KB1 | NM_003161 | S4759/RPS6KB.p3 | CACACCAACCAATAATTTCGCATT | 876 |
| RXRA | NM_002957 | S8463/RXRA.f1 | GCTCTGTTGTGTCCTGTTGC | 877 |
| RXRA | NM_002957 | S8464/RXRA.r1 | GTACGGAGAAGCCACTTCACA | 878 |
| RXRA | NM_002957 | S8465/RXRA.p1 | TCAGTCACAGGAAGGCCAGAGCC | 879 |
| RXRB | NM_021976 | S8490/RXRB.f1 | CGAGGAGATGCCTGTGGA | 880 |
| RXRB | NM_021976 | S8491/RXRB.r1 | CAACGCCCTGGTCACTCT | 881 |
| RXRB | NM_021976 | S8492/RXRB.p1 | CTGTTCCACAGCAAGCTCTGCCTC | 882 |
| S100A10 | NM_002966 | S9950/S100A1.f1 | ACACCAAAATGCCATCTCAA | 883 |
| S100A10 | NM_002966 | S9951/S100A1.r1 | TTTATCCCCAGCGAATTTGT | 884 |
| S100A10 | NM_002966 | S9952/S100A1.p1 | CACGCCATGGAAACCATGATGTTT | 885 |
| SEC61A | NM_013336 | S8648/SEC61A.f1 | CTTCTGAGCCCGTCTCCC | 886 |
| SEC61A | NM_013336 | S8649/SEC61A.r1 | GAGAGCTCCCCTTCCGAG | 887 |
| SEC61A | NM_013336 | S8650/SEC61A.p1 | CGCTTCTGGAGCAGCTTCCTCAAC | 888 |
| SEMA3F | NM_004186 | S2857/SEMA3F.f3 | CGCGAGCCCCTCATTATACA | 889 |
| SEMA3F | NM_004186 | S2858/SEMA3F.r3 | CACTCGCCGTTGACATCCT | 890 |
| SEMA3F | NM_004186 | S4972/SEMA3F.p3 | CTCCCCACAGCGCATCGAGGAA | 891 |
| SFN | NM_006142 | S9953/SFN.f1 | GAGAGAGCCAGTCTGATCCA | 892 |
| SFN | NM_006142 | S9954/SFN.r1 | AGGCTGCCATGTCCTCATA | 893 |
| SFN | NM_006142 | S9955/SFN.p1 | CTGCTCTGCCAGCTTGGCCTTC | 894 |
| SGCB | NM_000232 | S5752/SGCB.f1 | CAGTGGAGACCAGTTGGGTAGTG | 895 |
| SGCB | NM_000232 | S5753/SGCB.r1 | CCTTGAAGAGCGTCCCATCA | 896 |
| SGCB | NM_000232 | S5754/SGCB.p1 | | 897 |
| SGK | NM_005627 | S8308/SGK.f1 | TCCGCAAGACACCTCCTG | 898 |
| SGK | NM_005627 | S8309/SGK.r1 | TGAAGTCATCCTTGGCCC | 899 |
| SGK | NM_005627 | S8310/SGK.p1 | TGTCCTGTCCTTCTGCAGGAGGC | 900 |
| SGKL | NM_170709 | T2183/SGKL.f1 | TGCATTCGTTGGTTTCTCTT | 901 |
| SGKL | NM_170709 | T2184/SGKL.r1 | TTTCTGAATGGCAAACTGCT | 902 |
| SGKL | NM_170709 | T2185/SGKL.p1 | | 903 |
| SHC1 | NM_003029 | S6456/SHC1.f1 | CCAACACCTTCTTGGCTTCT | 904 |
| SHC1 | NM_003029 | S6457/SHC1.r1 | CTGTTATCCCAACCCAAACC | 905 |
| SHC1 | NM_003029 | S6458/SHC1.p1 | CCTGTGTTCTTGCTGAGCACCCTC | 906 |
| SIR2 | NM_012238 | S1575/SIR2.f2 | AGCTGGGGTGTCTGTTTCAT | 907 |
| SIR2 | NM_012238 | S1576/SIR2.r2 | ACAGCAAGGCGAGCATAAAT | 908 |
| SIR2 | NM_012238 | S4885/SIR2.p2 | CCTGACTTCAGGTCAAGGGATGG | 909 |
| SLC1A3 | NM_004172 | S8469/SLC1A3.f1 | GTGGGGAGCCCATCATCT | 910 |
| SLC1A3 | NM_004172 | S8470/SLC1A3.r1 | CCAGTCCACACTGAGTGCAT | 911 |
| SLC1A3 | NM_004172 | S8471/SLC1A3.p1 | CCAAGCCATCACAGGCTCTGCATA | 912 |
| SLC25A3 | NM_213611 | T0278/SLC25A.f2 | TCTGCCAGTGCTGAATTCTT | 913 |
| SLC25A3 | NM_213611 | T0279/SLC25A.r2 | TTCGAACCTTAGCAGCTTCC | 914 |
| SLC25A3 | NM_213611 | T0280/SLC25A.p2 | TGCTGACATTGCCCTGGCTCCTAT | 915 |
| SLC35B1 | NM_005827 | S8642/SLC35B.f1 | CCCAACTCAGGTCCTTGGTA | 916 |
| SLC35B1 | NM_005827 | S8643/SLC35B.r1 | CAAGAGGGTCACCCCAAG | 917 |
| SLC35B1 | NM_005827 | S8644/SLC35B.p1 | ATCCTGCAAGCCAATCCCAGTCAT | 918 |
| SLC7A11 | NM_014331 | T2045/SLC7A1.f1 | AGATGCATACTTGGAAGCACAG | 919 |
| SLC7A11 | NM_014331 | T2046/SLC7A1.r1 | AACCTAGGACCAGGTAACCACA | 920 |
| SLC7A11 | NM_014331 | T2047/SLC7A1.p1 | CATATCACACTGGGAGGCAATGCA | 921 |
| SLC7A5 | NM_003486 | S9244/SLC7A5.f2 | GCGCAGAGGCCAGTTAAA | 922 |
| SLC7A5 | NM_003486 | S9245/SLC7A5.r2 | AGCTGAGCTGTGGGTTGC | 923 |
| SLC7A5 | NM_003486 | S9246/SLC7A5.p2 | AGATCACCTCCTCGAACCCACTCC | 924 |
| SNAI2 | NM_003068 | S7824/SNAI2.f1 | GGCTGGCCAAACATAAGCA | 925 |
| SNAI2 | NM_003068 | S7825/SNAI2.r1 | | 926 |
| SNAI2 | NM_003068 | S7826/SNAI2.p1 | | 927 |
| SNCA | NM_007308 | T2320/SNCA.f1 | AGTGACAAATGTTGGAGGAGC | 928 |
| SNCA | NM_007308 | T2321/SNCA.r1 | CCCTCCACTGTCTTCTGGG | 929 |
| SNCA | NM_007308 | T2322/SNCA.p1 | TACTGCTGTCACACCCGTCACCAC | 930 |
| SNCG | NM_003087 | T1704/SNCG.f1 | ACCCACCATGGATGTCTTC | 931 |
| SNCG | NM_003087 | T1705/SNCG.r1 | CCTGCTTGGTCTTTTCCAC | 932 |
| SNCG | NM_003087 | T1706/SNCG.p1 | AAGAAGGGCTTCTCCATCGCCAAG | 933 |
| SOD1 | NM_000454 | S7683/SOD1.f1 | TGAAGAGAGGCATGTTGGAG | 934 |
| SOD1 | NM_000454 | S7684/SOD1.r1 | AATAGACACATCGGCCACAC | 935 |
| SOD1 | NM_000454 | S7685/SOD1.p1 | TTTGTCAGCAGTCACATTGCCCAA | 936 |
| SRI | NM_003130 | T2177/SRI.f1 | ATACAGCACCAATGGAAAGATCAC | 937 |
| SRI | NM_003130 | T2178/SRI.r1 | TGTCTGTAAGAGCCCTCAGTTTGA | 938 |
| SRI | NM_003130 | T2179/SRI.p1 | TTCGACGACTACATCGCCTGCTGC | 939 |
| STAT1 | NM_007315 | S1542/STAT1.f3 | GGGCTCAGCTTTCAGAAGTG | 940 |
| STAT1 | NM_007315 | S1543/STAT1.r3 | ACATGTTCAGCTGGTCCACA | 941 |
| STAT1 | NM_007315 | S4878/STAT1.p3 | TGGCAGTTTTCTTCTGTCACCAAAA | 942 |
| STAT3 | NM_003150 | S1545/STAT3.f1 | TCACATGCCACTTTGGTGTT | 943 |
| STAT3 | NM_003150 | S1546/STAT3.r1 | CTTGCAGGAAGCGGCTATAC | 944 |
| STAT3 | NM_003150 | S4881/STAT3.p1 | TCCTGGGAGAGATTGACCAGCA | 945 |
| STK10 | NM_005990 | T2099/STK10.f1 | CAAGAGGGACTCGGACTGC | 946 |
| STK10 | NM_005990 | T2100/STK10.r1 | CAGGTCAGTGGAGAGATTGGT | 947 |
| STK10 | NM_005990 | T2101/STK10.p1 | CCTCTGCACCTCTGAGAGCATGGA | 948 |
| STK11 | NM_000455 | S9454/STK11.f1 | GGACTCGGAGACGCTGTG | 949 |
| STK11 | NM_000455 | S9455/STK11.r1 | GGGATCCTTCGCAACTTCTT | 950 |
| STK11 | NM_000455 | S9456/STK11.p1 | TTCTTGAGGATCTTGACGGCCCTC | 951 |
| STK15 | NM_003600 | S0794/STK15.f2 | CATCTTCCAGGAGGACCACT | 952 |
| STK15 | NM_003600 | S0795/STK15.r2 | TCCGACCTTCAATCATTTCA | 953 |
| STK15 | NM_003600 | S4745/STK15.p2 | CTCTGTGGCACCCTGGACTACCTG | 954 |
| STMN1 | NM_005563 | S5838/STMN1.f1 | AATACCCAACGCACAAATGA | 955 |
| STMN1 | NM_005563 | S5839/STMN1.r1 | GGAGACAATGCAAACCACAC | 956 |
| STMN1 | NM_005563 | S5840/STMN1.p1 | CACGTTCTCTGCCCCGTTTCTTG | 957 |
| STMY3 | NM_005940 | S2067/STMY3.f3 | CCTGGAGGCTGCAACATACC | 958 |
| STMY3 | NM_005940 | S2068/STMY3.r3 | TACAATGGCTTTGGAGGATAGCA | 959 |
| STMY3 | NM_005940 | S4746/STMY3.p3 | | 960 |
| SURV | NM_001168 | S0259/SURV.f2 | TGTTTTGATTCCCGGGCTTA | 961 |
| SURV | NM_001168 | S0261/SURV.r2 | CAAAGCTGTCAGCTCTAGCAAAAG | 962 |
| SURV | NM_001168 | S4747/SURV.p2 | | 963 |
| TACC3 | NM_006342 | S7124/TACC3.f1 | CACCCTTGGACTGGAAAACT | 964 |
| TACC3 | NM_006342 | S7125/TACC3.r1 | CCTTGATGAGCTGTTGGTTC | 965 |
| TACC3 | NM_006342 | S7126/TACC3.p1 | CACACCCGGTCTGGACACAGAAAG | 966 |
| TBCA | NM_004607 | T2284/TBCA.f1 | GATCCTCGCGTGAGACAGA | 967 |
| TBCA | NM_004607 | T2285/TBCA.r1 | CACTTTTTCTTTGACCAACCG | 968 |
| TBCA | NM_004607 | T2286/TBCA.p1 | TTCACCACGCCGGTCTTGATCTT | 969 |
| TBCC | NM_003192 | T2302/TBCC.f1 | CTGTTTTCCTGGAGGACTGC | 970 |
| TBCC | NM_003192 | T2303/TBCC.r1 | ACTGTGTATGCGGAGCTGTT | 971 |
| TBCC | NM_003192 | T2304/TBCC.p1 | CCACTGCCAGCACGCAGTCAC | 972 |
| TBCD | NM_005993 | T2287/TBCD.f1 | CAGCCAGGTGTACGAGACATT | 973 |
| TBCD | NM_005993 | T2288/TBCD.r1 | ACCTCGTCCAGCACATCC | 974 |
| TBCD | NM_005993 | T2289/TBCD.p1 | CTCACCTACAGTGACGTCGTGGGC | 975 |
| TBCE | NM_003193 | T2290/TBCE.f1 | TCCCGAGAGAGGAAAGCAT | 976 |
| TBCE | NM_003193 | T2291/TBCE.r1 | GTCGGGTGCCTGCATTTA | 977 |
| TBCE | NM_003193 | T2292/TBCE.p1 | ATACACAGTCCCTTCGTGGCTCCC | 978 |
| TBD | NM_016261 | S3347/TBD.f2 | CCTGGTTGAAGCCTGTTAATGC | 979 |
| TBD | NM_016261 | S3348/TBD.r2 | | 980 |
| TBD | NM_016261 | S4864/TBD.p2 | CCGCTGGGTTTTCCACACGTTGA | 981 |
| TCP1 | NM_030752 | T2296/TCP1.f1 | CCAGTGTGTGTAACAGGGTCAC | 982 |
| TCP1 | NM_030752 | T2297/TCP1.r1 | TATAGCCTTGGGCCACCC | 983 |
| TCP1 | NM_030752 | T2298/TCP1.p1 | AGAATTCGACAGCCAGATGCTCCA | 984 |
| TFRC | NM_003234 | S1352/TFRC.f3 | GCCAACTGCTTTCATTTGTG | 985 |
| TFRC | NM_003234 | S1353/TFRC.r3 | ACTCAGGCCCATTTCCTTTA | 986 |
| TFRC | NM_003234 | S4748/TFRC.p3 | AGGGATCTGAACCAATACAGAGCA GACA | 987 |
| THBS1 | NM_003246 | S6474/THBS1.f1 | CATCCGCAAAGTGACTGAAGAG | 988 |
| THBS1 | NM_003246 | S6475/THBS1.r1 | | 989 |
| THBS1 | NM_003246 | S6476/THBS1.p1 | CCAATGAGCTGAGGCGGCCTCC | 990 |
| TK1 | NM_003258 | S0866/TK1.f2 | GCCGGGAAGACCGTAATTGT | 991 |
| TK1 | NM_003258 | S0927/TK1.r2 | CAGCGGCACCAGGTTCAG | 992 |
| TK1 | NM_003258 | S4798/TK1.p2 | | 993 |
| TOP2A | NM_001067 | S0271/TOP2A.f4 | AATCCAAGGGGGAGAGTGAT | 994 |
| TOP2A | NM_001067 | S0273/TOP2A.r4 | GTACAGATTTTGCCCGAGGA | 995 |
| TOP2A | NM_001067 | S4777/TOP2A.p4 | | 996 |
| TOP3B | NM_003935 | T2114/TOP3B.f1 | GTGATGCCTTCCCTGTGG | 997 |
| TOP3B | NM_003935 | T2115/TOP3B.r1 | TCAGGTAGTCGGGTGGGTT | 998 |
| TOP3B | NM_003935 | T2116/TOP3B.p1 | TGCTTCTCCAGCATCTTCACCTCG | 999 |
| TP | NM_001953 | S0277/TP.f3 | CTATATGCAGCCAGAGATGTGACA | 1000 |
| TP | NM_001953 | S0279/TP.r3 | CCACGAGTTTCTTACTGAGAATGG | 1001 |
| TP | NM_001953 | S4779/TP.p3 | ACAGCCTGCCACTCATCACAGCC | 1002 |
| TP53BP1 | NM_005657 | S1747/TP53BP.f2 | TGCTGTTGCTGAGTCTGTTG | 1003 |
| TP53BP1 | NM_005657 | S1748/TP53BP.r2 | CTTGCCTGGCTTCACAGATA | 1004 |
| TP53BP1 | NM_005657 | S4924/TP53BP.p2 | CCAGTCCCCAGAAGACCATGTCTG | 1005 |
| TPT1 | NM_003295 | S9098/TPT1.f1 | GGTGTCGATATTGTCATGAACC | 1006 |
| TPT1 | NM_003295 | S9099/TPT1.r1 | | 1007 |
| TPT1 | NM_003295 | S9100/TPT1.p1 | | 1008 |
| TRAG3 | NM_004909 | S5881/TRAG3.f1 | GACGCTGGTCTGGTGAAGATG | 1009 |
| TRAG3 | NM_004909 | S5882/TRAG3.r1 | TGGGTGGTTGTTGGACAATG | 1010 |
| TRAG3 | NM_004909 | S5883/TRAG3.p1 | CCAGGAAACCACGAGCCTCCAGC | 1011 |
| TRAIL | NM_003810 | S2539/TRAIL.f1 | CTTCACAGTGCTCCTGCAGTCT | 1012 |
| TRAIL | NM_003810 | S2540/TRAIL.r1 | CATCTGCTTCAGCTCGTTGGT | 1013 |
| TRAIL | NM_003810 | S4980/TRAIL.p1 | | 1014 |
| TS | NM_001071 | S0280/TS.f1 | GCCTCGGTGTGCCTTTCA | 1015 |
| TS | NM_001071 | S0282/TS.r1 | CGTGATGTGCGCAATCATG | 1016 |
| TS | NM_001071 | 54780/TS.p1 | CATCGCCAGCTACGCCCTGCTC | 1017 |
| TSPAN4 | NM_003271 | T2102/TSPAN4.f1 | CTGGTCAGCCTTCAGGGAC | 1018 |
| TSPAN4 | NM_003271 | T2103/TSPAN4.r1 | CTTCAGTTCTGGGCTGGC | 1019 |
| TSPAN4 | NM_003271 | T2104/TSPAN4.p1 | CTGAGCACCGCCTGGTCTCTTTC | 1020 |
| TTK | NM_003318 | S7247/TTK.f1 | TGCTTGTCAGTTGTCAACACCTT | 1021 |
| TTK | NM_003318 | S7248/TTK.r1 | TGGAGTGGCAAGTATTTGATGCT | 1022 |
| TTK | NM_003318 | S7249/TTK.p1 | TGGCCAACCTGCCTGTTTCCAGC | 1023 |
| TUBA1 | NM_006000 | S8578/TUBA1.f1 | TGTCACCCCGACTCAACGT | 1024 |
| TUBA1 | NM_006000 | S8579/TUBA1.r1 | ACGTGGACTGAGATGCATTCAC | 1025 |
| TUBA1 | NM_006000 | S8580/TUBA1.p1 | AGACGCACCGCCCGGACTCAC | 1026 |
| TUBA2 | NM_006001 | S8581/TUBA2.f1 | AGCTCAACATGCGTGAGTGT | 1027 |
| TUBA2 | NM_006001 | S8582/TUBA2.r1 | ATTGCCGATCTGGACTCCT | 1028 |
| TUBA2 | NM_006001 | S8583/TUBA2.p1 | ATCTCTATCCACGTGGGGCAGGC | 1029 |
| TUBA3 | NM_006009 | S8584/TUBA3.f1 | CTCTTACATCGACCGCCTAAGAG | 1030 |
| TUBA3 | NM_006009 | S8585/TUBA3.r1 | GCTGATGGCGGAGACGAA | 1031 |
| TUBA3 | NM_006009 | S8586/TUBA3.p1 | | 1032 |
| TUBA4 | NM_025019 | T2415/TUBA4.f3 | GAGGAGGGTGAGTTCTCCAA | 1033 |
| TUBA4 | NM_025019 | T2416/TUBA4.r3 | ATGCCCACCTCCTTGTAATC | 1034 |
| TUBA4 | NM_025019 | T2417/TUBA4.p3 | CCATGAGGATATGACTGCCCTGGA | 1035 |
| TUBA6 | NM_032704 | S8590/TUBA6.f1 | GTCCCTTCGCCTCCTTCAC | 1036 |
| TUBA6 | NM_032704 | S8591/TUBA6.r1 | CGTGGATGGAGATGCACTCA | 1037 |
| TUBA6 | NM_032704 | S8592/TUBA6.p1 | | 1038 |
| TUBA8 | NM_018943 | T2412/TUBA8.f2 | CGCCCTACCTATACCAACCT | 1039 |
| TUBA8 | NM_018943 | T2413/TUBA8.r2 | CGGAGAGAAGCAGTGATTGA | 1040 |
| TUBA8 | NM_018943 | T2414/TUBA8.p2 | | 1041 |
| TUBB | NM_001069 | S5820/TUBB.f1 | CGAGGACGAGGCTTAAAAAC | 1042 |
| TUBB | NM_001069 | S5821/TUBB.r1 | ACCATGCTTGAGGACAACAG | 1043 |
| TUBB | NM_001069 | S5822/TUBB.p1 | | 1044 |
| TUBB classIII | NM_006086 | S8090/TUBB c.f3 | CGCCCTCCTGCAGTATTTATG | 1045 |
| TUBB classIII | NM_006086 | S8091/TUBB c.r3 | ACAGAGACAGGAGCAGCTCACA | 1046 |
| TUBB classIII | NM_006086 | S8092/TUBB c.p3 | CCTCGTCCTCCCCACCTAGGCCA | 1047 |
| TUBB1 | NM_030773 | S8093/TUBB1.f1 | ACACTGACTGGCATCCTGCTT | 1048 |
| TUBB1 | NM_030773 | S8094/TUBB1.r1 | | 1049 |
| TUBB1 | NM_030773 | S8095/TUBB1.p1 | AGCCTCCAGAAGAGCCAGGTGCCT | 1050 |
| TUBB2 | NM_006088 | S8096/TUBB2.f1 | GTGGCCTAGAGCCTTCAGTC | 1051 |
| TUBB2 | NM_006088 | S8097/TUBB2.r1 | CAGGCTGGGAGTGAATAAAGA | 1052 |
| TUBB2 | NM_006088 | S8098/TUBB2.p1 | TTCACACTGCTTCCCTGCTTTCCC | 1053 |
| TUBB5 | NM_006087 | S8102/TUBB5.f1 | ACAGGCCCCATGCATCCT | 1054 |
| TUBB5 | NM_006087 | S8103/TUBB5.r1 | TGTTTCTCTCCCAGATAAGCTAAGG | 1055 |
| TUBB5 | NM_006087 | S8104/TUBB5.p1 | TGCCTCACTCCCCTCAGCCCC | 1056 |
| TUBBM | NM_032525 | S8105/TUBBM.f1 | CCCTATGGCCCTGAATGGT | 1057 |
| TUBBM | NM_032525 | S8106/TUBBM.r1 | | 1058 |
| TUBBM | NM_032525 | S8107/TUBBM.p1 | TGAGGGGCCGACACCAACACAAT | 1059 |
| TUBBOK | NM_178014 | S8108/TUBBOK.f1 | AGTGGAATCCTTCCCTTTCC | 1060 |
| TUBBOK | NM_178014 | S8109/TUBBOK.r1 | CCCTTGATCCCTTTCTCTGA | 1061 |
| TUBBOK | NM_178014 | S8110/TUBBOK.p1 | CCTCACTCAGCTCCTTTCCCCTGA | 1062 |
| TUBBP | NM_178012 | S8111/TUBBP.f1 | GGAAGGAAAGAAGCATGGTCTACT | 1063 |
| TUBBP | NM_178012 | S8112/TUBBP.r1 | AAAAAGTGACAGGCAACAGTGAAG | 1064 |
| TUBBP | NM_178012 | S8113/TUBBP.p1 | CACCAGAGACCCAGCGCACACCTA | 1065 |
| TUBG1 | NM_001070 | T2299/TUBG1.f1 | GATGCCGAGGGAAATCATC | 1066 |
| TUBG1 | NM_001070 | T2300/TUBG1.r1 | CCAGAACTCGAACCCAATCT | 1067 |
| TUBG1 | NM_001070 | T2301/TUBG1.p1 | ATTGCCGCACTGGCCCAACTGTAG | 1068 |
| TWIST1 | NM_000474 | S7929/TWIST1.f1 | GCGCTGCGGAAGATCATC | 1069 |
| TWIST1 | NM_000474 | S7930/TWIST1.r1 | GCTTGAGGGTCTGAATCTTGCT | 1070 |
| TWIST1 | NM_000474 | S7931/TWIST1.p1 | CCACGCTGCCCTCGGACAAGC | 1071 |
| TYR03 | NM_006293 | T2105/TYRO3.f1 | CAGTGTGGAGGGGATGGA | 1072 |
| TYRO3 | NM_006293 | T2106/TYRO3.r1 | CAAGTTCTGGACCACAGCC | 1073 |
| TYRO3 | NM_006293 | T2107/TYRO3.p1 | CTTCACCCACTGGATGTCAGGCTC | 1074 |
| UFM1 | NM_016617 | T1284/UFM1.f2 | AGTTGTCGTGTGTTCTGGATTCA | 1075 |
| UFM1 | NM_016617 | T1285/UFM1.r2 | CGTCAGCGTGATCTTAAAGGAA | 1076 |
| UFM1 | NM_016617 | T1286/UFM1.p2 | TCCGGCACCACCATGTCGAAGG | 1077 |
| upa | NM_002658 | S0283/upa.f3 | GTGGATGTGCCCTGAAGGA | 1078 |
| upa | NM_002658 | S0285/upa.r3 | CTGCGGATCCAGGGTAAGAA | 1079 |
| upa | NM_002658 | S4769/upa.p3 | | 1080 |
| V-RAF | NM_001654 | S5763N-RAF.f1 | GGTTGTGCTCTACGAGCTTATGAC | 1081 |
| V-RAF | NM_001654 | S5764N-RAF.r1 | CGGCCCACCATAAAGATAATCT | 1082 |
| V-RAF | NM_001654 | S5765/V-RAF.p1 | TGCCTTACAGCCACATTGGCTGCC | 1083 |
| VCAM1 | NM_001078 | S3505/VCAM1.f1 | TGGCTTCAGGAGCTGAATACC | 1084 |
| VCAM1 | NM_001078 | S3506/VCAM1.r1 | TGCTGTCGTGATGAGAAAATAGTG | 1085 |
| VCAM1 | NM_001078 | S3507/VCAM1.p1 | | 1086 |
| VEGF | NM_003376 | S0286/VEGF.f1 | CTGCTGTCTTGGGTGCATTG | 1087 |
| VEGF | NM_003376 | S0288/VEGF.r1 | GCAGCCTGGGACCACTTG | 1088 |
| VEGF | NM_003376 | S4782/VEGF.p1 | | 1089 |
| VEGFB | NM_003377 | S2724/VEGFB.f1 | TGACGATGGCCTGGAGTGT | 1090 |
| VEGFB | NM_003377 | S2725/VEGFB.r1 | GGTACCGGATCATGAGGATCTG | 1091 |
| VEGFB | NM_003377 | S4960/VEGFB.p1 | CTGGGCAGCACCAAGTCCGGA | 1092 |
| VEGFC | NM_005429 | S2251/VEGFC.f1 | CCTCAGCAAGACGTTATTTGAAATT | 1093 |
| VEGFC | NM_005429 | S2252/VEGFC.r1 | AAGTGTGATTGGCAAAACTGATTG | 1094 |
| VEGFC | NM_005429 | S4758/VEGFC.p1 | | 1095 |
| VHL | NM_000551 | T1359/VHL.f1 | CGGTTGGTGACTTGTCTGC | 1096 |
| VHL | NM_000551 | T1360/VHL.r1 | AAGACTTGTCCCTGCCTCAC | 1097 |
| VHL | NM_000551 | T1361/VHL.p1 | ATGCCTCAGTCTTCCCAAAGCAGG | 1098 |
| VIM | NM_003380 | S0790/VIM.f3 | TGCCCTTAAAGGAACCAATGA | 1099 |
| VIM | NM_003380 | S0791/VIM.r3 | GCTTCAACGGCAAAGTTCTCTT | 1100 |
| VIM | NM_003380 | S4810/VIM.p3 | ATTTCACGCATCTGGCGTTCCA | 1101 |
| WAVE3 | NM_006646 | T2640/WAVE3.f1 | CTCTCCAGTGTGGGCACC | 1102 |
| WAVE3 | NM_006646 | T2641/WAVE3.r1 | GCGGTGTAGCTCCCAGAGT | 1103 |
| WAVE3 | NM_006646 | T2642/WAVE3.p1 | CCAGAACAGATGCGAGCAGTCCAT | 1104 |
| Wnt-5a | NM_003392 | S6183/Wnt-5a.f1 | | 1105 |
| Wnt-5a | NM_003392 | S6184/Wnt-5a.r1 | TGTCGGAATTGATACTGGCATT | 1106 |
| Wnt-5a | NM_003392 | S6185/Wnt-5a.p1 | TTGATGCCTGTCTTCGCGCCTTCT | 1107 |
| XIAP | NM_001167 | S0289/XIAP.f1 | GCAGTTGGAAGACACAGGAAAGT | 1108 |
| XIAP | NM_001167 | S0291/XIAP.r1 | TGCGTGGCACTATTTTCAAGA | 1109 |
| XIAP | NM_001167 | S4752/XIAP.p1 | | 1110 |
| XIST | M97168 | S1844/XIST.f1 | CAGGTCAGGCAGAGGAAGTC | 1111 |
| XIST | M97168 | S1845/XIST.r1 | CCTAACAAGCCCCAAATCAA | 1112 |
| XIST | M97168 | S8271/XIST.p1 | | 1113 |
| ZW10 | NM_004724 | T2117/ZW10.f1 | TGGTCAGATGCTGCTGAAGT | 1114 |
| ZW10 | NM_004724 | T2118/ZW10.r1 | ATCACAGCATGAAGGGATGG | 1115 |
| ZW10 | NM_004724 | T2119/ZW10.p1 | TATCCTTAGGCCGCTGGCATCTTG | 1116 |
| ZWILCH | NM_017975 | T2057/ZWILCH.f1 | GAGGGAGCAGACAGTGGGT | 1117 |
| ZWILCH | NM_017975 | T2058/ZWILCH.r1 | TCAGAGCCCTTGCTAAGTCAC | 1118 |
| ZWILCH | NM_017975 | T2059/ZWILCH.p1 | CCACGATCTCCGTAACCATTTGCA | 1119 |
| ZWINT | NM_007057 | S8920/ZWINT.f1 | TAGAGGCCATCAAAATTGGC | 1120 |
| ZWINT | NM_007057 | S8921/ZWINT.r1 | TCCGTTTCCTCTGGGCTT | 1121 |
| ZWINT | NM_007057 | S8922/ZWINT.p1 | ACCAAGGCCCTGACTCAGATGGAG | 1122 |

| **Appendix 2** | | | |
|---|---|---|---|
| Gene Name | Accession # | Amplicon Sequence | SEQ ID NO: |
| ABCA9 | NM_080283 | | 401 |
| ABCB1 | NM_000927 | | 402 |
| ABCB5 | NM_178559 | | 403 |
| ABCC10 | NM_03345 0 | | 404 |
| ABCC11 | NM_032583 | | 405 |
| ABCC5 | NM_005688 | | 406 |
| ABCD1 | NM_000033 | | 407 |
| ACTG2 | NM_001615 | | 408 |
| ACTR2 | NM_005722 | | 409 |
| ACTR3 | NM_005721 | | 410 |
| AK055699 | NM_194317 | | 411 |
| AKT1 | NM_005163 | | 412 |
| AKT2 | NM_001626 | | 413 |
| AKT3 | NM_005465 | | 414 |
| ANXA4 | NM_001153 | | 415 |
| APC | NM_000038 | | 416 |
| APEX-1 | NM_001641 | | 417 |
| APOC1 | NM_001645 | | 418 |
| APOD | NM_001647 | | 419 |
| APOE | NM_000041 | | 420 |
| APRT | NM_000485 | | 421 |
| ARHA | NM_001664 | | 422 |
| AURKB | NM_004217 | | 423 |
| B-actin | NM_001101 | | 424 |
| BAD | NM_032989 | | 425 |
| BAG1 | NM_004323 | | 426 |
| Bak | NM_001188 | | 427 |
| Bax | NM_004324 | | 428 |
| BBC3 | NM_014417 | | 429 |
| B-Catenin | NM_001904 | | 430 |
| Bcl2 | NM_000633 | | 431 |
| BCL2L11 | NM_138621 | | 432 |
| BCL2L13 | NM_015367 | | 433 |
| Bclx | NM_001191 | | 434 |
| BCRP | NM_004827 | | 435 |
| BID | NM_001196 | | 436 |
| BIN1 | NM_004305 | | 437 |
| BRCA1 | NM_007295 | | 438 |
| BRCA2 | NM_000059 | | 439 |
| BUB1 | NM_004336 | | 440 |
| BUB1B | NM_001211 | | 441 |
| BUB3 | NM_004725 | | 442 |
| C14orf10 | NM_017917 | | 443 |
| C20_orf1 | NM_012112 | | 444 |
| CA9 | NM_001216 | | 445 |
| CALD1 | NM_004342 | | 446 |
| CAPZA1 | NM_006135 | | 447 |
| CAV1 | NM_001753 | | 448 |
| CCNB1 | NM_031966 | | 449 |
| CCND1 | NM_053056 | | 450 |
| CCNE2 | NM_057749 | | 451 |
| CCT3 | NM_0010088 00 | | 452 |
| CD14 | NM_000591 | | 453 |
| CD31 | NM_000442 | | 454 |
| CD3z | NM_000734 | | 455 |
| CD63 | NM_001780 | | 456 |
| CD68 | NM_001251 | | 457 |
| CDC2 | NM_001786 | | 458 |
| CDC20 | NM_001255 | | 459 |
| CDC25B | NM_021873 | | 460 |
| CDCA8 | NM_018101 | | 461 |
| CDH1 | NM_004360 | | 462 |
| CDK5 | NM_004935 | | 463 |
| CDKN1C | NM_000076 | | 464 |
| CEGP1 | NM_020974 | | 465 |
| CENPA | NM_001809 | | 466 |
| CENPE | NM_001813 | | 467 |
| CENPF | NM_016343 | | 468 |
| CGA (CHGA official) | NM_001275 | | 469 |
| CHFR | NM_018223 | | 470 |
| Chk1 | NM_001274 | | 471 |
| Chk2 | NM_007194 | | 472 |
| cIAP2 | NM_001165 | | 473 |
| CKAP1 | NM_001281 | | 474 |
| CLU | NM_001831 | | 475 |
| cMet | NM_000245 | | 476 |
| cMYC | NM_002467 | | 477 |
| CNN | NM_001299 | | 478 |
| COL1A1 | NM_000088 | | 479 |
| COL1A2 | NM_000089 | | 480 |
| | | | |
| COL6A3 | NM_004369 | | 481 |
| Contig 51037 | NM_198477 | | 482 |
| COX2 | NM_000963 | | 483 |
| COX7C | NM_001867 | | 484 |
| CRABP1 | NM_004378 | | 485 |
| CRIP2 | NM_001312 | | 486 |
| CRYAB | NM_001885 | | 487 |
| CSF1 | NM_000757 | | 488 |
| CSNK1D | NM_001893 | | 489 |
| CST7 | NM_003650 | | 490 |
| CTSD | NM_001909 | | 491 |
| CTSL | NM_001912 | | 492 |
| CTSL2 | NM_001333 | | 493 |
| CXCR4 | NM_003467 | | 494 |
| CYBA | NM_000101 | | 495 |
| CYP1B1 | NM_000104 | | 496 |
| CYP2C8 | NM_000770 | | 497 |
| CYP3A4 | NM_017460 | | 498 |
| DDR1 | NM_001954 | | 499 |
| DIABLO | NM_019887 | | 500 |
| DIAPH 1 | NM_005219 | | 501 |
| DICER1 | NM_177438 | | 502 |
| DKFZp564 D0462; | NM_198569 | | 503 |
| DR4 | NM_003844 | | 504 |
| DR5 | NM_003842 | | 505 |
| DUSP1 | NM_004417 | | 506 |
| EEF1D | NM 001960 | | 507 |
| EGFR | NM_005228 | | 508 |
| EIF4E | NM_001968 | | 509 |
| EIF4EL3 | NM_004846 | | 510 |
| ELP3 | NM_018091 | | 511 |
| ER2 | NM_001437 | | 512 |
| ErbB3 | NM_001982 | | 513 |
| ERBB4 | NM_005235 | | 514 |
| ERCC1 | NM_001983 | | 515 |
| ERK1 | NM_002746 | | 516 |
| ESPL1 | NM_012291 | | 517 |
| EstR1 | NM_000125 | | 518 |
| fas | NM_000043 | | 519 |
| fasl | NM_000639 | | 520 |
| FASN | NM_004104 | | 521 |
| FBXO5 | NM_012177 | | 522 |
| FDFT1 | NM_004462 | | 523 |
| FGFR1 | NM_023109 | | 524 |
| FHIT | NM_002012 | | 525 |
| FIGF | NM_004469 | | 526 |
| FLJ20354 (DEPDC1 official) | NM_017779 | | 527 |
| FOS | NM_005252 | | 528 |
| FOXM1 | NM_021953 | | 529 |
| FUS | NM_004960 | | 530 |
| FYN | NM_002037 | | 531 |
| G1P3 | NM_002038 | | 532 |
| GADD45 | NM_001924 | | 533 |
| GADD45B | NM_015675 | | 534 |
| GAGE1 | NM_001468 | | 535 |
| GAPDH | NM_002046 | | 536 |
| GATA3 | NM_002051 | | 537 |
| GBP1 | NM_002053 | | 538 |
| GBP2 | NM_004120 | | 539 |
| GCLC | NM_001498 | | 540 |
| GDF15 | NM_004864 | | 541 |
| GGPS1 | NM_004837 | | 542 |
| GLRX | NM_002064 | | 543 |
| GNS | NM_002076 | | 544 |
| GPR56 | NM_005682 | | 545 |
| GPX1 | NM_000581 | | 546 |
| GRB7 | NM_005310 | | 547 |
| GSK3B | NM_002093 | | 548 |
| GSR | NM_000637 | | 549 |
| | | | |
| GSTM1 | NM_000561 | | 550 |
| GSTp | NM_000852 | | 551 |
| GUS | NM_000181 | | 552 |
| HDAC6 | NM_006044 | | 553 |
| HER2 | NM_004448 | | 554 |
| HIF1A | NM_001530 | | 555 |
| HNF3A | NM_004496 | | 556 |
| HRAS | NM_005343 | | 557 |
| HSPA1A | NM_005345 | | 558 |
| HSPA1B | NM_005346 | | 559 |
| HSPA1L | NM_005527 | | 560 |
| HSPA5 | NM_005347 | | 561 |
| HSPA9B | NM_004134 | | 562 |
| HSPB1 | NM_001540 | | 563 |
| HSPCA | NM_005348 | | 564 |
| ID1 | NM_002165 | | 565 |
| IFITM1 | NM_003641 | | 566 |
| IGF1R | NM_000875 | | 567 |
| IGFBP2 | NM_000597 | | 568 |
| IGFBP3 | NM_000598 | | 569 |
| IGFBP5 | NM_000599 | | 570 |
| IL2RA | NM_000417 | | 571 |
| IL6 | NM_000600 | | 572 |
| | | | |
| IL-7 | NM_000880 | | 573 |
| IL-8 | NM_000584 | | 574 |
| IL8RB | NM_001557 | | 575 |
| ILK | NM_0010147 94 | | 576 |
| ILT-2 | NM_006669 | | 577 |
| INCENP | NM_020238 | | 578 |
| IRAK2 | NM_001570 | | 579 |
| IRS1 | NM_005544 | | 580 |
| ITGB1 | NM_002211 | | 581 |
| K-Alpha-1 | NM_006082 | | 582 |
| KDR | NM_002253 | | 583 |
| Ki-67 | NM_002417 | | 584 |
| KIF11 | NM_004523 | | 585 |
| KIF22 | NM_007317 | | 586 |
| KIF2C | NM_006845 | | 587 |
| KIFC1 | NM_002263 | | 588 |
| KLK10 | NM_002776 | | 589 |
| KNS2 | NM_005552 | | 590 |
| KNTC1 | NM_014708 | | 591 |
| KNTC2 | NM_006101 | | 592 |
| KRT14 | NM_000526 | | 593 |
| KRT17 | NM_000422 | | 594 |
| KRT19 | NM_002276 | | 595 |
| KRT5 | NM_000424 | | 596 |
| L1CAM | NM_000425 | | 597 |
| LAMC2 | NM_005562 | | 598 |
| LAPTM4B | NM_018407 | | 599 |
| LIMK1 | NM_016735 | | 600 |
| LIMK2 | NM_005569 | | 601 |
| MAD1L1 | NM_003550 | | 602 |
| MAD2L1 | NM_002358 | | 603 |
| MAD2L1BP | NM_014628 | | 604 |
| MAD2L2 | NM_006341 | | 605 |
| MAGE2 | NM_005361 | | 606 |
| MAGE6 | NM_005363 | | 607 |
| MAP2 | NM_002374 | | 608 |
| MAP2K3 | NM_002756 | | 609 |
| MAP4 | NM_002375 | | 610 |
| MAP6 | NM_033063 | | 611 |
| MAPK14 | NM_139012 | | 612 |
| MAPK8 | NM_002750 | | 613 |
| MAPRE1 | NM_012325 | | 614 |
| MAPT | NM_016835 | | 615 |
| Maspin | NM_002639 | | 616 |
| MCL1 | NM_021960 | | 617 |
| MCM2 | NM_004526 | | 618 |
| MCM6 | NM_005915 | | 619 |
| MCP1 | NM_002982 | | 620 |
| MGMT | NM_002412 | | 621 |
| MMP12 | NM_002426 | | 622 |
| MMP2 | NM_004530 | | 623 |
| MMP9 | NM_004994 | | 624 |
| MRE11A | NM_005590 | | 625 |
| MRP1 | NM_004996 | | 626 |
| MRP2 | NM_000392 | | 627 |
| MRP3 | NM_003786 | | 628 |
| MSH3 | NM_002439 | | 629 |
| MUC1 | NM_002456 | | 630 |
| MX1 | NM_002462 | | 631 |
| MYBL2 | NM_002466 | | 632 |
| MYH11 | NM_002474 | | 633 |
| NEK2 | NM_002497 | | 634 |
| NFKBp50 | NM_003998 | | 635 |
| NFKBp65 | NM_021975 | | 636 |
| NME6 | NM_005793 | | 637 |
| NPC2 | NM_006432 | | 638 |
| NPD009 (ABAT official) | NM_020686 | | 639 |
| NTSR2 | NM_012344 | | 640 |
| NUSAP1 | NM_016359 | | 641 |
| p21 | NM_000389 | | 642 |
| p27 | NM_004064 | | 643 |
| PCTK1 | NM_006201 | | 644 |
| PDGFRb | NM_002609 | | 645 |
| PFDN5 | NM_145897 | | 646 |
| PGK1 | NM_000291 | | 647 |
| PHB | NM_002634 | | 648 |
| PI3KC2A | NM_002645 | | 649 |
| PIM1 | NM_002648 | | 650 |
| PIM2 | NM_006875 | | 651 |
| PLAUR | NM_002659 | | 652 |
| PLD3 | NM_012268 | | 653 |
| PLK | NM_005030 | | 654 |
| PMS1 | NM_000534 | | 655 |
| PMS2 | NM_000535 | | 656 |
| PP591 | NM_025207 | | 657 |
| PPP2CA | NM_002715 | | 658 |
| PR | NM_000926 | | 659 |
| PRDX1 | NM_002574 | | 660 |
| PRDX2 | NM_005809 | | 661 |
| PRKCA | NM_002737 | | 662 |
| PRKCD | NM_006254 | | 663 |
| PRKCG | NM_002739 | | 664 |
| PRKCH | NM_006255 | | 665 |
| pS2 | NM_003225 | | 666 |
| PTEN | NM_000314 | | 667 |
| PTPD1 | NM_007039 | | 668 |
| PTTG1 | NM_004219 | | 669 |
| RAB27B | NM_004163 | | 670 |
| RAB31 | NM_006868 | | 671 |
| RAB6C | NM_032144 | | 672 |
| RAD1 | NM_002853 | | 673 |
| RAD54L | NM_003579 | | 674 |
| RAF1 | NM_002880 | | 675 |
| RALBP1 | NM_006788 | | 676 |
| RAP1GDS1 | NM_021159 | | 677 |
| RASSF1 | NM_007182 | | 678 |
| RB1 | NM_000321 | | 679 |
| RBM17 | NM_032905 | | 680 |
| RCC1 | NM_001269 | | 681 |
| REG1A | NM_002909 | | 682 |
| RELB | NM_006509 | | 683 |
| RhoB | NM_004040 | | 684 |
| rhoc | NM_175744 | | 685 |
| RIZ1 | NM_012231 | | 686 |
| ROCK1 | NM_005406 | | 687 |
| RPL37A | NM_000998 | | 688 |
| RPLPO | NM_001002 | | 689 |
| RPN2 | NM_002951 | | 690 |
| | | | |
| RPS6KB1 | NM_003161 | | 691 |
| RXRA | NM_002957 | | 692 |
| RXRB | NM_021976 | | 693 |
| S100A10 | NM_002966 | | 694 |
| SEC61A | NM_013336 | | 695 |
| SEMA3F | NM_004186 | | 696 |
| SFN | NM_006142 | | 697 |
| SGCB | NM_000232 | | 698 |
| SGK | NM_005627 | | 699 |
| SGKL | NM_170709 | | 700 |
| SHC1 | NM_003029 | | 701 |
| SIR2 | NM_012238 | | 702 |
| SLC1A3 | NM_004172 | | 703 |
| SLC25A3 | NM_213611 | | 704 |
| SLC35B1 | NM_005827 | | 705 |
| SLC7A11 | NM_014331 | | 706 |
| SLC7A5 | NM_003486 | | 707 |
| SNAI2 | NM_003068 | | 708 |
| SNCA | NM_007308 | | 709 |
| SNCG | NM_003087 | | 710 |
| SOD1 | NM_000454 | | 711 |
| SRC | NM_005417 | | 712 |
| SRI | NM_003130 | | 713 |
| STAT1 | NM_007315 | | 714 |
| | | | |
| STAT3 | NM_003150 | | 715 |
| STK10 | NM_005990 | | 716 |
| STK11 | NM_000455 | | 717 |
| STK15 | NM_003600 | | 718 |
| STMN1 | NM_005563 | | 719 |
| STMY3 | NM_005940 | | 720 |
| SURV | NM_001168 | | 721 |
| TACC3 | NM_006342 | | 722 |
| TBCA | NM_004607 | | 723 |
| TBCC | NM_003192 | | 724 |
| TBCD | NM_005993 | | 725 |
| TBCE | NM_003193 | | 726 |
| TBD | NM_016261 | | 727 |
| TCP1 | NM_030752 | | 728 |
| TFRC | NM_003234 | | 729 |
| THBS1 | NM_003246 | | 730 |
| TK1 | NM_003258 | | 731 |
| TOP2A | NM_001067 | | 732 |
| TOP3B | NM_003935 | | 733 |
| TP | NM_001953 | | 734 |
| TP53BP1 | NM_005657 | | 735 |
| TPT1 | NM_003295 | | 736 |
| TRAG3 | NM_004909 | | 737 |
| TRAIL | NM_003810 | | 738 |
| TS | NM_001071 | | 739 |
| TSPAN4 | NM_003271 | | 740 |
| TTK | NM_003318 | | 741 |
| TUBA1 | NM_006000 | | 742 |
| TUBA2 | NM_006001 | | 743 |
| TUBA3 | NM_006009 | | 744 |
| TUBA4 | NM_025019 | | 745 |
| TUBA6 | NM_032704 | | 746 |
| TUBA8 | NM_018943 | | 747 |
| TUBB | NM_001069 | | 748 |
| TUBB classIII | NM_006086 | | 749 |
| TUBB1 | NM_030773 | | 750 |
| TUBB2 | NM_006088 | | 751 |
| TUBB5 | NM_006087 | | 752 |
| TUBBM | NM_032525 | | 753 |
| TUBBOK | NM_178014 | | 754 |
| TUBBP | NM_178012 | | 755 |
| TUBG1 | NM_001070 | | 756 |
| TWIST1 | NM_000474 | | 757 |
| TYR03 | NM_006293 | | 758 |
| UFM1 | NM_016617 | | 759 |
| upa | NM_002658 | | 760 |
| VCAM1 | NM_001078 | | 761 |
| | | | |
| VEGF | NM_003376 | | 762 |
| VEGFB | NM_003377 | | 763 |
| VEGFC | NM_005429 | | 764 |
| VHL | NM_000551 | | 765 |
| VIM | NM_003380 | | 766 |
| V-RAF | NM_001654 | | 767 |
| WAVE3 | NM_006646 | | 768 |
| Wnt-5a | NM_003392 | | 769 |
| XIAP | NM_001167 | | 770 |
| XIST | NR_001564 | | 771 |
| ZW10 | NM_004724 | | 772 |
| ZWILCH | NM_017975 | | 773 |
| ZWINT | NM_007057 | | 774 |

| **Table 1** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | SLC1A3 | 0.0002 | -0.7577 | 27 | RAD1 | 0.0115 | -0.6673 | 52 | ACTR2 | 0.0297 | -0.8754 |
| 2 | TBCC | 0.0006 | -1.0289 | 28 | MRE11A | 0.0120 | -0.6253 | 53 | WNT5A | 0.0321 | 0.5036 |
| 3 | EIF4E2 | 0.0009 | -1.2038 | 29 | DDR1 | 0.0122 | -0.5660 | 54 | HSPA1L | 0.0321 | -1.8702 |
| 4 | TUBB | 0.0017 | -0.7332 | 30 | STK10 | 0.0123 | -0.6002 | 55 | APOC1 | 0.0324 | -0.3434 |
| 5 | TSPAN4 | 0.0027 | -0.7211 | 31 | LILRB1 | 0.0125 | -0.4674 | 56 | ZWINT | 0.0326 | -0.3966 |
| 6 | VHL | 0.0034 | -0.7450 | 32 | BBC3 | 0.0128 | -0.4481 | 57 | APEX1 | 0.0330 | -0.7200 |
| 7 | BAX | 0.0039 | -1.0224 | 33 | BUB3 | 0.0144 | -0.5476 | 58 | KALPHA1 | 0.0351 | -0.7627 |
| 8 | CD247 | 0.0044 | -0.4656 | 34 | CDCA8 | 0.0145 | -0.3759 | 59 | ABCC10 | 0.0354 | -0.5667 |
| 9 | CAPZA1 | 0.0044 | -1.1182 | 35 | TOP3B | 0.0164 | -0.7292 | 60 | PHB | 0.0380 | -0.5832 |
| 10 | STMN1 | 0.0052 | -0.4350 | 36 | RPN2 | 0.0166 | -0.8121 | 61 | TUBB2C | 0.0380 | -0.6664 |
| 11 | ABCC1 | 0.0054 | -0.7653 | 37 | ILK | 0.0169 | -0.6920 | 62 | RALBP1 | 0.0382 | -0.5989 |
| 12 | ZW10 | 0.0055 | -0.8228 | 38 | GBP1 | 0.0170 | -0.3496 | 63 | VEGF | 0.0397 | -0.3673 |
| 13 | HSPA1B | 0.0058 | -0.4740 | 39 | TUBB3 | 0.0173 | -0.3037 | 64 | MCL1 | 0.0398 | -0.6137 |
| 14 | MAPRE1 | 0.0060 | -0.7833 | 40 | NTSR2 | 0.0175 | -2.4355 | 65 | HSPA1A | 0.0402 | -0.3451 |
| 15 | PLD3 | 0.0061 | -0.8595 | 41 | BID | 0.0175 | -0.6228 | 66 | BUB1 | 0.0404 | -0.2911 |
| 16 | APRT | 0.0062 | -0.7714 | 42 | BCL2L13 | 0.0189 | -0.7228 | 67 | MAD2L1 | 0.0412 | -0.3336 |
| 17 | BAK1 | 0.0064 | -0.7515 | 43 | TPX2 | 0.0196 | -0.3148 | 68 | CENPF | 0.0418 | -0.2979 |
| 18 | TUBA6 | 0.0067 | -0.7006 | 44 | ABCC5 | 0.0203 | -0.3906 | 69 | IL2RA | 0.0427 | -0.5023 |
| 19 | CST7 | 0.0069 | -0.4243 | 45 | HDAC6 | 0.0226 | -0.7782 | 70 | TUBA3 | 0.0429 | -0.4528 |
| 20 | SHC1 | 0.0080 | -0.6632 | 46 | CD68 | 0.0226 | -0.6531 | 71 | ACTB | 0.0439 | -0.8259 |
| 21 | ZWILCH | 0.0088 | -0.6902 | 47 | NEK2 | 0.0232 | -0.3657 | 72 | KIF22 | 0.0447 | -0.5427 |
| 22 | SRC | 0.0089 | -0.7011 | 48 | DICER1 | 0.0233 | -0.5537 | 73 | CXCR4 | 0.0462 | -0.4239 |
| 23 | GADD45B | 0.0102 | -0.5253 | 49 | RHOA | 0.0268 | -0.7407 | 74 | STAT1 | 0.0472 | -0.3555 |
| 24 | LIMK2 | 0.0106 | -0.7784 | 50 | TYMS | 0.0291 | -0.3577 | 75 | IL7 | 0.0473 | -0.3973 |
| 25 | CENPA | 0.0106 | -0.3588 | 51 | CCT3 | 0.0292 | -0.5989 | 76 | CHFR | 0.0499 | -0.5387 |
| 26 | CHEK2 | 0.0109 | -0.6737 | | | | | | | | |

| **Table 2** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | DDR1 | <.0001 | -1.2307 | 34 | ILK | 0.0084 | -1.1481 | 66 | RXRA | 0.0247 | -0.7973 |
| 2 | EIF4E2 | 0.0001 | -1.8076 | 35 | NTSR2 | 0.0090 | -4.0522 | 67 | ABCD1 | 0.0259 | -0.7533 |
| 3 | TBCC | 0.0001 | -1.5303 | 36 | TOP3B | 0.0091 | -1.0744 | 68 | MAPK3 | 0.0269 | -0.7322 |
| 4 | STK10 | 0.0005 | -1.2320 | 37 | PLD3 | 0.0095 | -1.1126 | 69 | CDCA8 | 0.0275 | -0.5210 |
| 5 | ZW10 | 0.0006 | -1.3917 | 38 | DICER1 | 0.0095 | -0.8849 | 70 | DUSP1 | 0.0284 | -0.3398 |
| 6 | BBC3 | 0.0010 | -0.9034 | 39 | VHL | 0.0104 | -0.9357 | 71 | ABCC1 | 0.0287 | -0.8003 |
| 7 | BAX | 0.0011 | -1.4992 | 40 | GCLC | 0.0108 | -0.7822 | 72 | PRKCH | 0.0291 | -0.6680 |
| 8 | BAK1 | 0.0011 | -1.3122 | 41 | RAD1 | 0.0108 | -1.0141 | 73 | PRDX1 | 0.0301 | -0.8823 |
| 9 | TSPAN4 | 0.0013 | -1.1930 | 42 | GATA3 | 0.0112 | -0.4400 | 74 | TUBA3 | 0.0306 | -0.7331 |
| 10 | SLC1A3 | 0.0014 | -0.9828 | 43 | CXCR4 | 0.0120 | -0.7032 | 75 | VEGFB | 0.0317 | -0.7487 |
| 11 | SHC1 | 0.0015 | -1.1395 | 44 | NME6 | 0.0121 | -0.9873 | 76 | LILRB1 | 0.0320 | -0.5617 |
| 12 | CHFR | 0.0016 | -1.3371 | 45 | UFM1 | 0.0125 | -0.9686 | 77 | LAPTM4B | 0.0321 | -0.4994 |
| 13 | RHOB | 0.0018 | -0.7059 | 46 | BUB3 | 0.0126 | -0.9054 | 78 | HSPA9B | 0.0324 | -0.9660 |
| 14 | TUBA6 | 0.0019 | -1.1071 | 47 | CD14 | 0.0130 | -0.8152 | 79 | ECGF1 | 0.0329 | -0.5807 |
| 15 | BCL2L13 | 0.0023 | -1.3181 | 48 | MRE11A | 0.0130 | -0.8915 | 80 | GDF15 | 0.0332 | -0.3646 |
| 16 | MAPRE1 | 0.0029 | -1.2233 | 49 | CST7 | 0.0131 | -0.5204 | 81 | ACTR2 | 0.0347 | -1.1827 |
| 17 | GADD45B | 0.0034 | -0.9174 | 50 | APOC1 | 0.0134 | -0.5630 | 82 | IL7 | 0.0349 | -0.5623 |
| 18 | HSPA1B | 0.0036 | -0.6406 | 51 | GNS | 0.0136 | -1.0979 | 83 | HDAC6 | 0.0380 | -0.9486 |
| 19 | FAS | 0.0037 | -0.8571 | 52 | ABCC5 | 0.0146 | -0.5595 | 84 | ZWILCH | 0.0384 | -0.7296 |
| 20 | TUBB | 0.0040 | -1.0178 | 53 | AKT2 | 0.0150 | -1.0824 | 85 | CHEK2 | 0.0392 | -0.7502 |
| 21 | HSPA1A | 0.0041 | -0.6648 | 54 | APRT | 0.0150 | -0.9231 | 86 | REG1A | 0.0398 | -3.4734 |
| 22 | MCL1 | 0.0041 | -1.1459 | 55 | PLAU | 0.0157 | -0.6705 | 87 | APC | 0.0411 | -0.8324 |
| 23 | CCT3 | 0.0048 | -1.0709 | 56 | RCC1 | 0.0163 | -0.9073 | 88 | SLC35B1 | 0.0411 | -0.6801 |
| 24 | VEGF | 0.0049 | -0.8411 | 57 | CAPZA1 | 0.0165 | -1.3542 | 89 | NEK2 | 0.0415 | -0.4609 |
| 25 | TUBB2C | 0.0051 | -1.4181 | 58 | RELA | 0.0168 | -0.8534 | 90 | ACTB | 0.0418 | -1.1482 |
| 26 | AKT1 | 0.0053 | -1.1175 | 59 | NFKB1 | 0.0179 | -0.9847 | 91 | BUB1 | 0.0423 | -0.4612 |
| 27 | MAD2L1BP | 0.0055 | -1.0691 | 60 | RASSF1 | 0.0186 | -0.8078 | 92 | PPP2CA | 0.0423 | -0.9474 |
| 28 | RPN2 | 0.0056 | -1.2688 | 61 | BCL2L11 | 0.0209 | -0.9394 | 93 | TNFRSF 10A | 0.0448 | -0.6415 |
| 29 | RHOA | 0.0063 | -1.3773 | 62 | CSNK1D | 0.0211 | -1.2276 | 94 | TBCD | 0.0456 | -0.6196 |
| 30 | MAP2K3 | 0.0063 | -0.9616 | 63 | SRC | 0.0220 | -0.8341 | 95 | ERBB4 | 0.0460 | -0.2830 |
| 31 | BID | 0.0067 | -1.0502 | 64 | LIMK2 | 0.0221 | -1.0830 | 96 | CDC25B | 0.0467 | -0.5660 |
| 32 | APOE | 0.0074 | -0.8130 | 65 | SIRT1 | 0.0229 | -0.7236 | 97 | STMN1 | 0.0472 | -0.4684 |
| 33 | ESR1 | 0.0077 | -0.3456 | | | | | | | | |

| **Table 3** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | DDR1 | <.0001 | -1.3498 | 56 | APRT | 0.0027 | -1.2629 | 111 | RBM 17 | 0.0171 | -1.3981 |
| 2 | ZW 10 | <.0001 | -2.1657 | 57 | MAP2K3 | 0.0031 | -1.1297 | 112 | TP53BP1 | 0.0184 | -0.9442 |
| 3 | RELA | <.0001 | -1.5759 | 58 | MGC52057 | 0.0033 | -1.0906 | 113 | CD247 | 0.0188 | -0.5768 |
| 4 | BAX | <.0001 | -1.8857 | 59 | MAPK3 | 0.0033 | -1.0390 | 114 | ABCA9 | 0.0190 | -0.5489 |
| 5 | RHOB | <.0001 | -1.1694 | 60 | APC | 0.0034 | -1.2719 | 115 | NTSR2 | 0.0192 | -3.9043 |
| 6 | TSPAN4 | <.0001 | -1.7067 | 61 | RAD1 | 0.0036 | -1.2744 | 116 | FOS | 0.0195 | -0.4437 |
| 7 | BBC3 | <.0001 | -1.2017 | 62 | COL6A3 | 0.0039 | -0.8240 | 117 | TNFRSF10A | 0.0196 | -0.7666 |
| 8 | SHC1 | <.0001 | -1.4625 | 63 | RXRB | 0.0039 | -1.2638 | 118 | MSH3 | 0.0200 | -0.9585 |
| 9 | CAPZA1 | <.0001 | -2.4068 | 64 | CCT3 | 0.0040 | -1.3329 | 119 | PTEN | 0.0202 | -1.0307 |
| 10 | STK10 | 0.0001 | -1.4013 | 65 | ABCC3 | 0.0040 | -0.8170 | 120 | GBP2 | 0.0204 | -0.6414 |
| 11 | TBCC | 0.0001 | -1.6385 | 66 | GPX1 | 0.0042 | -1.5547 | 121 | STK11 | 0.0206 | -0.9807 |
| 12 | EIF4E2 | 0.0002 | -1.9122 | 67 | TUBB2C | 0.0042 | -1.6184 | 122 | ERBB4 | 0.0213 | -0.3933 |
| 13 | MCL1 | 0.0003 | -1.6617 | 68 | HSPA1A | 0.0043 | -0.7875 | 123 | TFF1 | 0.0220 | -0.2020 |
| 14 | RASSF1 | 0.0003 | -1.3201 | 69 | AKT1 | 0.0045 | -1.1777 | 124 | ABCC1 | 0.0222 | -0.9438 |
| 15 | VEGF | 0.0003 | -1.0800 | 70 | TUBA6 | 0.0046 | -1.2048 | 125 | IL7 | 0.0223 | -0.6920 |
| 16 | SLC1A3 | 0.0004 | -1.0855 | 71 | TOP3B | 0.0048 | -1.1950 | 126 | CDC25B | 0.0228 | -0.7338 |
| 17 | DICER1 | 0.0004 | -1.4236 | 72 | CSNK1D | 0.0049 | -1.6201 | 127 | TUBD1 | 0.0234 | -0.6092 |
| 18 | ILK | 0.0004 | -1.7221 | 73 | SOD1 | 0.0049 | -1.2383 | 128 | BIRC4 | 0.0236 | -0.9072 |
| 19 | FAS | 0.0005 | -1.1671 | 74 | BUB3 | 0.0050 | -1.0111 | 129 | ACTR3 | 0.0246 | -1.3384 |
| 20 | RAB6C | 0.0005 | -1.6154 | 75 | MAP4 | 0.0052 | -1.5220 | 130 | SLC35B1 | 0.0253 | -0.7793 |
| 21 | ESR1 | 0.0006 | -0.4845 | 76 | NFKB1 | 0.0060 | -1.2355 | 131 | COL1A1 | 0.0256 | -0.4945 |
| 22 | MRE11A | 0.0006 | -1.2537 | 77 | SEC61A1 | 0.0060 | -1.4777 | 132 | FOXA1 | 0.0262 | -0.4554 |
| 23 | APOE | 0.0006 | -1.0602 | 78 | MAD1 L1 | 0.0060 | -1.1168 | 133 | DUSP1 | 0.0264 | -0.4205 |
| 24 | BAK1 | 0.0006 | -1.4288 | 79 | PRKCH | 0.0073 | -0.8259 | 134 | CXCR4 | 0.0265 | -0.6550 |
| 25 | UFM1 | 0.0006 | -1.4110 | 80 | RXRA | 0.0074 | -0.9693 | 135 | IL2RA | 0.0268 | -0.9731 |
| 26 | AKT2 | 0.0007 | -1.6213 | 81 | PLAU | 0.0074 | -0.7987 | 136 | GGPS1 | 0.0268 | -0.7915 |
| 27 | SIRT1 | 0.0007 | -1.1651 | 82 | CD63 | 0.0074 | -1.3830 | 137 | KNS2 | 0.0281 | -0.8758 |
| 28 | BCL2L13 | 0.0008 | -1.5059 | 83 | CD14 | 0.0075 | -0.9409 | 138 | RB1 | 0.0289 | -0.9291 |
| 29 | ACTR2 | 0.0008 | -1.9690 | 84 | RHOC | 0.0077 | -1.0341 | 139 | BCL2L1 | 0.0289 | -0.9123 |
| 30 | LIMK2 | 0.0009 | -1.6937 | 85 | STAT1 | 0.0093 | -0.7663 | 140 | XIST | 0.0294 | -0.6529 |
| 31 | HDAC6 | 0.0010 | -1.5715 | 86 | NPC2 | 0.0094 | -1.2302 | 141 | BIRC3 | 0.0294 | -0.4739 |
| 32 | RPN2 | 0.0010 | -1.5839 | 87 | NME6 | 0.0095 | -1.2091 | 142 | BID | 0.0303 | -0.8691 |
| 33 | PLD3 | 0.0010 | -1.5460 | 88 | PDGFRB | 0.0096 | -0.7932 | 143 | BCL2 | 0.0303 | -0.5525 |
| 34 | CHGA | 0.0011 | -0.8275 | 89 | MGMT | 0.0098 | -1.0325 | 144 | STAT3 | 0.0311 | -0.9289 |
| 35 | RHOA | 0.0011 | -1.6934 | 90 | GBP1 | 0.0098 | -0.5896 | 145 | PECAM1 | 0.0319 | -0.6803 |
| 36 | MAPK14 | 0.0014 | -1.6611 | 91 | ERCC1 | 0.0105 | -1.2240 | 146 | DIABLO | 0.0328 | -0.9572 |
| 37 | ECGF1 | 0.0014 | -0.8835 | 92 | RCC1 | 0.0107 | -1.0453 | 147 | CYBA | 0.0333 | -0.6642 |
| 38 | MAPRE1 | 0.0016 | -1.3329 | 93 | FUS | 0.0117 | -1.2869 | 148 | TBCE | 0.0336 | -0.7411 |
| 39 | HSPA1B | 0.0017 | -0.8048 | 94 | TUBA3 | 0.0117 | -0.8905 | 149 | CYP1B1 | 0.0337 | -0.6013 |
| 40 | GATA3 | 0.0017 | -0.6153 | 95 | CHEK2 | 0.0120 | -1.0057 | 150 | APEX1 | 0.0357 | -1.0916 |
| 41 | PPP2CA | 0.0017 | -1.6176 | 96 | APOC1 | 0.0123 | -0.6422 | 151 | TBCD | 0.0383 | -0.5893 |
| 42 | ABCD1 | 0.0018 | -1.1669 | 97 | ABCC10 | 0.0124 | -0.9400 | 152 | HRAS | 0.0390 | -0.8411 |
| 43 | MAD2L1 BP | 0.0018 | -1.1725 | 98 | SRC | 0.0128 | -1.1170 | 153 | TNFRSF10B | 0.0394 | -0.7293 |
| 44 | VHL | 0.0022 | -1.1855 | 99 | TUBB | 0.0136 | -0.9398 | 154 | ELP3 | 0.0398 | -0.9560 |
| 45 | GCLC | 0.0023 | -1.1240 | 100 | FLAD1 | 0.0139 | -1.0396 | 155 | PIK3C2A | 0.0408 | -0.9158 |
| 46 | ACTB | 0.0023 | -1.8754 | 101 | MAD2L2 | 0.0141 | -1.0834 | 156 | HSPA5 | 0.0417 | -1.5232 |
| 47 | BCL2L11 | 0.0024 | -1.5415 | 102 | LAPTM4B | 0.0149 | -0.5932 | 157 | VEGFC | 0.0427 | -0.7309 |
| 48 | PRDX1 | 0.0025 | -1.3943 | 103 | REG1A | 0.0150 | -5.1214 | 158 | CRABP1 | 0.0440 | 0.2492 |
| 49 | LILRB1 | 0.0025 | -0.8462 | 104 | PRKCD | 0.0152 | -1.0120 | 159 | MMP11 | 0.0456 | -0.3894 |
| 50 | GNS | 0.0025 | -1.3307 | 105 | CST7 | 0.0157 | -0.5499 | 160 | SGK | 0.0456 | -0.6740 |
| 51 | CHFR | 0.0026 | -1.3292 | 106 | IGFBP2 | 0.0161 | -0.5019 | 161 | CTSD | 0.0463 | -0.7166 |
| 52 | CD68 | 0.0026 | -1.1941 | 107 | FYN | 0.0162 | -0.7670 | 162 | BAD | 0.0479 | -0.6436 |
| 53 | LIMK1 | 0.0026 | -1.5655 | 108 | KDR | 0.0168 | -0.8204 | 163 | PTPN21 | 0.0484 | -0.5636 |
| 54 | GADD45B | 0.0027 | -1.0162 | 109 | STMN1 | 0.0169 | -0.6791 | 164 | HSPA9B | 0.0487 | -0.9657 |
| 55 | VEGFB | 0.0027 | -1.1252 | 110 | ZWILCH | 0.0170 | -0.8897 | 165 | PMS1 | 0.0498 | -0.9283 |

| **Table 4** | | | | | | | | | | | |
|---|---|---|---|---|---|---|---|---|---|---|---|
| | Gene | p-value | Estimated Coefficient | | Gene | p-value | Estimated Coefficient | | Gene | p-value | Estimated Coefficient |
| 1 | CD247 | 0.0101 | -0.6642 | 4 | ACTG2 | 0.0280 | -0.2775 | 7 | CHEK2 | 0.0438 | -0.9595 |
| 2 | TYMS | 0.0225 | -0.5949 | 5 | CCND1 | 0.0355 | 0.4802 | 8 | STMN1 | 0.0441 | -0.5369 |
| 3 | IGF1R | 0.0270 | -0.5243 | 6 | CAPZA1 | 0.0401 | -1.1408 | 9 | ZWILCH | 0.0476 | -0.8264 |

| **Table 5** | | | |
|---|---|---|---|
| **Official Symbol** | **Name** | **Entrez** | **Role** |
| CHUK | Conserved helix-loop-helix ubiquitous kinase | 1147 | Activates |
| BCL3 | B-cell CLL/lymphoma 3 | 602 | Transcriptional co-activator |
| FADD | Fas (TNFRSF6)-associated via death domain | 8772 | Stimulates pathway |
| IKBKB | Inhibitor of kappa light polypeptide gene enhancer in B-cells, kinase beta | 3551 | Activates; triggers degradation of NFKBIA, NFKBIB |
| IKBKG | Inhibitor of kappa light polypeptide gene enhancer in B-cells, kinase gamma | 8517 | Activates; triggers degradation of NFKBIA, NFKBIB |
| IL1A | Interleukin 1, alpha | 3552 | Stimulates pathway |
| IL1R1 | Interleukin 1 receptor, type I | 3554 | Stimulates pathway |
| IRAK1 | Interleukin-1 receptor-associated kinase 1 | 3654 | Stimulates pathway |
| NFKB1 | Nuclear factor of kappa light polypeptide gene enhancer in B-cells 1 (p105) | 4790 | Core subunit |
| NFKB2 | Nuclear factor of kappa light polypeptide gene enhancer in B-cells 2 (p49/p100) | 4791 | Core subunit |
| NFKBIA | Nuclear factor of kappa light polypeptide gene enhancer in B-cells inhibitor, alpha | 4792 | Inhibits |
| NFKBIB | Nuclear factor of kappa light polypeptide gene enhancer in B-cells inhibitor, beta | 4793 | Inhibits |
| NFKBIE | nuclear factor of kappa light polypeptide gene enhancer in B-cells inhibitor, epsilon | 4794 | Inhibits |
| REL | v-rel reticuloendotheliosis viral oncogene homolog (avian) | 5966 | Transcriptional co-activator |
| RELA | V-rel reticuloendotheliosis viral oncogene homolog A, nuclear factor of kappa light polypeptide gene enhancer in B-cells 3, p65 (avian) | 5970 | Transcriptional co-activator |
| RELB | v-rel reticuloendotheliosis viral oncogene homolog B, nuclear factor of kappa light polypeptide gene enhancer in B-cells 3 (avian) | 5971 | Transcriptional co-activator |
| RHOC | ras homolog gene family, member C | 389 | Induce activation of pathway |
| TNFAIP3 | Tumor necrosis factor, alpha-induced protein 3 | 7128 | Activates |
| TNFRSF1A | Tumor necrosis factor receptor superfamily, member 1A | 7132 | Activates |
| TNFRSF1B | TNFRSF1A-associated via death domain | 7133 | Activates |
| TRAF6 | TNF receptor-associated factor 6 | 7189 | Activates CHUK |

## Claims

1. A method of predicting whether a breast cancer patient will exhibit a beneficial response to chemotherapy, comprising
measuring an expression level of TBCC, or its expression product, in a tumour sample obtained from the patient,
using the expression level to determine a likelihood of a beneficial response to a treatment including a taxane, wherein expression of TBCC is positively correlated with increased likelihood of a beneficial response to a treatment including a taxane, and
generating a report including information based on the likelihood of a beneficial response to chemotherapy including a taxane.

2. A method of predicting whether a breast cancer patient will exhibit a beneficial response to chemotherapy, comprising
measuring an expression level of TBCC, or its expression product, in a tumour sample obtained from the patient,
using the expression level to determine a likelihood of a beneficial response to a treatment including a cyclophosphamide, wherein expression of TBCC is negatively correlated with increased likelihood of a beneficial response to a treatment including a cyclophosphamide, and
generating a report including information based on the likelihood of a beneficial response to chemotherapy including a cyclophosphamide.

3. A method as claimed in claim 1 or 2, wherein the breast cancer is hormone-receptor positive.

4. A method as claimed in claim 1 or 2, wherein the chemotherapy includes an anthracycline.

5. A method as claimed in claim 4, wherein the anthracycline is doxorubicin.

6. A method as claimed in claim 1, 3 or 4, wherein the taxane is docetaxel.

7. A method as claimed in any preceding claim, wherein said measuring is by quantitative polymerase chain reaction (qPCR).

8. A method as claimed in any preceding claim, wherein said measuring is by detection of an intron-based sequence of an RNA transcript of the gene, wherein the expression of which correlates with the expression of a corresponding exon sequence.

9. A method as claimed in any preceding claim, wherein the tumour sample is a formalin-fixed and paraffin-embedded (FPE) tumour sample.

10. A method as claimed in any of claims 1 to 8, wherein the tumour sample is a frozen tumour sample.

11. A method as claimed in any of claims 1 to 6, wherein said measuring is by microarray.

12. A method as claimed in any of claims 1 to 6, wherein said measuring is by nucleic acid sequencing.

## Patentansprüche

1. Verfahren zur Vorhersage davon, ob ein Brustkrebspatient positiv auf eine Chemotherapie anspricht, wobei man
ein TBCC-Expressionsniveau oder sein Expressionsprodukt in einer von einem Patienten erhaltenen Tumorprobe misst;
das Expressionsniveau dazu verwendet, eine Wahrscheinlichkeit eines positiven Ansprechens auf eine Behandlung unter Einschluss eines Taxans zu bestimmen, wobei die Expression von TBCC eine positive Korrelation mit einer erhöhten Wahrscheinlichkeit eines positiven Ansprechens auf eine Behandlung unter Einschluss eines Taxans aufweist; und
einen Bericht erstellt, der auf die Wahrscheinlichkeit eines positiven Ansprechens auf eine Chemotherapie unter Einschluss eines Taxans bezogene Informationen enthält.

2. Verfahren zur Vorhersage davon, ob ein Brustkrebspatient positiv auf eine Chemotherapie anspricht, wobei man
ein TBCC-Expressionsniveau oder sein Expressionsprodukt in einer von einem Patienten erhaltenen Tumorprobe misst;
das Expressionsniveau dazu verwendet, eine Wahrscheinlichkeit eines positiven Ansprechens auf eine Behandlung unter Einschluss eines Cyclophosphamids zu bestimmen, wobei die Expression von TBCC eine negative Korrelation mit einer erhöhten Wahrscheinlichkeit eines positiven Ansprechens auf eine Behandlung unter Einschluss eines Cyclophosphamids aufweist; und
einen Bericht erstellt, der auf die Wahrscheinlichkeit eines positiven Ansprechens auf eine Chemotherapie unter Einschluss eines Cyclophosphamids bezogene Informationen enthält.

3. Verfahren nach Anspruch 1 oder 2, wobei es sich um eine hormonrezeptor-positive Brustkrebserkrankung handelt.

4. Verfahren nach Anspruch 1 oder 2, wobei es sich um eine Chemotherapie unter Einschluss eines Anthracyclins handelt.

5. Verfahren nach Anspruch 4, wobei es sich bei dem Anthracyclin um Doxorubicin handelt.

6. Verfahren nach Anspruch 1, 3 oder 4, wobei es sich bei dem Taxan um Docetaxel handelt.

7. Verfahren nach einem vorhergehenden Anspruch, wobei die Messung mittels quantitativer Polymerasekettenreaktion (qPCR) erfolgt.

8. Verfahren nach einem vorhergehenden Anspruch, wobei die Messung mittels Nachweis einer intronbasierten Sequenz eines RNA-Transkripts des Gens erfolgt, wobei ihre Expression mit der Expression einer entsprechenden Exonsequenz korreliert.

9. Verfahren nach einem vorhergehenden Anspruch, wobei es sich bei der Tumorprobe um eine mit Formalin fixierte und in Paraffin eingebettete (FPE-) Tumorprobe handelt.

10. Verfahren nach einem der Ansprüche 1 bis 8, wobei es sich bei der Tumorprobe um eine gefrorene Tumorprobe handelt.

11. Verfahren nach einem der Ansprüche 1 bis 6, wobei das Messen mittels Mikroarray erfolgt.

12. Verfahren nach einem der Ansprüche 1 bis 6, wobei das Messen mittels Nukleinsäuresequenzierung erfolgt.

## Revendications

1. Procédé consistant à prédire si un patient atteint de cancer du sein présente une réponse bénéfique à une chimiothérapie, comprenant
la mesure d'un niveau d'expression de TBCC, ou son produit d'expression, dans un échantillon de tumeur obtenu à partir du patient ;
l'utilisation du niveau d'expression pour déterminer une probabilité d'une réponse bénéfique à un traitement comprenant un taxane, dans lequel l'expression de TBCC est positivement corrélée avec une probabilité augmentée d'une réponse bénéfique à un traitement comprenant un taxane ; et
la génération d'un rapport comprenant des informations basées sur la probabilité d'une réponse bénéfique à une chimiothérapie comprenant un taxane.

2. Procédé consistant à prédire si un patient atteint de cancer du sein présente une réponse bénéfique à une chimiothérapie, comprenant
la mesure d'un niveau d'expression de TBCC, ou son produit d'expression, dans un échantillon de tumeur obtenu à partir du patient ;
l'utilisation du niveau d'expression pour déterminer une probabilité d'une réponse bénéfique à un traitement comprenant un cyclophosphamide, dans lequel l'expression de TBCC est négativement corrélée avec une probabilité augmentée d'une réponse bénéfique à un traitement comprenant un cyclophosphamide ; et
la génération d'un rapport comprenant des informations basées sur la probabilité d'une réponse bénéfique à une chimiothérapie comprenant un cyclophosphamide.

3. Procédé selon la revendication 1 ou 2, dans lequel le cancer du sein est à récepteur hormonal positif.

4. Procédé selon la revendication 1 ou 2, dans lequel la chimiothérapie comprend une anthracycline.

5. Procédé selon la revendication 4, dans lequel l'anthracycline est la doxorubicine.

6. Procédé selon la revendication 1, 3 ou 4, dans lequel le taxane est le docétaxel.

7. Procédé selon l'une quelconque des revendications précédentes, dans lequel ladite mesure est effectuée par réaction de polymérase en chaîne quantitative (qPCR).

8. Procédé selon l'une quelconque des revendications précédentes, dans lequel ladite mesure est effectuée par détection d'une séquence à base d'introduction d'un produit de transcription d'ARN du gène, dont l'expression est corrélée avec l'expression d'une séquence d'exon correspondante.

9. Procédé selon l'une quelconque des revendications précédentes, dans lequel l'échantillon de tumeur est un échantillon de tumeur fixé au formol et inclus dans la paraffine (FPE).

10. Procédé selon l'une quelconque des revendications 1 à 8, dans lequel l'échantillon de tumeur est un échantillon de tumeur congelé.

11. Procédé selon l'une quelconque des revendications 1 à 6, dans lequel ladite mesure est par microréseau.

12. Procédé selon l'une quelconque des revendications 1 à 6, dans lequel ladite mesure est par détermination des séquences nucléotidiques.
